# EUROPEAN PATENT SPECIFICATION

(11) **EP 2 826 787 B1**
(45) Date of publication and mention of the grant of the patent: **28.10.2020**
(21) Application number: 14184972.9
(22) Date of filing: 05.06.2003
(51) Int. Cl.: C07K 14/415

(54) **Therapeutic epitopes and uses thereof**
Therapeutische Epitope und deren Anwendungen
Epitopes thérapeutiques et leurs utilisations

(30) Priority: 05.06.2002 GB 0212885
(43) Date of publication of application: 21.01.2015
(62) Divisional of application: 10181717.9
(73) Proprietor: Oxford University Innovation Limited, Botley Oxford OX2 0JB (GB)
(72) Inventor: Anderson, Robert Paul, Parkville Victoria 3050 (AU); Jewel, Derek Parry, Oxford Oxfordshire OX2 6HE (GB); Hill, Adrian Vivian Sinton, Oxford Oxfordshire OX3 7BN (GB)
(74) Representative: J A Kemp LLP

(56) References cited:
- EP-A1- 1 332 760
- WO-A2-01/25793
- WO-A2-02/083722
- VADER L W ET AL: "Specificity of tissue transglutaminase explains cereal toxicity in celiac disease", THE JOURNAL OF EXPERIMENTAL MEDICINE, ROCKEFELLER UNIVERSITY PRESS, US, vol. 195, no. 5, 4 March 2002 (2002-03-04) , pages 643-649, XP002204499, ISSN: 0022-1007, DOI: 10.1084/JEM.20012028
- JUNG G ET AL: "From combinatorial libraries to MHC ligand motifs, T-cell superagonists and antagonists", BIOLOGICALS, ACADEMIC PRESS LTD., LONDON, GB, vol. 29, no. 3-4, 1 September 2001 (2001-09-01), pages 179-181, XP002242277, ISSN: 1045-1056, DOI: 10.1006/BIOL.2001.0299
- SKERRITT J H ET AL: "Antigenicity of wheat prolamins: Detailed epitope analysis using a panel of monoclonal antibodies", JOURNAL OF CEREAL SCIENCE, ACADEMIC PRESS LTD, GB, vol. 32, no. 3, 1 November 2000 (2000-11-01), pages 259-279, XP002188676, ISSN: 0733-5210, DOI: 10.1006/JCRS.2000.0316

### SEQUENCE LISTING

<110> ISIS INNOVATION LIMITED
   ANDERSON, Robert Paul
   HILL, Adrian Vivian Sinton
   JEWELL, Derek Parry
<120> THERAPEUTIC EPITOPES AND USES THEREOF
<130> 142769 / P035468WO
<140> PCT/GB03/02450
   <141> 2003-06-05
<150> GB 0212885.8
   <151> 2002-06-05
<160> 758
<170> SeqWin99, version 1.02
<210> 1
   <211> 7
   <212> PRT
   <213> Artificial Sequence
<220>
   <223> peptide
<400> 1
   Pro Gln Pro Glu Leu Pro Tyr
   1 5
<210> 2
   <211> 17
   <212> **PRT**
   <213> Artificial Sequence
<220>
   <223> peptide
<400> 2
   Gln Leu Gln Pro Phe Pro Gln Pro Glu Leu Pro Tyr Pro Gln Pro Gln
   1 5 10 15
   Ser
<210> 3
   <211> 266
   <212> PRT
   <213> Homo sapiens
<400> 3
   Val Arg Val Pro Val Pro Gln Leu Gln Pro Gln Asn Pro Ser Gln Gln
   1 5 10 15
   Gln Pro Gln Glu Gln Val Pro Leu Val Gln Gln Gln Gln Phe Pro Gly
   20 25 30
   Gln Gln Gln Gln Phe Pro Pro Gln Gln Pro Tyr Pro Gln Pro Gln Pro
   35 40 45
   Phe Pro Ser Gln Gln Pro Tyr Leu Gln Leu Gln Pro Phe Pro Gln Pro
       50                  55                  60
   Gln Leu Pro Tyr Pro Gln Pro Gln Ser Phe Pro Pro Gln Gln Pro Tyr
   65                  70                  75                  80
   Pro Gln Pro Gln Pro Gln Tyr Ser Gln Pro Gln Gln Pro Ile Ser Gln
                   85                  90                  95
   Gln Gln Ala Gln Gln Gln Gln Gln Gln Gln Gln Gln Gln Gln Gln Gln
               100                 105                 110
   Gln Ile Leu Gln Gln Ile Leu Gln Gln Gln Leu Ile Pro Cys Met Asp
           115                 120                 125
   Val Val Leu Gln Gln His Asn Ile Ala His Ala Arg Ser Gln Val Leu
       130                 135                 140
   Gln Gln Ser Thr Tyr Gln Leu Leu Gln Glu Leu Cys Cys Gln His Leu
   145                 150                 155                 160
   Trp Gln Ile Pro Glu Gln Ser Gln Cys Gln Ala Ile His Asn Val Val
                   165                 170                 175
   His Ala Ile Ile Leu His Gln Gln Gln Lys Gln Gln Gln Gln Pro Ser
               180                 185                 190
   Ser Gln Val Ser Phe Gln Gln Pro Leu Gln Gln Tyr Pro Leu Gly Gln
           195                 200                 205
   Gly Ser Phe Arg Pro Ser Gln Gln Asn Pro Gln Ala Gln Gly Ser Val
       210                 215                 220
   Gln Pro Gln Gln Leu Pro Gln Phe Glu Glu Ile Arg Asn Leu Ala Leu
   225                 230                 235                 240
   Gln Thr Leu Pro Ala Met Cys Asn Val Tyr Ile Ala Pro Tyr Cys Thr
                   245                 250                 255
   Ile Ala Pro Phe Gly Ile Phe Gly Thr Asn
               260                 265
<210> 4
   <211> 7
   <212> PRT
   <213> Artificial Sequence
<220>
   <223> peptide
<400> 4
   Pro Gln Pro Gln Leu Pro Tyr
   1               5
<210> 5
   <211> 20
   <212> PRT
   <213> Artificial Sequence
<220>
   <223> peptide
<400> 5
   Leu Gln Leu Gln Pro Phe Pro Gln Pro Gln Leu Pro Tyr Pro Gln Pro
   1               5                   10                  15
   Gln Ser Phe Pro
               20
<210> 6
   <211> 17
   <212> PRT
   <213> Artificial Sequence
<220>
   <223> peptide
<400> 6
   Glu Leu Gln Pro Phe Pro Gln Pro Glu Leu Pro Tyr Pro Gln Pro Gln
   1               5                   10                  15
   Ser
<210> 7
   <211> 17
   <212> PRT
   <213> Artificial Sequence
<220>
   <223> peptide
<400> 7
   Gln Leu Gln Pro Phe Pro Gln Pro Glu Leu Pro Tyr Pro Gln Pro Glu
   1               5                   10                  15
   Ser
<210> 8
   <211> 17
   <212> PRT
   <213> Artificial Sequence
<220>
   <223> peptide
<400> 8
   Glu Leu Gln Pro Phe Pro Gln Pro Glu Leu Pro Tyr Pro Gln Pro Glu
   1               5                   10                  15
   Ser
<210> 9
   <211> 5
   <212> PRT
   <213> Artificial Sequence
<220>
   <223> peptide
<400> 9
   Gln Pro Gln Leu Pro
   1 5
<210> 10
   <211> 17
   <212> PRT
   <213> Artificial Sequence
<220>
   <223> peptide
<400> 10
   Gln Leu Gln Pro Phe Pro Gln Pro Gln Leu Pro Tyr Pro Gln Pro Gln
   1 5 10 15
   Ser
<210> 11
   <211> 20
   <212> PRT
   <213> Artificial Sequence
<220>
   <223> peptide
<400> 11
   Leu Gln Leu Gln Pro Phe Pro Gln Pro Glu Leu Pro Tyr Pro Gln Pro
   1 5 10 15
   Gln Ser Phe Pro
   20
<210> 12
   <211> 5
   <212> PRT
   <213> Artificial Sequence
<220>
   <223> peptide
<400> 12
   Pro Gln Leu Pro Tyr
   1 5
<210> 13
   <211> 12
   <212> PRT
   <213> Artificial Sequence
<220>
   <223> peptide
<400> 13
   Gln Leu Gln Pro Phe Pro Gln Pro Glu Leu Pro Tyr
   1 5 10
<210> 14
   <211> 11
   <212> PRT
   <213> Artificial Sequence
<220>
   <223> peptide
<400> 14
   Pro Phe Pro Gln Pro Glu Leu Pro Tyr Pro Gln
   1 5 10
<210> 15
   <211> 14
   <212> PRT
   <213> Artificial Sequence
<220>
   <223> peptide
<400> 15
   Pro Arg Ala Pro Trp Ile Glu Gln Glu Gly Pro Glu Tyr Trp
   1 5 10
<210> 16
   <211> 16
   <212> PRT
   <213> Artificial Sequence
<220>
   <223> peptide
<400> 16
   Ile Asp Val Trp Leu Gly Gly Leu Leu Ala Glu Asn Phe Leu Pro Tyr
   1 5 10 15
<210> 17
   <211> 17
   <212> PRT
   <213> Artificial Sequence
<220>
   <223> peptide
<400> 17
   Pro Gln Pro Gln Pro Phe Pro Pro Glu Leu Pro Tyr Pro Gln Pro Gln
   1 5 10 15
   Ser
<210> 18
   <211> 9
   <212> PRT
   <213> Artificial Sequence
<220>
   <223> peptide
<400> 18
   Phe Pro Gln Pro Gln Leu Pro Tyr Pro
   1 5
<210> 19
   <211> 9
   <212> PRT
   <213> Artificial Sequence
<220>
   <223> peptide
<400> 19
   Phe Pro Gln Pro Gln Gln Pro Phe Pro
   1 5
<210> 20
   <211> 9
   <212> PRT
   <213> Artificial Sequence
<220>
   <223> peptide
<400> 20
   Pro Gln Gln Pro Gln Gln Pro Phe Pro
   1 5
<210> 21
   <211> 12
   <212> PRT
   <213> Artificial Sequence
<220>
   <223> peptide
<400> 21
   Leu Gln Pro Gln Asn Pro Ser Gln Gln Gln Pro Gln
   1 5 10
<210> 22
   <211> 12
   <212> PRT
   <213> Artificial Sequence
<220>
   <223> peptide
<400> 22
   Leu Gln Pro Glu Asn Pro Ser Gln Glu Gln Pro Glu
   1               5                   10
<210> 23
   <211> 17
   <212> PRT
   <213> Artificial Sequence
<220>
   <223> peptide
<220>
   <222> 1-7 and 12-17
   <223> Xaa is any amino acid
<220>
   <222> 10
   <223> Xaa is Ile, Leu, Met or Pro
<220>
   <222> 11
   <223> Xaa is Pro, Ser or Thr
<400> 23
   Xaa Xaa Xaa Xaa Xaa Xaa Xaa Pro Gln Xaa Xaa Xaa Xaa Xaa Xaa Xaa
   1               5                   10                  15
   Xaa
<210> 24
   <211> 20
   <212> PRT
   <213> Artificial Sequence
<220>
   <223> peptide
<400> 24
   Val Leu Gln Gln His Asn Ile Ala His Gly Ser Ser Gln Val Leu Gln
   1               5                   10                  15
   Glu Ser Thr Tyr
               20
<210> 25
   <211> 20
   <212> PRT
   <213> Artificial Sequence
<220>
   <223> peptide
<400> 25
   Ile Lys Asp Phe His Val Tyr Phe Arg Glu Ser Arg Asp Ala Leu Trp
   1               5                   10                  15
   Lys Gly Pro Gly
               20
<210> 26
   <211> 17
   <212> PRT
   <213> Artificial Sequence
<220>
   <223> peptide
<400> 26
   Gln Leu Gln Pro Phe Pro Gln Pro Gln Leu Pro Tyr Pro Gln Pro Gln
   1               5                   10                  15
   Pro
<210> 27
   <211> 17
   <212> PRT
   <213> Artificial Sequence
<220>
   <223> peptide
<400> 27
   Gln Leu Gln Pro Phe Pro Gln Pro Glu Leu Pro Tyr Pro Gln Pro Gln
   1               5                   10                  15
   Pro
<210> 28
   <211> 17
   <212> PRT
   <213> Artificial Sequence
<220>
   <223> peptide
<400> 28
   Gln Leu Gln Pro Phe Pro Gln Pro Gln Leu Pro Tyr Ser Gln Pro Gln
   1 5 10 15
   Pro
<210> 29
   <211> 17
   <212> PRT
   <213> Artificial Sequence
<220>
   <223> peptide
<400> 29
   Gln Leu Gln Pro Phe Pro Gln Pro Glu Leu Pro Tyr Ser Gln Pro Gln
   1 5 10 15
   Pro
<210> 30
   <211> 20
   <212> PRT
   <213> Artificial Sequence
<220>
   <223> peptide
<400> 30
   Gln Gln Thr Tyr Pro Gln Arg Pro Gln Gln Pro Phe Pro Gln Thr Gln
   1 5 10 15
   Gln Pro Gln Gln
   20
<210> 31
   <211> 20
   <212> PRT
   <213> Artificial Sequence
<220>
   <223> peptide
<400> 31
   Pro Gln Gln Pro Gln Gln Pro Gln Gln Pro Phe Pro Gln Pro Gln Gln
   1 5 10 15
   Pro Phe Pro Trp
   20
<210> 32
   <211> 20
   <212> PRT
   <213> Artificial Sequence
<220>
   <223> peptide
<400> 32
   Gln Gln Pro Gln Gln Pro Phe Pro Gln Pro Gln Gln Pro Gln Leu Pro
   1               5                   10                  15
   Phe Pro Gln Gln
               20
<210> 33
   <211> 20
   <212> PRT
   <213> Artificial Sequence
<220>
   <223> peptide
<400> 33
   Gln Ala Phe Pro Gln Pro Gln Gln Thr Phe Pro His Gln Pro Gln Gln
   1               5                   10                  15
   Gln Phe Pro Gln
               20
<210> 34
   <211> 20
   <212> PRT
   <213> Artificial Sequence
<220>
   <223> peptide
<400> 34
   Thr Gln Gln Pro Gln Gln Pro Phe Pro Gln Gln Pro Gln Gln Pro Phe
   1               5                   10                  15
   Pro Gln Thr Gln
               20
<210> 35
   <211> 20
   <212> PRT
   <213> Artificial Sequence
<220>
   <223> peptide
<400> 35
   Pro Ile Gln Pro Gln Gln Pro Phe Pro Gln Gln Pro Gln Gln Pro Gln
   1               5                   10                  15
   Gln Pro Phe Pro
               20
<210> 36
   <211> 20
   <212> PRT
   <213> Artificial Sequence
<220>
   <223> peptide
<400> 36
   Pro Gln Gln Ser Phe Ser Tyr Gln Gln Gln Pro Phe Pro Gln Gln Pro
   1               5                   10                  15
   Tyr Pro Gln Gln
               20
<210> 37
   <211> 4
   <212> PRT
   <213> Artificial Sequence
<220>
   <223> peptide
<220>
   <222> 2
   <223> Xaa is any amino acid
<400> 37
   Gln Xaa Pro Phe
   1
<210> 38
   <211> 4
   <212> PRT
   <213> Artificial Sequence
<220>
   <223> peptide
<220>
   <222> 2-3
   <223> Xaa is any amino acid
<220>
   <222> 4
   <223> Xaa is Phe or Tyr
<400> 38
   Gln Xaa Xaa Xaa
   1
<210> 39
   <211> 12
   <212> PRT
   <213> Artificial Sequence
<220>
   <223> peptide
<400> 39
   Gln Gln Pro Phe Pro Gln Pro Gln Gln Pro Phe Pro
   1               5                   10
<210> 40
   <211> 13
   <212> PRT
   <213> Artificial Sequence
<220>
   <223> peptide
<400> 40
   Gln Gln Pro Phe Pro Gln Gln Pro Gln Gln Pro Phe Pro
   1               5                   10
<210> 41
   <211> 12
   <212> PRT
   <213> Artificial Sequence
<220>
   <223> peptide
<400> 41
   Pro Ile Gln Pro Gln Gln Pro Phe Pro Gln Gln Pro
   1               5                   10
<210> 42
   <211> 12
   <212> PRT
   <213> Artificial Sequence
<220>
   <223> peptide
<400> 42
   Leu Gln Pro Gln Asn Pro Ser Gln Gln Gln Pro Gln
   1               5                   10
<210> 43
   <211> 12
   <212> PRT
   <213> Artificial Sequence
<220>
   <223> peptide
<400> 43
   Leu Gln Pro Gln Asn Pro Ser Gln Gln Gln Pro Gln
   1 5 10
<210> 44
   <211> 18
   <212> PRT
   <213> Artificial Sequence
<220>
   <223> peptide
<400> 44
   Gln Gln Tyr Pro Ser Gly Gln Gly Ser Phe Gln Pro Ser Gln Gln Asn
   1 5 10 15
   Pro Gln
<210> 45
   <211> 6
   <212> PRT
   <213> Artificial Sequence
<220>
   <223> peptide
<220>
   <222> 3
   <223> Xaa is Gln or Leu
<220>
   <222> 5
   <223> Xaa is Phe or Tyr
<400> 45
   Pro Gln Xaa Pro Xaa Pro
   1 5
<210> 46
   <211> 12
   <212> PRT
   <213> Artificial Sequence
<220>
   <223> peptide
<400> 46
   Leu Gln Pro Gln Asn Pro Ser Gln Gln Gln Pro Gln
   1 5 10
<210> 47
   <211> 14
   <212> PRT
   <213> Artificial Sequence
<220>
   <223> peptide
<400> 47
   Pro Gln Pro Glu Leu Pro Tyr Pro Gln Pro Glu Leu Pro Tyr
   1               5                   10
<210> 48
   <211> 20
   <212> PRT
   <213> Artificial Sequence
<220>
   <223> peptide
<400> 48
   Gln Gln Leu Pro Gln Pro Glu Gln Pro Gln Gln Ser Phe Pro Glu Gln
   1               5                   10                  15
   Glu Arg Pro Phe
               20
<210> 49
   <211> 17
   <212> PRT
   <213> Artificial Sequence
<220>
   <223> peptide
<400> 49
   Gln Leu Gln Pro Phe Pro Gln Pro Glu Leu Pro Tyr Pro Gln Pro Gln
   1               5                   10                  15
   Leu
<210> 50
   <211> 22
   <212> PRT
   <213> Artificial Sequence
<220>
   <223> peptide
<400> 50
   Pro Gln Gln Pro Gln Gln Pro Gln Gln Pro Phe Pro Gln Pro Gln Gln
   1               5                   10                  15
   Pro Phe Pro Trp Gln Pro
               20
<210> 51
   <211> 17
   <212> PRT
   <213> Artificial Sequence
<220>
   <223> peptide
<400> 51
   Gln Leu Gln Pro Phe Pro Gln Pro Gln Leu Pro Tyr Pro Gln Pro Gln
   1               5                   10                  15
   Leu
<210> 52
   <211> 15
   <212> PRT
   <213> Artificial Sequence
<220>
   <223> peptide
<400> 52
   Gln Pro Gln Gln Pro Phe Pro Gln Pro Gln Gln Pro Phe Pro Trp
   1               5                   10                  15
<210> 53
   <211> 12
   <212> PRT
   <213> Artificial Sequence
<220>
   <223> peptide
<400> 53
   Gln Leu Gln Pro Phe Pro Gln Pro Gln Leu Pro Tyr
   1               5                   10
<210> 54
   <211> 9
   <212> PRT
   <213> Artificial Sequence
<220>
   <223> peptide
<400> 54
   Pro Phe Pro Gln Pro Gln Leu Pro Tyr
   1               5
<210> 55
   <211> 9
   <212> PRT
   <213> Artificial Sequence
<220>
   <223> peptide
<400> 55
   Pro Gln Pro Gln Leu Pro Tyr Pro Gln
   1 5
<210> 56
   <211> 9
   <212> PRT
   <213> Artificial Sequence
<220>
   <223> peptide
<400> 56
   Pro Tyr Pro Gln Pro Gln Leu Pro Tyr
   1 5
<210> 57
   <211> 11
   <212> PRT
   <213> Artificial Sequence
<220>
   <223> peptide
<400> 57
   Pro Gln Gln Pro Tyr Pro Gln Pro Gln Pro Gln
   1 5 10
<210> 58
   <211> 9
   <212> PRT
   <213> Artificial Sequence
<220>
   <223> peptide
<400> 58
   Pro Gln Gln Ser Phe Pro Gln Gln Gln
   1 5
<210> 59
   <211> 8
   <212> PRT
   <213> Artificial Sequence
<220>
   <223> peptide
<400> 59
   Ile Ile Pro Gln Gln Pro Ala Gln
   1 5
<210> 60
   <211> 13
   <212> PRT
   <213> Artificial Sequence
<220>
   <223> peptide
<400> 60
   Phe Pro Gln Gln Pro Gln Gln Pro Tyr Pro Gln Gln Pro
   1               5                   10
<210> 61
   <211> 12
   <212> PRT
   <213> Artificial Sequence
<220>
   <223> peptide
<400> 61
   Phe Ser Gln Pro Gln Gln Gln Phe Pro Gln Pro Gln
   1               5                   10
<210> 62
   <211> 20
   <212> PRT
   <213> Artificial Sequence
<220>
   <223> peptide
<400> 62
   Leu Gln Pro Gln Gln Pro Phe Pro Gln Gln Pro Gln Gln Pro Tyr Pro
   1               5                   10                  15
   Gln Gln Pro Gln
               20
<210> 63
   <211> 13
   <212> PRT
   <213> Artificial Sequence
<220>
   <223> peptide
<400> 63
   Gln Ser Glu Gln Ser Gln Gln Pro Phe Pro Gln Gln Phe
   1               5                   10
<210> 64
   <211> 9
   <212> PRT
   <213> Artificial Sequence
<220>
   <223> peptide
<220>
   <222> 2
   <223> Xaa is Ile or Leu
<400> 64
   Gln Xaa Pro Gln Gln Pro Gln Gln Phe
   1 5
<210> 65
   <211> 10
   <212> PRT
   <213> Artificial Sequence
<220>
   <223> peptide
<400> 65
   Pro Phe Ser Gln Gln Gln Gln Ser Pro Phe
   1 5 10
<210> 66
   <211> 8
   <212> PRT
   <213> Artificial Sequence
<220>
   <223> peptide
<400> 66
   Pro Phe Ser Gln Gln Gln Gln Gln
   1 5
<210> 67
   <211> 17
   <212> PRT
   <213> Artificial Sequence
<220>
   <223> peptide
<400> 67
   Gln Leu Gln Pro Phe Pro Gln Pro Gln Leu Pro Tyr Leu Gln Pro Gln
   1 5 10 15
   Pro
<210> 68
   <211> 17
   <212> PRT
   <213> Artificial Sequence
<220>
   <223> peptide
<400> 68
   Gln Leu Gln Pro Phe Pro Arg Pro Gln Leu Pro Tyr Pro Gln Pro Gln
   1 5 10 15
   Pro
<210> 69
   <211> 17
   <212> PRT
   <213> Artificial Sequence
<220>
   <223> peptide
<400> 69
   Gln Leu Gln Pro Phe Leu Gln Pro Gln Leu Pro Tyr Ser Gln Pro Gln
   1 5 10 15
   Pro
<210> 70
   <211> 17
   <212> PRT
   <213> Artificial Sequence
<220>
   <223> peptide
<400> 70
   Gln Leu Gln Pro Phe Ser Gln Pro Gln Leu Pro Tyr Ser Gln Pro Gln
   1 5 10 15
   Pro
<210> 71
   <211> 17
   <212> PRT
   <213> Artificial Sequence
<220>
   <223> peptide
<400> 71
   Gln Leu Gln Pro Phe Pro Gln Pro Gln Leu Ser Tyr Ser Gln Pro Gln
   1 5 10 15
   Pro
<210> 72
   <211> 17
   <212> PRT
   <213> Artificial Sequence
<220>
   <223> peptide
<400> 72
   Pro Gln Leu Pro Tyr Pro Gln Pro Gln Leu Pro Tyr Pro Gln Pro Gln
   1 5 10 15
   Pro
<210> 73
   <211> 17
   <212> PRT
   <213> Artificial Sequence
<220>
   <223> peptide
<400> 73
   Pro Gln Leu Pro Tyr Pro Gln Pro Gln Leu Pro Tyr Pro Gln Pro Gln
   1 5 10 15
   Leu
<210> 74
   <211> 17
   <212> PRT
   <213> Artificial Sequence
<220>
   <223> peptide
<400> 74
   Pro Gln Pro Gln Pro Phe Leu Pro Gln Leu Pro Tyr Pro Gln Pro Gln
   1 5 10 15
   Ser
<210> 75
   <211> 17
   <212> PRT
   <213> Artificial Sequence
<220>
   <223> peptide
<400> 75
   Pro Gln Pro Gln Pro Phe Pro Pro Gln Leu Pro Tyr Pro Gln Pro Gln
   1 5 10 15
   Ser
<210> 76
   <211> 17
   <212> PRT
   <213> Artificial Sequence
<220>
   <223> peptide
<400> 76
   Pro Gln Pro Gln Pro Phe Pro Pro Gln Leu Pro Tyr Pro Gln Tyr Gln
   1               5                   10                  15
   Pro
<210> 77
   <211> 17
   <212> PRT
   <213> Artificial Sequence
<220>
   <223> peptide
<400> 77
   Pro Gln Pro Gln Pro Phe Pro Pro Gln Leu Pro Tyr Pro Gln Pro Pro
   1               5                   10                  15
   Pro
<210> 78
   <211> 18
   <212> PRT
   <213> Artificial Sequence
<220>
   <223> peptide
<400> 78
   Val Pro Gln Leu Gln Pro Gln Asn Pro Ser Gln Gln Gln Pro Gln Glu
   1               5                   10                  15
   Gln Val
<210> 79
   <211> 18
   <212> PRT
   <213> Artificial Sequence
<220>
   <223> peptide
<400> 79
   Val Pro Gln Leu Gln Pro Glu Asn Pro Ser Gln Gln Gln Pro Gln Glu
   1               5                   10                  15
   Gln Val
<210> 80
   <211> 18
   <212> PRT
   <213> Artificial Sequence
<220>
   <223> peptide
<400> 80
   Val Pro Gln Leu Gln Pro Arg Asn Pro Ser Gln Gln Gln Pro Gln Glu
   1               5                   10                  15
   Gln Val
<210> 81
   <211> 18
   <212> PRT
   <213> Artificial Sequence
<220>
   <223> peptide
<400> 81
   Val Pro Gln Leu Gln Pro Gln Asn Pro Ser Gln Glu Gln Pro Gln Glu
   1               5                   10                  15
   Gln Val
<210> 82
   <211> 18
   <212> PRT
   <213> Artificial Sequence
<220>
   <223> peptide
<400> 82
   Val Pro Gln Leu Gln Pro Gln Asn Pro Ser Gln Arg Gln Pro Gln Glu
   1               5                   10                  15
   Gln Val
<210> 83
   <211> 18
   <212> PRT
   <213> Artificial Sequence
<220>
   <223> peptide
<400> 83
   Val Pro Gln Leu Gln Pro Gln Asn Pro Ser Gln Gln Gln Pro Glu Glu
   1               5                   10                  15
   Gln Val
<210> 84
   <211> 18
   <212> PRT
   <213> Artificial Sequence
<220>
   <223> peptide
<400> 84
   Val Pro Gln Leu Gln Pro Gln Asn Pro Ser Gln Gln Gln Pro Arg Glu
   1               5                   10                  15
   Gln Val
<210> 85
   <211> 18
   <212> PRT
   <213> Artificial Sequence
<220>
   <223> peptide
<400> 85
   Val Pro Gln Leu Gln Pro Gln Asn Pro Ser Gln Glu Gln Pro Glu Glu
   1               5                   10                  15
   Gln Val
<210> 86
   <211> 18
   <212> PRT
   <213> Artificial Sequence
<220>
   <223> peptide
<400> 86
   Val Pro Gln Leu Gln Pro Glu Asn Pro Ser Gln Gln Gln Pro Glu Glu
   1               5                   10                  15
   Gln Val
<210> 87
   <211> 18
   <212> PRT
   <213> Artificial Sequence
<220>
   <223> peptide
<400> 87
   Val Pro Gln Leu Gln Pro Glu Asn Pro Ser Gln Glu Gln Pro Gln Glu
   1               5                   10                  15
   Gln Val
<210> 88
   <211> 18
   <212> PRT
   <213> Artificial Sequence
<220>
   <223> peptide
<400> 88
   Val Pro Gln Leu Gln Pro Glu Asn Pro Ser Gln Glu Gln Pro Glu Glu
   1               5                   10                  15
   Gln Val
<210> 89
   <211> 15
   <212> PRT
   <213> Artificial Sequence
<220>
   <223> peptide
<400> 89
   Arg Trp Pro Val Pro Gln Leu Gln Pro Gln Asn Pro Ser Gln Gln
   1               5                   10                  15
<210> 90
   <211> 15
   <212> PRT
   <213> Artificial Sequence
<220>
   <223> peptide
<400> 90
   Trp Pro Val Pro Gln Leu Gln Pro Gln Asn Pro Ser Gln Gln Gln
   1               5                   10                  15
<210> 91
   <211> 15
   <212> PRT
   <213> Artificial Sequence
<220>
   <223> peptide
<400> 91
   Pro Val Pro Gln Leu Gln Pro Gln Asn Pro Ser Gln Gln Gln Pro
   1 5 10 15
<210> 92
   <211> 15
   <212> PRT
   <213> Artificial Sequence
<220>
   <223> peptide
<400> 92
   Val Pro Gln Leu Gln Pro Gln Asn Pro Ser Gln Gln Gln Pro Gln
   1 5 10 15
<210> 93
   <211> 15
   <212> PRT
   <213> Artificial Sequence
<220>
   <223> peptide
<400> 93
   Pro Gln Leu Gln Pro Gln Asn Pro Ser Gln Gln Gln Pro Gln Glu
   1 5 10 15
<210> 94
   <211> 15
   <212> PRT
   <213> Artificial Sequence
<220>
   <223> peptide
<400> 94
   Gln Leu Gln Pro Gln Asn Pro Ser Gln Gln Gln Pro Gln Glu Gln
   1 5 10 15
<210> 95
   <211> 15
   <212> PRT
   <213> Artificial Sequence
<220>
   <223> peptide
<400> 95
   Leu Gln Pro Gln Asn Pro Ser Gln Gln Gln Pro Gln Glu Gln Val
   1 5 10 15
<210> 96
   <211> 15
   <212> PRT
   <213> Artificial Sequence
<220>
   <223> peptide
<400> 96
   Gln Pro Gln Asn Pro Ser Gln Gln Gln Pro Gln Glu Gln Val Pro
   1 5 10 15
<210> 97
   <211> 17
   <212> PRT
   <213> Artificial Sequence
<220>
   <223> peptide
<400> 97
   Pro Gln Pro Gln Pro Phe Pro Pro Gln Leu Pro Tyr Pro Gln Thr Gln
   1 5 10 15
   Pro
<210> 98
   <211> 19
   <212> PRT
   <213> Artificial Sequence
<220>
   <223> peptide
<400> 98
   Leu Gly Gln Gln Gln Pro Phe Pro Pro Gln Gln Pro Tyr Pro Gln Pro
   1 5 10 15
   Gln Pro Phe
<210> 99
   <211> 18
   <212> PRT
   <213> Artificial Sequence
<220>
   <223> peptide
<400> 99
   Gln Gln Tyr Pro Ser Gly Glu Gly Ser Phe Gln Pro Ser Gln Glu Asn
   1 5 10 15
   Pro Gln
<210> 100
   <211> 15
   <212> PRT
   <213> Artificial Sequence
<220>
   <223> peptide
<400> 100
   Gly Gln Gln Gly Tyr Tyr Pro Thr Ser Pro Gln Gln Ser Gly Gln
   1               5                   10                  15
<210> 101
   <211> 17
   <212> PRT
   <213> Artificial Sequence
<220>
   <223> peptide
<400> 101
   Pro Gln Leu Pro Tyr Pro Gln Pro Glu Leu Pro Tyr Pro Gln Pro Gln
   1               5                   10                  15
   Pro
<210> 102
   <211> 14
   <212> PRT
   <213> Artificial Sequence
<220>
   <223> peptide
<400> 102
   Pro Gln Pro Gln Leu Pro Tyr Pro Gln Pro Gln Leu Pro Tyr
   1               5                   10
<210> 103
   <211> 20
   <212> PRT
   <213> Artificial Sequence
<220>
   <223> peptide
<400> 103
   Gln Gln Leu Pro Gln Pro Gln Gln Pro Gln Gln Ser Phe Pro Gln Gln
   1               5                   10                  15
   Gln Arg Pro Phe
               20
<210> 104
   <211> 17
   <212> PRT
   <213> Artificial Sequence
<220>
   <223> peptide
<400> 104
   Gln Leu Gln Pro Phe Pro Gln Pro Gln Leu Pro Tyr Leu Gln Pro Gln
   1               5                   10                  15
   Ser
<210> 105
   <211> 17
   <212> PRT
   <213> Artificial Sequence
<220>
   <223> peptide
<400> 105
   Gln Pro Gln Pro Phe Pro Pro Pro Gln Leu Pro Tyr Pro Gln Thr Gln
   1               5                   10                  15
   Pro
<210> 106
   <211> 17
   <212> PRT
   <213> Artificial Sequence
<220>
   <223> peptide
<400> 106
   Pro Gln Pro Gln Pro Phe Pro Pro Gln Leu Pro Tyr Pro Gln Thr Gln
   1               5                   10                  15
   Ser
<210> 107
   <211> 20
   <212> PRT
   <213> Artificial Sequence
<220>
   <223> peptide
<400> 107
   Ala Val Arg Phe Pro Val Pro Gln Leu Gln Pro Gln Asn Pro Ser Gln
   1 5 10 15
   Gln Leu Pro Gln
   20
<210> 108
   <211> 20
   <212> PRT
   <213> Artificial Sequence
<220>
   <223> peptide
<400> 108
   Met Val Arg Val Pro Val Pro Gln Leu Gln Pro Gln Asn Pro Ser Gln
   1 5 10 15
   Gln Gln Pro Gln
   20
<210> 109
   <211> 20
   <212> PRT
   <213> Artificial Sequence
<220>
   <223> peptide
<400> 109
   Met Val Arg Val Pro Val Pro Gln Leu Gln Pro Gln Asn Pro Ser Gln
   1 5 10 15
   Gln His Pro Gln
   20
<210> 110
   <211> 20
   <212> PRT
   <213> Artificial Sequence
<220>
   <223> peptide
<400> 110
   Met Val Arg Val Pro Met Pro Gln Leu Gln Pro Gln Asp Pro Ser Gln
   1 5 10 15
   Gln Gln Pro Gln
   20
<210> 111
   <211> 20
   <212> PRT
   <213> Artificial Sequence
<220>
   <223> peptide
<400> 111
   Met Val Arg Val Thr Val Pro Gln Leu Gln Pro Gln Asn Pro Ser Gln
   1               5                   10                  15
   Gln Gln Pro Gln
               20
<210> 112
   <211> 20
   <212> PRT
   <213> Artificial Sequence
<220>
   <223> peptide
<400> 112
   Ala Val Arg Val Ser Val Pro Gln Leu Gln Pro Gln Asn Pro Ser Gln
   1               5                   10                  15
   Gln Gln Pro Gln
               20
<210> 113
   <211> 20
   <212> PRT
   <213> Artificial Sequence
<220>
   <223> peptide
<400> 113
   Ala Val Arg Val Pro Val Pro Gln Leu Gln Pro Gln Asn Pro Ser Gln
   1               5                   10                  15
   Gln Gln Pro Gln
               20
<210> 114
   <211> 20
   <212> PRT
   <213> Artificial Sequence
<220>
   <223> peptide
<400> 114
   Ala Val Arg Trp Pro Val Pro Gln Leu Gln Pro Gln Asn Pro Ser Gln
   1               5                   10                  15
   Gln Gln Pro Gln
               20
<210> 115
   <211> 20
   <212> PRT
   <213> Artificial Sequence
<220>
   <223> peptide
<400> 115
   Ala Val Arg Val Pro Val Pro Gln Leu Gln Leu Gln Asn Pro Ser Gln
   1               5                   10                  15
   Gln Gln Pro Gln
               20
<210> 116
   <211> 20
   <212> PRT
   <213> Artificial Sequence
<220>
   <223> peptide
<400> 116
   Met Val Arg Val Pro Val Pro Gln Leu Gln Leu Gln Asn Pro Ser Gln
   1               5                   10                  15
   Gln Gln Pro Gln
               20
<210> 117
   <211> 20
   <212> PRT
   <213> Artificial Sequence
<220>
   <223> peptide
<400> 117
   Ala Val Arg Val Pro Val Pro Gln Pro Gln Pro Gln Asn Pro Ser Gln
   1               5                   10                  15
   Pro Gln Pro Gln
               20
<210> 118
   <211> 20
   <212> PRT
   <213> Artificial Sequence
<220>
   <223> peptide
<400> 118
   Ala Val Arg Val Pro Val Pro Gln Leu Gln Pro Lys Asn Pro Ser Gln
   1               5                   10                  15
   Gln Gln Pro Gln
               20
<210> 119
   <211> 20
   <212> PRT
   <213> Artificial Sequence
<220>
   <223> peptide
<400> 119
   Leu Gln Pro Gln Asn Pro Ser Gln Gln Leu Pro Gln Glu Gln Val Pro
   1               5                   10                  15
   Leu Val Gln Gln
               20
<210> 120
   <211> 20
   <212> PRT
   <213> Artificial Sequence
<220>
   <223> peptide
<400> 120
   Leu Gln Pro Gln Asn Pro Ser Gln Gln Gln Pro Gln Glu Gln Val Pro
   1               5                   10                  15
   Leu Val Gln Gln
               20
<210> 121
   <211> 20
   <212> PRT
   <213> Artificial Sequence
<220>
   <223> peptide
<400> 121
   Leu Gln Pro Gln Asn Pro Ser Gln Gln His Pro Gln Glu Gln Val Pro
   1               5                   10                  15
   Leu Val Gln Gln
               20
<210> 122
   <211> 20
   <212> PRT
   <213> Artificial Sequence
<220>
   <223> peptide
<400> 122
   Leu Gln Pro Gln Asp Pro Ser Gln Gln Gln Pro Gln Glu Gln Val Pro
   1               5                   10                  15
   Leu Val Gln Gln
               20
<210> 123
   <211> 20
   <212> PRT
   <213> Artificial Sequence
<220>
   <223> peptide
<400> 123
   Leu Gln Pro Gln Asn Pro Ser Gln Gln Gln Pro Gln Lys Gln Val Pro
   1               5                   10                  15
   Leu Val Gln Gln
               20
<210> 124
   <211> 20
   <212> PRT
   <213> Artificial Sequence
<220>
   <223> peptide
<400> 124
   Leu Gln Leu Gln Asn Pro Ser Gln Gln Gln Pro Gln Glu Gln Val Pro
   1               5                   10                  15
   Leu Val Gln Glu
               20
<210> 125
   <211> 20
   <212> PRT
   <213> Artificial Sequence
<220>
   <223> peptide
<400> 125
   Leu Gln Leu Gln Asn Pro Ser Gln Gln Gln Pro Gln Glu Gln Val Pro
   1               5                   10                  15
   Leu Val Gln Glu
               20
<210> 126
   <211> 20
   <212> PRT
   <213> Artificial Sequence
<220>
   <223> peptide
<400> 126
   Pro Gln Pro Gln Asn Pro Ser Gln Pro Gln Pro Gln Gly Gln Val Pro
   1               5                   10                  15
   Leu Val Gln Gln
               20
<210> 127
   <211> 20
   <212> PRT
   <213> Artificial Sequence
<220>
   <223> peptide
<400> 127
   Pro Gln Pro Gln Asn Pro Ser Gln Pro Gln Pro Gln Arg Gln Val Pro
   1               5                   10                  15
   Leu Val Gln Gln
               20
<210> 128
   <211> 20
   <212> PRT
   <213> Artificial Sequence
<220>
   <223> peptide
<400> 128
   Leu Gln Pro Lys Asn Pro Ser Gln Gln Gln Pro Gln Glu Gln Val Pro
   1               5                   10                  15
   Leu Val Gln Gln
               20
<210> 129
   <211> 20
   <212> PRT
   <213> Artificial Sequence
<220>
   <223> peptide
<400> 129
   Leu Gln Pro Gln Asn Pro Ser Gln Gln Gln Pro Gln Glu Gln Val Pro
   1 5 10 15
   Leu Met Gln Gln
   20
<210> 130
   <211> 20
   <212> PRT
   <213> Artificial Sequence
<220>
   <223> peptide
<400> 130
   Gln Leu Pro Gln Glu Gln Val Pro Leu Val Gln Gln Gln Gln Phe Leu
   1 5 10 15
   Gly Gln Gln Gln
   20
<210> 131
   <211> 20
   <212> PRT
   <213> Artificial Sequence
<220>
   <223> peptide
<400> 131
   Gln His Pro Gln Glu Gln Val Pro Leu Val Gln Gln Gln Gln Phe Leu
   1 5 10 15
   Gly Gln Gln Gln
   20
<210> 132
   <211> 20
   <212> PRT
   <213> Artificial Sequence
<220>
   <223> peptide
<400> 132
   Gln Gln Pro Gln Glu Gln Val Pro Leu Val Gln Gln Gln Gln Phe Leu
   1 5 10 15
   Gly Gln Gln Gln
   20
<210> 133
   <211> 20
   <212> PRT
   <213> Artificial Sequence
<220>
   <223> peptide
<400> 133
   Gln Gln Pro Gln Glu Gln Val Pro Leu Val Gln Gln Gln Gln Phe Leu
   1               5                   10                  15
   Gly Gln Gln Gln
               20
<210> 134
   <211> 20
   <212> PRT
   <213> Artificial Sequence
<220>
   <223> peptide
<400> 134
   Gln Gln Pro Gln Glu Gln Val Pro Leu Val Gln Gln Gln Gln Phe Pro
   1               5                   10                  15
   Gly Gln Gln Gln
               20
<210> 135
   <211> 20
   <212> PRT
   <213> Artificial Sequence
<220>
   <223> peptide
<400> 135
   Gln Gln Pro Gln Lys Gln Val Pro Leu Val Gln Gln Gln Gln Phe Pro
   1               5                   10                  15
   Gly Gln Gln Gln
               20
<210> 136
   <211> 20
   <212> PRT
   <213> Artificial Sequence
<220>
   <223> peptide
<400> 136
   Gln Gln Pro Gln Glu Gln Val Pro Leu Val Gln Glu Gln Gln Phe Gln
   1               5                   10                  15
   Gly Gln Gln Gln
               20
<210> 137
   <211> 20
   <212> PRT
   <213> Artificial Sequence
<220>
   <223> peptide
<400> 137
   Pro Gln Pro Gln Gly Gln Val Pro Leu Val Gln Gln Gln Gln Phe Pro
   1               5                   10                  15
   Gly Gln Gln Gln
               20
<210> 138
   <211> 20
   <212> PRT
   <213> Artificial Sequence
<220>
   <223> peptide
<400> 138
   Pro Gln Pro Gln Arg Gln Val Pro Leu Val Gln Gln Gln Gln Phe Pro
   1               5                   10                  15
   Gly Gln Gln Gln
               20
<210> 139
   <211> 20
   <212> PRT
   <213> Artificial Sequence
<220>
   <223> peptide
<400> 139
   Gln Gln Pro Gln Glu Gln Val Pro Leu Met Gln Gln Gln Gln Gln Phe
   1               5                   10                  15
   Pro Gly Gln Gln
               20
<210> 140
   <211> 20
   <212> PRT
   <213> Artificial Sequence
<220>
   <223> peptide
<400> 140
   Leu Val Gln Gln Gln Gln Phe Leu Gly Gln Gln Gln Pro Phe Pro Pro
   1 5 10 15
   Gln Gln Pro Tyr
   20
<210> 141
   <211> 20
   <212> PRT
   <213> Artificial Sequence
<220>
   <223> peptide
<400> 141
   Leu Val Gln Gln Gln Gln Phe Leu Gly Gln Gln Gln Ser Phe Pro Pro
   1 5 10 15
   Gln Gln Pro Tyr
   20
<210> 142
   <211> 20
   <212> PRT
   <213> Artificial Sequence
<220>
   <223> peptide
<400> 142
   Leu Val Gln Gln Gln Gln Phe Leu Gly Gln Gln Gln Pro Phe Pro Pro
   1 5 10 15
   Gln Gln Pro Tyr
   20
<210> 143
   <211> 20
   <212> PRT
   <213> Artificial Sequence
<220>
   <223> peptide
<400> 143
   Leu Val Gln Gln Gln Gln Phe Pro Gly Gln Gln Gln Pro Phe Pro Pro
   1 5 10 15
   Gln Gln Pro Tyr
   20
<210> 144
   <211> 20
   <212> PRT
   <213> Artificial Sequence
<220>
   <223> peptide
<400> 144
   Leu Val Gln Glu Gln Gln Phe Gln Gly Gln Gln Gln Pro Phe Pro Pro
   1               5                   10                  15
   Gln Gln Pro Tyr
               20
<210> 145
   <211> 20
   <212> PRT
   <213> Artificial Sequence
<220>
   <223> peptide
<400> 145
   Leu Val Gln Gln Gln Gln Phe Pro Gly Gln Gln Gln Gln Phe Pro Pro
   1               5                   10                  15
   Gln Gln Pro Tyr
               20
<210> 146
   <211> 20
   <212> PRT
   <213> Artificial Sequence
<220>
   <223> peptide
<400> 146
   Leu Met Gln Gln Gln Gln Gln Phe Pro Gly Gln Gln Glu Gln Phe Pro
   1               5                   10                  15
   Pro Gln Gln Pro
               20
<210> 147
   <211> 20
   <212> PRT
   <213> Artificial Sequence
<220>
   <223> peptide
<400> 147
   Leu Met Gln Gln Gln Gln Gln Phe Pro Gly Gln Gln Glu Arg Phe Pro
   1               5                   10                  15
   Pro Gln Gln Pro
               20
<210> 148
   <211> 20
   <212> PRT
   <213> Artificial Sequence
<220>
   <223> peptide
<400> 148
   Gly Gln Gln Gln Pro Phe Pro Pro Gln Gln Pro Tyr Pro Gln Pro Gln
   1               5                   10                  15
   Pro Phe Pro Ser
               20
<210> 149
   <211> 20
   <212> PRT
   <213> Artificial Sequence
<220>
   <223> peptide
<400> 149
   Gly Gln Gln Gln Pro Phe Pro Pro Gln Gln Pro Tyr Pro Gln Pro Gln
   1               5                   10                  15
   Phe Pro Ser Gln
               20
<210> 150
   <211> 20
   <212> PRT
   <213> Artificial Sequence
<220>
   <223> peptide
<400> 150
   Gly Gln Gln Gln Ser Phe Pro Pro Gln Gln Pro Tyr Pro Gln Pro Gln
   1               5                   10                  15
   Pro Phe Pro Ser
               20
<210> 151
   <211> 20
   <212> PRT
   <213> Artificial Sequence
<220>
   <223> peptide
<400> 151
   Gly Gln Gln Gln Pro Phe Pro Pro Gln Gln Pro Tyr Pro Gln Gln Gln
   1 5 10 15
   Pro Phe Pro Ser
   20
<210> 152
   <211> 20
   <212> PRT
   <213> Artificial Sequence
<220>
   <223> peptide
<400> 152
   Gly Gln Gln Gln Gln Phe Pro Pro Gln Gln Pro Tyr Pro Gln Pro Gln
   1 5 10 15
   Pro Phe Pro Ser
   20
<210> 153
   <211> 20
   <212> PRT
   <213> Artificial Sequence
<220>
   <223> peptide
<400> 153
   Gly Gln Gln Glu Gln Phe Pro Pro Gln Gln Pro Tyr Pro His Gln Gln
   1 5 10 15
   Pro Phe Pro Ser
   20
<210> 154
   <211> 20
   <212> PRT
   <213> Artificial Sequence
<220>
   <223> peptide
<400> 154
   Gly Gln Gln Glu Arg Phe Pro Pro Gln Gln Pro Tyr Pro His Gln Gln
   1 5 10 15
   Pro Phe Pro Ser
   20
<210> 155
   <211> 20
   <212> PRT
   <213> Artificial Sequence
<220>
   <223> peptide
<400> 155
   Gln Gln Pro Tyr Pro Gln Pro Gln Pro Phe Pro Ser Gln Leu Pro Tyr
   1               5                   10                  15
   Leu Gln Leu Gln
               20
<210> 156
   <211> 20
   <212> PRT
   <213> Artificial Sequence
<220>
   <223> peptide
<400> 156
   Gln Gln Pro Tyr Pro Gln Pro Gln Phe Pro Ser Gln Leu Pro Tyr Leu
   1               5                   10                  15
   Gln Leu Gln Pro
               20
<210> 157
   <211> 20
   <212> PRT
   <213> Artificial Sequence
<220>
   <223> peptide
<400> 157
   Gln Gln Pro Tyr Pro Gln Pro Gln Pro Phe Pro Ser Gln Gln Pro Tyr
   1               5                   10                  15
   Leu Gln Leu Gln
               20
<210> 158
   <211> 20
   <212> PRT
   <213> Artificial Sequence
<220>
   <223> peptide
<400> 158
   Gln Gln Pro Tyr Pro Gln Gln Gln Pro Phe Pro Ser Gln Gln Pro Tyr
   1               5                   10                  15
   Met Gln Leu Gln
               20
<210> 159
   <211> 20
   <212> PRT
   <213> Artificial Sequence
<220>
   <223> peptide
<400> 159
   Gln Gln Pro Tyr Pro His Gln Gln Pro Phe Pro Ser Gln Gln Pro Tyr
   1               5                   10                  15
   Pro Gln Pro Gln
               20
<210> 160
   <211> 20
   <212> PRT
   <213> Artificial Sequence
<220>
   <223> peptide
<400> 160
   Pro Phe Pro Ser Gln Leu Pro Tyr Leu Gln Leu Gln Pro Phe Pro Gln
   1               5                   10                  15
   Pro Gln Leu Pro
               20
<210> 161
   <211> 20
   <212> PRT
   <213> Artificial Sequence
<220>
   <223> peptide
<400> 161
   Pro Phe Pro Ser Gln Gln Pro Tyr Leu Gln Leu Gln Pro Phe Pro Gln
   1               5                   10                  15
   Pro Gln Leu Pro
               20
<210> 162
   <211> 20
   <212> PRT
   <213> Artificial Sequence
<220>
   <223> peptide
<400> 162
   Pro Phe Pro Ser Gln Gln Pro Tyr Leu Gln Leu Gln Pro Phe Ser Gln
   1 5 10 15
   Pro Gln Leu Pro
   20
<210> 163
   <211> 20
   <212> PRT
   <213> Artificial Sequence
<220>
   <223> peptide
<400> 163
   Pro Phe Pro Ser Gln Gln Pro Tyr Leu Gln Leu Gln Pro Phe Leu Gln
   1 5 10 15
   Pro Gln Leu Pro
   20
<210> 164
   <211> 20
   <212> PRT
   <213> Artificial Sequence
<220>
   <223> peptide
<400> 164
   Pro Phe Pro Ser Gln Gln Pro Tyr Leu Gln Leu Gln Pro Phe Leu Gln
   1 5 10 15
   Pro Gln Pro Phe
   20
<210> 165
   <211> 20
   <212> PRT
   <213> Artificial Sequence
<220>
   <223> peptide
<400> 165
   Pro Phe Pro Ser Gln Gln Pro Tyr Leu Gln Leu Gln Pro Phe Pro Gln
   1 5 10 15
   Pro Gln Leu Pro
   20
<210> 166
   <211> 20
   <212> PRT
   <213> Artificial Sequence
<220>
   <223> peptide
<400> 166
   Pro Phe Pro Ser Gln Gln Pro Tyr Met Gln Leu Gln Pro Phe Pro Gln
   1               5                   10                  15
   Pro Gln Leu Pro
               20
<210> 167
   <211> 20
   <212> PRT
   <213> Artificial Sequence
<220>
   <223> peptide
<400> 167
   Pro Phe Pro Ser Gln Gln Pro Tyr Met Gln Leu Gln Pro Phe Pro Gln
   1               5                   10                  15
   Pro Gln Pro Phe
               20
<210> 168
   <211> 20
   <212> PRT
   <213> Artificial Sequence
<220>
   <223> peptide
<400> 168
   Pro Phe Pro Ser Gln Gln Pro Tyr Leu Gln Leu Gln Pro Phe Pro Gln
   1               5                   10                  15
   Pro Gln Pro Phe
               20
<210> 169
   <211> 20
   <212> PRT
   <213> Artificial Sequence
<220>
   <223> peptide
<400> 169
   Pro Phe Pro Ser Gln Gln Pro Tyr Leu Gln Leu Gln Pro Phe Pro Arg
   1               5                   10                  15
   Pro Gln Leu Pro
               20
<210> 170
   <211> 20
   <212> PRT
   <213> Artificial Sequence
<220>
   <223> peptide
<400> 170
   Pro Phe Pro Ser Gln Gln Pro Tyr Pro Gln Pro Gln Pro Phe Pro Pro
   1               5                   10                  15
   Gln Leu Pro Tyr
               20
<210> 171
   <211> 20
   <212> PRT
   <213> Artificial Sequence
<220>
   <223> peptide
<400> 171
   Pro Phe Pro Ser Gln Gln Pro Tyr Pro Gln Pro Gln Pro Phe Pro Gln
   1               5                   10                  15
   Pro Gln Pro Phe
               20
<210> 172
   <211> 20
   <212> PRT
   <213> Artificial Sequence
<220>
   <223> peptide
<400> 172
   Leu Gln Leu Gln Pro Phe Pro Gln Pro Gln Leu Pro Tyr Ser Gln Pro
   1               5                   10                  15
   Gln Pro Phe Arg
               20
<210> 173
   <211> 20
   <212> PRT
   <213> Artificial Sequence
<220>
   <223> peptide
<400> 173
   Leu Gln Leu Gln Pro Phe Pro Gln Pro Gln Leu Pro Tyr Ser Gln Pro
   1               5                   10                  15
   Gln Gln Phe Arg
               20
<210> 174
   <211> 20
   <212> PRT
   <213> Artificial Sequence
<220>
   <223> peptide
<400> 174
   Leu Gln Leu Gln Pro Phe Pro Gln Pro Gln Leu Pro Tyr Leu Gln Pro
   1               5                   10                  15
   Gln Pro Phe Arg
               20
<210> 175
   <211> 20
   <212> PRT
   <213> Artificial Sequence
<220>
   <223> peptide
<400> 175
   Leu Gln Leu Gln Pro Phe Pro Gln Pro Gln Leu Ser Tyr Ser Gln Pro
   1               5                   10                  15
   Gln Pro Phe Arg
               20
<210> 176
   <211> 20
   <212> PRT
   <213> Artificial Sequence
<220>
   <223> peptide
<400> 176
   Leu Gln Leu Gln Pro Phe Ser Gln Pro Gln Leu Pro Tyr Ser Gln Pro
   1               5                   10                  15
   Gln Pro Phe Arg
               20
<210> 177
   <211> 20
   <212> PRT
   <213> Artificial Sequence
<220>
   <223> peptide
<400> 177
   Leu Gln Leu Gln Pro Phe Leu Gln Pro Gln Leu Pro Tyr Ser Gln Pro
   1 5 10 15
   Gln Pro Phe Arg
   20
<210> 178
   <211> 20
   <212> PRT
   <213> Artificial Sequence
<220>
   <223> peptide
<400> 178
   Leu Gln Leu Gln Pro Phe Leu Gln Pro Gln Pro Phe Pro Pro Gln Leu
   1 5 10 15
   Pro Tyr Ser Gln
   20
<210> 179
   <211> 20
   <212> PRT
   <213> Artificial Sequence
<220>
   <223> peptide
<400> 179
   Leu Gln Leu Gln Pro Phe Pro Gln Pro Gln Leu Pro Tyr Pro Gln Pro
   1 5 10 15
   Gln Leu Pro Tyr
   20
<210> 180
   <211> 20
   <212> PRT
   <213> Artificial Sequence
<220>
   <223> peptide
<400> 180
   Met Gln Leu Gln Pro Phe Pro Gln Pro Gln Leu Pro Tyr Pro Gln Pro
   1 5 10 15
   Gln Leu Pro Tyr
   20
<210> 181
   <211> 20
   <212> PRT
   <213> Artificial Sequence
<220>
   <223> peptide
<400> 181
   Met Gln Leu Gln Pro Phe Pro Gln Pro Gln Pro Phe Pro Pro Gln Leu
   1               5                   10                  15
   Pro Tyr Pro Gln
               20
<210> 182
   <211> 20
   <212> PRT
   <213> Artificial Sequence
<220>
   <223> peptide
<400> 182
   Leu Gln Leu Gln Pro Phe Pro Gln Pro Gln Leu Pro Tyr Pro Gln Pro
   1               5                   10                  15
   Gln Pro Phe Arg
               20
<210> 183
   <211> 20
   <212> PRT
   <213> Artificial Sequence
<220>
   <223> peptide
<400> 183
   Leu Gln Leu Gln Pro Phe Pro Gln Pro Gln Pro Phe Pro Pro Gln Leu
   1               5                   10                  15
   Pro Tyr Pro Gln
               20
<210> 184
   <211> 20
   <212> PRT
   <213> Artificial Sequence
<220>
   <223> peptide
<400> 184
   Leu Gln Leu Gln Pro Phe Pro Arg Pro Gln Leu Pro Tyr Pro Gln Pro
   1               5                   10                  15
   Gln Pro Phe Arg
               20
<210> 185
   <211> 20
   <212> PRT
   <213> Artificial Sequence
<220>
   <223> peptide
<400> 185
   Leu Gln Leu Gln Pro Phe Pro Gln Pro Gln Pro Phe Leu Pro Gln Leu
   1               5                   10                  15
   Pro Tyr Pro Gln
               20
<210> 186
   <211> 20
   <212> PRT
   <213> Artificial Sequence
<220>
   <223> peptide
<400> 186
   Leu Gln Leu Gln Pro Phe Pro Gln Pro Gln Pro Phe Pro Pro Gln Leu
   1               5                   10                  15
   Pro Tyr Pro Gln
               20
<210> 187
   <211> 20
   <212> PRT
   <213> Artificial Sequence
<220>
   <223> peptide
<400> 187
   Pro Gln Pro Gln Pro Phe Pro Pro Gln Leu Pro Tyr Pro Gln Thr Gln
   1               5                   10                  15
   Pro Phe Pro Pro
               20
<210> 188
   <211> 20
   <212> PRT
   <213> Artificial Sequence
<220>
   <223> peptide
<400> 188
   Pro Gln Pro Gln Pro Phe Pro Gln Pro Gln Pro Phe Pro Pro Gln Leu
   1               5                   10                  15
   Pro Tyr Pro Gln
               20
<210> 189
   <211> 20
   <212> PRT
   <213> Artificial Sequence
<220>
   <223> peptide
<400> 189
   Pro Gln Leu Pro Tyr Ser Gln Pro Gln Pro Phe Arg Pro Gln Gln Pro
   1               5                   10                  15
   Tyr Pro Gln Pro
               20
<210> 190
   <211> 20
   <212> PRT
   <213> Artificial Sequence
<220>
   <223> peptide
<400> 190
   Pro Gln Leu Pro Tyr Ser Gln Pro Gln Gln Phe Arg Pro Gln Gln Pro
   1               5                   10                  15
   Tyr Pro Gln Pro
               20
<210> 191
   <211> 20
   <212> PRT
   <213> Artificial Sequence
<220>
   <223> peptide
<400> 191
   Pro Gln Leu Pro Tyr Leu Gln Pro Gln Pro Phe Arg Pro Gln Gln Pro
   1               5                   10                  15
   Tyr Pro Gln Pro
               20
<210> 192
   <211> 20
   <212> PRT
   <213> Artificial Sequence
<220>
   <223> peptide
<400> 192
   Pro Gln Leu Ser Tyr Ser Gln Pro Gln Pro Phe Arg Pro Gln Gln Pro
   1               5                   10                  15
   Tyr Pro Gln Pro
               20
<210> 193
   <211> 20
   <212> PRT
   <213> Artificial Sequence
<220>
   <223> peptide
<400> 193
   Pro Gln Leu Ser Tyr Ser Gln Pro Gln Pro Phe Arg Pro Gln Gln Leu
   1               5                   10                  15
   Tyr Pro Gln Pro
               20
<210> 194
   <211> 20
   <212> PRT
   <213> Artificial Sequence
<220>
   <223> peptide
<400> 194
   Pro Gln Pro Phe Pro Pro Gln Leu Pro Tyr Ser Gln Pro Gln Pro Phe
   1               5                   10                  15
   Arg Pro Gln Gln
               20
<210> 195
   <211> 20
   <212> PRT
   <213> Artificial Sequence
<220>
   <223> peptide
<400> 195
   Pro Gln Leu Pro Tyr Pro Gln Pro Gln Leu Pro Tyr Pro Gln Pro Gln
   1 5 10 15
   Leu Pro Tyr Pro
   20
<210> 196
   <211> 20
   <212> PRT
   <213> Artificial Sequence
<220>
   <223> peptide
<400> 196
   Pro Gln Leu Pro Tyr Pro Gln Pro Gln Leu Pro Tyr Pro Gln Pro Gln
   1 5 10 15
   Pro Phe Arg Pro
   20
<210> 197
   <211> 20
   <212> PRT
   <213> Artificial Sequence
<220>
   <223> peptide
<400> 197
   Pro Gln Pro Phe Pro Pro Gln Leu Pro Tyr Pro Gln Pro Gln Leu Pro
   1 5 10 15
   Tyr Pro Gln Pro
   20
<210> 198
   <211> 20
   <212> PRT
   <213> Artificial Sequence
<220>
   <223> peptide
<400> 198
   Pro Gln Leu Pro Tyr Pro Gln Pro Gln Pro Phe Arg Pro Gln Gln Pro
   1 5 10 15
   Tyr Pro Gln Pro
   20
<210> 199
   <211> 20
   <212> PRT
   <213> Artificial Sequence
<220>
   <223> peptide
<400> 199
   Pro Gln Pro Phe Pro Pro Gln Leu Pro Tyr Pro Gln Pro Gln Pro Phe
   1 5 10 15
   Arg Pro Gln Gln
   20
<210> 200
   <211> 20
   <212> PRT
   <213> Artificial Sequence
<220>
   <223> peptide
<400> 200
   Pro Gln Pro Phe Pro Pro Gln Leu Pro Tyr Pro Gln Pro Pro Pro Phe
   1 5 10 15
   Ser Pro Gln Gln
   20
<210> 201
   <211> 20
   <212> PRT
   <213> Artificial Sequence
<220>
   <223> peptide
<400> 201
   Pro Gln Pro Phe Leu Pro Gln Leu Pro Tyr Pro Gln Pro Gln Ser Phe
   1 5 10 15
   Pro Pro Gln Gln
   20
<210> 202
   <211> 20
   <212> PRT
   <213> Artificial Sequence
<220>
   <223> peptide
<400> 202
   Pro Gln Pro Phe Pro Pro Gln Leu Pro Tyr Pro Gln Pro Gln Ser Phe
   1 5 10 15
   Pro Pro Gln Gln
   20
<210> 203
   <211> 20
   <212> PRT
   <213> Artificial Sequence
<220>
   <223> peptide
<400> 203
   Gln Leu Pro Tyr Pro Gln Thr Gln Pro Phe Pro Pro Gln Gln Pro Tyr
   1               5                   10                  15
   Pro Gln Pro Gln
               20
<210> 204
   <211> 20
   <212> PRT
   <213> Artificial Sequence
<220>
   <223> peptide
<400> 204
   Pro Gln Pro Phe Pro Pro Gln Leu Pro Tyr Pro Gln Thr Gln Pro Phe
   1               5                   10                  15
   Pro Pro Gln Gln
               20
<210> 205
   <211> 20
   <212> PRT
   <213> Artificial Sequence
<220>
   <223> peptide
<400> 205
   Leu Pro Tyr Pro Gln Pro Gln Pro Phe Arg Pro Gln Gln Pro Tyr Pro
   1               5                   10                  15
   Gln Ser Gln Pro
               20
<210> 206
   <211> 20
   <212> PRT
   <213> Artificial Sequence
<220>
   <223> peptide
<400> 206
   Leu Pro Tyr Pro Gln Pro Gln Pro Phe Arg Pro Gln Gln Ser Tyr Pro
   1               5                   10                  15
   Gln Pro Gln Pro
               20
<210> 207
   <211> 20
   <212> PRT
   <213> Artificial Sequence
<220>
   <223> peptide
<400> 207
   Leu Pro Tyr Pro Gln Pro Pro Pro Phe Ser Pro Gln Gln Pro Tyr Pro
   1               5                   10                  15
   Gln Pro Gln Pro
               20
<210> 208
   <211> 20
   <212> PRT
   <213> Artificial Sequence
<220>
   <223> peptide
<400> 208
   Leu Pro Gln Leu Pro Tyr Pro Gln Pro Gln Ser Phe Pro Pro Gln Gln
   1               5                   10                  15
   Pro Tyr Pro Gln
               20
<210> 209
   <211> 20
   <212> PRT
   <213> Artificial Sequence
<220>
   <223> peptide
<400> 209
   Pro Pro Gln Leu Pro Tyr Pro Gln Thr Gln Pro Phe Pro Pro Gln Gln
   1               5                   10                  15
   Pro Tyr Pro Gln
               20
<210> 210
   <211> 20
   <212> PRT
   <213> Artificial Sequence
<220>
   <223> peptide
<400> 210
   Gln Pro Phe Arg Pro Gln Gln Pro Tyr Pro Gln Pro Gln Pro Gln Tyr
   1               5                   10                  15
   Ser Gln Pro Gln
               20
<210> 211
   <211> 20
   <212> PRT
   <213> Artificial Sequence
<220>
   <223> peptide
<400> 211
   Gln Pro Phe Arg Pro Gln Gln Leu Tyr Pro Gln Pro Gln Pro Gln Tyr
   1               5                   10                  15
   Ser Gln Pro Gln
               20
<210> 212
   <211> 20
   <212> PRT
   <213> Artificial Sequence
<220>
   <223> peptide
<400> 212
   Gln Pro Phe Arg Pro Gln Gln Pro Tyr Pro Gln Ser Gln Pro Gln Tyr
   1               5                   10                  15
   Ser Gln Pro Gln
               20
<210> 213
   <211> 20
   <212> PRT
   <213> Artificial Sequence
<220>
   <223> peptide
<400> 213
   Gln Pro Phe Arg Pro Gln Gln Ser Tyr Pro Gln Pro Gln Pro Gln Tyr
   1               5                   10                  15
   Ser Gln Pro Gln
               20
<210> 214
   <211> 20
   <212> PRT
   <213> Artificial Sequence
<220>
   <223> peptide
<400> 214
   Pro Pro Phe Ser Pro Gln Gln Pro Tyr Pro Gln Pro Gln Pro Gln Tyr
   1               5                   10                  15
   Pro Gln Pro Gln
               20
<210> 215
   <211> 20
   <212> PRT
   <213> Artificial Sequence
<220>
   <223> peptide
<400> 215
   Gln Ser Phe Pro Pro Gln Gln Pro Tyr Pro Gln Gln Arg Pro Lys Tyr
   1               5                   10                  15
   Leu Gln Pro Gln
               20
<210> 216
   <211> 20
   <212> PRT
   <213> Artificial Sequence
<220>
   <223> peptide
<400> 216
   Gln Ser Phe Pro Pro Gln Gln Pro Tyr Pro Gln Gln Arg Pro Met Tyr
   1               5                   10                  15
   Leu Gln Pro Gln
               20
<210> 217
   <211> 20
   <212> PRT
   <213> Artificial Sequence
<220>
   <223> peptide
<400> 217
   Gln Ser Phe Pro Pro Gln Gln Pro Tyr Pro Gln Gln Gln Pro Gln Tyr
   1 5 10 15
   Leu Gln Pro Gln
   20
<210> 218
   <211> 20
   <212> PRT
   <213> Artificial Sequence
<220>
   <223> peptide
<400> 218
   Gln Pro Phe Pro Pro Gln Gln Pro Tyr Pro Gln Pro Gln Pro Gln Tyr
   1 5 10 15
   Pro Gln Pro Gln
   20
<210> 219
   <211> 20
   <212> PRT
   <213> Artificial Sequence
<220>
   <223> peptide
<400> 219
   Tyr Pro Gln Pro Gln Pro Gln Tyr Ser Gln Pro Gln Gln Pro Ile Ser
   1 5 10 15
   Gln Gln Gln Gln
   20
<210> 220
   <211> 20
   <212> PRT
   <213> Artificial Sequence
<220>
   <223> peptide
<400> 220
   Tyr Pro Gln Pro Gln Pro Gln Tyr Ser Gln Pro Gln Glu Pro Ile Ser
   1 5 10 15
   Gln Gln Gln Gln
   20
<210> 221
   <211> 20
   <212> PRT
   <213> Artificial Sequence
<220>
   <223> peptide
<400> 221
   Tyr Pro Gln Ser Gln Pro Gln Tyr Ser Gln Pro Gln Gln Pro Ile Ser
   1               5                   10                  15
   Gln Gln Gln Gln
               20
<210> 222
   <211> 20
   <212> PRT
   <213> Artificial Sequence
<220>
   <223> peptide
<400> 222
   Tyr Pro Gln Pro Gln Pro Gln Tyr Pro Gln Pro Gln Gln Pro Ile Ser
   1               5                   10                  15
   Gln Gln Gln Ala
               20
<210> 223
   <211> 20
   <212> PRT
   <213> Artificial Sequence
<220>
   <223> peptide
<400> 223
   Tyr Pro Gln Gln Arg Pro Lys Tyr Leu Gln Pro Gln Gln Pro Ile Ser
   1               5                   10                  15
   Gln Gln Gln Ala
               20
<210> 224
   <211> 20
   <212> PRT
   <213> Artificial Sequence
<220>
   <223> peptide
<400> 224
   Tyr Pro Gln Gln Arg Pro Met Tyr Leu Gln Pro Gln Gln Pro Ile Ser
   1               5                   10                  15
   Gln Gln Gln Ala
               20
<210> 225
   <211> 20
   <212> PRT
   <213> Artificial Sequence
<220>
   <223> peptide
<400> 225
   Tyr Pro Gln Gln Gln Pro Gln Tyr Leu Gln Pro Gln Gln Pro Ile Ser
   1               5                   10                  15
   Gln Gln Gln Ala
               20
<210> 226
   <211> 20
   <212> PRT
   <213> Artificial Sequence
<220>
   <223> peptide
<400> 226
   Ser Gln Pro Gln Gln Pro Ile Ser Gln Gln Gln Gln Gln Gln Gln Gln
   1               5                   10                  15
   Gln Gln Gln Gln
               20
<210> 227
   <211> 20
   <212> PRT
   <213> Artificial Sequence
<220>
   <223> peptide
<400> 227
   Ser Gln Pro Gln Glu Pro Ile Ser Gln Gln Gln Gln Gln Gln Gln Gln
   1               5                   10                  15
   Gln Gln Gln Ile
               20
<210> 228
   <211> 20
   <212> PRT
   <213> Artificial Sequence
<220>
   <223> peptide
<400> 228
   Pro Gln Pro Gln Gln Pro Ile Ser Gln Gln Gln Ala Gln Gln Gln Gln
   1               5                   10                  15
   Gln Gln Gln Gln
               20
<210> 229
   <211> 20
   <212> PRT
   <213> Artificial Sequence
<220>
   <223> peptide
<400> 229
   Gln Gln Gln Gln Gln Gln Gln Gln Gln Gln Gln Gln Gln Gln Gln Gln
   1               5                   10                  15
   Ile Leu Gln Gln
               20
<210> 230
   <211> 20
   <212> PRT
   <213> Artificial Sequence
<220>
   <223> peptide
<400> 230
   Gln Gln Gln Gln Gln Gln Gln Gln Gln Gln Gln Gln Gln Glu Gln Gln
   1               5                   10                  15
   Ile Leu Gln Gln
               20
<210> 231
   <211> 20
   <212> PRT
   <213> Artificial Sequence
<220>
   <223> peptide
<400> 231
   Gln Gln Gln Gln Gln Gln Gln Gln Gln Gln Gln Gln Gln Gln Gln Gln
   1               5                   10                  15
   Ile Ile Gln Gln
               20
<210> 232
   <211> 20
   <212> PRT
   <213> Artificial Sequence
<220>
   <223> peptide
<400> 232
   Gln Gln Gln Gln Gln Gln Gln Gln Gln Gln Lys Gln Gln Gln Gln Gln
   1 5 10 15
   Gln Gln Gln Ile
   20
<210> 233
   <211> 20
   <212> PRT
   <213> Artificial Sequence
<220>
   <223> peptide
<400> 233
   Ala Gln Gln Gln Gln Gln Gln Gln Gln Gln Gln Gln Gln Gln Gln Gln
   1 5 10 15
   Thr Leu Gln Gln
   20
<210> 234
   <211> 20
   <212> PRT
   <213> Artificial Sequence
<220>
   <223> peptide
<400> 234
   Gln Gln Gln Gln Gln Gln Gln Gln Ile Leu Gln Gln Ile Leu Gln Gln
   1 5 10 15
   Gln Leu Ile Pro
   20
<210> 235
   <211> 20
   <212> PRT
   <213> Artificial Sequence
<220>
   <223> peptide
<400> 235
   Gln Gln Gln Gln Gln Glu Gln Gln Ile Leu Gln Gln Ile Leu Gln Gln
   1 5 10 15
   Gln Leu Ile Pro
   20
<210> 236
   <211> 20
   <212> PRT
   <213> Artificial Sequence
<220>
   <223> peptide
<400> 236
   Gln Gln Gln Gln Gln Glu Gln Gln Ile Leu Gln Gln Met Leu Gln Gln
   1               5                   10                  15
   Gln Leu Ile Pro
               20
<210> 237
   <211> 20
   <212> PRT
   <213> Artificial Sequence
<220>
   <223> peptide
<400> 237
   Gln Gln Gln Gln Gln Glu Gln Gln Ile Leu Gln Gln Ile Leu Gln Gln
   1               5                   10                  15
   Gln Leu Thr Pro
               20
<210> 238
   <211> 20
   <212> PRT
   <213> Artificial Sequence
<220>
   <223> peptide
<400> 238
   Gln Gln Gln Gln Gln Gln Gln Gln Ile Ile Gln Gln Ile Leu Gln Gln
   1               5                   10                  15
   Gln Leu Ile Pro
               20
<210> 239
   <211> 20
   <212> PRT
   <213> Artificial Sequence
<220>
   <223> peptide
<400> 239
   Gln Gln Lys Gln Gln Gln Gln Gln Gln Gln Gln Ile Leu Gln Gln Ile
   1 5 10 15
   Leu Gln Gln Gln
   20
<210> 240
   <211> 20
   <212> PRT
   <213> Artificial Sequence
<220>
   <223> peptide
<400> 240
   Gln Gln Gln Gln Gln Gln Gln Gln Ile Leu Pro Gln Ile Leu Gln Gln
   1 5 10 15
   Gln Leu Ile Pro
   20
<210> 241
   <211> 20
   <212> PRT
   <213> Artificial Sequence
<220>
   <223> peptide
<400> 241
   Gln Gln Gln Gln Gln Gln Gln Gln Thr Leu Gln Gln Ile Leu Gln Gln
   1 5 10 15
   Gln Leu Ile Pro
   20
<210> 242
   <211> 20
   <212> PRT
   <213> Artificial Sequence
<220>
   <223> peptide
<400> 242
   Ile Leu Gln Gln Ile Leu Gln Gln Gln Leu Ile Pro Cys Met Asp Val
   1 5 10 15
   Val Leu Gln Gln
   20
<210> 243
   <211> 20
   <212> PRT
   <213> Artificial Sequence
<220>
   <223> peptide
<400> 243
   Ile Leu Gln Gln Met Leu Gln Gln Gln Leu Ile Pro Cys Met Asp Val
   1               5                   10                  15
   Val Leu Gln Gln
               20
<210> 244
   <211> 20
   <212> PRT
   <213> Artificial Sequence
<220>
   <223> peptide
<400> 244
   Ile Leu Gln Gln Ile Leu Gln Gln Gln Leu Thr Pro Cys Met Asp Val
   1               5                   10                  15
   Val Leu Gln Gln
               20
<210> 245
   <211> 20
   <212> PRT
   <213> Artificial Sequence
<220>
   <223> peptide
<400> 245
   Ile Leu Gln Gln Ile Leu Gln Gln Gln Leu Ile Pro Cys Arg Asp Val
   1               5                   10                  15
   Val Leu Gln Gln
               20
<210> 246
   <211> 20
   <212> PRT
   <213> Artificial Sequence
<220>
   <223> peptide
<400> 246
   Ile Leu Pro Gln Ile Leu Gln Gln Gln Leu Ile Pro Cys Arg Asp Val
   1               5                   10                  15
   Val Leu Gln Gln
               20
<210> 247
   <211> 20
   <212> PRT
   <213> Artificial Sequence
<220>
   <223> peptide
<400> 247
   Thr Leu Gln Gln Ile Leu Gln Gln Gln Leu Ile Pro Cys Arg Asp Val
   1               5                   10                  15
   Val Leu Gln Gln
               20
<210> 248
   <211> 20
   <212> PRT
   <213> Artificial Sequence
<220>
   <223> peptide
<400> 248
   Gln Leu Ile Pro Cys Met Asp Val Val Leu Gln Gln His Asn Ile Ala
   1               5                   10                  15
   His Gly Arg Ser
               20
<210> 249
   <211> 20
   <212> PRT
   <213> Artificial Sequence
<220>
   <223> peptide
<400> 249
   Gln Leu Ile Pro Cys Met Asp Val Val Leu Gln Gln His Asn Lys Ala
   1               5                   10                  15
   His Gly Arg Ser
               20
<210> 250
   <211> 20
   <212> PRT
   <213> Artificial Sequence
<220>
   <223> peptide
<400> 250
   Gln Leu Ile Pro Cys Met Asp Val Val Leu Gln Gln His Asn Leu Ala
   1 5 10 15
   His Gly Arg Ser
   20
<210> 251
   <211> 20
   <212> PRT
   <213> Artificial Sequence
<220>
   <223> peptide
<400> 251
   Gln Leu Ile Pro Cys Met Asp Val Val Leu Gln Gln His Asn Ile Val
   1 5 10 15
   His Gly Arg Ser
   20
<210> 252
   <211> 20
   <212> PRT
   <213> Artificial Sequence
<220>
   <223> peptide
<400> 252
   Gln Leu Thr Pro Cys Met Asp Val Val Leu Gln Gln His Asn Ile Ala
   1 5 10 15
   Arg Gly Arg Ser
   20
<210> 253
   <211> 20
   <212> PRT
   <213> Artificial Sequence
<220>
   <223> peptide
<400> 253
   Gln Leu Ile Pro Cys Met Asp Val Val Leu Gln Gln His Asn Ile Val
   1 5 10 15
   His Gly Lys Ser
   20
<210> 254
   <211> 20
   <212> PRT
   <213> Artificial Sequence
<220>
   <223> peptide
<400> 254
   Gln Leu Ile Pro Cys Arg Asp Val Val Leu Gln Gln His Ser Ile Ala
   1               5                   10                  15
   Tyr Gly Ser Ser
               20
<210> 255
   <211> 20
   <212> PRT
   <213> Artificial Sequence
<220>
   <223> peptide
<400> 255
   Gln Leu Ile Pro Cys Arg Asp Val Val Leu Gln Gln His Ser Ile Ala
   1               5                   10                  15
   His Gly Ser Ser
               20
<210> 256
   <211> 20
   <212> PRT
   <213> Artificial Sequence
<220>
   <223> peptide
<400> 256
   Gln Leu Ile Pro Cys Arg Asp Val Val Leu Gln Gln His Asn Ile Ala
   1               5                   10                  15
   His Gly Ser Ser
               20
<210> 257
   <211> 20
   <212> PRT
   <213> Artificial Sequence
<220>
   <223> peptide
<400> 257
   Gln Leu Ile Pro Cys Arg Asp Val Val Leu Gln Gln His Asn Ile Ala
   1               5                   10                  15
   His Ala Arg Ser
               20
<210> 258
   <211> 20
   <212> PRT
   <213> Artificial Sequence
<220>
   <223> peptide
<400> 258
   Gln Leu Ile Pro Cys Arg Asp Val Val Leu Gln Gln His Asn Ile Ala
   1               5                   10                  15
   His Ala Ser Ser
               20
<210> 259
   <211> 20
   <212> PRT
   <213> Artificial Sequence
<220>
   <223> peptide
<400> 259
   Val Leu Gln Gln His Asn Ile Ala His Gly Arg Ser Gln Val Leu Gln
   1               5                   10                  15
   Gln Ser Thr Tyr
               20
<210> 260
   <211> 20
   <212> PRT
   <213> Artificial Sequence
<220>
   <223> peptide
<400> 260
   Val Leu Gln Gln His Asn Lys Ala His Gly Arg Ser Gln Val Leu Gln
   1               5                   10                  15
   Gln Ser Thr Tyr
               20
<210> 261
   <211> 20
   <212> PRT
   <213> Artificial Sequence
<220>
   <223> peptide
<400> 261
   Val Leu Gln Gln His Asn Leu Ala His Gly Arg Ser Gln Val Leu Gln
   1 5 10 15
   Gln Ser Thr Tyr
   20
<210> 262
   <211> 20
   <212> PRT
   <213> Artificial Sequence
<220>
   <223> peptide
<400> 262
   Val Leu Gln Gln His Asn Ile Val His Gly Arg Ser Gln Val Leu Gln
   1 5 10 15
   Gln Ser Thr Tyr
   20
<210> 263
   <211> 20
   <212> PRT
   <213> Artificial Sequence
<220>
   <223> peptide
<400> 263
   Val Leu Gln Gln His Asn Ile Ala Arg Gly Arg Ser Gln Val Leu Gln
   1 5 10 15
   Gln Ser Thr Tyr
   20
<210> 264
   <211> 20
   <212> PRT
   <213> Artificial Sequence
<220>
   <223> peptide
<400> 264
   Val Leu Gln Gln His Asn Ile Val His Gly Lys Ser Gln Val Leu Gln
   1 5 10 15
   Gln Ser Thr Tyr
   20
<210> 265
   <211> 20
   <212> PRT
   <213> Artificial Sequence
<220>
   <223> peptide
<400> 265
   Val Leu Gln Gln His Ser Ile Ala Tyr Gly Ser Ser Gln Val Leu Gln
   1               5                   10                  15
   Gln Ser Thr Tyr
               20
<210> 266
   <211> 20
   <212> PRT
   <213> Artificial Sequence
<220>
   <223> peptide
<400> 266
   Val Leu Gln Gln His Ser Ile Ala His Gly Ser Ser Gln Val Leu Gln
   1               5                   10                  15
   Gln Ser Thr Tyr
               20
<210> 267
   <211> 20
   <212> PRT
   <213> Artificial Sequence
<220>
   <223> peptide
<400> 267
   Val Leu Gln Gln His Asn Ile Ala His Gly Ser Ser Gln Val Leu Gln
   1               5                   10                  15
   Glu Ser Thr Tyr
               20
<210> 268
   <211> 20
   <212> PRT
   <213> Artificial Sequence
<220>
   <223> peptide
<400> 268
   Val Leu Gln Gln His Asn Ile Ala His Ala Arg Ser Gln Val Leu Gln
   1               5                   10                  15
   Gln Ser Thr Tyr
               20
<210> 269
   <211> 20
   <212> PRT
   <213> Artificial Sequence
<220>
   <223> peptide
<400> 269
   Val Leu Gln Gln His Asn Ile Ala His Ala Ser Ser Gln Val Leu Gln
   1               5                   10                  15
   Gln Ser Thr Tyr
               20
<210> 270
   <211> 20
   <212> PRT
   <213> Artificial Sequence
<220>
   <223> peptide
<400> 270
   Val Leu Gln Gln His Asn Ile Ala His Ala Ser Ser Gln Val Leu Gln
   1               5                   10                  15
   Gln Ser Ser Tyr
               20
<210> 271
   <211> 20
   <212> PRT
   <213> Artificial Sequence
<220>
   <223> peptide
<400> 271
   His Gly Arg Ser Gln Val Leu Gln Gln Ser Thr Tyr Gln Leu Leu Gln
   1               5                   10                  15
   Glu Leu Cys Cys
               20
<210> 272
   <211> 20
   <212> PRT
   <213> Artificial Sequence
<220>
   <223> peptide
<400> 272
   His Gly Arg Ser Gln Val Leu Gln Gln Ser Thr Tyr Gln Leu Leu Arg
   1 5 10 15
   Glu Leu Cys Cys
   20
<210> 273
   <211> 20
   <212> PRT
   <213> Artificial Sequence
<220>
   <223> peptide
<400> 273
   His Gly Arg Ser Gln Val Leu Gln Gln Ser Thr Tyr Gln Leu Leu Arg
   1 5 10 15
   Glu Leu Cys Cys
   20
<210> 274
   <211> 20
   <212> PRT
   <213> Artificial Sequence
<220>
   <223> peptide
<400> 274
   His Gly Lys Ser Gln Val Leu Gln Gln Ser Thr Tyr Gln Leu Leu Gln
   1 5 10 15
   Glu Leu Cys Cys
   20
<210> 275
   <211> 20
   <212> PRT
   <213> Artificial Sequence
<220>
   <223> peptide
<400> 275
   Tyr Gly Ser Ser Gln Val Leu Gln Gln Ser Thr Tyr Gln Leu Val Gln
   1 5 10 15
   Gln Leu Cys Cys
   20
<210> 276
   <211> 20
   <212> PRT
   <213> Artificial Sequence
<220>
   <223> peptide
<400> 276
   His Gly Ser Ser Gln Val Leu Gln Gln Ser Thr Tyr Gln Leu Val Gln
   1               5                   10                  15
   Gln Phe Cys Cys
               20
<210> 277
   <211> 20
   <212> PRT
   <213> Artificial Sequence
<220>
   <223> peptide
<400> 277
   His Gly Ser Ser Gln Val Leu Gln Glu Ser Thr Tyr Gln Leu Val Gln
   1               5                   10                  15
   Gln Leu Cys Cys
               20
<210> 278
   <211> 20
   <212> PRT
   <213> Artificial Sequence
<220>
   <223> peptide
<400> 278
   His Ala Arg Ser Gln Val Leu Gln Gln Ser Thr Tyr Gln Pro Leu Gln
   1               5                   10                  15
   Gln Leu Cys Cys
               20
<210> 279
   <211> 20
   <212> PRT
   <213> Artificial Sequence
<220>
   <223> peptide
<400> 279
   His Ala Ser Ser Gln Val Leu Gln Gln Ser Thr Tyr Gln Leu Leu Gln
   1               5                   10                  15
   Gln Leu Cys Cys
               20
<210> 280
   <211> 20
   <212> PRT
   <213> Artificial Sequence
<220>
   <223> peptide
<400> 280
   His Ala Ser Ser Gln Val Leu Gln Gln Ser Ser Tyr Gln Gln Leu Gln
   1 5 10 15
   Gln Leu Cys Cys
   20
<210> 281
   <211> 20
   <212> PRT
   <213> Artificial Sequence
<220>
   <223> peptide
<400> 281
   Gln Ser Thr Tyr Gln Leu Leu Gln Glu Leu Cys Cys Gln His Leu Trp
   1 5 10 15
   Gln Ile Pro Glu
   20
<210> 282
   <211> 20
   <212> PRT
   <213> Artificial Sequence
<220>
   <223> peptide
<400> 282
   Gln Ser Thr Tyr Gln Leu Leu Arg Glu Leu Cys Cys Gln His Leu Trp
   1 5 10 15
   Gln Ile Pro Glu
   20
<210> 283
   <211> 20
   <212> PRT
   <213> Artificial Sequence
<220>
   <223> peptide
<400> 283
   Gln Ser Thr Tyr Gln Leu Leu Arg Glu Leu Cys Cys Gln His Leu Trp
   1 5 10 15
   Gln Ile Pro Glu
   20
<210> 284
   <211> 20
   <212> PRT
   <213> Artificial Sequence
<220>
   <223> peptide
<400> 284
   Gln Ser Thr Tyr Gln Leu Val Gln Gln Leu Cys Cys Gln Gln Leu Trp
   1 5 10 15
   Gln Ile Pro Glu
   20
<210> 285
   <211> 20
   <212> PRT
   <213> Artificial Sequence
<220>
   <223> peptide
<400> 285
   Gln Ser Thr Tyr Gln Leu Val Gln Gln Phe Cys Cys Gln Gln Leu Trp
   1 5 10 15
   Gln Ile Pro Glu
   20
<210> 286
   <211> 20
   <212> PRT
   <213> Artificial Sequence
<220>
   <223> peptide
<400> 286
   Gln Ser Thr Tyr Gln Pro Leu Gln Gln Leu Cys Cys Gln Gln Leu Trp
   1 5 10 15
   Gln Ile Pro Glu
   20
<210> 287
   <211> 20
   <212> PRT
   <213> Artificial Sequence
<220>
   <223> peptide
<400> 287
   Gln Ser Thr Tyr Gln Leu Leu Gln Gln Leu Cys Cys Gln Gln Leu Leu
   1               5                   10                  15
   Gln Ile Pro Glu
               20
<210> 288
   <211> 20
   <212> PRT
   <213> Artificial Sequence
<220>
   <223> peptide
<400> 288
   Gln Ser Ser Tyr Gln Gln Leu Gln Gln Leu Cys Cys Gln Gln Leu Phe
   1               5                   10                  15
   Gln Ile Pro Glu
               20
<210> 289
   <211> 20
   <212> PRT
   <213> Artificial Sequence
<220>
   <223> peptide
<400> 289
   Glu Leu Cys Cys Gln His Leu Trp Gln Ile Pro Glu Gln Ser Gln Cys
   1               5                   10                  15
   Gln Ala Ile His
               20
<210> 290
   <211> 20
   <212> PRT
   <213> Artificial Sequence
<220>
   <223> peptide
<400> 290
   Glu Leu Cys Cys Gln His Leu Trp Gln Ile Leu Glu Gln Ser Gln Cys
   1               5                   10                  15
   Gln Ala Ile His
               20
<210> 291
   <211> 20
   <212> PRT
   <213> Artificial Sequence
<220>
   <223> peptide
<400> 291
   Glu Leu Cys Cys Gln His Leu Trp Gln Ile Pro Glu Lys Leu Gln Cys
   1 5 10 15
   Gln Ala Ile His
   20
<210> 292
   <211> 20
   <212> PRT
   <213> Artificial Sequence
<220>
   <223> peptide
<400> 292
   Gln Leu Cys Cys Gln Gln Leu Trp Gln Ile Pro Glu Gln Ser Arg Cys
   1 5 10 15
   Gln Ala Ile His
   20
<210> 293
   <211> 20
   <212> PRT
   <213> Artificial Sequence
<220>
   <223> peptide
<400> 293
   Gln Phe Cys Cys Gln Gln Leu Trp Gln Ile Pro Glu Gln Ser Arg Cys
   1 5 10 15
   Gln Ala Ile His
   20
<210> 294
   <211> 20
   <212> PRT
   <213> Artificial Sequence
<220>
   <223> peptide
<400> 294
   Gln Leu Cys Cys Gln Gln Leu Leu Gln Ile Pro Glu Gln Ser Arg Cys
   1               5                   10                  15
   Gln Ala Ile His
               20
<210> 295
   <211> 20
   <212> PRT
   <213> Artificial Sequence
<220>
   <223> peptide
<400> 295
   Gly Leu Cys Cys Gln Gln Leu Leu Gln Ile Pro Glu Gln Ser Gln Cys
   1               5                   10                  15
   Gln Ala Ile His
               20
<210> 296
   <211> 20
   <212> PRT
   <213> Artificial Sequence
<220>
   <223> peptide
<400> 296
   Gln Leu Cys Cys Gln Gln Leu Phe Gln Ile Pro Glu Gln Ser Arg Cys
   1               5                   10                  15
   Gln Ala Ile His
               20
<210> 297
   <211> 20
   <212> PRT
   <213> Artificial Sequence
<220>
   <223> peptide
<400> 297
   Gln Ile Pro Glu Gln Ser Gln Cys Gln Ala Ile His Asn Val Val His
   1               5                   10                  15
   Ala Ile Ile Leu
               20
<210> 298
   <211> 20
   <212> PRT
   <213> Artificial Sequence
<220>
   <223> peptide
<400> 298
   Gln Ile Pro Glu Gln Ser Gln Cys Gln Ala Ile Gln Asn Val Val His
   1 5 10 15
   Ala Ile Ile Leu
   20
<210> 299
   <211> 20
   <212> PRT
   <213> Artificial Sequence
<220>
   <223> peptide
<400> 299
   Gln Ile Leu Glu Gln Ser Gln Cys Gln Ala Ile His Asn Val Val His
   1 5 10 15
   Ala Ile Ile Leu
   20
<210> 300
   <211> 20
   <212> PRT
   <213> Artificial Sequence
<220>
   <223> peptide
<400> 300
   Gln Ile Pro Glu Gln Ser Gln Cys Gln Ala Ile His Lys Val Val His
   1 5 10 15
   Ala Ile Ile Leu
   20
<210> 301
   <211> 20
   <212> PRT
   <213> Artificial Sequence
<220>
   <223> peptide
<400> 301
   Gln Ile Pro Glu Lys Leu Gln Cys Gln Ala Ile His Asn Val Val His
   1 5 10 15
   Ala Ile Ile Leu
   20
<210> 302
   <211> 20
   <212> PRT
   <213> Artificial Sequence
<220>
   <223> peptide
<400> 302
   Gln Ile Pro Glu Gln Ser Arg Cys Gln Ala Ile His Asn Val Val His
   1               5                   10                  15
   Ala Ile Ile Leu
               20
<210> 303
   <211> 20
   <212> PRT
   <213> Artificial Sequence
<220>
   <223> peptide
<400> 303
   Gln Ile Pro Glu Gln Ser Gln Cys Gln Ala Ile His Asn Val Ala His
   1               5                   10                  15
   Ala Ile Ile Met
               20
<210> 304
   <211> 20
   <212> PRT
   <213> Artificial Sequence
<220>
   <223> peptide
<400> 304
   Gln Ile Pro Glu Gln Ser Arg Cys Gln Ala Ile His Asn Val Val His
   1               5                   10                  15
   Ala Ile Ile Leu
               20
<210> 305
   <211> 20
   <212> PRT
   <213> Artificial Sequence
<220>
   <223> peptide
<400> 305
   Gln Ala Ile His Asn Val Val His Ala Ile Ile Leu His Gln Gln Gln
   1 5 10 15
   Lys Gln Gln Gln
   20
<210> 306
   <211> 20
   <212> PRT
   <213> Artificial Sequence
<220>
   <223> peptide
<400> 306
   Gln Ala Ile His Asn Val Val His Ala Ile Ile Leu His Gln Gln Gln
   1 5 10 15
   Gln Lys Gln Gln
   20
<210> 307
   <211> 20
   <212> PRT
   <213> Artificial Sequence
<220>
   <223> peptide
<400> 307
   Gln Ala Ile Gln Asn Val Val His Ala Ile Ile Leu His Gln Gln Gln
   1 5 10 15
   Lys Gln Gln Gln
   20
<210> 308
   <211> 20
   <212> PRT
   <213> Artificial Sequence
<220>
   <223> peptide
<400> 308
   Gln Ala Ile His Lys Val Val His Ala Ile Ile Leu His Gln Gln Gln
   1 5 10 15
   Lys Gln Gln Gln
   20
<210> 309
   <211> 20
   <212> PRT
   <213> Artificial Sequence
<220>
   <223> peptide
<400> 309
   Gln Ala Ile His Asn Val Val His Ala Ile Ile Leu His Gln Gln Gln
   1               5                   10                  15
   Gln Gln Gln Gln
               20
<210> 310
   <211> 20
   <212> PRT
   <213> Artificial Sequence
<220>
   <223> peptide
<400> 310
   Gln Ala Ile His Asn Val Val His Ala Ile Ile Leu His Gln Gln His
   1               5                   10                  15
   His His His Gln
               20
<210> 311
   <211> 20
   <212> PRT
   <213> Artificial Sequence
<220>
   <223> peptide
<400> 311
   Gln Ala Ile His Asn Val Val His Ala Ile Ile Leu His Gln Gln Gln
   1               5                   10                  15
   Arg Gln Gln Gln
               20
<210> 312
   <211> 20
   <212> PRT
   <213> Artificial Sequence
<220>
   <223> peptide
<400> 312
   Gln Ala Ile His Asn Val Val His Ala Ile Ile Met His Gln Gln Glu
   1               5                   10                  15
   Gln Gln Gln Gln
               20
<210> 313
   <211> 20
   <212> PRT
   <213> Artificial Sequence
<220>
   <223> peptide
<400> 313
   Gln Ala Ile His Asn Val Ala His Ala Ile Ile Met His Gln Gln Gln
   1               5                   10                  15
   Gln Gln Gln Gln
               20
<210> 314
   <211> 20
   <212> PRT
   <213> Artificial Sequence
<220>
   <223> peptide
<400> 314
   Gln Ala Ile His Asn Val Val His Ala Ile Ile Leu His His His Gln
   1               5                   10                  15
   Gln Gln Gln Gln
               20
<210> 315
   <211> 20
   <212> PRT
   <213> Artificial Sequence
<220>
   <223> peptide
<400> 315
   Ala Ile Ile Leu His Gln Gln Gln Lys Gln Gln Gln Gln Pro Ser Ser
   1               5                   10                  15
   Gln Val Ser Phe
               20
<210> 316
   <211> 20
   <212> PRT
   <213> Artificial Sequence
<220>
   <223> peptide
<400> 316
   Ala Ile Ile Leu His Gln Gln Gln Gln Lys Gln Gln Gln Gln Pro Ser
   1               5                   10                  15
   Ser Gln Phe Ser
               20
<210> 317
   <211> 20
   <212> PRT
   <213> Artificial Sequence
<220>
   <223> peptide
<400> 317
   Ala Ile Ile Leu His Gln Gln Gln Lys Gln Gln Gln Gln Leu Ser Ser
   1               5                   10                  15
   Gln Val Ser Phe
               20
<210> 318
   <211> 20
   <212> PRT
   <213> Artificial Sequence
<220>
   <223> peptide
<400> 318
   Ala Ile Ile Leu His Gln Gln Gln Lys Gln Gln Gln Pro Ser Ser Gln
   1               5                   10                  15
   Val Ser Phe Gln
               20
<210> 319
   <211> 20
   <212> PRT
   <213> Artificial Sequence
<220>
   <223> peptide
<400> 319
   Ala Ile Ile Leu His Gln Gln Gln Gln Gln Gln Gln Glu Gln Lys Gln
   1               5                   10                  15
   Gln Leu Gln Gln
               20
<210> 320
   <211> 20
   <212> PRT
   <213> Artificial Sequence
<220>
   <223> peptide
<400> 320
   Ala Ile Ile Leu His Gln Gln Gln Gln Gln Gln Gln Gln Gln Gln Gln
   1               5                   10                  15
   Gln Pro Leu Ser
               20
<210> 321
   <211> 20
   <212> PRT
   <213> Artificial Sequence
<220>
   <223> peptide
<400> 321
   Ala Ile Ile Leu His Gln Gln His His His His Gln Gln Gln Gln Gln
   1               5                   10                  15
   Gln Gln Gln Gln
               20
<210> 322
   <211> 20
   <212> PRT
   <213> Artificial Sequence
<220>
   <223> peptide
<400> 322
   Ala Ile Ile Leu His Gln Gln His His His His Gln Glu Gln Lys Gln
   1               5                   10                  15
   Gln Leu Gln Gln
               20
<210> 323
   <211> 20
   <212> PRT
   <213> Artificial Sequence
<220>
   <223> peptide
<400> 323
   Ala Ile Ile Leu His Gln Gln Gln Arg Gln Gln Gln Pro Ser Ser Gln
   1               5                   10                  15
   Val Ser Leu Gln
               20
<210> 324
   <211> 20
   <212> PRT
   <213> Artificial Sequence
<220>
   <223> peptide
<400> 324
   Ala Ile Ile Met His Gln Gln Glu Gln Gln Gln Gln Leu Gln Gln Gln
   1               5                   10                  15
   Gln Gln Gln Gln
               20
<210> 325
   <211> 20
   <212> PRT
   <213> Artificial Sequence
<220>
   <223> peptide
<400> 325
   Ala Ile Ile Met His Gln Gln Gln Gln Gln Gln Gln Glu Gln Lys Gln
   1               5                   10                  15
   Gln Leu Gln Gln
               20
<210> 326
   <211> 20
   <212> PRT
   <213> Artificial Sequence
<220>
   <223> peptide
<400> 326
   Ala Ile Ile Leu His His His Gln Gln Gln Gln Gln Gln Pro Ser Ser
   1               5                   10                  15
   Gln Val Ser Tyr
               20
<210> 327
   <211> 20
   <212> PRT
   <213> Artificial Sequence
<220>
   <223> peptide
<400> 327
   Lys Gln Gln Gln Gln Pro Ser Ser Gln Val Ser Phe Gln Gln Pro Leu
   1               5                   10                  15
   Gln Gln Tyr Pro
               20
<210> 328
   <211> 20
   <212> PRT
   <213> Artificial Sequence
<220>
   <223> peptide
<400> 328
   Lys Gln Gln Gln Gln Pro Ser Ser Gln Phe Ser Phe Gln Gln Pro Leu
   1               5                   10                  15
   Gln Gln Tyr Pro
               20
<210> 329
   <211> 20
   <212> PRT
   <213> Artificial Sequence
<220>
   <223> peptide
<400> 329
   Lys Gln Gln Gln Gln Leu Ser Ser Gln Val Ser Phe Gln Gln Pro Gln
   1               5                   10                  15
   Gln Gln Tyr Pro
               20
<210> 330
   <211> 20
   <212> PRT
   <213> Artificial Sequence
<220>
   <223> peptide
<400> 330
   Lys Gln Gln Gln Pro Ser Ser Gln Val Ser Phe Gln Gln Pro Gln Gln
   1               5                   10                  15
   Gln Tyr Pro Leu
               20
<210> 331
   <211> 20
   <212> PRT
   <213> Artificial Sequence
<220>
   <223> peptide
<400> 331
   Gln Gln Gln Gln Glu Gln Lys Gln Gln Leu Gln Gln Gln Gln Gln Gln
   1 5 10 15
   Gln Gln Gln Leu
   20
<210> 332
   <211> 20
   <212> PRT
   <213> Artificial Sequence
<220>
   <223> peptide
<400> 332
   His His His Gln Glu Gln Lys Gln Gln Leu Gln Gln Gln Gln Gln Gln
   1 5 10 15
   Gln Gln Gln Leu
   20
<210> 333
   <211> 20
   <212> PRT
   <213> Artificial Sequence
<220>
   <223> peptide
<400> 333
   Arg Gln Gln Gln Pro Ser Ser Gln Val Ser Leu Gln Gln Pro Gln Gln
   1 5 10 15
   Gln Tyr Pro Ser
   20
<210> 334
   <211> 20
   <212> PRT
   <213> Artificial Sequence
<220>
   <223> peptide
<400> 334
   Gln Gln Gln Gln Leu Gln Gln Gln Gln Gln Gln Gln Leu Gln Gln Gln
   1 5 10 15
   Gln Gln Gln Gln
   20
<210> 335
   <211> 20
   <212> PRT
   <213> Artificial Sequence
<220>
   <223> peptide
<400> 335
   Gln Gln Gln Gln Gln Pro Ser Ser Gln Val Ser Tyr Gln Gln Pro Gln
   1 5 10 15
   Glu Gln Tyr Pro
   20
<210> 336
   <211> 20
   <212> PRT
   <213> Artificial Sequence
<220>
   <223> peptide
<400> 336
   Gln Leu Gln Gln Gln Gln Gln Gln Gln Gln Gln Leu Gln Gln Gln Gln
   1 5 10 15
   Gln Lys Gln Gln
   20
<210> 337
   <211> 20
   <212> PRT
   <213> Artificial Sequence
<220>
   <223> peptide
<400> 337
   Gln Gln Gln Leu Gln Gln Gln Gln Gln Lys Gln Gln Gln Gln Pro Ser
   1 5 10 15
   Ser Gln Val Ser
   20
<210> 338
   <211> 20
   <212> PRT
   <213> Artificial Sequence
<220>
   <223> peptide
<400> 338
   Gln Gln Gln Gln Gln Gln Gln Gln Gln Pro Leu Ser Gln Val Ser Phe
   1 5 10 15
   Gln Gln Pro Gln
   20
<210> 339
   <211> 20
   <212> PRT
   <213> Artificial Sequence
<220>
   <223> peptide
<400> 339
   Gln Gln Gln Gln Gln Gln Gln Gln Gln Pro Leu Ser Gln Val Cys Phe
   1 5 10 15
   Gln Gln Ser Gln
   20
<210> 340
   <211> 20
   <212> PRT
   <213> Artificial Sequence
<220>
   <223> peptide
<400> 340
   His His His Gln Gln Gln Gln Gln Gln Gln Gln Gln Gln Pro Leu Ser
   1 5 10 15
   Gln Val Ser Phe
   20
<210> 341
   <211> 20
   <212> PRT
   <213> Artificial Sequence
<220>
   <223> peptide
<400> 341
   Gln Gln Gln Gln Gln Gln Gln Gln Gln Pro Ser Ser Gln Val Ser Phe
   1 5 10 15
   Gln Gln Pro Gln
   20
<210> 342
   <211> 20
   <212> PRT
   <213> Artificial Sequence
<220>
   <223> peptide
<400> 342
   Gln Pro Leu Ser Gln Val Ser Phe Gln Gln Pro Gln Gln Gln Tyr Pro
   1 5 10 15
   Ser Gly Gln Gly
   20
<210> 343
   <211> 20
   <212> PRT
   <213> Artificial Sequence
<220>
   <223> peptide
<400> 343
   Gln Pro Leu Ser Gln Val Cys Phe Gln Gln Ser Gln Gln Gln Tyr Pro
   1 5 10 15
   Ser Gly Gln Gly
   20
<210> 344
   <211> 20
   <212> PRT
   <213> Artificial Sequence
<220>
   <223> peptide
<400> 344
   Gln Pro Ser Ser Gln Val Ser Phe Gln Gln Pro Gln Gln Gln Tyr Pro
   1 5 10 15
   Ser Ser Gln Val
   20
<210> 345
   <211> 20
   <212> PRT
   <213> Artificial Sequence
<220>
   <223> peptide
<400> 345
   Gln Val Ser Phe Gln Gln Pro Leu Gln Gln Tyr Pro Leu Gly Gln Gly
   1 5 10 15
   Ser Phe Arg Pro
   20
<210> 346
   <211> 20
   <212> PRT
   <213> Artificial Sequence
<220>
   <223> peptide
<400> 346
   Gln Phe Ser Phe Gln Gln Pro Leu Gln Gln Tyr Pro Leu Gly Gln Gly
   1               5                   10                  15
   Ser Phe Arg Pro
               20
<210> 347
   <211> 20
   <212> PRT
   <213> Artificial Sequence
<220>
   <223> peptide
<400> 347
   Gln Val Ser Phe Gln Gln Pro Gln Gln Gln Tyr Pro Leu Gly Gln Gly
   1               5                   10                  15
   Ser Phe Arg Pro
               20
<210> 348
   <211> 20
   <212> PRT
   <213> Artificial Sequence
<220>
   <223> peptide
<400> 348
   Gln Val Ser Phe Gln Gln Pro Gln Gln Gln Tyr Pro Ser Gly Gln Gly
   1               5                   10                  15
   Ser Phe Gln Pro
               20
<210> 349
   <211> 20
   <212> PRT
   <213> Artificial Sequence
<220>
   <223> peptide
<400> 349
   Gln Val Cys Phe Gln Gln Ser Gln Gln Gln Tyr Pro Ser Gly Gln Gly
   1 5 10 15
   Ser Phe Gln Pro
   20
<210> 350
   <211> 20
   <212> PRT
   <213> Artificial Sequence
<220>
   <223> peptide
<400> 350
   Gln Val Ser Phe Gln Gln Pro Gln Gln Gln Tyr Pro Ser Gly Gln Gly
   1 5 10 15
   Phe Phe Gln Pro
   20
<210> 351
   <211> 20
   <212> PRT
   <213> Artificial Sequence
<220>
   <223> peptide
<400> 351
   Gln Val Ser Phe Gln Gln Pro Gln Gln Gln Tyr Pro Ser Gly Gln Gly
   1 5 10 15
   Phe Phe Gln Pro
   20
<210> 352
   <211> 20
   <212> PRT
   <213> Artificial Sequence
<220>
   <223> peptide
<400> 352
   Gln Val Ser Leu Gln Gln Pro Gln Gln Gln Tyr Pro Ser Gly Gln Gly
   1 5 10 15
   Phe Phe Gln Pro
   20
<210> 353
   <211> 20
   <212> PRT
   <213> Artificial Sequence
<220>
   <223> peptide
<400> 353
   Gln Val Ser Phe Gln Gln Pro Gln Gln Gln Tyr Pro Ser Ser Gln Val
   1 5 10 15
   Ser Phe Gln Pro
   20
<210> 354
   <211> 20
   <212> PRT
   <213> Artificial Sequence
<220>
   <223> peptide
<400> 354
   Gln Val Ser Phe Gln Gln Pro Gln Gln Gln Tyr Pro Ser Ser Gln Gly
   1 5 10 15
   Ser Phe Gln Pro
   20
<210> 355
   <211> 20
   <212> PRT
   <213> Artificial Sequence
<220>
   <223> peptide
<400> 355
   Gln Val Ser Tyr Gln Gln Pro Gln Glu Gln Tyr Pro Ser Gly Gln Val
   1 5 10 15
   Ser Phe Gln Ser
   20
<210> 356
   <211> 20
   <212> PRT
   <213> Artificial Sequence
<220>
   <223> peptide
<400> 356
   Gln Gln Tyr Pro Leu Gly Gln Gly Ser Phe Arg Pro Ser Gln Gln Asn
   1 5 10 15
   Pro Gln Ala Gln
   20
<210> 357
   <211> 20
   <212> PRT
   <213> Artificial Sequence
<220>
   <223> peptide
<400> 357
   Gln Gln Tyr Pro Leu Gly Gln Gly Ser Phe Arg Pro Ser Gln Gln Asn
   1               5                   10                  15
   Ser Gln Ala Gln
               20
<210> 358
   <211> 20
   <212> PRT
   <213> Artificial Sequence
<220>
   <223> peptide
<400> 358
   Gln Gln Tyr Pro Ser Gly Gln Gly Ser Phe Gln Pro Ser Gln Gln Asn
   1               5                   10                  15
   Pro Gln Ala Gln
               20
<210> 359
   <211> 20
   <212> PRT
   <213> Artificial Sequence
<220>
   <223> peptide
<400> 359
   Gln Gln Tyr Pro Ser Gly Gln Gly Phe Phe Gln Pro Ser Gln Gln Asn
   1               5                   10                  15
   Pro Gln Ala Gln
               20
<210> 360
   <211> 20
   <212> PRT
   <213> Artificial Sequence
<220>
   <223> peptide
<400> 360
   Gln Gln Tyr Pro Ser Gly Gln Gly Phe Phe Gln Pro Phe Gln Gln Asn
   1               5                   10                  15
   Pro Gln Ala Gln
               20
<210> 361
   <211> 20
   <212> PRT
   <213> Artificial Sequence
<220>
   <223> peptide
<400> 361
   Gln Gln Tyr Pro Ser Gly Gln Gly Phe Phe Gln Pro Ser Gln Gln Asn
   1               5                   10                  15
   Pro Gln Ala Gln
               20
<210> 362
   <211> 20
   <212> PRT
   <213> Artificial Sequence
<220>
   <223> peptide
<400> 362
   Gln Gln Tyr Pro Ser Ser Gln Val Ser Phe Gln Pro Ser Gln Leu Asn
   1               5                   10                  15
   Pro Gln Ala Gln
               20
<210> 363
   <211> 20
   <212> PRT
   <213> Artificial Sequence
<220>
   <223> peptide
<400> 363
   Gln Gln Tyr Pro Ser Ser Gln Gly Ser Phe Gln Pro Ser Gln Gln Asn
   1               5                   10                  15
   Pro Gln Ala Gln
               20
<210> 364
   <211> 20
   <212> PRT
   <213> Artificial Sequence
<220>
   <223> peptide
<400> 364
   Glu Gln Tyr Pro Ser Gly Gln Val Ser Phe Gln Ser Ser Gln Gln Asn
   1               5                   10                  15
   Pro Gln Ala Gln
               20
<210> 365
   <211> 20
   <212> PRT
   <213> Artificial Sequence
<220>
   <223> peptide
<400> 365
   Ser Phe Arg Pro Ser Gln Gln Asn Pro Leu Ala Gln Gly Ser Val Gln
   1               5                   10                  15
   Pro Gln Gln Leu
               20
<210> 366
   <211> 20
   <212> PRT
   <213> Artificial Sequence
<220>
   <223> peptide
<400> 366
   Ser Phe Arg Pro Ser Gln Gln Asn Pro Gln Ala Gln Gly Ser Val Gln
   1               5                   10                  15
   Pro Gln Gln Leu
               20
<210> 367
   <211> 20
   <212> PRT
   <213> Artificial Sequence
<220>
   <223> peptide
<400> 367
   Ser Phe Arg Pro Ser Gln Gln Asn Pro Gln Thr Gln Gly Ser Val Gln
   1               5                   10                  15
   Pro Gln Gln Leu
               20
<210> 368
   <211> 20
   <212> PRT
   <213> Artificial Sequence
<220>
   <223> peptide
<400> 368
   Ser Phe Arg Pro Ser Gln Gln Asn Ser Gln Ala Gln Gly Ser Val Gln
   1               5                   10                  15
   Pro Gln Gln Leu
               20
<210> 369
   <211> 20
   <212> PRT
   <213> Artificial Sequence
<220>
   <223> peptide
<400> 369
   Ser Phe Arg Pro Ser Gln Gln Asn Pro Gln Asp Gln Gly Ser Val Gln
   1               5                   10                  15
   Pro Gln Gln Leu
               20
<210> 370
   <211> 20
   <212> PRT
   <213> Artificial Sequence
<220>
   <223> peptide
<400> 370
   Ser Phe Arg Pro Ser Gln Gln Asn Pro Arg Ala Gln Gly Ser Val Gln
   1               5                   10                  15
   Pro Gln Gln Leu
               20
<210> 371
   <211> 20
   <212> PRT
   <213> Artificial Sequence
<220>
   <223> peptide
<400> 371
   Ser Phe Gln Pro Ser Gln Gln Asn Pro Gln Ala Gln Gly Ser Val Gln
   1 5 10 15
   Pro Gln Gln Leu
   20
<210> 372
   <211> 20
   <212> PRT
   <213> Artificial Sequence
<220>
   <223> peptide
<400> 372
   Phe Phe Gln Pro Ser Gln Gln Asn Pro Gln Ala Gln Gly Ser Phe Gln
   1 5 10 15
   Pro Gln Gln Leu
   20
<210> 373
   <211> 20
   <212> PRT
   <213> Artificial Sequence
<220>
   <223> peptide
<400> 373
   Phe Phe Gln Pro Phe Gln Gln Asn Pro Gln Ala Gln Gly Ser Phe Gln
   1 5 10 15
   Pro Gln Gln Leu
   20
<210> 374
   <211> 20
   <212> PRT
   <213> Artificial Sequence
<220>
   <223> peptide
<400> 374
   Phe Phe Gln Pro Ser Gln Gln Asn Pro Gln Ala Gln Gly Ser Val Gln
   1 5 10 15
   Pro Gln Gln Leu
   20
<210> 375
   <211> 20
   <212> PRT
   <213> Artificial Sequence
<220>
   <223> peptide
<400> 375
   Ser Phe Gln Pro Ser Gln Leu Asn Pro Gln Ala Gln Gly Ser Val Gln
   1               5                   10                  15
   Pro Gln Gln Leu
               20
<210> 376
   <211> 20
   <212> PRT
   <213> Artificial Sequence
<220>
   <223> peptide
<400> 376
   Ser Phe Gln Pro Ser Gln Leu Asn Pro Gln Ala Gln Gly Ser Val Gln
   1               5                   10                  15
   Pro Gln Gln Leu
               20
<210> 377
   <211> 20
   <212> PRT
   <213> Artificial Sequence
<220>
   <223> peptide
<400> 377
   Ser Phe Gln Pro Ser Gln Gln Asn Pro Gln Ala Gln Gly Ser Val Gln
   1               5                   10                  15
   Pro Gln Gln Leu
               20
<210> 378
   <211> 20
   <212> PRT
   <213> Artificial Sequence
<220>
   <223> peptide
<400> 378
   Ser Phe Gln Ser Ser Gln Gln Asn Pro Gln Ala Gln Gly Ser Val Gln
   1               5                   10                  15
   Pro Gln Gln Leu
               20
<210> 379
   <211> 20
   <212> PRT
   <213> Artificial Sequence
<220>
   <223> peptide
<400> 379
   Pro Gln Ala Gln Gly Ser Val Gln Pro Gln Gln Leu Pro Gln Phe Glu
   1               5                   10                  15
   Glu Ile Arg Asn
               20
<210> 380
   <211> 20
   <212> PRT
   <213> Artificial Sequence
<220>
   <223> peptide
<400> 380
   Pro Gln Thr Gln Gly Ser Val Gln Pro Gln Gln Leu Pro Gln Phe Glu
   1               5                   10                  15
   Glu Ile Arg Asn
               20
<210> 381
   <211> 20
   <212> PRT
   <213> Artificial Sequence
<220>
   <223> peptide
<400> 381
   Pro Gln Ala Gln Gly Ser Val Gln Pro Gln Gln Leu Pro Gln Phe Glu
   1               5                   10                  15
   Ile Arg Asn Leu
               20
<210> 382
   <211> 20
   <212> PRT
   <213> Artificial Sequence
<220>
   <223> peptide
<400> 382
   Pro Leu Ala Gln Gly Ser Val Gln Pro Gln Gln Leu Pro Gln Phe Glu
   1 5 10 15
   Glu Ile Arg Asn
   20
<210> 383
   <211> 20
   <212> PRT
   <213> Artificial Sequence
<220>
   <223> peptide
<400> 383
   Pro Gln Asp Gln Gly Ser Val Gln Pro Gln Gln Leu Pro Gln Phe Glu
   1 5 10 15
   Glu Ile Arg Asn
   20
<210> 384
   <211> 20
   <212> PRT
   <213> Artificial Sequence
<220>
   <223> peptide
<400> 384
   Pro Arg Ala Gln Gly Ser Val Gln Pro Gln Gln Leu Pro Gln Phe Glu
   1 5 10 15
   Glu Ile Arg Asn
   20
<210> 385
   <211> 20
   <212> PRT
   <213> Artificial Sequence
<220>
   <223> peptide
<400> 385
   Pro Gln Ala Gln Gly Ser Phe Gln Pro Gln Gln Leu Pro Gln Phe Glu
   1 5 10 15
   Glu Ile Arg Asn
   20
<210> 386
   <211> 20
   <212> PRT
   <213> Artificial Sequence
<220>
   <223> peptide
<400> 386
   Pro Gln Ala Gln Gly Ser Phe Gln Pro Gln Gln Leu Pro Gln Phe Glu
   1               5                   10                  15
   Ala Ile Arg Asn
               20
<210> 387
   <211> 20
   <212> PRT
   <213> Artificial Sequence
<220>
   <223> peptide
<400> 387
   Pro Gln Ala Gln Gly Ser Val Gln Pro Gln Gln Leu Pro Gln Phe Ala
   1               5                   10                  15
   Glu Ile Arg Asn
               20
<210> 388
   <211> 20
   <212> PRT
   <213> Artificial Sequence
<220>
   <223> peptide
<400> 388
   Pro Gln Ala Gln Gly Ser Val Gln Pro Gln Gln Leu Pro Gln Phe Gln
   1               5                   10                  15
   Glu Ile Arg Asn
               20
<210> 389
   <211> 20
   <212> PRT
   <213> Artificial Sequence
<220>
   <223> peptide
<400> 389
   Pro Gln Gln Leu Pro Gln Phe Glu Glu Ile Arg Asn Leu Ala Leu Gln
   1               5                   10                  15
   Thr Leu Pro Ala
               20
<210> 390
   <211> 20
   <212> PRT
   <213> Artificial Sequence
<220>
   <223> peptide
<400> 390
   Pro Gln Gln Leu Pro Gln Phe Glu Ile Arg Asn Leu Ala Leu Gln Thr
   1               5                   10                  15
   Leu Pro Ala Met
               20
<210> 391
   <211> 16
   <212> PRT
   <213> Artificial Sequence
<220>
   <223> peptide
<400> 391
   Pro Gln Gln Leu Pro Gln Phe Glu Glu Ile Arg Asn Leu Ala Arg Lys
   1               5                   10                  15
<210> 392
   <211> 20
   <212> PRT
   <213> Artificial Sequence
<220>
   <223> peptide
<400> 392
   Pro Gln Gln Leu Pro Gln Phe Glu Glu Ile Arg Asn Leu Ala Leu Glu
   1               5                   10                  15
   Thr Leu Pro Ala
               20
<210> 393
   <211> 20
   <212> PRT
   <213> Artificial Sequence
<220>
   <223> peptide
<400> 393
   Pro Gln Gln Leu Pro Gln Phe Glu Ala Ile Arg Asn Leu Ala Leu Gln
   1 5 10 15
   Thr Leu Pro Ala
   20
<210> 394
   <211> 20
   <212> PRT
   <213> Artificial Sequence
<220>
   <223> peptide
<400> 394
   Pro Gln Gln Leu Pro Gln Phe Ala Glu Ile Arg Asn Leu Ala Leu Gln
   1 5 10 15
   Thr Leu Pro Ala
   20
<210> 395
   <211> 20
   <212> PRT
   <213> Artificial Sequence
<220>
   <223> peptide
<400> 395
   Pro Gln Gln Leu Pro Gln Phe Gln Glu Ile Arg Asn Leu Ala Leu Gln
   1 5 10 15
   Thr Leu Pro Ala
   20
<210> 396
   <211> 20
   <212> PRT
   <213> Artificial Sequence
<220>
   <223> peptide
<400> 396
   Glu Ile Arg Asn Leu Ala Leu Gln Thr Leu Pro Ala Met Cys Asn Val
   1 5 10 15
   Tyr Ile Pro Pro
   20
<210> 397
   <211> 20
   <212> PRT
   <213> Artificial Sequence
<220>
   <223> peptide
<400> 397
   Glu Ile Arg Asn Leu Ala Leu Gln Thr Leu Pro Ser Met Cys Asn Val
   1               5                   10                  15
   Tyr Ile Pro Pro
               20
<210> 398
   <211> 20
   <212> PRT
   <213> Artificial Sequence
<220>
   <223> peptide
<400> 398
   Glu Ile Arg Asn Leu Ala Leu Glu Thr Leu Pro Ala Met Cys Asn Val
   1               5                   10                  15
   Tyr Ile Pro Pro
               20
<210> 399
   <211> 20
   <212> PRT
   <213> Artificial Sequence
<220>
   <223> peptide
<400> 399
   Glu Ile Arg Asn Leu Ala Leu Gln Thr Leu Pro Arg Met Cys Asn Val
   1               5                   10                  15
   Tyr Ile Pro Pro
               20
<210> 400
   <211> 20
   <212> PRT
   <213> Artificial Sequence
<220>
   <223> peptide
<400> 400
   Thr Leu Pro Ala Met Cys Asn Val Tyr Ile Pro Pro Tyr Cys Thr Ile
   1               5                   10                  15
   Ala Pro Phe Gly
               20
<210> 401
   <211> 20
   <212> PRT
   <213> Artificial Sequence
<220>
   <223> peptide
<400> 401
   Thr Leu Pro Ser Met Cys Asn Val Tyr Ile Pro Pro Tyr Cys Thr Ile
   1               5                   10                  15
   Ala Pro Phe Gly
               20
<210> 402
   <211> 20
   <212> PRT
   <213> Artificial Sequence
<220>
   <223> peptide
<400> 402
   Thr Leu Pro Ala Met Cys Asn Val Tyr Ile Pro Pro Tyr Cys Thr Ile
   1               5                   10                  15
   Val Pro Phe Gly
               20
<210> 403
   <211> 20
   <212> PRT
   <213> Artificial Sequence
<220>
   <223> peptide
<400> 403
   Thr Leu Pro Ala Met Cys Asn Val Tyr Ile Pro Pro Tyr Cys Ala Met
   1               5                   10                  15
   Ala Pro Phe Gly
               20
<210> 404
   <211> 20
   <212> PRT
   <213> Artificial Sequence
<220>
   <223> peptide
<400> 404
   Thr Leu Pro Ala Met Cys Asn Val Tyr Ile Pro Pro Tyr Cys Thr Ile
   1 5 10 15
   Thr Pro Phe Gly
   20
<210> 405
   <211> 20
   <212> PRT
   <213> Artificial Sequence
<220>
   <223> peptide
<400> 405
   Thr Leu Pro Ala Met Cys Asn Val Tyr Ile Pro Pro Tyr Cys Thr Ile
   1 5 10 15
   Ala Pro Val Gly
   20
<210> 406
   <211> 20
   <212> PRT
   <213> Artificial Sequence
<220>
   <223> peptide
<400> 406
   Thr Leu Pro Ala Met Cys Asn Val Tyr Ile Pro Pro Tyr Cys Ser Thr
   1 5 10 15
   Thr Ile Ala Pro
   20
<210> 407
   <211> 20
   <212> PRT
   <213> Artificial Sequence
<220>
   <223> peptide
<400> 407
   Thr Leu Pro Arg Met Cys Asn Val Tyr Ile Pro Pro Tyr Cys Ser Thr
   1 5 10 15
   Thr Ile Ala Pro
   20
<210> 408
   <211> 20
   <212> PRT
   <213> Artificial Sequence
<220>
   <223> peptide
<400> 408
   Thr Leu Pro Arg Met Cys Asn Val Tyr Ile Pro Pro Tyr Cys Ser Thr
   1               5                   10                  15
   Thr Thr Ala Pro
               20
<210> 409
   <211> 20
   <212> PRT
   <213> Artificial Sequence
<220>
   <223> peptide
<400> 409
   Thr Leu Pro Ala Met Cys Asn Val Tyr Ile Pro Pro His Cys Ser Thr
   1               5                   10                  15
   Thr Ile Ala Pro
               20
<210> 410
   <211> 19
   <212> PRT
   <213> Artificial Sequence
<220>
   <223> peptide
<400> 410
   Tyr Ile Pro Pro Tyr Cys Thr Ile Ala Pro Phe Gly Ile Phe Gly Thr
   1               5                   10                  15
   Asn Tyr Arg
<210> 411
   <211> 19
   <212> PRT
   <213> Artificial Sequence
<220>
   <223> peptide
<400> 411
   Tyr Ile Pro Pro Tyr Cys Thr Ile Val Pro Phe Gly Ile Phe Gly Thr
   1               5                   10                  15
   Asn Tyr Arg
<210> 412
   <211> 19
   <212> PRT
   <213> Artificial Sequence
<220>
   <223> peptide
<400> 412
   Tyr Ile Pro Pro Tyr Cys Ala Met Ala Pro Phe Gly Ile Phe Gly Thr
   1 5 10 15
   Asn Tyr Arg
<210> 413
   <211> 19
   <212> PRT
   <213> Artificial Sequence
<220>
   <223> peptide
<400> 413
   Tyr Ile Pro Pro Tyr Cys Thr Met Ala Pro Phe Gly Ile Phe Gly Thr
   1 5 10 15
   Asn Tyr Arg
<210> 414
   <211> 17
   <212> PRT
   <213> Artificial Sequence
<220>
   <223> peptide
<400> 414
   Tyr Ile Pro Pro Tyr Cys Thr Ile Thr Pro Phe Gly Ile Phe Gly Thr
   1 5 10 15
   Asn
<210> 415
   <211> 19
   <212> PRT
   <213> Artificial Sequence
<220>
   <223> peptide
<400> 415
   Tyr Ile Pro Pro Tyr Cys Thr Ile Ala Pro Val Gly Ile Phe Gly Thr
   1 5 10 15
   Asn Tyr Arg
<210> 416
   <211> 19
   <212> PRT
   <213> Artificial Sequence
<220>
   <223> peptide
<400> 416
   Tyr Ile Pro Pro Tyr Cys Ser Thr Thr Ile Ala Pro Val Gly Ile Phe
   1               5                   10                  15
   Gly Thr Asn
<210> 417
   <211> 19
   <212> PRT
   <213> Artificial Sequence
<220>
   <223> peptide
<400> 417
   Tyr Ile Pro Pro Tyr Cys Ser Thr Thr Thr Ala Pro Phe Gly Ile Phe
   1               5                   10                  15
   Gly Thr Asn
<210> 418
   <211> 19
   <212> PRT
   <213> Artificial Sequence
<220>
   <223> peptide
<400> 418
   Tyr Ile Pro Pro His Cys Ser Thr Thr Ile Ala Pro Phe Gly Ile Phe
   1               5                   10                  15
   Gly Thr Asn
<210> 419
   <211> 19
   <212> PRT
   <213> Artificial Sequence
<220>
   <223> peptide
<400> 419
   Tyr Ile Pro Pro His Cys Ser Thr Thr Ile Ala Pro Phe Gly Ile Ser
   1 5 10 15
   Gly Thr Asn
<210> 420
   <211> 20
   <212> PRT
   <213> Artificial Sequence
<220>
   <223> peptide
<400> 420
   Ile Pro Pro Tyr Cys Ser Thr Thr Ile Ala Pro Phe Gly Ile Phe Gly
   1 5 10 15
   Thr Asn Tyr Arg
   20
<210> 421
   <211> 20
   <212> PRT
   <213> Artificial Sequence
<220>
   <223> peptide
<400> 421
   Gly Thr Ala Asn Met Gln Val Asp Pro Ser Ser Gln Val Gln Trp Pro
   1 5 10 15
   Gln Gln Gln Pro
   20
<210> 422
   <211> 20
   <212> PRT
   <213> Artificial Sequence
<220>
   <223> peptide
<400> 422
   Gly Thr Ala Asn Ile Gln Val Asp Pro Ser Gly Gln Val Gln Trp Leu
   1 5 10 15
   Gln Gln Gln Leu
   20
<210> 423
   <211> 20
   <212> PRT
   <213> Artificial Sequence
<220>
   <223> peptide
<400> 423
   Ala Thr Ala Asn Met Gln Val Asp Pro Ser Gly Gln Val Pro Trp Pro
   1 5 10 15
   Gln Gln Gln Pro
   20
<210> 424
   <211> 20
   <212> PRT
   <213> Artificial Sequence
<220>
   <223> peptide
<400> 424
   Met Asn Ile Gln Val Asp Pro Ser Gly Gln Val Pro Trp Pro Gln Gln
   1 5 10 15
   Gln Pro Phe Pro
   20
<210> 425
   <211> 20
   <212> PRT
   <213> Artificial Sequence
<220>
   <223> peptide
<400> 425
   Ala Thr Ala Asn Met Gln Ala Asp Pro Ser Gly Gln Val Gln Trp Pro
   1 5 10 15
   Gln Gln Gln Pro
   20
<210> 426
   <211> 20
   <212> PRT
   <213> Artificial Sequence
<220>
   <223> peptide
<400> 426
   Thr Thr Ala Asn Ile Gln Val Asp Pro Ser Gly Gln Val Gln Trp Pro
   1 5 10 15
   Gln Gln Gln Gln
   20
<210> 427
   <211> 20
   <212> PRT
   <213> Artificial Sequence
<220>
   <223> peptide
<400> 427
   Ala Thr Ala Asn Met Gln Val Asp Pro Ser Gly Gln Val Gln Trp Pro
   1               5                   10                  15
   Gln Gln Gln Pro
               20
<210> 428
   <211> 19
   <212> PRT
   <213> Artificial Sequence
<220>
   <223> peptide
<400> 428
   Gln Ile Val Phe Pro Ser Gly Gln Val Gln Trp Pro Gln Gln Gln Gln
   1               5                   10                  15
   Pro Phe Pro
<210> 429
   <211> 20
   <212> PRT
   <213> Artificial Sequence
<220>
   <223> peptide
<400> 429
   Pro Ser Ser Gln Val Gln Trp Pro Gln Gln Gln Pro Val Pro Gln Pro
   1               5                   10                  15
   His Gln Pro Phe
               20
<210> 430
   <211> 20
   <212> PRT
   <213> Artificial Sequence
<220>
   <223> peptide
<400> 430
   Pro Ser Gly Gln Val Gln Trp Leu Gln Gln Gln Leu Val Pro Gln Leu
   1 5 10 15
   Gln Gln Pro Leu
   20
<210> 431
   <211> 20
   <212> PRT
   <213> Artificial Sequence
<220>
   <223> peptide
<400> 431
   Pro Ser Gly Gln Val Pro Trp Pro Gln Gln Gln Pro Phe Pro Gln Pro
   1 5 10 15
   His Gln Pro Phe
   20
<210> 432
   <211> 20
   <212> PRT
   <213> Artificial Sequence
<220>
   <223> peptide
<400> 432
   Pro Ser Gly Gln Val Gln Trp Pro Gln Gln Gln Pro Phe Leu Gln Pro
   1 5 10 15
   His Gln Pro Phe
   20
<210> 433
   <211> 20
   <212> PRT
   <213> Artificial Sequence
<220>
   <223> peptide
<400> 433
   Pro Ser Gly Gln Val Gln Trp Pro Gln Gln Gln Gln Pro Phe Pro Gln
   1 5 10 15
   Pro Gln Gln Pro
   20
<210> 434
   <211> 20
   <212> PRT
   <213> Artificial Sequence
<220>
   <223> peptide
<400> 434
   Pro Ser Gly Gln Val Gln Trp Pro Gln Gln Gln Pro Phe Arg Gln Pro
   1               5                   10                  15
   Gln Gln Pro Phe
               20
<210> 435
   <211> 20
   <212> PRT
   <213> Artificial Sequence
<220>
   <223> peptide
<400> 435
   Pro Ser Gly Gln Val Gln Trp Pro Gln Gln Gln Pro Phe Pro Gln Pro
   1               5                   10                  15
   Gln Gln Pro Phe
               20
<210> 436
   <211> 20
   <212> PRT
   <213> Artificial Sequence
<220>
   <223> peptide
<400> 436
   Gln Gln Gln Pro Val Pro Gln Pro His Gln Pro Phe Ser Gln Gln Pro
   1               5                   10                  15
   Gln Gln Thr Phe
               20
<210> 437
   <211> 20
   <212> PRT
   <213> Artificial Sequence
<220>
   <223> peptide
<400> 437
   Gln Gln Gln Leu Val Pro Gln Leu Gln Gln Pro Leu Ser Gln Gln Pro
   1               5                   10                  15
   Gln Gln Thr Phe
               20
<210> 438
   <211> 20
   <212> PRT
   <213> Artificial Sequence
<220>
   <223> peptide
<400> 438
   Gln Gln Gln Pro Phe Pro Gln Pro His Gln Pro Phe Ser Gln Gln Pro
   1               5                   10                  15
   Gln Gln Thr Phe
               20
<210> 439
   <211> 20
   <212> PRT
   <213> Artificial Sequence
<220>
   <223> peptide
<400> 439
   Gln Gln Gln Pro Phe Leu Gln Pro His Gln Pro Phe Ser Gln Gln Pro
   1               5                   10                  15
   Gln Gln Ile Phe
               20
<210> 440
   <211> 20
   <212> PRT
   <213> Artificial Sequence
<220>
   <223> peptide
<400> 440
   Gln Gln Gln Gln Pro Phe Pro Gln Pro Gln Gln Pro Phe Ser Gln Gln
   1               5                   10                  15
   Pro Gln Gln Ile
               20
<210> 441
   <211> 20
   <212> PRT
   <213> Artificial Sequence
<220>
   <223> peptide
<400> 441
   Gln Gln Gln Gln Pro Phe Pro Gln Pro Gln Gln Pro Gln Gln Pro Phe
   1 5 10 15
   Pro Gln Pro Gln
   20
<210> 442
   <211> 20
   <212> PRT
   <213> Artificial Sequence
<220>
   <223> peptide
<400> 442
   Gln Gln Gln Pro Phe Arg Gln Pro Gln Gln Pro Phe Tyr Gln Gln Pro
   1 5 10 15
   Gln His Thr Phe
   20
<210> 443
   <211> 20
   <212> PRT
   <213> Artificial Sequence
<220>
   <223> peptide
<400> 443
   Gln Gln Gln Pro Phe Pro Gln Pro Gln Gln Pro Phe Cys Gln Gln Pro
   1 5 10 15
   Gln Arg Thr Ile
   20
<210> 444
   <211> 20
   <212> PRT
   <213> Artificial Sequence
<220>
   <223> peptide
<400> 444
   Gln Gln Gln Pro Phe Pro Gln Pro Gln Gln Pro Phe Cys Glu Gln Pro
   1 5 10 15
   Gln Arg Thr Ile
   20
<210> 445
   <211> 20
   <212> PRT
   <213> Artificial Sequence
<220>
   <223> peptide
<400> 445
   His Gln Pro Phe Ser Gln Gln Pro Gln Gln Thr Phe Pro Gln Pro Gln
   1 5 10 15
   Gln Thr Phe Pro
   20
<210> 446
   <211> 20
   <212> PRT
   <213> Artificial Sequence
<220>
   <223> peptide
<400> 446
   Gln Gln Pro Leu Ser Gln Gln Pro Gln Gln Thr Phe Pro Gln Pro Gln
   1 5 10 15
   Gln Thr Phe Pro
   20
<210> 447
   <211> 20
   <212> PRT
   <213> Artificial Sequence
<220>
   <223> peptide
<400> 447
   His Gln Pro Phe Ser Gln Gln Pro Gln Gln Ile Phe Pro Gln Pro Gln
   1 5 10 15
   Gln Thr Phe Pro
   20
<210> 448
   <211> 20
   <212> PRT
   <213> Artificial Sequence
<220>
   <223> peptide
<400> 448
   Gln Gln Pro Phe Ser Gln Gln Pro Gln Gln Ile Phe Pro Gln Pro Gln
   1 5 10 15
   Gln Thr Phe Pro
   20
<210> 449
   <211> 20
   <212> PRT
   <213> Artificial Sequence
<220>
   <223> peptide
<400> 449
   Gln Gln Pro Gln Gln Pro Phe Pro Gln Pro Gln Gln Pro Gln Leu Pro
   1               5                   10                  15
   Phe Pro Gln Gln
               20
<210> 450
   <211> 20
   <212> PRT
   <213> Artificial Sequence
<220>
   <223> peptide
<400> 450
   Gln Gln Pro Phe Tyr Gln Gln Pro Gln His Thr Phe Pro Gln Pro Gln
   1               5                   10                  15
   Gln Thr Cys Pro
               20
<210> 451
   <211> 20
   <212> PRT
   <213> Artificial Sequence
<220>
   <223> peptide
<400> 451
   Gln Gln Pro Phe Cys Gln Gln Pro Gln Arg Thr Ile Pro Gln Pro His
   1               5                   10                  15
   Gln Thr Phe His
               20
<210> 452
   <211> 20
   <212> PRT
   <213> Artificial Sequence
<220>
   <223> peptide
<400> 452
   Gln Gln Pro Phe Cys Gln Gln Pro Gln Gln Thr Ile Pro Gln Pro His
   1 5 10 15
   Gln Thr Phe His
   20
<210> 453
   <211> 20
   <212> PRT
   <213> Artificial Sequence
<220>
   <223> peptide
<400> 453
   Gln Gln Pro Phe Cys Glu Gln Pro Gln Arg Thr Ile Pro Gln Pro His
   1 5 10 15
   Gln Thr Phe His
   20
<210> 454
   <211> 20
   <212> PRT
   <213> Artificial Sequence
<220>
   <223> peptide
<400> 454
   Gln Gln Thr Phe Pro Gln Pro Gln Gln Thr Phe Pro His Gln Pro Gln
   1 5 10 15
   Gln Gln Phe Pro
   20
<210> 455
   <211> 20
   <212> PRT
   <213> Artificial Sequence
<220>
   <223> peptide
<400> 455
   Gln Gln Ile Phe Pro Gln Pro Gln Gln Thr Phe Pro His Gln Pro Gln
   1 5 10 15
   Gln Gln Phe Pro
   20
<210> 456
   <211> 20
   <212> PRT
   <213> Artificial Sequence
<220>
   <223> peptide
<400> 456
   Gln Gln Ile Phe Pro Gln Pro Gln Gln Thr Phe Pro His Gln Pro Gln
   1               5                   10                  15
   Gln Ala Phe Pro
               20
<210> 457
   <211> 20
   <212> PRT
   <213> Artificial Sequence
<220>
   <223> peptide
<400> 457
   Gln Arg Thr Ile Pro Gln Pro His Gln Thr Phe His His Gln Pro Gln
   1               5                   10                  15
   Gln Thr Phe Pro
               20
<210> 458
   <211> 20
   <212> PRT
   <213> Artificial Sequence
<220>
   <223> peptide
<400> 458
   Gln Thr Phe Pro His Gln Pro Gln Gln Ala Phe Pro Gln Pro Gln Gln
   1               5                   10                  15
   Thr Phe Pro His
               20
<210> 459
   <211> 20
   <212> PRT
   <213> Artificial Sequence
<220>
   <223> peptide
<400> 459
   Gln Thr Phe His His Gln Pro Gln Gln Thr Phe Pro Gln Pro Gln Gln
   1               5                   10                  15
   Thr Tyr Pro His
               20
<210> 460
   <211> 20
   <212> PRT
   <213> Artificial Sequence
<220>
   <223> peptide
<400> 460
   Gln Thr Phe His His Gln Pro Gln Gln Thr Phe Pro Gln Pro Glu Gln
   1               5                   10                  15
   Thr Tyr Pro His
               20
<210> 461
   <211> 20
   <212> PRT
   <213> Artificial Sequence
<220>
   <223> peptide
<400> 461
   Gln Ala Phe Pro Gln Pro Gln Gln Thr Phe Pro His Gln Pro Gln Gln
   1               5                   10                  15
   Gln Phe Pro Gln
               20
<210> 462
   <211> 20
   <212> PRT
   <213> Artificial Sequence
<220>
   <223> peptide
<400> 462
   Gln His Thr Phe Pro Gln Pro Gln Gln Thr Cys Pro His Gln Pro Gln
   1               5                   10                  15
   Gln Gln Phe Pro
               20
<210> 463
   <211> 20
   <212> PRT
   <213> Artificial Sequence
<220>
   <223> peptide
<400> 463
   Gln Thr Phe Pro Gln Pro Gln Gln Thr Tyr Pro His Gln Pro Gln Gln
   1               5                   10                  15
   Gln Phe Pro Gln
               20
<210> 464
   <211> 20
   <212> PRT
   <213> Artificial Sequence
<220>
   <223> peptide
<400> 464
   Gln Thr Phe Pro Gln Pro Glu Gln Thr Tyr Pro His Gln Pro Gln Gln
   1               5                   10                  15
   Gln Phe Pro Gln
               20
<210> 465
   <211> 20
   <212> PRT
   <213> Artificial Sequence
<220>
   <223> peptide
<400> 465
   Gln Thr Phe Pro His Gln Pro Gln Gln Gln Phe Pro Gln Pro Gln Gln
   1               5                   10                  15
   Pro Gln Gln Gln
               20
<210> 466
   <211> 20
   <212> PRT
   <213> Artificial Sequence
<220>
   <223> peptide
<400> 466
   Gln Thr Phe Pro His Gln Pro Gln Gln Gln Val Pro Gln Pro Gln Gln
   1               5                   10                  15
   Pro Gln Gln Pro
               20
<210> 467
   <211> 20
   <212> PRT
   <213> Artificial Sequence
<220>
   <223> peptide
<400> 467
   Gln Thr Phe Pro His Gln Pro Gln Gln Gln Phe Ser Gln Pro Gln Gln
   1               5                   10                  15
   Pro Gln Gln Gln
               20
<210> 468
   <211> 20
   <212> PRT
   <213> Artificial Sequence
<220>
   <223> peptide
<400> 468
   Gln Thr Cys Pro His Gln Pro Gln Gln Gln Phe Pro Gln Pro Gln Gln
   1               5                   10                  15
   Pro Gln Gln Pro
               20
<210> 469
   <211> 20
   <212> PRT
   <213> Artificial Sequence
<220>
   <223> peptide
<400> 469
   Gln Thr Tyr Pro His Gln Pro Gln Gln Gln Phe Pro Gln Thr Gln Gln
   1               5                   10                  15
   Pro Gln Gln Pro
               20
<210> 470
   <211> 20
   <212> PRT
   <213> Artificial Sequence
<220>
   <223> peptide
<400> 470
   Gln Gln Phe Pro Gln Pro Gln Gln Pro Gln Gln Gln Phe Leu Gln Pro
   1               5                   10                  15
   Gln Gln Pro Phe
               20
<210> 471
   <211> 20
   <212> PRT
   <213> Artificial Sequence
<220>
   <223> peptide
<400> 471
   Gln Gln Val Pro Gln Pro Gln Gln Pro Gln Gln Pro Phe Leu Gln Pro
   1               5                   10                  15
   Gln Gln Pro Phe
               20
<210> 472
   <211> 20
   <212> PRT
   <213> Artificial Sequence
<220>
   <223> peptide
<400> 472
   Gln Gln Phe Ser Gln Pro Gln Gln Pro Gln Gln Gln Phe Ile Gln Pro
   1               5                   10                  15
   Gln Gln Pro Phe
               20
<210> 473
   <211> 20
   <212> PRT
   <213> Artificial Sequence
<220>
   <223> peptide
<400> 473
   Gln Gln Phe Pro Gln Pro Gln Gln Pro Gln Gln Gln Phe Leu Gln Pro
   1               5                   10                  15
   Arg Gln Pro Phe
               20
<210> 474
   <211> 20
   <212> PRT
   <213> Artificial Sequence
<220>
   <223> peptide
<400> 474
   Gln Gln Phe Pro Gln Pro Gln Gln Pro Gln Gln Pro Phe Pro Gln Gln
   1 5 10 15
   Pro Gln Gln Gln
   20
<210> 475
   <211> 20
   <212> PRT
   <213> Artificial Sequence
<220>
   <223> peptide
<400> 475
   Gln Gln Phe Pro Gln Thr Gln Gln Pro Gln Gln Pro Phe Pro Gln Pro
   1 5 10 15
   Gln Gln Thr Phe
   20
<210> 476
   <211> 20
   <212> PRT
   <213> Artificial Sequence
<220>
   <223> peptide
<400> 476
   Pro Gln Gln Gln Phe Leu Gln Pro Gln Gln Pro Phe Pro Gln Gln Pro
   1 5 10 15
   Gln Gln Pro Tyr
   20
<210> 477
   <211> 20
   <212> PRT
   <213> Artificial Sequence
<220>
   <223> peptide
<400> 477
   Pro Gln Gln Gln Phe Ile Gln Pro Gln Gln Pro Phe Pro Gln Gln Pro
   1 5 10 15
   Gln Gln Thr Tyr
   20
<210> 478
   <211> 20
   <212> PRT
   <213> Artificial Sequence
<220>
   <223> peptide
<400> 478
   Pro Gln Gln Gln Phe Ile Gln Pro Gln Gln Pro Gln Gln Thr Tyr Pro
   1               5                   10                  15
   Gln Arg Pro Gln
               20
<210> 479
   <211> 20
   <212> PRT
   <213> Artificial Sequence
<220>
   <223> peptide
<400> 479
   Pro Gln Gln Gln Phe Leu Gln Pro Arg Gln Pro Phe Pro Gln Gln Pro
   1               5                   10                  15
   Gln Gln Pro Tyr
               20
<210> 480
   <211> 20
   <212> PRT
   <213> Artificial Sequence
<220>
   <223> peptide
<400> 480
   Pro Gln Gln Pro Phe Pro Gln Gln Pro Gln Gln Gln Phe Pro Gln Pro
   1               5                   10                  15
   Gln Gln Pro Gln
               20
<210> 481
   <211> 20
   <212> PRT
   <213> Artificial Sequence
<220>
   <223> peptide
<400> 481
   Pro Gln Gln Pro Phe Pro Gln Pro Gln Gln Pro Gln Gln Pro Phe Pro
   1               5                   10                  15
   Gln Pro Gln Gln
               20
<210> 482
   <211> 20
   <212> PRT
   <213> Artificial Sequence
<220>
   <223> peptide
<400> 482
   Pro Gln Gln Pro Phe Pro Gln Pro Gln Gln Thr Phe Pro Gln Gln Pro
   1               5                   10                  15
   Gln Leu Pro Phe
               20
<210> 483
   <211> 20
   <212> PRT
   <213> Artificial Sequence
<220>
   <223> peptide
<400> 483
   Pro Gln Gln Gln Phe Pro Gln Pro Gln Gln Pro Gln Gln Pro Phe Pro
   1               5                   10                  15
   Gln Gln Pro Gln
               20
<210> 484
   <211> 20
   <212> PRT
   <213> Artificial Sequence
<220>
   <223> peptide
<400> 484
   Gln Gln Pro Gln Gln Pro Phe Pro Gln Gln Pro Gln Gln Gln Phe Pro
   1               5                   10                  15
   Gln Pro Gln Gln
               20
<210> 485
   <211> 20
   <212> PRT
   <213> Artificial Sequence
<220>
   <223> peptide
<400> 485
   Gln Gln Pro Gln Gln Gln Phe Pro Gln Pro Gln Gln Pro Gln Gln Pro
   1 5 10 15
   Phe Pro Gln Pro
   20
<210> 486
   <211> 20
   <212> PRT
   <213> Artificial Sequence
<220>
   <223> peptide
<400> 486
   Gln Pro Gln Gln Pro Gln Gln Pro Phe Pro Gln Pro Gln Gln Pro Gln
   1 5 10 15
   Leu Pro Phe Pro
   20
<210> 487
   <211> 20
   <212> PRT
   <213> Artificial Sequence
<220>
   <223> peptide
<400> 487
   Gln Gln Pro Phe Pro Gln Gln Pro Gln Gln Pro Tyr Pro Gln Gln Pro
   1 5 10 15
   Gln Gln Pro Phe
   20
<210> 488
   <211> 20
   <212> PRT
   <213> Artificial Sequence
<220>
   <223> peptide
<400> 488
   Gln Gln Pro Phe Pro Gln Gln Pro Gln Gln Pro Phe Pro Gln Thr Gln
   1 5 10 15
   Gln Pro Gln Gln
   20
<210> 489
   <211> 20
   <212> PRT
   <213> Artificial Sequence
<220>
   <223> peptide
<400> 489
   Gln Gln Pro Phe Pro Gln Gln Pro Gln Gln Thr Tyr Pro Gln Arg Pro
   1 5 10 15
   Gln Gln Pro Phe
   20
<210> 490
   <211> 20
   <212> PRT
   <213> Artificial Sequence
<220>
   <223> peptide
<400> 490
   Arg Gln Pro Phe Pro Gln Gln Pro Gln Gln Pro Tyr Pro Gln Gln Pro
   1 5 10 15
   Gln Gln Pro Phe
   20
<210> 491
   <211> 20
   <212> PRT
   <213> Artificial Sequence
<220>
   <223> peptide
<400> 491
   Gln Gln Pro Phe Pro Gln Pro Gln Gln Pro Gln Leu Pro Phe Pro Gln
   1 5 10 15
   Gln Pro Gln Gln
   20
<210> 492
   <211> 20
   <212> PRT
   <213> Artificial Sequence
<220>
   <223> peptide
<400> 492
   Gln Gln Pro Phe Pro Gln Pro Gln Gln Ala Gln Leu Pro Phe Pro Gln
   1 5 10 15
   Gln Pro Gln Gln
   20
<210> 493
   <211> 20
   <212> PRT
   <213> Artificial Sequence
<220>
   <223> peptide
<400> 493
   Gln Gln Thr Phe Pro Gln Gln Pro Gln Leu Pro Phe Pro Gln Gln Pro
   1               5                   10                  15
   Gln Gln Pro Phe
               20
<210> 494
   <211> 20
   <212> PRT
   <213> Artificial Sequence
<220>
   <223> peptide
<400> 494
   Gln Gln Pro Tyr Pro Gln Gln Pro Gln Gln Pro Phe Pro Gln Thr Gln
   1               5                   10                  15
   Gln Pro Gln Gln
               20
<210> 495
   <211> 20
   <212> PRT
   <213> Artificial Sequence
<220>
   <223> peptide
<400> 495
   Gln Gln Pro Phe Pro Gln Thr Gln Gln Pro Gln Gln Pro Phe Pro Gln
   1               5                   10                  15
   Gln Pro Gln Gln
               20
<210> 496
   <211> 20
   <212> PRT
   <213> Artificial Sequence
<220>
   <223> peptide
<400> 496
   Gln Gln Thr Tyr Pro Gln Arg Pro Gln Gln Pro Phe Pro Gln Thr Gln
   1 5 10 15
   Gln Pro Gln Gln
   20
<210> 497
   <211> 20
   <212> PRT
   <213> Artificial Sequence
<220>
   <223> peptide
<400> 497
   Gln Pro Gln Leu Pro Phe Pro Gln Gln Pro Gln Gln Gln Pro Gln Gln
   1 5 10 15
   Pro Phe Pro Gln
   20
<210> 498
   <211> 20
   <212> PRT
   <213> Artificial Sequence
<220>
   <223> peptide
<400> 498
   Gln Ala Gln Leu Pro Phe Pro Gln Gln Pro Gln Gln Pro Leu Pro Gln
   1 5 10 15
   Pro Gln Gln Pro
   20
<210> 499
   <211> 20
   <212> PRT
   <213> Artificial Sequence
<220>
   <223> peptide
<400> 499
   Gln Leu Pro Phe Pro Gln Gln Pro Gln Gln Pro Phe Pro Gln Pro Gln
   1 5 10 15
   Gln Pro Gln Gln
   20
<210> 500
   <211> 20
   <212> PRT
   <213> Artificial Sequence
<220>
   <223> peptide
<400> 500
   Gln Pro Gln Gln Pro Phe Pro Gln Gln Pro Gln Gln Pro Phe Pro Gln
   1 5 10 15
   Thr Gln Gln Pro
   20
<210> 501
   <211> 20
   <212> PRT
   <213> Artificial Sequence
<220>
   <223> peptide
<400> 501
   Gln Pro Gln Gln Pro Phe Pro Gln Thr Gln Gln Pro Gln Gln Pro Phe
   1 5 10 15
   Pro Gln Gln Pro
   20
<210> 502
   <211> 20
   <212> PRT
   <213> Artificial Sequence
<220>
   <223> peptide
<400> 502
   Thr Gln Gln Pro Gln Gln Pro Phe Pro Gln Gln Pro Gln Gln Pro Phe
   1 5 10 15
   Pro Gln Thr Gln
   20
<210> 503
   <211> 20
   <212> PRT
   <213> Artificial Sequence
<220>
   <223> peptide
<400> 503
   Pro Gln Gln Pro Gln Gln Pro Phe Pro Gln Thr Gln Gln Pro Gln Gln
   1 5 10 15
   Pro Phe Pro Gln
   20
<210> 504
   <211> 20
   <212> PRT
   <213> Artificial Sequence
<220>
   <223> peptide
<400> 504
   Gln Gln Pro Phe Pro Gln Thr Gln Gln Pro Gln Gln Leu Phe Pro Gln
   1               5                   10                  15
   Ser Gln Gln Pro
               20
<210> 505
   <211> 20
   <212> PRT
   <213> Artificial Sequence
<220>
   <223> peptide
<400> 505
   Gln Gln Pro Phe Pro Gln Thr Gln Gln Pro Gln Gln Pro Phe Pro Gln
   1               5                   10                  15
   Leu Gln Gln Pro
               20
<210> 506
   <211> 20
   <212> PRT
   <213> Artificial Sequence
<220>
   <223> peptide
<400> 506
   Gln Gln Pro Phe Pro Gln Thr Gln Gln Pro Gln Gln Pro Phe Pro Gln
   1               5                   10                  15
   Ser Gln Gln Pro
               20
<210> 507
   <211> 20
   <212> PRT
   <213> Artificial Sequence
<220>
   <223> peptide
<400> 507
   Gln Gln Pro Phe Pro Gln Thr Gln Gln Pro Gln Gln Pro Phe Pro Gln
   1 5 10 15
   Ser Lys Gln Pro
   20
<210> 508
   <211> 20
   <212> PRT
   <213> Artificial Sequence
<220>
   <223> peptide
<400> 508
   Gln Gln Pro Phe Pro Gln Pro Gln Gln Pro Gln Gln Pro Phe Pro Gln
   1 5 10 15
   Leu Gln Gln Pro
   20
<210> 509
   <211> 20
   <212> PRT
   <213> Artificial Sequence
<220>
   <223> peptide
<400> 509
   Gln Gln Pro Leu Pro Gln Pro Gln Gln Pro Gln Gln Pro Phe Pro Gln
   1 5 10 15
   Ser Gln Gln Pro
   20
<210> 510
   <211> 20
   <212> PRT
   <213> Artificial Sequence
<220>
   <223> peptide
<400> 510
   Gln Gln Pro Phe Pro Gln Pro Gln Gln Pro Gln Gln Pro Phe Pro Gln
   1 5 10 15
   Ser Gln Gln Pro
   20
<210> 511
   <211> 20
   <212> PRT
   <213> Artificial Sequence
<220>
   <223> peptide
<400> 511
   Gln Pro Gln Gln Leu Phe Pro Gln Ser Gln Gln Pro Gln Gln Gln Phe
   1               5                   10                  15
   Ser Gln Pro Gln
               20
<210> 512
   <211> 20
   <212> PRT
   <213> Artificial Sequence
<220>
   <223> peptide
<400> 512
   Gln Pro Gln Gln Pro Phe Pro Gln Leu Gln Gln Pro Gln Gln Pro Phe
   1               5                   10                  15
   Pro Gln Pro Gln
               20
<210> 513
   <211> 20
   <212> PRT
   <213> Artificial Sequence
<220>
   <223> peptide
<400> 513
   Gln Pro Gln Gln Pro Phe Pro Gln Ser Gln Gln Pro Gln Gln Pro Phe
   1               5                   10                  15
   Pro Gln Pro Gln
               20
<210> 514
   <211> 20
   <212> PRT
   <213> Artificial Sequence
<220>
   <223> peptide
<400> 514
   Gln Pro Gln Gln Pro Phe Pro Gln Ser Lys Gln Pro Gln Gln Pro Phe
   1               5                   10                  15
   Pro Gln Pro Gln
               20
<210> 515
   <211> 20
   <212> PRT
   <213> Artificial Sequence
<220>
   <223> peptide
<400> 515
   Gln Pro Gln Gln Pro Phe Pro Gln Leu Gln Gln Pro Gln Gln Pro Leu
   1               5                   10                  15
   Pro Gln Pro Gln
               20
<210> 516
   <211> 20
   <212> PRT
   <213> Artificial Sequence
<220>
   <223> peptide
<400> 516
   Ser Gln Gln Pro Gln Gln Gln Phe Ser Gln Pro Gln Gln Gln Phe Pro
   1               5                   10                  15
   Gln Pro Gln Gln
               20
<210> 517
   <211> 20
   <212> PRT
   <213> Artificial Sequence
<220>
   <223> peptide
<400> 517
   Leu Gln Gln Pro Gln Gln Pro Phe Pro Gln Pro Gln Gln Gln Leu Pro
   1               5                   10                  15
   Gln Pro Gln Gln
               20
<210> 518
   <211> 20
   <212> PRT
   <213> Artificial Sequence
<220>
   <223> peptide
<400> 518
   Ser Gln Gln Pro Gln Gln Pro Phe Pro Gln Pro Gln Gln Gln Phe Pro
   1 5 10 15
   Gln Pro Gln Gln
   20
<210> 519
   <211> 20
   <212> PRT
   <213> Artificial Sequence
<220>
   <223> peptide
<400> 519
   Ser Lys Gln Pro Gln Gln Pro Phe Pro Gln Pro Gln Gln Pro Gln Gln
   1 5 10 15
   Ser Phe Pro Gln
   20
<210> 520
   <211> 20
   <212> PRT
   <213> Artificial Sequence
<220>
   <223> peptide
<400> 520
   Leu Gln Gln Pro Gln Gln Pro Leu Pro Gln Pro Gln Gln Pro Gln Gln
   1 5 10 15
   Pro Phe Pro Gln
   20
<210> 521
   <211> 20
   <212> PRT
   <213> Artificial Sequence
<220>
   <223> peptide
<400> 521
   Ser Gln Gln Pro Gln Gln Pro Phe Pro Gln Pro Gln Gln Pro Gln Gln
   1 5 10 15
   Ser Phe Pro Gln
   20
<210> 522
   <211> 20
   <212> PRT
   <213> Artificial Sequence
<220>
   <223> peptide
<400> 522
   Ser Gln Pro Gln Gln Gln Phe Pro Gln Pro Gln Gln Pro Gln Gln Ser
   1               5                   10                  15
   Phe Pro Gln Gln
               20
<210> 523
   <211> 20
   <212> PRT
   <213> Artificial Sequence
<220>
   <223> peptide
<400> 523
   Pro Gln Pro Gln Gln Gln Leu Pro Gln Pro Gln Gln Pro Gln Gln Ser
   1               5                   10                  15
   Phe Pro Gln Gln
               20
<210> 524
   <211> 20
   <212> PRT
   <213> Artificial Sequence
<220>
   <223> peptide
<400> 524
   Pro Gln Pro Gln Gln Gln Phe Pro Gln Pro Gln Gln Pro Gln Gln Ser
   1               5                   10                  15
   Phe Pro Gln Gln
               20
<210> 525
   <211> 20
   <212> PRT
   <213> Artificial Sequence
<220>
   <223> peptide
<400> 525
   Pro Gln Pro Gln Gln Pro Gln Gln Ser Phe Pro Gln Gln Gln Pro Ser
   1               5                   10                  15
   Leu Ile Gln Gln
               20
<210> 526
   <211> 20
   <212> PRT
   <213> Artificial Sequence
<220>
   <223> peptide
<400> 526
   Pro Gln Pro Gln Gln Pro Gln Gln Pro Phe Pro Gln Gln Gln Gln Pro
   1               5                   10                  15
   Leu Ile Gln Pro
               20
<210> 527
   <211> 20
   <212> PRT
   <213> Artificial Sequence
<220>
   <223> peptide
<400> 527
   Pro Gln Pro Gln Gln Pro Gln Gln Ser Phe Pro Gln Gln Gln Gln Pro
   1               5                   10                  15
   Leu Ile Gln Pro
               20
<210> 528
   <211> 20
   <212> PRT
   <213> Artificial Sequence
<220>
   <223> peptide
<400> 528
   Gln Pro Gln Gln Pro Gln Gln Ser Phe Pro Gln Gln Gln Pro Pro Phe
   1               5                   10                  15
   Ile Gln Pro Ser
               20
<210> 529
   <211> 20
   <212> PRT
   <213> Artificial Sequence
<220>
   <223> peptide
<400> 529
   Gln Pro Gln Gln Pro Gln Gln Ser Phe Pro Gln Gln Gln Arg Pro Phe
   1 5 10 15
   Ile Gln Pro Ser
   20
<210> 530
   <211> 20
   <212> PRT
   <213> Artificial Sequence
<220>
   <223> peptide
<400> 530
   Gln Pro Gln Gln Pro Gln Gln Ser Phe Pro Gln Gln Gln Pro Ser Leu
   1 5 10 15
   Ile Gln Gln Ser
   20
<210> 531
   <211> 20
   <212> PRT
   <213> Artificial Sequence
<220>
   <223> peptide
<400> 531
   Phe Pro Gln Gln Gln Pro Pro Phe Ile Gln Pro Ser Leu Gln Gln Gln
   1 5 10 15
   Val Asn Pro Cys
   20
<210> 532
   <211> 20
   <212> PRT
   <213> Artificial Sequence
<220>
   <223> peptide
<400> 532
   Phe Pro Gln Gln Gln Arg Pro Phe Ile Gln Pro Ser Leu Gln Gln Gln
   1 5 10 15
   Leu Asn Pro Cys
   20
<210> 533
   <211> 20
   <212> PRT
   <213> Artificial Sequence
<220>
   <223> peptide
<400> 533
   Phe Pro Gln Gln Gln Pro Ser Leu Ile Gln Gln Ser Leu Gln Gln Gln
   1               5                   10                  15
   Leu Asn Pro Cys
               20
<210> 534
   <211> 20
   <212> PRT
   <213> Artificial Sequence
<220>
   <223> peptide
<400> 534
   Phe Pro Gln Gln Gln Gln Pro Leu Ile Gln Pro Tyr Leu Gln Gln Gln
   1               5                   10                  15
   Met Asn Pro Cys
               20
<210> 535
   <211> 20
   <212> PRT
   <213> Artificial Sequence
<220>
   <223> peptide
<400> 535
   Phe Pro Gln Gln Gln Gln Pro Ala Ile Gln Ser Phe Leu Gln Gln Gln
   1               5                   10                  15
   Met Asn Pro Cys
               20
<210> 536
   <211> 20
   <212> PRT
   <213> Artificial Sequence
<220>
   <223> peptide
<400> 536
   Ile Gln Pro Ser Leu Gln Gln Gln Val Asn Pro Cys Lys Asn Phe Leu
   1               5                   10                  15
   Leu Gln Gln Cys
               20
<210> 537
   <211> 20
   <212> PRT
   <213> Artificial Sequence
<220>
   <223> peptide
<400> 537
   Ile Gln Pro Ser Leu Gln Gln Gln Leu Asn Pro Cys Lys Asn Ile Leu
   1               5                   10                  15
   Leu Gln Gln Ser
               20
<210> 538
   <211> 20
   <212> PRT
   <213> Artificial Sequence
<220>
   <223> peptide
<400> 538
   Ile Gln Gln Ser Leu Gln Gln Gln Leu Asn Pro Cys Lys Asn Phe Leu
   1               5                   10                  15
   Leu Gln Gln Cys
               20
<210> 539
   <211> 20
   <212> PRT
   <213> Artificial Sequence
<220>
   <223> peptide
<400> 539
   Ile Gln Pro Tyr Leu Gln Gln Gln Met Asn Pro Cys Lys Asn Tyr Leu
   1               5                   10                  15
   Leu Gln Gln Cys
               20
<210> 540
   <211> 20
   <212> PRT
   <213> Artificial Sequence
<220>
   <223> peptide
<400> 540
   Ile Gln Ser Phe Leu Gln Gln Gln Met Asn Pro Cys Lys Asn Phe Leu
   1               5                   10                  15
   Leu Gln Gln Cys
               20
<210> 541
   <211> 20
   <212> PRT
   <213> Artificial Sequence
<220>
   <223> peptide
<400> 541
   Val Asn Pro Cys Lys Asn Phe Leu Leu Gln Gln Cys Lys Pro Val Ser
   1               5                   10                  15
   Leu Val Ser Ser
               20
<210> 542
   <211> 20
   <212> PRT
   <213> Artificial Sequence
<220>
   <223> peptide
<400> 542
   Leu Asn Pro Cys Lys Asn Ile Leu Leu Gln Gln Ser Lys Pro Ala Ser
   1               5                   10                  15
   Leu Val Ser Ser
               20
<210> 543
   <211> 20
   <212> PRT
   <213> Artificial Sequence
<220>
   <223> peptide
<400> 543
   Leu Asn Pro Cys Lys Asn Phe Leu Leu Gln Gln Cys Lys Pro Val Ser
   1               5                   10                  15
   Leu Val Ser Ser
               20
<210> 544
   <211> 20
   <212> PRT
   <213> Artificial Sequence
<220>
   <223> peptide
<400> 544
   Met Asn Pro Cys Lys Asn Tyr Leu Leu Gln Gln Cys Asn Pro Val Ser
   1 5 10 15
   Leu Val Ser Ser
   20
<210> 545
   <211> 20
   <212> PRT
   <213> Artificial Sequence
<220>
   <223> peptide
<400> 545
   Met Asn Pro Cys Lys Asn Phe Leu Leu Gln Gln Cys Asn His Val Ser
   1 5 10 15
   Leu Val Ser Ser
   20
<210> 546
   <211> 20
   <212> PRT
   <213> Artificial Sequence
<220>
   <223> peptide
<400> 546
   Leu Gln Gln Cys Lys Pro Val Ser Leu Val Ser Ser Leu Trp Ser Met
   1 5 10 15
   Ile Trp Pro Gln
   20
<210> 547
   <211> 20
   <212> PRT
   <213> Artificial Sequence
<220>
   <223> peptide
<400> 547
   Leu Gln Gln Ser Lys Pro Ala Ser Leu Val Ser Ser Leu Trp Ser Ile
   1 5 10 15
   Ile Trp Pro Gln
   20
<210> 548
   <211> 20
   <212> PRT
   <213> Artificial Sequence
<220>
   <223> peptide
<400> 548
   Leu Gln Gln Cys Lys Pro Val Ser Leu Val Ser Ser Leu Trp Ser Met
   1               5                   10                  15
   Ile Leu Pro Arg
               20
<210> 549
   <211> 20
   <212> PRT
   <213> Artificial Sequence
<220>
   <223> peptide
<400> 549
   Leu Gln Gln Cys Asn Pro Val Ser Leu Val Ser Ser Leu Val Ser Met
   1               5                   10                  15
   Ile Leu Pro Arg
               20
<210> 550
   <211> 20
   <212> PRT
   <213> Artificial Sequence
<220>
   <223> peptide
<400> 550
   Leu Gln Gln Cys Asn His Val Ser Leu Val Ser Ser Leu Val Ser Ile
   1               5                   10                  15
   Ile Leu Pro Arg
               20
<210> 551
   <211> 20
   <212> PRT
   <213> Artificial Sequence
<220>
   <223> peptide
<400> 551
   Leu Val Ser Ser Leu Trp Ser Met Ile Trp Pro Gln Ser Asp Cys Gln
   1               5                   10                  15
   Val Met Arg Gln
               20
<210> 552
   <211> 20
   <212> PRT
   <213> Artificial Sequence
<220>
   <223> peptide
<400> 552
   Leu Val Ser Ser Leu Trp Ser Ile Ile Trp Pro Gln Ser Asp Cys Gln
   1               5                   10                  15
   Val Met Arg Gln
               20
<210> 553
   <211> 20
   <212> PRT
   <213> Artificial Sequence
<220>
   <223> peptide
<400> 553
   Leu Val Ser Ser Leu Trp Ser Met Ile Leu Pro Arg Ser Asp Cys Gln
   1               5                   10                  15
   Val Met Arg Gln
               20
<210> 554
   <211> 20
   <212> PRT
   <213> Artificial Sequence
<220>
   <223> peptide
<400> 554
   Leu Val Ser Ser Leu Trp Ser Ile Ile Leu Pro Pro Ser Asp Cys Gln
   1               5                   10                  15
   Val Met Arg Gln
               20
<210> 555
   <211> 20
   <212> PRT
   <213> Artificial Sequence
<220>
   <223> peptide
<400> 555
   Leu Val Ser Ser Leu Val Ser Met Ile Leu Pro Arg Ser Asp Cys Lys
   1 5 10 15
   Val Met Arg Gln
   20
<210> 556
   <211> 20
   <212> PRT
   <213> Artificial Sequence
<220>
   <223> peptide
<400> 556
   Leu Val Ser Ser Leu Val Ser Met Ile Leu Pro Arg Ser Asp Cys Gln
   1 5 10 15
   Val Met Gln Gln
   20
<210> 557
   <211> 20
   <212> PRT
   <213> Artificial Sequence
<220>
   <223> peptide
<400> 557
   Leu Val Ser Ser Leu Val Ser Ile Ile Leu Pro Arg Ser Asp Cys Gln
   1 5 10 15
   Val Met Gln Gln
   20
<210> 558
   <211> 20
   <212> PRT
   <213> Artificial Sequence
<220>
   <223> peptide
<400> 558
   Ile Trp Pro Gln Ser Asp Cys Gln Val Met Arg Gln Gln Cys Cys Gln
   1 5 10 15
   Gln Leu Ala Gln
   20
<210> 559
   <211> 20
   <212> PRT
   <213> Artificial Sequence
<220>
   <223> peptide
<400> 559
   Ile Leu Pro Arg Ser Asp Cys Gln Val Met Arg Gln Gln Cys Cys Gln
   1               5                   10                  15
   Gln Leu Ala Gln
               20
<210> 560
   <211> 20
   <212> PRT
   <213> Artificial Sequence
<220>
   <223> peptide
<400> 560
   Ile Leu Pro Pro Ser Asp Cys Gln Val Met Arg Gln Gln Cys Cys Gln
   1               5                   10                  15
   Gln Leu Ala Gln
               20
<210> 561
   <211> 20
   <212> PRT
   <213> Artificial Sequence
<220>
   <223> peptide
<400> 561
   Ile Leu Pro Arg Ser Asp Cys Lys Val Met Arg Gln Gln Cys Cys Gln
   1               5                   10                  15
   Gln Leu Ala Arg
               20
<210> 562
   <211> 20
   <212> PRT
   <213> Artificial Sequence
<220>
   <223> peptide
<400> 562
   Ile Leu Pro Arg Ser Asp Cys Gln Val Met Gln Gln Gln Cys Cys Gln
   1 5 10 15
   Gln Leu Ala Gln
   20
<210> 563
   <211> 20
   <212> PRT
   <213> Artificial Sequence
<220>
   <223> peptide
<400> 563
   Val Met Arg Gln Gln Cys Cys Gln Gln Leu Ala Gln Ile Pro Gln Gln
   1 5 10 15
   Leu Gln Cys Ala
   20
<210> 564
   <211> 20
   <212> PRT
   <213> Artificial Sequence
<220>
   <223> peptide
<400> 564
   Val Met Arg Gln Gln Cys Cys Gln Gln Leu Ala Arg Ile Pro Gln Gln
   1 5 10 15
   Leu Gln Cys Ala
   20
<210> 565
   <211> 20
   <212> PRT
   <213> Artificial Sequence
<220>
   <223> peptide
<400> 565
   Val Met Gln Gln Gln Cys Cys Gln Gln Leu Ala Gln Ile Pro Arg Gln
   1 5 10 15
   Leu Gln Cys Ala
   20
<210> 566
   <211> 20
   <212> PRT
   <213> Artificial Sequence
<220>
   <223> peptide
<400> 566
   Val Met Gln Gln Gln Cys Cys Gln Gln Leu Ala Gln Ile Pro Gln Gln
   1 5 10 15
   Leu Gln Cys Ala
   20
<210> 567
   <211> 20
   <212> PRT
   <213> Artificial Sequence
<220>
   <223> peptide
<400> 567
   Gln Leu Ala Gln Ile Pro Gln Gln Leu Gln Cys Ala Ala Ile His Thr
   1 5 10 15
   Ile Ile His Ser
   20
<210> 568
   <211> 20
   <212> PRT
   <213> Artificial Sequence
<220>
   <223> peptide
<400> 568
   Gln Leu Ala Gln Ile Pro Gln Gln Leu Gln Cys Ala Ala Ile His Thr
   1 5 10 15
   Val Ile His Ser
   20
<210> 569
   <211> 20
   <212> PRT
   <213> Artificial Sequence
<220>
   <223> peptide
<400> 569
   Gln Leu Ala Gln Ile Pro Gln Gln Leu Gln Cys Ala Ala Ile His Ser
   1 5 10 15
   Val Val His Ser
   20
<210> 570
   <211> 20
   <212> PRT
   <213> Artificial Sequence
<220>
   <223> peptide
<400> 570
   Gln Leu Ala Gln Ile Pro Gln Gln Leu Gln Cys Ala Ala Ile His Ser
   1               5                   10                  15
   Ile Val His Ser
               20
<210> 571
   <211> 20
   <212> PRT
   <213> Artificial Sequence
<220>
   <223> peptide
<400> 571
   Gln Leu Ala Arg Ile Pro Gln Gln Leu Gln Cys Ala Ala Ile His Gly
   1               5                   10                  15
   Ile Val His Ser
               20
<210> 572
   <211> 20
   <212> PRT
   <213> Artificial Sequence
<220>
   <223> peptide
<400> 572
   Gln Leu Ala Gln Ile Pro Arg Gln Leu Gln Cys Ala Ala Ile His Ser
   1               5                   10                  15
   Val Val His Ser
               20
<210> 573
   <211> 20
   <212> PRT
   <213> Artificial Sequence
<220>
   <223> peptide
<400> 573
   Gln Leu Ala Gln Ile Pro Gln Gln Leu Gln Cys Ala Ala Ile His Ser
   1 5 10 15
   Val Ala His Ser
   20
<210> 574
   <211> 20
   <212> PRT
   <213> Artificial Sequence
<220>
   <223> peptide
<400> 574
   Leu Gln Cys Ala Ala Ile His Thr Ile Ile His Ser Ile Ile Met Gln
   1 5 10 15
   Gln Glu Gln Gln
   20
<210> 575
   <211> 20
   <212> PRT
   <213> Artificial Sequence
<220>
   <223> peptide
<400> 575
   Leu Gln Cys Ala Ala Ile His Thr Val Ile His Ser Ile Ile Met Gln
   1 5 10 15
   Gln Glu Gln Gln
   20
<210> 576
   <211> 20
   <212> PRT
   <213> Artificial Sequence
<220>
   <223> peptide
<400> 576
   Leu Gln Cys Ala Ala Ile His Ser Val Val His Ser Ile Ile Met Gln
   1 5 10 15
   Gln Gln Gln Gln
   20
<210> 577
   <211> 20
   <212> PRT
   <213> Artificial Sequence
<220>
   <223> peptide
<400> 577
   Leu Gln Cys Ala Ala Ile His Ser Ile Val His Ser Ile Ile Met Gln
   1 5 10 15
   Gln Glu Gln Gln
   20
<210> 578
   <211> 20
   <212> PRT
   <213> Artificial Sequence
<220>
   <223> peptide
<400> 578
   Leu Gln Cys Ala Ala Ile His Ser Val Val His Ser Ile Ile Met Gln
   1 5 10 15
   Gln Glu Gln Gln
   20
<210> 579
   <211> 20
   <212> PRT
   <213> Artificial Sequence
<220>
   <223> peptide
<400> 579
   Leu Gln Cys Ala Ala Ile His Gly Ile Val His Ser Ile Ile Met Gln
   1 5 10 15
   Gln Glu Gln Gln
   20
<210> 580
   <211> 20
   <212> PRT
   <213> Artificial Sequence
<220>
   <223> peptide
<400> 580
   Leu Gln Cys Ala Ala Ile His Ser Val Ala His Ser Ile Ile Met Gln
   1 5 10 15
   Gln Glu Gln Gln
   20
<210> 581
   <211> 20
   <212> PRT
   <213> Artificial Sequence
<220>
   <223> peptide
<400> 581
   Ile Ile His Ser Ile Ile Met Gln Gln Glu Gln Gln Glu Gln Gln Gln
   1               5                   10                  15
   Gly Met His Ile
               20
<210> 582
   <211> 20
   <212> PRT
   <213> Artificial Sequence
<220>
   <223> peptide
<400> 582
   Val Ile His Ser Ile Ile Met Gln Gln Glu Gln Gln Gln Gly Met His
   1               5                   10                  15
   Ile Leu Leu Pro
               20
<210> 583
   <211> 20
   <212> PRT
   <213> Artificial Sequence
<220>
   <223> peptide
<400> 583
   Val Val His Ser Ile Ile Met Gln Gln Gln Gln Gln Gln Gln Gln Gln
   1               5                   10                  15
   Gln Gly Ile Asp
               20
<210> 584
   <211> 20
   <212> PRT
   <213> Artificial Sequence
<220>
   <223> peptide
<400> 584
   Ile Val His Ser Ile Ile Met Gln Gln Glu Gln Gln Glu Gln Arg Gln
   1               5                   10                  15
   Gly Val Gln Ile
               20
<210> 585
   <211> 20
   <212> PRT
   <213> Artificial Sequence
<220>
   <223> peptide
<400> 585
   Val Val His Ser Ile Ile Met Gln Gln Glu Gln Gln Glu Gln Leu Gln
   1               5                   10                  15
   Gly Val Gln Ile
               20
<210> 586
   <211> 20
   <212> PRT
   <213> Artificial Sequence
<220>
   <223> peptide
<400> 586
   Ile Val His Ser Ile Ile Met Gln Gln Glu Gln Gln Gln Gln Gln Gln
   1               5                   10                  15
   Gln Gln Gln Gln
               20
<210> 587
   <211> 20
   <212> PRT
   <213> Artificial Sequence
<220>
   <223> peptide
<400> 587
   Val Val His Ser Ile Val Met Gln Gln Glu Gln Gln Gln Gly Ile Gln
   1               5                   10                  15
   Ile Leu Arg Pro
               20
<210> 588
   <211> 20
   <212> PRT
   <213> Artificial Sequence
<220>
   <223> peptide
<400> 588
   Val Ala His Ser Ile Ile Met Gln Gln Glu Gln Gln Gln Gly Val Pro
   1               5                   10                  15
   Ile Leu Arg Pro
               20
<210> 589
   <211> 20
   <212> PRT
   <213> Artificial Sequence
<220>
   <223> peptide
<400> 589
   Gln Glu Gln Gln Glu Gln Gln Gln Gly Met His Ile Leu Leu Pro Leu
   1               5                   10                  15
   Tyr Gln Gln Gln
               20
<210> 590
   <211> 20
   <212> PRT
   <213> Artificial Sequence
<220>
   <223> peptide
<400> 590
   Gln Gln Gln Gln Gln Gln Gln Gln Gln Gly Ile Asp Ile Phe Leu Pro
   1               5                   10                  15
   Leu Ser Gln His
               20
<210> 591
   <211> 20
   <212> PRT
   <213> Artificial Sequence
<220>
   <223> peptide
<400> 591
   Gln Gln Gln Gln Gln Gln Gln Gln Gln Gly Met His Ile Phe Leu Pro
   1               5                   10                  15
   Leu Ser Gln Gln
               20
<210> 592
   <211> 20
   <212> PRT
   <213> Artificial Sequence
<220>
   <223> peptide
<400> 592
   Gln Glu Gln Gln Glu Gln Arg Gln Gly Val Gln Ile Leu Val Pro Leu
   1 5 10 15
   Ser Gln Gln Gln
   20
<210> 593
   <211> 20
   <212> PRT
   <213> Artificial Sequence
<220>
   <223> peptide
<400> 593
   Gln Glu Gln Gln Glu Gln Leu Gln Gly Val Gln Ile Leu Val Pro Leu
   1 5 10 15
   Ser Gln Gln Gln
   20
<210> 594
   <211> 20
   <212> PRT
   <213> Artificial Sequence
<220>
   <223> peptide
<400> 594
   Gln Glu Gln Gln Gln Gln Gln Gln Gln Gln Gln Gln Gln Gln Gln Gly
   1 5 10 15
   Ile Gln Ile Met
   20
<210> 595
   <211> 20
   <212> PRT
   <213> Artificial Sequence
<220>
   <223> peptide
<400> 595
   Gln Glu Gln Gln Gln Gly Ile Gln Ile Leu Arg Pro Leu Phe Gln Leu
   1 5 10 15
   Val Gln Gly Gln
   20
<210> 596
   <211> 20
   <212> PRT
   <213> Artificial Sequence
<220>
   <223> peptide
<400> 596
   Gln Glu Gln Gln Gln Gly Val Pro Ile Leu Arg Pro Leu Phe Gln Leu
   1 5 10 15
   Ala Gln Gly Leu
   20
<210> 597
   <211> 20
   <212> PRT
   <213> Artificial Sequence
<220>
   <223> peptide
<400> 597
   Gln Gln Gln Gln Gln Gln Gln Gly Ile Gln Ile Met Arg Pro Leu Phe
   1 5 10 15
   Gln Leu Val Gln
   20
<210> 598
   <211> 20
   <212> PRT
   <213> Artificial Sequence
<220>
   <223> peptide
<400> 598
   Gly Met His Ile Leu Leu Pro Leu Tyr Gln Gln Gln Gln Val Gly Gln
   1 5 10 15
   Gly Thr Leu Val
   20
<210> 599
   <211> 20
   <212> PRT
   <213> Artificial Sequence
<220>
   <223> peptide
<400> 599
   Gly Ile Asp Ile Phe Leu Pro Leu Ser Gln His Glu Gln Val Gly Gln
   1               5                   10                  15
   Gly Ser Leu Val
               20
<210> 600
   <211> 20
   <212> PRT
   <213> Artificial Sequence
<220>
   <223> peptide
<400> 600
   Gly Met His Ile Phe Leu Pro Leu Ser Gln Gln Gln Gln Val Gly Gln
   1               5                   10                  15
   Gly Ser Leu Val
               20
<210> 601
   <211> 20
   <212> PRT
   <213> Artificial Sequence
<220>
   <223> peptide
<400> 601
   Gly Val Gln Ile Leu Val Pro Leu Ser Gln Gln Gln Gln Val Gly Gln
   1               5                   10                  15
   Gly Thr Leu Val
               20
<210> 602
   <211> 20
   <212> PRT
   <213> Artificial Sequence
<220>
   <223> peptide
<400> 602
   Gly Val Gln Ile Leu Val Pro Leu Ser Gln Gln Gln Gln Val Gly Gln
   1               5                   10                  15
   Gly Ile Leu Val
               20
<210> 603
   <211> 20
   <212> PRT
   <213> Artificial Sequence
<220>
   <223> peptide
<400> 603
   Gly Ile Gln Ile Met Arg Pro Leu Phe Gln Leu Val Gln Gly Gln Gly
   1               5                   10                  15
   Ile Ile Gln Pro
               20
<210> 604
   <211> 20
   <212> PRT
   <213> Artificial Sequence
<220>
   <223> peptide
<400> 604
   Gly Ile Gln Ile Leu Arg Pro Leu Phe Gln Leu Val Gln Gly Gln Gly
   1               5                   10                  15
   Ile Ile Gln Pro
               20
<210> 605
   <211> 20
   <212> PRT
   <213> Artificial Sequence
<220>
   <223> peptide
<400> 605
   Gly Val Pro Ile Leu Arg Pro Leu Phe Gln Leu Ala Gln Gly Leu Gly
   1               5                   10                  15
   Ile Ile Gln Pro
               20
<210> 606
   <211> 20
   <212> PRT
   <213> Artificial Sequence
<220>
   <223> peptide
<400> 606
   Tyr Gln Gln Gln Gln Val Gly Gln Gly Thr Leu Val Gln Gly Gln Gly
   1 5 10 15
   Ile Ile Gln Pro
   20
<210> 607
   <211> 20
   <212> PRT
   <213> Artificial Sequence
<220>
   <223> peptide
<400> 607
   Ser Gln His Glu Gln Val Gly Gln Gly Ser Leu Val Gln Gly Gln Gly
   1 5 10 15
   Ile Ile Gln Pro
   20
<210> 608
   <211> 20
   <212> PRT
   <213> Artificial Sequence
<220>
   <223> peptide
<400> 608
   Ser Gln Gln Gln Gln Val Gly Gln Gly Ser Leu Val Gln Gly Gln Gly
   1 5 10 15
   Ile Ile Gln Pro
   20
<210> 609
   <211> 20
   <212> PRT
   <213> Artificial Sequence
<220>
   <223> peptide
<400> 609
   Ser Gln Gln Gln Gln Val Gly Gln Gly Thr Leu Val Gln Gly Gln Gly
   1 5 10 15
   Ile Ile Gln Pro
   20
<210> 610
   <211> 20
   <212> PRT
   <213> Artificial Sequence
<220>
   <223> peptide
<400> 610
   Ser Gln Gln Gln Gln Val Gly Gln Gly Ile Leu Val Gln Gly Gln Gly
   1 5 10 15
   Ile Ile Gln Pro
   20
<210> 611
   <211> 20
   <212> PRT
   <213> Artificial Sequence
<220>
   <223> peptide
<400> 611
   Gly Thr Leu Val Gln Gly Gln Gly Ile Ile Gln Pro Gln Gln Pro Ala
   1 5 10 15
   Gln Leu Glu Ala
   20
<210> 612
   <211> 20
   <212> PRT
   <213> Artificial Sequence
<220>
   <223> peptide
<400> 612
   Gly Ser Leu Val Gln Gly Gln Gly Ile Ile Gln Pro Gln Gln Pro Ala
   1 5 10 15
   Gln Leu Glu Ala
   20
<210> 613
   <211> 20
   <212> PRT
   <213> Artificial Sequence
<220>
   <223> peptide
<400> 613
   Phe Gln Leu Val Gln Gly Gln Gly Ile Ile Gln Pro Gln Gln Pro Ala
   1 5 10 15
   Gln Leu Glu Val
   20
<210> 614
   <211> 20
   <212> PRT
   <213> Artificial Sequence
<220>
   <223> peptide
<400> 614
   Phe Gln Leu Ala Gln Gly Leu Gly Ile Ile Gln Pro Gln Gln Pro Ala
   1               5                   10                  15
   Gln Leu Glu Gly
               20
<210> 615
   <211> 20
   <212> PRT
   <213> Artificial Sequence
<220>
   <223> peptide
<400> 615
   Ile Ile Gln Pro Gln Gln Pro Ala Gln Leu Glu Ala Ile Arg Ser Leu
   1               5                   10                  15
   Val Leu Gln Thr
               20
<210> 616
   <211> 20
   <212> PRT
   <213> Artificial Sequence
<220>
   <223> peptide
<400> 616
   Ile Ile Gln Pro Gln Gln Pro Ala Gln Leu Glu Val Ile Arg Ser Leu
   1               5                   10                  15
   Val Leu Gln Thr
               20
<210> 617
   <211> 20
   <212> PRT
   <213> Artificial Sequence
<220>
   <223> peptide
<400> 617
   Ile Ile Gln Pro Gln Gln Pro Ala Gln Leu Glu Val Ile Arg Ser Ser
   1               5                   10                  15
   Val Leu Gln Thr
               20
<210> 618
   <211> 20
   <212> PRT
   <213> Artificial Sequence
<220>
   <223> peptide
<400> 618
   Ile Ile Gln Pro Gln Gln Pro Ala Gln Tyr Glu Val Ile Arg Ser Leu
   1               5                   10                  15
   Val Leu Arg Thr
               20
<210> 619
   <211> 20
   <212> PRT
   <213> Artificial Sequence
<220>
   <223> peptide
<400> 619
   Ile Ile Gln Pro Gln Gln Pro Ala Gln Leu Glu Gly Ile Arg Ser Leu
   1               5                   10                  15
   Val Leu Lys Thr
               20
<210> 620
   <211> 20
   <212> PRT
   <213> Artificial Sequence
<220>
   <223> peptide
<400> 620
   Gln Leu Glu Ala Ile Arg Ser Leu Val Leu Gln Thr Leu Pro Thr Met
   1               5                   10                  15
   Cys Asn Val Tyr
               20
<210> 621
   <211> 20
   <212> PRT
   <213> Artificial Sequence
<220>
   <223> peptide
<400> 621
   Gln Leu Glu Ala Ile Arg Ser Leu Val Leu Gln Thr Leu Pro Ser Met
   1 5 10 15
   Cys Asn Val Tyr
   20
<210> 622
   <211> 20
   <212> PRT
   <213> Artificial Sequence
<220>
   <223> peptide
<400> 622
   Gln Leu Glu Val Ile Arg Ser Leu Val Leu Gln Thr Leu Ala Thr Met
   1 5 10 15
   Cys Asn Val Tyr
   20
<210> 623
   <211> 20
   <212> PRT
   <213> Artificial Sequence
<220>
   <223> peptide
<400> 623
   Gln Leu Glu Val Ile Arg Ser Ser Val Leu Gln Thr Leu Ala Thr Met
   1 5 10 15
   Cys Asn Val Tyr
   20
<210> 624
   <211> 20
   <212> PRT
   <213> Artificial Sequence
<220>
   <223> peptide
<400> 624
   Gln Leu Glu Val Ile Arg Ser Leu Val Leu Gly Thr Leu Pro Thr Met
   1 5 10 15
   Cys Asn Val Phe
   20
<210> 625
   <211> 20
   <212> PRT
   <213> Artificial Sequence
<220>
   <223> peptide
<400> 625
   Gln Tyr Glu Val Ile Arg Ser Leu Val Leu Arg Thr Leu Pro Asn Met
   1               5                   10                  15
   Cys Asn Val Tyr
               20
<210> 626
   <211> 20
   <212> PRT
   <213> Artificial Sequence
<220>
   <223> peptide
<400> 626
   Gln Leu Glu Gly Ile Arg Ser Leu Val Leu Lys Thr Leu Pro Thr Met
   1               5                   10                  15
   Cys Asn Val Tyr
               20
<210> 627
   <211> 20
   <212> PRT
   <213> Artificial Sequence
<220>
   <223> peptide
<400> 627
   Val Leu Gln Thr Leu Pro Thr Met Cys Asn Val Tyr Val Pro Pro Glu
   1               5                   10                  15
   Cys Ser Ile Ile
               20
<210> 628
   <211> 20
   <212> PRT
   <213> Artificial Sequence
<220>
   <223> peptide
<400> 628
   Val Leu Gln Thr Leu Pro Ser Met Cys Asn Val Tyr Val Pro Pro Glu
   1               5                   10                  15
   Cys Ser Ile Met
               20
<210> 629
   <211> 20
   <212> PRT
   <213> Artificial Sequence
<220>
   <223> peptide
<400> 629
   Val Leu Gln Thr Leu Ala Thr Met Cys Asn Val Tyr Val Pro Pro Tyr
   1               5                   10                  15
   Cys Ser Thr Ile
               20
<210> 630
   <211> 20
   <212> PRT
   <213> Artificial Sequence
<220>
   <223> peptide
<400> 630
   Val Leu Gly Thr Leu Pro Thr Met Cys Asn Val Phe Val Pro Pro Glu
   1               5                   10                  15
   Cys Ser Thr Thr
               20
<210> 631
   <211> 20
   <212> PRT
   <213> Artificial Sequence
<220>
   <223> peptide
<400> 631
   Val Leu Arg Thr Leu Pro Asn Met Cys Asn Val Tyr Val Arg Pro Asp
   1               5                   10                  15
   Cys Ser Thr Ile
               20
<210> 632
   <211> 20
   <212> PRT
   <213> Artificial Sequence
<220>
   <223> peptide
<400> 632
   Val Leu Lys Thr Leu Pro Thr Met Cys Asn Val Tyr Val Pro Pro Asp
   1 5 10 15
   Cys Ser Thr Ile
   20
<210> 633
   <211> 20
   <212> PRT
   <213> Artificial Sequence
<220>
   <223> peptide
<400> 633
   Cys Asn Val Tyr Val Pro Pro Glu Cys Ser Ile Ile Lys Ala Pro Phe
   1 5 10 15
   Ser Ser Val Val
   20
<210> 634
   <211> 20
   <212> PRT
   <213> Artificial Sequence
<220>
   <223> peptide
<400> 634
   Cys Asn Val Tyr Val Pro Pro Glu Cys Ser Ile Met Arg Ala Pro Phe
   1 5 10 15
   Ala Ser Ile Val
   20
<210> 635
   <211> 20
   <212> PRT
   <213> Artificial Sequence
<220>
   <223> peptide
<400> 635
   Cys Asn Val Tyr Val Pro Pro Tyr Cys Ser Thr Ile Arg Ala Pro Phe
   1 5 10 15
   Ala Ser Ile Val
   20
<210> 636
   <211> 20
   <212> PRT
   <213> Artificial Sequence
<220>
   <223> peptide
<400> 636
   Cys Asn Val Phe Val Pro Pro Glu Cys Ser Thr Thr Lys Ala Pro Phe
   1               5                   10                  15
   Ala Ser Ile Val
               20
<210> 637
   <211> 20
   <212> PRT
   <213> Artificial Sequence
<220>
   <223> peptide
<400> 637
   Cys Asn Val Tyr Val Arg Pro Asp Cys Ser Thr Ile Asn Ala Pro Phe
   1               5                   10                  15
   Ala Ser Ile Val
               20
<210> 638
   <211> 20
   <212> PRT
   <213> Artificial Sequence
<220>
   <223> peptide
<400> 638
   Cys Asn Val Tyr Val Pro Pro Asp Cys Ser Thr Ile Asn Val Pro Tyr
   1               5                   10                  15
   Ala Asn Ile Asp
               20
<210> 639
   <211> 18
   <212> PRT
   <213> Artificial Sequence
<220>
   <223> peptide
<400> 639
   Cys Ser Ile Ile Lys Ala Pro Phe Ser Ser Val Val Ala Gly Ile Gly
   1 5 10 15
   Gly Gln
<210> 640
   <211> 18
   <212> PRT
   <213> Artificial Sequence
<220>
   <223> peptide
<400> 640
   Cys Ser Ile Met Arg Ala Pro Phe Ala Ser Ile Val Ala Gly Ile Gly
   1 5 10 15
   Gly Gln
<210> 641
   <211> 20
   <212> PRT
   <213> Artificial Sequence
<220>
   <223> peptide
<400> 641
   Cys Ser Thr Ile Arg Ala Pro Phe Ala Ser Ile Val Ala Gly Ile Gly
   1 5 10 15
   Gly Gln Tyr Arg
   20
<210> 642
   <211> 18
   <212> PRT
   <213> Artificial Sequence
<220>
   <223> peptide
<400> 642
   Cys Ser Thr Ile Arg Ala Pro Phe Ala Ser Ile Val Ala Ser Ile Gly
   1 5 10 15
   Gly Gln
<210> 643
   <211> 18
   <212> PRT
   <213> Artificial Sequence
<220>
   <223> peptide
<400> 643
   Cys Ser Thr Thr Lys Ala Pro Phe Ala Ser Ile Val Ala Asp Ile Gly
   1 5 10 15
   Gly Gln
<210> 644
   <211> 18
   <212> PRT
   <213> Artificial Sequence
<220>
   <223> peptide
<400> 644
   Cys Ser Thr Ile Asn Ala Pro Phe Ala Ser Ile Val Ala Gly Ile Ser
   1 5 10 15
   Gly Gln
<210> 645
   <211> 18
   <212> PRT
   <213> Artificial Sequence
<220>
   <223> peptide
<400> 645
   Cys Ser Thr Ile Asn Val Pro Tyr Ala Asn Ile Asp Ala Gly Ile Gly
   1 5 10 15
   Gly Gln
<210> 646
   <211> 20
   <212> PRT
   <213> Artificial Sequence
<220>
   <223> peptide
<400> 646
   Pro Gln Gln Pro Phe Pro Leu Gln Pro Gln Gln Ser Phe Leu Trp Gln
   1 5 10 15
   Ser Gln Gln Pro
   20
<210> 647
   <211> 20
   <212> PRT
   <213> Artificial Sequence
<220>
   <223> peptide
<400> 647
   Pro Gln Gln Ser Phe Leu Trp Gln Ser Gln Gln Pro Phe Leu Gln Gln
   1 5 10 15
   Pro Gln Gln Pro
   20
<210> 648
   <211> 20
   <212> PRT
   <213> Artificial Sequence
<220>
   <223> peptide
<400> 648
   Ser Gln Gln Pro Phe Leu Gln Gln Pro Gln Gln Pro Ser Pro Gln Pro
   1 5 10 15
   Gln Gln Val Val
   20
<210> 649
   <211> 20
   <212> PRT
   <213> Artificial Sequence
<220>
   <223> peptide
<400> 649
   Pro Gln Gln Pro Ser Pro Gln Pro Gln Gln Val Val Gln Ile Ile Ser
   1 5 10 15
   Pro Ala Thr Pro
   20
<210> 650
   <211> 20
   <212> PRT
   <213> Artificial Sequence
<220>
   <223> peptide
<400> 650
   Gln Gln Val Val Gln Ile Ile Ser Pro Ala Thr Pro Thr Thr Ile Pro
   1 5 10 15
   Ser Ala Gly Lys
   20
<210> 651
   <211> 20
   <212> PRT
   <213> Artificial Sequence
<220>
   <223> peptide
<400> 651
   Pro Ala Thr Pro Thr Thr Ile Pro Ser Ala Gly Lys Pro Thr Ser Ala
   1 5 10 15
   Pro Phe Pro Gln
   20
<210> 652
   <211> 20
   <212> PRT
   <213> Artificial Sequence
<220>
   <223> peptide
<400> 652
   Ser Ala Gly Lys Pro Thr Ser Ala Pro Phe Pro Gln Gln Gln Gln Gln
   1 5 10 15
   His Gln Gln Leu
   20
<210> 653
   <211> 20
   <212> PRT
   <213> Artificial Sequence
<220>
   <223> peptide
<400> 653
   Pro Phe Pro Gln Gln Gln Gln Gln His Gln Gln Leu Ala Gln Gln Gln
   1 5 10 15
   Ile Pro Val Val
   20
<210> 654
   <211> 20
   <212> PRT
   <213> Artificial Sequence
<220>
   <223> peptide
<400> 654
   His Gln Gln Leu Ala Gln Gln Gln Ile Pro Val Val Gln Pro Ser Ile
   1 5 10 15
   Leu Gln Gln Leu
   20
<210> 655
   <211> 20
   <212> PRT
   <213> Artificial Sequence
<220>
   <223> peptide
<400> 655
   Ile Pro Val Val Gln Pro Ser Ile Leu Gln Gln Leu Asn Pro Cys Lys
   1 5 10 15
   Val Phe Leu Gln
   20
<210> 656
   <211> 20
   <212> PRT
   <213> Artificial Sequence
<220>
   <223> peptide
<400> 656
   Leu Gln Gln Leu Asn Pro Cys Lys Val Phe Leu Gln Gln Gln Cys Ser
   1 5 10 15
   Pro Val Ala Met
   20
<210> 657
   <211> 20
   <212> PRT
   <213> Artificial Sequence
<220>
   <223> peptide
<400> 657
   Val Phe Leu Gln Gln Gln Cys Ser Pro Val Ala Met Pro Gln Arg Leu
   1 5 10 15
   Ala Arg Ser Gln
   20
<210> 658
   <211> 20
   <212> PRT
   <213> Artificial Sequence
<220>
   <223> peptide
<400> 658
   Pro Val Ala Met Pro Gln Arg Leu Ala Arg Ser Gln Met Leu Gln Gln
   1               5                   10                  15
   Ser Ser Cys His
               20
<210> 659
   <211> 20
   <212> PRT
   <213> Artificial Sequence
<220>
   <223> peptide
<400> 659
   Ala Arg Ser Gln Met Leu Gln Gln Ser Ser Cys His Val Met Gln Gln
   1               5                   10                  15
   Gln Cys Cys Gln
               20
<210> 660
   <211> 20
   <212> PRT
   <213> Artificial Sequence
<220>
   <223> peptide
<400> 660
   Ser Ser Cys His Val Met Gln Gln Gln Cys Cys Gln Gln Leu Pro Gln
   1               5                   10                  15
   Ile Pro Gln Gln
               20
<210> 661
   <211> 20
   <212> PRT
   <213> Artificial Sequence
<220>
   <223> peptide
<400> 661
   Gln Cys Cys Gln Gln Leu Pro Gln Ile Pro Gln Gln Ser Arg Tyr Gln
   1               5                   10                  15
   Ala Ile Arg Ala
               20
<210> 662
   <211> 20
   <212> PRT
   <213> Artificial Sequence
<220>
   <223> peptide
<400> 662
   Pro Gln Ile Pro Gln Gln Ser Arg Tyr Glu Ala Ile Arg Ala Ile Ile
   1 5 10 15
   Tyr Ser Ile Ile
   20
<210> 663
   <211> 20
   <212> PRT
   <213> Artificial Sequence
<220>
   <223> peptide
<400> 663
   Ile Pro Gln Gln Ser Arg Tyr Gln Ala Ile Arg Ala Ile Ile Tyr Ser
   1 5 10 15
   Ile Ile Leu Gln
   20
<210> 664
   <211> 20
   <212> PRT
   <213> Artificial Sequence
<220>
   <223> peptide
<400> 664
   Ala Ile Arg Ala Ile Ile Tyr Ser Ile Ile Leu Gln Glu Gln Gln Gln
   1 5 10 15
   Val Gln Gly Ser
   20
<210> 665
   <211> 20
   <212> PRT
   <213> Artificial Sequence
<220>
   <223> peptide
<400> 665
   Ile Ile Leu Gln Glu Gln Gln Gln Val Gln Gly Ser Ile Gln Ser Gln
   1 5 10 15
   Gln Gln Gln Pro
   20
<210> 666
   <211> 20
   <212> PRT
   <213> Artificial Sequence
<220>
   <223> peptide
<400> 666
   Val Gln Gly Ser Ile Gln Ser Gln Gln Gln Gln Pro Gln Gln Leu Gly
   1 5 10 15
   Gln Cys Val Ser
   20
<210> 667
   <211> 20
   <212> PRT
   <213> Artificial Sequence
<220>
   <223> peptide
<400> 667
   Gln Gln Gln Pro Gln Gln Leu Gly Gln Cys Val Ser Gln Pro Gln Gln
   1 5 10 15
   Gln Ser Gln Gln
   20
<210> 668
   <211> 20
   <212> PRT
   <213> Artificial Sequence
<220>
   <223> peptide
<400> 668
   Gln Cys Val Ser Gln Pro Gln Gln Gln Ser Gln Gln Gln Leu Gly Gln
   1 5 10 15
   Gln Pro Gln Gln
   20
<210> 669
   <211> 20
   <212> PRT
   <213> Artificial Sequence
<220>
   <223> peptide
<400> 669
   Gln Ser Gln Gln Gln Leu Gly Gln Gln Pro Gln Gln Gln Gln Leu Ala
   1 5 10 15
   Gln Gly Thr Phe
   20
<210> 670
   <211> 20
   <212> PRT
   <213> Artificial Sequence
<220>
   <223> peptide
<400> 670
   Gln Pro Gln Gln Gln Gln Leu Ala Gln Gly Thr Phe Leu Gln Pro His
   1 5 10 15
   Gln Ile Ala Gln
   20
<210> 671
   <211> 20
   <212> PRT
   <213> Artificial Sequence
<220>
   <223> peptide
<400> 671
   Gln Gly Thr Phe Leu Gln Pro His Gln Ile Ala Gln Leu Glu Val Met
   1 5 10 15
   Thr Ser Ile Ala
   20
<210> 672
   <211> 20
   <212> PRT
   <213> Artificial Sequence
<220>
   <223> peptide
<400> 672
   Gln Ile Ala Gln Leu Glu Val Met Thr Ser Ile Ala Leu Arg Ile Leu
   1 5 10 15
   Pro Thr Met Cys
   20
<210> 673
   <211> 20
   <212> PRT
   <213> Artificial Sequence
<220>
   <223> peptide
<400> 673
   Thr Ser Ile Ala Leu Arg Ile Leu Pro Thr Met Cys Ser Val Asn Val
   1               5                   10                  15
   Pro Leu Tyr Arg
               20
<210> 674
   <211> 20
   <212> PRT
   <213> Artificial Sequence
<220>
   <223> peptide
<400> 674
   Pro Thr Met Cys Ser Val Asn Val Pro Leu Tyr Arg Thr Thr Thr Ser
   1               5                   10                  15
   Val Pro Phe Gly
               20
<210> 675
   <211> 20
   <212> PRT
   <213> Artificial Sequence
<220>
   <223> peptide
<400> 675
   Pro Leu Tyr Arg Thr Thr Thr Ser Val Pro Phe Gly Val Gly Thr Gly
   1               5                   10                  15
   Val Gly Ala Tyr
               20
<210> 676
   <211> 20
   <212> PRT
   <213> Artificial Sequence
<220>
   <223> peptide
<400> 676
   Thr Ile Thr Arg Thr Phe Pro Ile Pro Thr Ile Ser Ser Asn Asn Asn
   1 5 10 15
   His His Phe Arg
   20
<210> 677
   <211> 20
   <212> PRT
   <213> Artificial Sequence
<220>
   <223> peptide
<400> 677
   Pro Thr Ile Ser Ser Asn Asn Asn His His Phe Arg Ser Asn Ser Asn
   1 5 10 15
   His His Phe His
   20
<210> 678
   <211> 20
   <212> PRT
   <213> Artificial Sequence
<220>
   <223> peptide
<400> 678
   His His Phe Arg Ser Asn Ser Asn His His Phe His Ser Asn Asn Asn
   1 5 10 15
   Gln Phe Tyr Arg
   20
<210> 679
   <211> 20
   <212> PRT
   <213> Artificial Sequence
<220>
   <223> peptide
<400> 679
   His His Phe His Ser Asn Asn Asn Gln Phe Tyr Arg Asn Asn Asn Ser
   1 5 10 15
   Pro Gly His Asn
   20
<210> 680
   <211> 20
   <212> PRT
   <213> Artificial Sequence
<220>
   <223> peptide
<400> 680
   Gln Phe Tyr Arg Asn Asn Asn Ser Pro Gly His Asn Asn Pro Leu Asn
   1 5 10 15
   Asn Asn Asn Ser
   20
<210> 681
   <211> 20
   <212> PRT
   <213> Artificial Sequence
<220>
   <223> peptide
<400> 681
   Pro Gly His Asn Asn Pro Leu Asn Asn Asn Asn Ser Pro Asn Asn Asn
   1 5 10 15
   Ser Pro Ser Asn
   20
<210> 682
   <211> 20
   <212> PRT
   <213> Artificial Sequence
<220>
   <223> peptide
<400> 682
   Asn Asn Asn Ser Pro Asn Asn Asn Ser Pro Ser Asn His His Asn Asn
   1 5 10 15
   Ser Pro Asn Asn
   20
<210> 683
   <211> 20
   <212> PRT
   <213> Artificial Sequence
<220>
   <223> peptide
<400> 683
   Ser Pro Ser Asn His His Asn Asn Ser Pro Asn Asn Asn Phe Gln Tyr
   1 5 10 15
   His Thr His Pro
   20
<210> 684
   <211> 20
   <212> PRT
   <213> Artificial Sequence
<220>
   <223> peptide
<400> 684
   Ser Pro Asn Asn Asn Phe Gln Tyr His Thr His Pro Ser Asn His Lys
   1               5                   10                  15
   Asn Leu Pro His
               20
<210> 685
   <211> 20
   <212> PRT
   <213> Artificial Sequence
<220>
   <223> peptide
<400> 685
   His Thr His Pro Ser Asn His Lys Asn Leu Pro His Thr Asn Asn Ile
   1               5                   10                  15
   Gln Gln Gln Gln
               20
<210> 686
   <211> 20
   <212> PRT
   <213> Artificial Sequence
<220>
   <223> peptide
<400> 686
   Asn Leu Pro His Thr Asn Asn Ile Gln Gln Gln Gln Pro Pro Phe Ser
   1               5                   10                  15
   Gln Gln Gln Gln
               20
<210> 687
   <211> 20
   <212> PRT
   <213> Artificial Sequence
<220>
   <223> peptide
<400> 687
   Gln Gln Gln Gln Pro Pro Phe Ser Gln Gln Gln Gln Pro Pro Phe Ser
   1 5 10 15
   Gln Gln Gln Gln
   20
<210> 688
   <211> 20
   <212> PRT
   <213> Artificial Sequence
<220>
   <223> peptide
<400> 688
   Gln Gln Gln Gln Pro Pro Phe Ser Gln Gln Gln Gln Pro Val Leu Pro
   1 5 10 15
   Gln Gln Ser Pro
   20
<210> 689
   <211> 20
   <212> PRT
   <213> Artificial Sequence
<220>
   <223> peptide
<400> 689
   Gln Gln Gln Gln Pro Val Leu Pro Gln Gln Ser Pro Phe Ser Gln Gln
   1 5 10 15
   Gln Gln Leu Val
   20
<210> 690
   <211> 20
   <212> PRT
   <213> Artificial Sequence
<220>
   <223> peptide
<400> 690
   Gln Gln Ser Pro Phe Ser Gln Gln Gln Gln Leu Val Leu Pro Pro Gln
   1 5 10 15
   Gln Gln Gln Gln
   20
<210> 691
   <211> 20
   <212> PRT
   <213> Artificial Sequence
<220>
   <223> peptide
<400> 691
   Gln Gln Leu Val Leu Pro Pro Gln Gln Gln Gln Gln Gln Leu Val Gln
   1               5                   10                  15
   Gln Gln Ile Pro
               20
<210> 692
   <211> 20
   <212> PRT
   <213> Artificial Sequence
<220>
   <223> peptide
<400> 692
   Gln Gln Gln Gln Gln Leu Val Gln Gln Gln Ile Pro Ile Val Gln Pro
   1               5                   10                  15
   Ser Val Leu Gln
               20
<210> 693
   <211> 20
   <212> PRT
   <213> Artificial Sequence
<220>
   <223> peptide
<400> 693
   Gln Gln Ile Pro Ile Val Gln Pro Ser Val Leu Gln Gln Leu Asn Pro
   1               5                   10                  15
   Cys Lys Val Phe
               20
<210> 694
   <211> 20
   <212> PRT
   <213> Artificial Sequence
<220>
   <223> peptide
<400> 694
   Ser Val Leu Gln Gln Leu Asn Pro Cys Lys Val Phe Leu Gln Gln Gln
   1               5                   10                  15
   Cys Ser Pro Val
               20
<210> 695
   <211> 20
   <212> PRT
   <213> Artificial Sequence
<220>
   <223> peptide
<400> 695
   Cys Lys Val Phe Leu Gln Gln Gln Cys Ser Pro Val Ala Met Pro Gln
   1               5                   10                  15
   Arg Leu Ala Arg
               20
<210> 696
   <211> 20
   <212> PRT
   <213> Artificial Sequence
<220>
   <223> peptide
<400> 696
   Cys Ser Pro Val Ala Met Pro Gln Arg Leu Ala Arg Ser Gln Met Trp
   1               5                   10                  15
   Gln Gln Ser Ser
               20
<210> 697
   <211> 20
   <212> PRT
   <213> Artificial Sequence
<220>
   <223> peptide
<400> 697
   Arg Leu Ala Arg Ser Gln Met Trp Gln Gln Ser Ser Cys His Val Met
   1               5                   10                  15
   Gln Gln Gln Cys
               20
<210> 698
   <211> 20
   <212> PRT
   <213> Artificial Sequence
<220>
   <223> peptide
<400> 698
   Gln Gln Ser Ser Cys His Val Met Gln Gln Gln Cys Cys Gln Gln Leu
   1               5                   10                  15
   Gln Gln Ile Pro
               20
<210> 699
   <211> 20
   <212> PRT
   <213> Artificial Sequence
<220>
   <223> peptide
<400> 699
   Gln Gln Gln Cys Cys Gln Gln Leu Gln Gln Ile Pro Glu Gln Ser Arg
   1               5                   10                  15
   Tyr Glu Ala Ile
               20
<210> 700
   <211> 20
   <212> PRT
   <213> Artificial Sequence
<220>
   <223> peptide
<400> 700
   Gln Gln Ile Pro Glu Gln Ser Arg Tyr Glu Ala Ile Arg Ala Ile Ile
   1               5                   10                  15
   Tyr Ser Ile Ile
               20
<210> 701
   <211> 20
   <212> PRT
   <213> Artificial Sequence
<220>
   <223> peptide
<400> 701
   Tyr Glu Ala Ile Arg Ala Ile Ile Tyr Ser Ile Ile Leu Gln Glu Gln
   1               5                   10                  15
   Gln Gln Gly Phe
               20
<210> 702
   <211> 20
   <212> PRT
   <213> Artificial Sequence
<220>
   <223> peptide
<400> 702
   Tyr Ser Ile Ile Leu Gln Glu Gln Gln Gln Gly Phe Val Gln Pro Gln
   1 5 10 15
   Gln Gln Gln Pro
   20
<210> 703
   <211> 20
   <212> PRT
   <213> Artificial Sequence
<220>
   <223> peptide
<400> 703
   Gln Gln Gly Phe Val Gln Pro Gln Gln Gln Gln Pro Gln Gln Ser Gly
   1 5 10 15
   Gln Gly Val Ser
   20
<210> 704
   <211> 20
   <212> PRT
   <213> Artificial Sequence
<220>
   <223> peptide
<400> 704
   Gln Gln Gln Pro Gln Gln Ser Gly Gln Gly Val Ser Gln Ser Gln Gln
   1 5 10 15
   Gln Ser Gln Gln
   20
<210> 705
   <211> 20
   <212> PRT
   <213> Artificial Sequence
<220>
   <223> peptide
<400> 705
   Gln Gly Val Ser Gln Ser Gln Gln Gln Ser Gln Gln Gln Leu Gly Gln
   1 5 10 15
   Cys Ser Phe Gln
   20
<210> 706
   <211> 20
   <212> PRT
   <213> Artificial Sequence
<220>
   <223> peptide
<400> 706
   Gln Ser Gln Gln Gln Leu Gly Gln Cys Ser Phe Gln Gln Pro Gln Gln
   1 5 10 15
   Gln Leu Gly Gln
   20
<210> 707
   <211> 20
   <212> PRT
   <213> Artificial Sequence
<220>
   <223> peptide
<400> 707
   Cys Ser Phe Gln Gln Pro Gln Gln Gln Leu Gly Gln Gln Pro Gln Gln
   1 5 10 15
   Gln Gln Gln Gln
   20
<210> 708
   <211> 20
   <212> PRT
   <213> Artificial Sequence
<220>
   <223> peptide
<400> 708
   Gln Leu Gly Gln Gln Pro Gln Gln Gln Gln Gln Gln Gln Val Leu Gln
   1 5 10 15
   Gly Thr Phe Leu
   20
<210> 709
   <211> 20
   <212> PRT
   <213> Artificial Sequence
<220>
   <223> peptide
<400> 709
   Gln Gln Gln Gln Gln Val Leu Gln Gly Thr Phe Leu Gln Pro His Gln
   1 5 10 15
   Ile Ala His Leu
   20
<210> 710
   <211> 20
   <212> PRT
   <213> Artificial Sequence
<220>
   <223> peptide
<400> 710
   Gly Thr Phe Leu Gln Pro His Gln Ile Ala His Leu Glu Ala Val Thr
   1 5 10 15
   Ser Ile Ala Leu
   20
<210> 711
   <211> 20
   <212> PRT
   <213> Artificial Sequence
<220>
   <223> peptide
<400> 711
   Ile Ala His Leu Glu Ala Val Thr Ser Ile Ala Leu Arg Thr Leu Pro
   1 5 10 15
   Thr Met Cys Ser
   20
<210> 712
   <211> 20
   <212> PRT
   <213> Artificial Sequence
<220>
   <223> peptide
<400> 712
   Ser Ile Ala Leu Arg Thr Leu Pro Thr Met Cys Ser Val Asn Val Pro
   1 5 10 15
   Leu Tyr Ser Ala
   20
<210> 713
   <211> 20
   <212> PRT
   <213> Artificial Sequence
<220>
   <223> peptide
<400> 713
   Thr Met Cys Ser Val Asn Val Pro Leu Tyr Ser Ala Thr Thr Ser Val
   1 5 10 15
   Pro Phe Gly Val
   20
<210> 714
   <211> 19
   <212> PRT
   <213> Artificial Sequence
<220>
   <223> peptide
<400> 714
   Leu Tyr Ser Ala Thr Thr Ser Val Pro Phe Gly Val Gly Thr Gly Val
   1 5 10 15
   Gly Ala Tyr
<210> 715
   <211> 20
   <212> PRT
   <213> Artificial Sequence
<220>
   <223> peptide
<400> 715
   Ser Cys Ile Ser Gly Leu Glu Arg Pro Trp Gln Gln Gln Pro Leu Pro
   1 5 10 15
   Pro Gln Gln Ser
   20
<210> 716
   <211> 20
   <212> PRT
   <213> Artificial Sequence
<220>
   <223> peptide
<400> 716
   Pro Trp Gln Gln Gln Pro Leu Pro Pro Gln Gln Ser Phe Ser Gln Gln
   1 5 10 15
   Pro Pro Phe Ser
   20
<210> 717
   <211> 20
   <212> PRT
   <213> Artificial Sequence
<220>
   <223> peptide
<400> 717
   Pro Gln Gln Ser Phe Ser Gln Gln Pro Pro Phe Ser Gln Gln Gln Gln
   1               5                   10                  15
   Gln Pro Leu Pro
               20
<210> 718
   <211> 20
   <212> PRT
   <213> Artificial Sequence
<220>
   <223> peptide
<400> 718
   Pro Pro Phe Ser Gln Gln Gln Gln Gln Pro Leu Pro Gln Gln Pro Ser
   1               5                   10                  15
   Phe Ser Gln Gln
               20
<210> 719
   <211> 20
   <212> PRT
   <213> Artificial Sequence
<220>
   <223> peptide
<400> 719
   Gln Pro Leu Pro Gln Gln Pro Ser Phe Ser Gln Gln Gln Pro Pro Phe
   1               5                   10                  15
   Ser Gln Gln Gln
               20
<210> 720
   <211> 20
   <212> PRT
   <213> Artificial Sequence
<220>
   <223> peptide
<400> 720
   Phe Ser Gln Gln Gln Pro Pro Phe Ser Gln Gln Gln Pro Ile Leu Ser
   1 5 10 15
   Gln Gln Pro Pro
   20
<210> 721
   <211> 20
   <212> PRT
   <213> Artificial Sequence
<220>
   <223> peptide
<400> 721
   Ser Gln Gln Gln Pro Ile Leu Ser Gln Gln Pro Pro Phe Ser Gln Gln
   1 5 10 15
   Gln Gln Pro Val
   20
<210> 722
   <211> 20
   <212> PRT
   <213> Artificial Sequence
<220>
   <223> peptide
<400> 722
   Ala Thr Ala Ala Arg Glu Leu Asn Pro Ser Asn Lys Glu Leu Gln Ser
   1 5 10 15
   Pro Gln Gln Ser
   20
<210> 723
   <211> 20
   <212> PRT
   <213> Artificial Sequence
<220>
   <223> peptide
<400> 723
   Pro Ser Asn Lys Glu Leu Gln Ser Pro Gln Gln Ser Phe Ser Tyr Gln
   1 5 10 15
   Gln Gln Pro Phe
   20
<210> 724
   <211> 20
   <212> PRT
   <213> Artificial Sequence
<220>
   <223> peptide
<400> 724
   Pro Gln Gln Ser Phe Ser Tyr Gln Gln Gln Pro Phe Pro Gln Gln Pro
   1 5 10 15
   Tyr Pro Gln Gln
   20
<210> 725
   <211> 20
   <212> PRT
   <213> Artificial Sequence
<220>
   <223> peptide
<400> 725
   Gln Gln Pro Phe Pro Gln Gln Pro Tyr Pro Gln Gln Pro Tyr Pro Ser
   1 5 10 15
   Gln Gln Pro Tyr
   20
<210> 726
   <211> 20
   <212> PRT
   <213> Artificial Sequence
<220>
   <223> peptide
<400> 726
   Tyr Pro Gln Gln Pro Tyr Pro Ser Gln Gln Pro Tyr Pro Ser Gln Gln
   1 5 10 15
   Pro Phe Pro Thr
   20
<210> 727
   <211> 20
   <212> PRT
   <213> Artificial Sequence
<220>
   <223> peptide
<400> 727
   Gln Gln Pro Tyr Pro Ser Gln Gln Pro Phe Pro Thr Pro Gln Gln Gln
   1 5 10 15
   Phe Pro Glu Gln
   20
<210> 728
   <211> 20
   <212> PRT
   <213> Artificial Sequence
<220>
   <223> peptide
<400> 728
   Pro Phe Pro Thr Pro Gln Gln Gln Phe Pro Glu Gln Ser Gln Gln Pro
   1               5                   10                  15
   Phe Thr Gln Pro
               20
<210> 729
   <211> 20
   <212> PRT
   <213> Artificial Sequence
<220>
   <223> peptide
<400> 729
   Phe Pro Glu Gln Ser Gln Gln Pro Phe Thr Gln Pro Gln Gln Pro Thr
   1               5                   10                  15
   Pro Ile Gln Pro
               20
<210> 730
   <211> 20
   <212> PRT
   <213> Artificial Sequence
<220>
   <223> peptide
<400> 730
   Phe Thr Gln Pro Gln Gln Pro Thr Pro Ile Gln Pro Gln Gln Pro Phe
   1               5                   10                  15
   Pro Gln Gln Pro
               20
<210> 731
   <211> 20
   <212> PRT
   <213> Artificial Sequence
<220>
   <223> peptide
<400> 731
   Pro Ile Gln Pro Gln Gln Pro Phe Pro Gln Gln Pro Gln Gln Pro Gln
   1 5 10 15
   Gln Pro Phe Pro
   20
<210> 732
   <211> 20
   <212> PRT
   <213> Artificial Sequence
<220>
   <223> peptide
<400> 732
   Pro Gln Gln Pro Gln Gln Pro Gln Gln Pro Phe Pro Gln Pro Gln Gln
   1 5 10 15
   Pro Phe Pro Trp
   20
<210> 733
   <211> 20
   <212> PRT
   <213> Artificial Sequence
<220>
   <223> peptide
<400> 733
   Gln Pro Phe Pro Gln Pro Gln Gln Pro Phe Pro Trp Gln Pro Gln Gln
   1 5 10 15
   Pro Phe Pro Gln
   20
<210> 734
   <211> 20
   <212> PRT
   <213> Artificial Sequence
<220>
   <223> peptide
<400> 734
   Pro Phe Pro Trp Gln Pro Gln Gln Pro Phe Pro Gln Thr Gln Gln Ser
   1 5 10 15
   Phe Pro Leu Gln
   20
<210> 735
   <211> 20
   <212> PRT
   <213> Artificial Sequence
<220>
   <223> peptide
<400> 735
   Pro Phe Pro Gln Thr Gln Gln Ser Phe Pro Leu Gln Pro Gln Gln Pro
   1 5 10 15
   Phe Pro Gln Gln
   20
<210> 736
   <211> 20
   <212> PRT
   <213> Artificial Sequence
<220>
   <223> peptide
<400> 736
   Phe Pro Leu Gln Pro Gln Gln Pro Phe Pro Gln Gln Pro Gln Gln Pro
   1 5 10 15
   Phe Pro Gln Pro
   20
<210> 737
   <211> 20
   <212> PRT
   <213> Artificial Sequence
<220>
   <223> peptide
<400> 737
   Phe Pro Gln Gln Pro Gln Gln Pro Phe Pro Gln Pro Gln Leu Pro Phe
   1 5 10 15
   Pro Gln Gln Ser
   20
<210> 738
   <211> 20
   <212> PRT
   <213> Artificial Sequence
<220>
   <223> peptide
<400> 738
   Phe Pro Gln Pro Gln Leu Pro Phe Pro Gln Gln Ser Glu Gln Ile Ile
   1 5 10 15
   Pro Gln Gln Leu
   20
<210> 739
   <211> 20
   <212> PRT
   <213> Artificial Sequence
<220>
   <223> peptide
<400> 739
   Pro Gln Gln Ser Glu Gln Ile Ile Pro Gln Gln Leu Gln Gln Pro Phe
   1               5                   10                  15
   Pro Leu Gln Pro
               20
<210> 740
   <211> 20
   <212> PRT
   <213> Artificial Sequence
<220>
   <223> peptide
<400> 740
   Pro Gln Gln Leu Gln Gln Pro Phe Pro Leu Gln Pro Gln Gln Pro Phe
   1               5                   10                  15
   Pro Gln Gln Pro
               20
<210> 741
   <211> 20
   <212> PRT
   <213> Artificial Sequence
<220>
   <223> peptide
<400> 741
   Pro Leu Gln Pro Gln Gln Pro Phe Pro Gln Gln Pro Gln Gln Pro Phe
   1               5                   10                  15
   Pro Gln Pro Gln
               20
<210> 742
   <211> 20
   <212> PRT
   <213> Artificial Sequence
<220>
   <223> peptide
<400> 742
   Pro Gln Gln Pro Gln Gln Pro Phe Pro Gln Pro Gln Gln Pro Ile Pro
   1 5 10 15
   Val Gln Pro Gln
   20
<210> 743
   <211> 20
   <212> PRT
   <213> Artificial Sequence
<220>
   <223> peptide
<400> 743
   Pro Gln Pro Gln Gln Pro Ile Pro Val Gln Pro Gln Gln Ser Phe Pro
   1 5 10 15
   Gln Gln Ser Gln
   20
<210> 744
   <211> 20
   <212> PRT
   <213> Artificial Sequence
<220>
   <223> peptide
<400> 744
   Val Gln Pro Gln Gln Ser Phe Pro Gln Gln Ser Gln Gln Ser Gln Gln
   1 5 10 15
   Pro Phe Ala Gln
   20
<210> 745
   <211> 20
   <212> PRT
   <213> Artificial Sequence
<220>
   <223> peptide
<400> 745
   Gln Gln Ser Gln Gln Ser Gln Gln Pro Phe Ala Gln Pro Gln Gln Leu
   1 5 10 15
   Phe Pro Glu Leu
   20
<210> 746
   <211> 20
   <212> PRT
   <213> Artificial Sequence
<220>
   <223> peptide
<400> 746
   Pro Phe Ala Gln Pro Gln Gln Leu Phe Pro Glu Leu Gln Gln Pro Ile
   1 5 10 15
   Pro Gln Gln Pro
   20
<210> 747
   <211> 20
   <212> PRT
   <213> Artificial Sequence
<220>
   <223> peptide
<400> 747
   Phe Pro Glu Leu Gln Gln Pro Ile Pro Gln Gln Pro Gln Gln Pro Phe
   1 5 10 15
   Pro Leu Gln Pro
   20
<210> 748
   <211> 20
   <212> PRT
   <213> Artificial Sequence
<220>
   <223> peptide
<400> 748
   Pro Gln Gln Pro Gln Gln Pro Phe Pro Leu Gln Pro Gln Gln Pro Phe
   1 5 10 15
   Pro Gln Gln Pro
   20
<210> 749
   <211> 20
   <212> PRT
   <213> Artificial Sequence
<220>
   <223> peptide
<400> 749
   Pro Leu Gln Pro Gln Gln Pro Phe Pro Gln Gln Pro Gln Gln Pro Phe
   1 5 10 15
   Pro Gln Gln Pro
   20
<210> 750
   <211> 20
   <212> PRT
   <213> Artificial Sequence
<220>
   <223> peptide
<400> 750
   Pro Gln Gln Pro Gln Gln Pro Phe Pro Gln Gln Pro Gln Gln Ser Phe
   1               5                   10                  15
   Pro Gln Gln Pro
               20
<210> 751
   <211> 20
   <212> PRT
   <213> Artificial Sequence
<220>
   <223> peptide
<400> 751
   Pro Gln Gln Pro Gln Gln Ser Phe Pro Gln Gln Pro Gln Gln Pro Tyr
   1               5                   10                  15
   Pro Gln Gln Gln
               20
<210> 752
   <211> 20
   <212> PRT
   <213> Artificial Sequence
<220>
   <223> peptide
<400> 752
   Pro Gln Gln Pro Gln Gln Pro Tyr Pro Gln Gln Gln Pro Tyr Gly Ser
   1               5                   10                  15
   Ser Leu Thr Ser
               20
<210> 753
   <211> 16
   <212> PRT
   <213> Artificial Sequence
<220>
   <223> peptide
<400> 753
   Pro Gln Gln Gln Pro Tyr Gly Ser Ser Leu Thr Ser Ile Gly Gly Gln
   1 5 10 15
<210> 754
   <211> 20
   <212> PRT
   <213> Artificial Sequence
<220>
   <223> peptide
<400> 754
   Ala Arg Gln Leu Asn Pro Ser Asp Gln Glu Leu Gln Ser Pro Gln Gln
   1 5 10 15
   Leu Tyr Pro Gln
   20
<210> 755
   <211> 20
   <212> PRT
   <213> Artificial Sequence
<220>
   <223> peptide
<400> 755
   Gln Glu Leu Gln Ser Pro Gln Gln Leu Tyr Pro Gln Gln Pro Tyr Pro
   1 5 10 15
   Gln Gln Pro Tyr
   20
<210> 756
   <211> 20
   <212> PRT
   <213> Artificial Sequence
<220>
   <223> peptide
<400> 756
   Ser Arg Leu Leu Ser Pro Arg Gly Lys Glu Leu His Thr Pro Gln Glu
   1 5 10 15
   Gln Phe Pro Gln
   20
<210> 757
   <211> 20
   <212> PRT
   <213> Artificial Sequence
<220>
   <223> peptide
<400> 757
   Lys Glu Leu His Thr Pro Gln Glu Gln Phe Pro Gln Gln Gln Gln Phe
   1 5 10 15
   Pro Gln Pro Gln
   20
<210> 758
   <211> 16
   <212> PRT
   <213> Artificial Sequence
<220>
   <223> peptide
<400> 758
   Gln Phe Pro Gln Gln Gln Gln Phe Pro Gln Pro Gln Gln Phe Pro Gln
   1 5 10 15

## Description

The invention relates to epitopes useful in the diagnosis and therapy of coeliac disease, including diagnostics, therapeutics, kits, and methods of using the foregoing.

An immune reaction to gliadin (a component of gluten) in the diet causes coeliac disease. It is known that immune responses in the intestinal tissue preferentially respond to gliadin which has been modified by an intestinal transglutaminase. Coeliac disease is diagnosed by detection of anti-endomysial antibodies, but this requires confirmation by the finding of a lymphocytic inflammation in intestinal biopsies. The taking of such a biopsy is inconvenient for the patient.

Investigators have previously assumed that only intestinal T cell responses provide an accurate indication of the immune response against gliadins. Therefore they have concentrated on the investigation of T cell responses in intestinal tissue¹. Gliadin epitopes which require transglutaminase modification (before they are recognised by the immune system) are known².

The inventors have found the immunodominant T cell A-gliadin epitope recognised by the immune system in coeliac disease, and have shown that this is recognised by T cells in the peripheral blood of individuals with coeliac disease (see WO 01/25793). Such T cells were found to be present at high enough frequencies to be detectable without restimulation (i.e. a 'fresh response' detection system could be used). The epitope was identified using a non-T cell cloning based method which provided a more accurate reflection of the epitopes being recognised. The immunodominant epitope requires transglutaminase modification (causing substitution of a particular glutamine to glutamate) before immune system recognition.

Based on this work the inventors have developed a test which can be used to diagnose coeliac disease at an early stage. The test may be carried out on a sample from peripheral blood and therefore an intestinal biopsy is not required. The test is more sensitive than the antibody tests which are currently being used.

The present disclosure thus provides a method of diagnosing coeliac disease, or susceptibility to coeliac disease, in an individual comprising:
(a) contacting a sample from the host with an agent selected from (i) the epitope comprising sequence which is: SEQ ID NO:1 (PQPELPY)or SEQ ID NO:2 (QLQPFPQPELPYPQPQS), or an equivalent sequence from a naturally occurring homologue of the gliadin represented by SEQ ID NO:3, (ii) an epitope comprising sequence comprising: SEQ ID NO:1, or an equivalent sequence from a naturally occurring homologue of the gliadin represented by SEQ ID NO:3 (shown in Table 1), which epitope is an isolated oligopeptide derived from a gliadin protein, (iii) an analogue of (i) or (ii) which is capable of being recognised by a T cell receptor that recognises (i) or (ii), which in the case of a peptide analogue is not more than 50 amino acids in length, or (iv) a product comprising two or more agents as defined in (i), (ii) or (iii), and (b) determining *in vitro* whether T cells in the sample recognise the agent, recognition by the T cells indicating that the individual has, or is susceptible to, coeliac disease.

Through comprehensive mapping of wheat gliadin T cell epitopes (see Example 13), the inventors have also found epitopes bioactive in coeliac disease in HLA-DQ2+ patients in other wheat gliadins, having similar core sequences (e.g., SEQ ID NOS:18-22) and similar full length sequences (e.g., SEQ ID NOS:31-36), as well as in rye secalins and barley hordeins (e.g., SEQ ID NOS:39-41); see also Tables 20 and 21. Additionally, several epitopes bioactive in coeliac disease in HLA-DQ8+ patients have been identified (e.g., SEQ ID NOS:42-44, 46). This comprehensive mapping thus provides the dominant epitopes recognized by T cells in coeliac patients. Thus, the above-described method and other methods described herein may be performed using any of these additional identified epitopes, and analogues and equivalents thereof; (i) and (ii) herein include these additional epitopes. That is, the agents disclosed herein also include these novel epitopes.

The disclosure also provides use of the agent for the preparation of a diagnostic means for use in a method of diagnosing coeliac disease, or susceptibility to coeliac disease, in an individual, said method comprising determining whether T cells of the individual recognise the agent, recognition by the T cells indicating that the individual has, or is susceptible to, coeliac disease.

The finding of an immunodominant epitope which is modified by transglutaminase (as well as the additional other epitopes defined herein) also allows diagnosis of coeliac disease based on determining whether other types of immune response to this epitope are present. Thus the disclosure also provides a method of diagnosing coeliac disease, or susceptibility to coeliac disease, in an individual comprising determining the presence of an antibody that binds to the epitope in a sample from the individual, the presence of the antibody indicating that the individual has, or is susceptible to, coeliac disease.

The disclosure additionally provides the agent, optionally in association with a carrier, for use in a method of treating or preventing coeliac disease by tolerising T cells which recognise the agent. Also disclosed is an antagonist of a T cell which has a T cell receptor that recognises (i) or (ii), optionally in association with a carrier, for use in a method of treating or preventing coeliac disease by antagonising such T cells. Additionally disclosed is the agent or an analogue that binds an antibody (that binds the agent) for use in a method of treating or preventing coeliac disease in an individual by tolerising the individual to prevent the production of such an antibody.

The disclosure provides a method of determining whether a composition is capable of causing coeliac disease comprising determining whether a protein capable of being modified by a transglutaminase to an oligopeptide sequence as defined above is present in the composition, the presence of the protein indicating that the composition is capable of causing coeliac disease.

The disclosure also provides a mutant gliadin protein whose wild-type sequence can be modified by a transglutaminase to a sequence that comprises an epitope comprising sequence as defined above, but which mutant gliadin protein has been modified in such a way that it does not contain sequence which can be modified by a transglutaminase to a sequence that comprises such an epitope comprising sequence; or a fragment of such a mutant gliadin protein which is at least 15 amino acids long and which comprises sequence which has been modified in said way.

The disclosure also provides a protein that comprises a sequence which is able to bind to a T cell receptor, which T cell receptor recognises the agent, and which sequence is able to cause antagonism of a T cell that carries such a T cell receptor.

Additionally the disclosure provides a food that comprises the proteins defined above.

### SUMMARY OF THE INVENTION

The present invention specifically provides the following:
[1] A peptide for use in a method of therapy, the peptide comprising at least one T-Cell epitope, wherein the peptide is not more than 50 amino acids in length and comprises a sequence obtainable by transglutaminase deamidating the sequence PQQPQQPFP; provided that the peptide:
   (i) is not an epitope that is selected from the group consisting of QQPYPEQPEQPFPQ, QQPFPEQPEQPFPQ, QLPFPEQPEQPFPQ and PFPQPEQPEQPFPQ; and
   (ii) is not a peptide obtained by tissue transglutaminase treatment of the peptide PQQPFPQPQQPQQPFPQSQQ.
[2] A peptide for use according to [1], which comprises the sequence PEQPEQPFP.
[3] A peptide for use in a method of therapy, the peptide comprising at least one T-Cell epitope, wherein the peptide is not more than 50 amino acids in length and comprises a sequence obtainable by transglutaminase deamidating a sequence selected from the group consisting of PQQPQQPQQPFPQPQQPFPW, QQPFPQQPQQPFP, PQQPQQPFPQPQQPIPVQPQ, TQQPQQPFPQQPQQPFPQTQ, PIQPQQPFPQQPQQPQQPFP, PQQPQQPQQPFPQPQQPFPWQP, QQQQPFPQPQQPQQPFPQPQ, QQFPQPQQPQQPFPQQPQQQ, PQQPFPQPQQPQQPFPQPQQ, PQQQFPQPQQPQQPFPQQPQ, QQPQQQFPQPQQPQQPFPQP, QPQQPQQPFPQPQQPQLPFP, QQPFPQQPQQPFPQTQQPQQ, QQPYPQQPQQPFPQTQQPQQ, QLPFPQQPQQPFPQPQQPQQ, QPQQPFPQQPQQPFPQTQQP, TQQPQQPFPQQPQQPFPQTQ, PQQPQQPFPQTQQPQQPFPQ, QQPFPQPQQPQQPFPQLQQP, QQPLPQPQQPQQPFPQSQQP, QQPFPQPQQPQQPFPQSQQP, LQQPQQPLPQPQQPQQPFPQ, PQPQQPQQPFPQQQQPLIQP, FPLQPQQPFPQQPQQPFPQP, FPQQPQQPFPQPQLPFPQQS, PLQPQQPFPQQPQQPFPQPQ, FPELQQPIPQQPQQPFPLQP, PQQPQQPFPLQPQQPFPQQP, PLQPQQPFPQQPQQPFPQQP and PQQPQQPFPQQPQQSFPQQP.
[4] A peptide for use according to [3], which comprises a sequence selected from the group consisting of PEQPEQPEQPFPQPEQPFPW, EQPFPEQPEQPFP, PEQPEQPFPQPEQPIPVQPQ, TEQPEQPFPEQPEQPFPQTQ, PIQPEQPFPEQPEQPEQPFP, PEQPEQPEQPFPQPEQPFPWQP, QQEQPFPQPEQPEQPFPQPQ, QEFPQPEQPEQPFPEQPQQQ, PEQPFPQPEQPEQPFPQPQQ, PEQEFPQPEQPEQPFPEQPQ, EQPEQEFPQPEQPEQPFPQP, QPEQPEQPFPQPEEPELPFP, EQPFPEQPEQPFPQTEQPQQ, EQPYPEQPEQPFPQTEQPQQ, ELPFPEQPEQPFPQPEQPQQ, QPEQPFPEQPEQPFPQTEQP, TEQPEQPFPEQPEQPFPQTQ, PEQPEQPFPQTEQPEQPFPQ, EQPFPQPEQPEQPFPQLEQP, EQPLPQPEQPEQPFPQSEQP, EQPFPQPEQPEQPFPQSEQP, LEQPEQPLPQPEQPEQPFPQ, PQPEQPEQPFPQQEEPLIQP, FPLQPEQPFPEQPEQPFPQP, FPEQPEQPFPEPELPFPQQS, PLQPEQPFPEQPEQPFPQPQ, FPELEQPIPEQPEQPFPLQP, PEQPEQPFPLQPEQPFPEQP, PLQPEQPFPEQPEQPFPEQP and PEQPEQPFPEQPEQSFPEQP.
[5] A peptide for use according to any one of [1] to [4], which comprises a wheat epitope, a barley epitope, and a rye epitope.
[6] The peptide as defined in any one of [1] to [5], for use in treating or preventing coeliac disease; provided that the peptide: (i) is not an epitope that is selected from the group consisting of QQPYPEQPEQPFPQ, QQPFPEQPEQPFPQ, QLPFPEQPEQPFPQ and PFPQPEQPEQPFPQ; and (ii) is not a peptide obtained by tissue transglutaminase treatment of the peptide PQQPFPQPQQPQQPFPQSQQ.
[7] A pharmaceutical composition comprising: (a) a peptide as defined in any one of [1]-[5], (b) a pharmaceutically acceptable carrier or diluent, and, optionally, (c) a peptide comprising at least one epitope comprising a sequence selected from PQPELPY (SEQ ID NO:1) and QLQPFPQPELPYPQPQS (SEQ ID NO:2); provided that the peptide in (a): (i) is not an epitope that is selected from the group consisting of QQPYPEQPEQPFPQ, QQPFPEQPEQPFPQ, QLPFPEQPEQPFPQ and PFPQPEQPEQPFPQ; and (ii) is not a peptide obtained by tissue transglutaminase treatment of the peptide PQQPFPQPQQPQQPFPQSQQ.
[8] An isolated peptide comprising at least one T-Cell epitope, wherein the peptide is not more than 50 amino acids in length and comprises a sequence obtainable by transglutaminase deamidating a sequence selected from the group consisting of PQQPQQPQQPFPQPQQPFPW, PQQPQQPFPQPQQPIPVQPQ, TQQPQQPFPQQPQQPFPQTQ, PIQPQQPFPQQPQQPQQPFP, PQQPQQPQQPFPQPQQPFPWQP, QQQQPFPQPQQPQQPFPQPQ, QQFPQPQQPQQPFPQQPQQQ, PQQPFPQPQQPQQPFPQPQQ, PQQQFPQPQQPQQPFPQQPQ, QQPQQQFPQPQQPQQPFPQP, QPQQPQQPFPQPQQPQLPFP, QQPFPQQPQQPFPQTQQPQQ, QQPYPQQPQQPFPQTQQPQQ, QLPFPQQPQQPFPQPQQPQQ, QPQQPFPQQPQQPFPQTQQP, TQQPQQPFPQQPQQPFPQTQ, PQQPQQPFPQTQQPQQPFPQ, QQPFPQPQQPQQPFPQLQQP, QQPLPQPQQPQQPFPQSQQP, QQPFPQPQQPQQPFPQSQQP, LQQPQQPLPQPQQPQQPFPQ, PQPQQPQQPFPQQQQPLIQP, FPLQPQQPFPQQPQQPFPQP, FPQQPQQPFPQPQLPFPQQS, PLQPQQPFPQQPQQPFPQPQ, FPELQQPIPQQPQQPFPLQP, PQQPQQPFPLQPQQPFPQQP, PLQPQQPFPQQPQQPFPQQP and PQQPQQPFPQQPQQSFPQQP.
[9] A peptide according to [8], which comprises a sequence selected from the group consisting of PEQPEQPEQPFPQPEQPFPW, PEQPEQPFPQPEQPIPVQPQ, TEQPEQPFPEQPEQPFPQTQ, PIQPEQPFPEQPEQPEQPFP, PEQPEQPEQPFPQPEQPFPWQP, QQEQPFPQPEQPEQPFPQPQ, QEFPQPEQPEQPFPEQPQQQ, PEQPFPQPEQPEQPFPQPQQ, PEQEFPQPEQPEQPFPEQPQ, EQPEQEFPQPEQPEQPFPQP, QPEQPEQPFPQPEEPELPFP, EQPFPEQPEQPFPQTEQPQQ, EQPYPEQPEQPFPQTEQPQQ, ELPFPEQPEQPFPQPEQPQQ, QPEQPFPEQPEQPFPQTEQP, TEQPEQPFPEQPEQPFPQTQ, PEQPEQPFPQTEQPEQPFPQ, EQPFPQPEQPEQPFPQLEQP, EQPLPQPEQPEQPFPQSEQP, EQPFPQPEQPEQPFPQSEQP, LEQPEQPLPQPEQPEQPFPQ, PQPEQPEQPFPQQEEPLIQP, FPLQPEQPFPEQPEQPFPQP, FPEQPEQPFPEPELPFPQQS, PLQPEQPFPEQPEQPFPQPQ, FPELEQPIPEQPEQPFPLQP, PEQPEQPFPLQPEQPFPEQP, PLQPEQPFPEQPEQPFPEQP and PEQPEQPFPEQPEQSFPEQP.
[10] A peptide according to [8] or [9], which comprises a wheat epitope, a barley epitope, and a rye epitope.
[11] A composition comprising a first HLA-DQ2-restricted agent and a second HLA-DQ8-restricted agent, wherein the composition comprises a peptide as defined in any one of [8] to [10].
[12] A composition comprising a peptide comprising a wheat epitope, a peptide comprising a barley epitope and/or a peptide comprising a rye epitope, wherein the composition comprises a peptide as defined in any one of [8] to [10].
[13] A composition comprising: (a) a peptide as defined in any one of [8] to [10]; and (b) a peptide comprising at least one epitope comprising a sequence selected from PQPELPY (SEQ ID NO:1) and QLQPFPQPELPYPQPQS (SEQ ID NO:2).
[14] A method of diagnosing coeliac disease, or susceptibility to coeliac disease, in an individual comprising:
   contacting a sample from the host with at least one peptide as defined in any one of [8] to [10]; and
   determining *in vitro* whether T cells in the sample recognise the peptide; recognition by the T cells indicating that the individual has, or is susceptible to, coeliac disease.
[15] A kit for carrying out a method according to [14], comprising a peptide as defined in any one of [8] to [10] and a means to detect the recognition of the peptide by a T cell, wherein optionally the means to detect recognition comprises an antibody to IFN-γ.

### BRIEF DESCRIPTION OF THE DRAWING

The present disclosure is illustrated by the accompanying drawings in which:

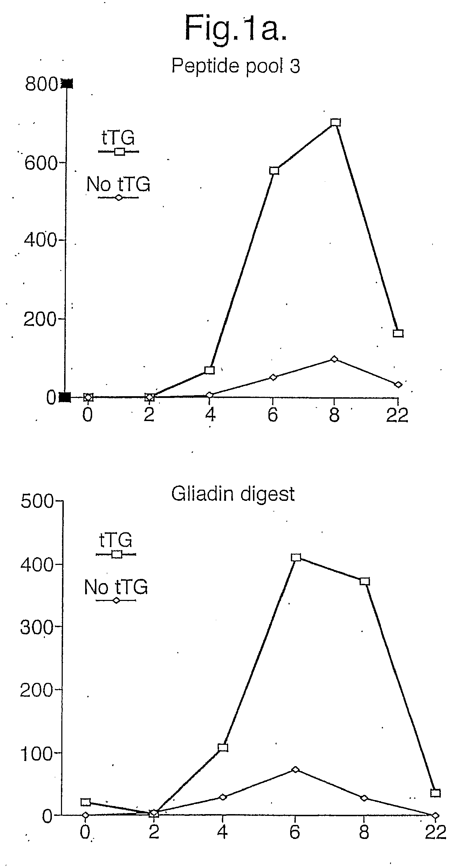

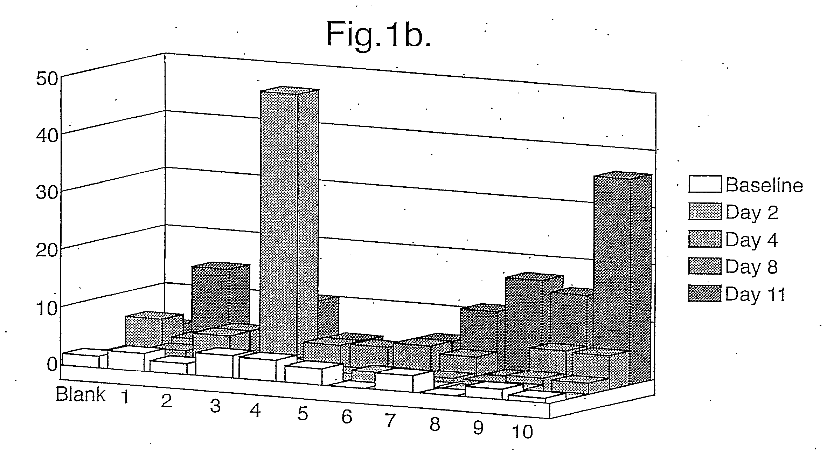

Figure 1 shows freshly isolated PBMC (peripheral blood mononuclear cell) IFNγ ELISPOT responses (vertical axis shows spot forming cells per 10⁶ PBMC) to transglutaminase (tTG)-treated and untreated peptide pool 3 (each peptide 10 µg/ml) including five overlapping 15mers spanning A-gliadin 51-85 (see Table 1) and a-chymotrypsin-digested gliadin (40 µg/ml) in coeliac disease Subject 1, initially in remission following a gluten free diet then challenged with 200g bread daily for three days from day 1 (a). PBMC IFNγ ELISPOT responses by Subject 2 to tTG-treated A-gliadin peptide pools 1-10 spanning the complete A-gliadin protein during ten day bread challenge (b). The horizontal axis shows days after commencing bread.

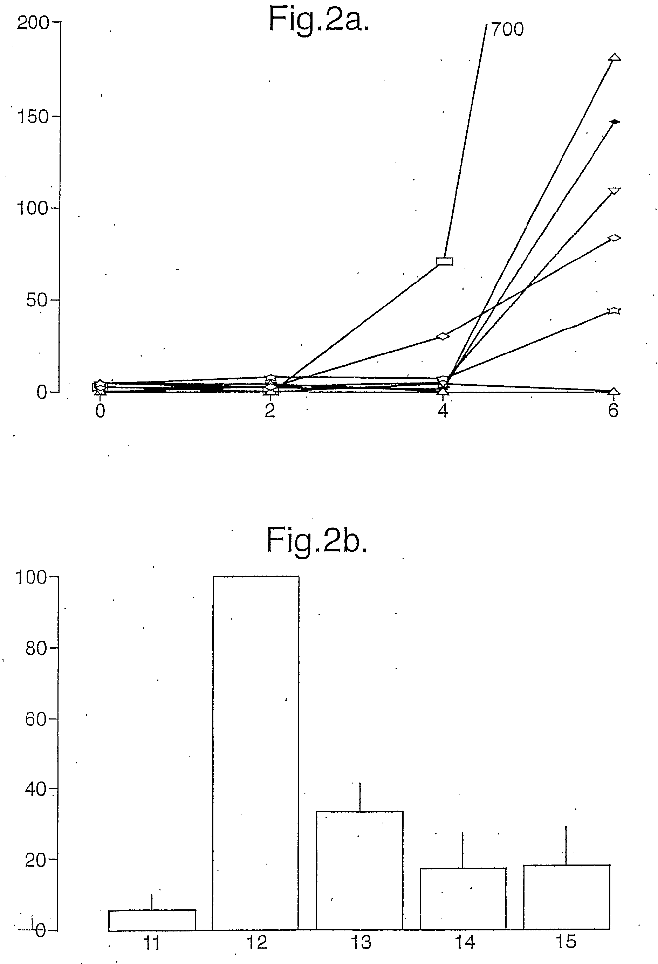

Figure 2 shows PBMC IFNγ ELISPOT responses to tTG-treated peptide pool 3 (spanning A-gliadin 51-85) in 7 individual coeliac disease subjects (vertical axis shows spot forming cells per 10⁶ PBMC), initially in remission on gluten free diet, challenged with bread for three days (days 1 to 3). The horizontal axis shows days after commencing bread. (a). PBMC IFNγ Elispot responses to tTG-treated overlapping 15mer peptides included in pool 3; bars represent the mean (± SEM) response to individual peptides (10 µg/ml) in 6 Coeliac disease subjects on day 6 or 7(b). (In individual subjects, ELISPOT responses to peptides were calculated as a % of response elicited by peptide 12 - as shown by the vertical axis.)
Figure 3 shows PBMC IFNγ ELISPOT responses to tTG-treated truncations of A-gliadin 56-75 (0.1 µM). Bars represent the mean (± SEM) in 5 Coeliac disease subjects. (In individual subjects, responses were calculated as the % of the maximal response elicited by any of the peptides tested.)
Figure 4 shows how the minimal structure of the dominant A-gliadin epitope was mapped using tTG-treated 7-17mer A-gliadin peptides (0.1 µM) including the sequence, PQPQLPY (SEQ ID NO:4) (A-gliadin 62-68) (a), and the same peptides without tTG treatment but with the substitution Q→E65 (b). Each line represents PBMC IFNγ ELISPOT responses in each of three Coeliac disease subjects on day 6 or 7 after bread was ingested on days 1-3. (In individual subjects, ELISPOT responses were calculated as a % of the response elicited by the 17mer, A-gliadin 57-73.)
Figure 5 shows the amino acids that were deamidated by tTG. A-gliadin 56-75 LQLQPFPQPQLPYPQPQSFP (SEQ ID NO:5) (0.1 µM) was incubated with tTG (50 µg/ml) at 37°C for 2 hours. A single product was identified and purified by reverse phase HPLC. Amino acid analysis allowed % deamidation (Q→E) of each Gln residue in A-gliadin 56-75 attributable to tTG to be calculated (vertical axis).
Figure 6 shows the effect of substituting Q→E in A-gliadin 57-73 at other positions in addition to Q65 using the 17mers: QLQPFPQPELPYPQPES (SEQ ID NO:6) (E57,65), QLQPFPQPELPYPQPES (SEQ ID NO:7) (E65,72), ELQPFPQPELPYPQPES (SEQ ID NO:8) (E57, 65, 72), and QLQPFPQPELPYPQPQS (SEQ ID NO:2) (E65) in three Coeliac disease subjects on day 6 or 7 after bread was ingested on days 1-3. Vertical axis shows % of the E65 response.
Figure 7 shows that tTG treated A-gliadin 56-75 (0.1 µM) elicited IFN-g ELISPOT responses in (a) CD4 and CD8 magnetic bead depleted PBMC. (Bars represent CD4 depleted PBMC responses as a % of CD8 depleted PBMC responses; spot forming cells per million CD8 depleted PBMC were: Subject 4: 29, and Subject 6: 535). (b) PBMC IFNγ ELISPOT responses (spot forming cells/million PBMC) after incubation with monoclonal antibodies to HLA-DR (L243), -DQ (L2) and -DP (B7.21) (10 µg/ml) 1h prior to tTG-treated 56-75 (0.1 µM) in two coeliac disease subjects homozygous for HLA-DQ a1*0501, b1*0201.
Figure 8 shows the effect of substituting Glu at position 65 for other amino acids in the immunodominant epitope. The vertical axis shows the % response in the 3 subjects in relation to the immunodominant epitope.
Figure 9 shows the immunoreactivity of naturally occurring gliadin peptides (measuring responses from 3 subjects) which contain the sequence PQLPY (SEQ ID NO:12) with (shaded) and without (clear) transglutaminase treatment.

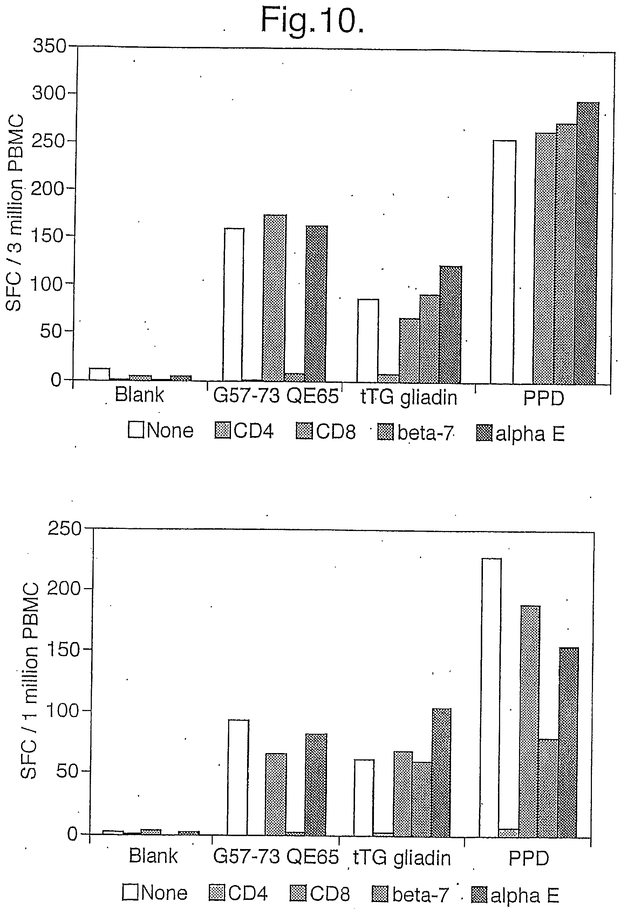

Figure 10 shows CD8, CD4, β₇, and α^{E} -specific immunomagnetic bead depletion of peripheral blood mononuclear cells from two coeliac subjects 6 days after commencing gluten challenge followed by interferon gamma ELISpot. A-gliadin 57-73 QE65 (25mcg/ml), tTG-treated chymotrypsin-digested gliadin (100 mcg/ml) or PPD (10 mcg/ml) were used as antigen.
Figure 11 shows the optimal T cell epitope length.
Figure 12 shows a comparison of A-gliadin 57-73 QE65 with other peptides in a dose response study.
Figure 13 shows a comparison of gliadin and A-gliadin 57-73 QE65 specific responses.
Figure 14 shows the bioactivity of gliadin polymorphisms in coeliac subjects.
Figures 15 and 16 show the defining of the core epitope sequence.
Figures 17 to 27 show the agonist activity of A-gliadin 57-73 QE65 variants.
Figure 28 shows responses in different patient groups.
Figure 29 shows bioactivity of prolamin homologues of A-gliadin 57-73.
Figure 30 shows, for healthy HLA-DQ2 subjects, the change in IFN-gamma ELISpot responses to tTG-deamidated gliadin peptide pools.
Figure 31 shows, for coeliac HLA-DQ2 subjects, the change in IFN-gamma ELISpot responses to tTG-deamidated gliadin peptide pools.
Figure 32 shows individual peptide contributions to "summed" gliadin peptide response.
Figure 33 shows, for coeliac HLA-DQ2/8 subject C08, gluten challenge induced IFNγ ELISpot responses to tTG-deamidated gliadin peptide pools.
Figure 34 shows, for coeliac HLA-DQ2/8 subject C07, gluten challenge induced IFNγ ELISpot responses to tTG-deamidated gliadin peptide pools.
Figure 35 shows, for coeliac HLA-DQ8/7 subject C12, gluten challenge induced IFNγ ELISpot responses to tTG-deamidated gliadin peptide pools.
Figure 36 shows, for coeliac HLA-DQ6/8 subject C11, gluten challenge induced IFNγ ELISpot responses to tTG-deamidated gliadin peptide pools.

### Detailed Description of the Invention

The term "coeliac disease" encompasses a spectrum of conditions caused by varying degrees of gluten sensitivity, including a severe form characterised by a flat small intestinal mucosa (hyperplastic villous atrophy) and other forms characterised by milder symptoms.

The individual mentioned above (in the context of diagnosis or therapy) is human. They may have coeliac disease (symptomatic or asymptomatic) or be suspected of having it. They may be on a gluten free diet. They may be in an acute phase response (for example they may have coeliac disease, but have only ingested gluten in the last 24 hours before which they had been on a gluten free diet for 14 to 28 days).

The individual may be susceptible to coeliac disease, such as a genetic susceptibility (determined for example by the individual having relatives with coeliac disease or possessing genes which cause predisposition to coeliac disease).

### The agent

The agent of the present disclosure is typically a peptide, for example of length 7 to 50 amino acids, such as 10 to 40, or 15 to 30 amino acids in length. The peptides of the present invention, and those for use in the method of therapy of the invention, and those used in the compositions, methods and kits of the invention, are as defined in the claims.

SEQ ID NO:1 is PQPELPY. SEQ ID NO:2 is QLQPFPQPELPYPQPQS. SEQ ID NO:3 is shown in Table 1 and is the sequence of a whole A-gliadin. The glutamate at position 4 of SEQ ID NO:1 (equivalent to position 9 of SEQ ID NO:2) is generated by transglutaminase treatment of A-gliadin.

According to the general disclosure, the agent may be the peptide represented by SEQ ID NO:1 or 2 or an epitope comprising sequence that comprises SEQ ID NO:1 which is an isolated oligopeptide derived from a gliadin protein; or an equivalent of these sequences from a naturally occurring gliadin protein which is a homologue of SEQ ID NO:3. Thus the epitope may be a derivative of the protein represented by SEQ ID NO:3. Such a derivative is typically a fragment of the gliadin, or a mutated derivative of the whole protein or fragment. Therefore the epitope does not include this naturally occurring whole gliadin protein, and does not include other whole naturally occurring gliadins.

The epitope may thus be a fragment of A-gliadin (e.g. SEQ ID NO:3), which comprises the sequence of SEQ ID NO:1, obtainable by treating (fully or partially) with transglutaminase, i.e. with 1, 2, 3 or more glutamines substituted to glutamates (including the substitution within SEQ ID NO:1).

Such fragments may be or may include the sequences represented by positions 55 to 70, 58 to 73, 61 to 77 of SEQ ID NO:3 shown in Table 1. Typically such fragments will be recognised by T cells to at least the same extent that the peptides represented by SEQ ID NO:1 or 2 are recognised in any of the assays described herein using samples from coeliac disease patients.

Additionally, the agent may be the peptide represented by any of SEQ ID NOS:18-22, 31-36, 39-44, and 46 or a protein comprising a sequence corresponding to any of SEQ ID NOS:18-22, 31-36, 39-44, and 46 (such as fragments of a gliadin comprising any of SEQ ID NOS:18-22, 31-36, 39-44, and 46, for example after the gliadin has been treated with transglutaminase). Bioactive fragments of such sequences are also agents of the disclosure. Sequences equivalent to any of SEQ ID NOS:18-22, 31-36, 39-44, and 46 or analogues of these sequences are also agents of the disclosure.

In the case where the epitope comprises a sequence equivalent to the above epitopes (including fragments) from another gliadin protein (e.g. any of the gliadin proteins mentioned herein or any gliadins which cause coeliac disease), such equivalent sequences will correspond to a fragment of a gliadin protein typically treated (partially or fully) with transglutaminase. Such equivalent peptides can be determined by aligning the sequences of other gliadin proteins with the gliadin from which the original epitope derives, such as with SEQ ID NO:3 (for example using any of the programs mentioned herein). Transglutaminase is commercially available (e.g. Sigma T-5398). Table 4 provides a few examples of suitable equivalent sequences.

The agent which is an analogue is capable of being recognised by a TCR which recognises (i) or (ii). Therefore generally when the analogue is added to T cells in the presence of (i) or (ii), typically also in the presence of an antigen presenting cell (APC) (such as any of the APCs mentioned herein), the analogue inhibits the recognition of (i) or (ii), i.e. the analogue is able to compete with (i) or (ii) in such a system.

The analogue may be one which is capable of binding the TCR which recognises (i) or (ii). Such binding can be tested by standard techniques. Such TCRs can be isolated from T cells which have been shown to recognise (i) or (ii) (e.g. using the method of the disclosure). Demonstration of the binding of the analogue to the TCRs can then shown by determining whether the TCRs inhibit the binding of the analogue to a substance that binds the analogue, e.g. an antibody to the analogue. Typically the analogue is bound to a class II MHC molecule (e.g. HLA-DQ2) in such an inhibition of binding assay.

Typically the analogue inhibits the binding of (i) or (ii) to a TCR. In this case the amount of (i) or (ii) which can bind the TCR in the presence of the analogue is decreased. This is because the analogue is able to bind the TCR and therefore competes with (i) or (ii) for binding to the TCR.

T cells for use in the above binding experiments can be isolated from patients with coeliac disease, for example with the aid of the method of the disclosure. Other binding characteristics of the analogue may also be the same as (i) or (ii), and thus typically the analogue binds to the same MHC class II molecule to which the peptide binds (HLA-DQ2 or -DQ8). The analogue typically binds to antibodies specific for (i) or (ii), and thus inhibits binding of (i) or (ii) to such antibodies.

The analogue is typically a peptide. It may have homology with (i) or (ii), typically at least 70% homology, preferably at least 80, 90%, 95%, 97% or 99% homology with (i) or (ii), for example over a region of at least 15 more (such as the entire length of the analogue and/or (i) or (ii), or across the region which contacts the TCR or binds the MHC molecule) contiguous amino acids. Methods of measuring protein homology are well known in the art and it will be understood by those of skill in the art that in the present context, homology is calculated on the basis of amino acid identity (sometimes referred to as "hard homology").

For example the UWGCG Package provides the BESTFIT program which can be used to calculate homology (for example used on its default settings) (Devereux et al (1984) Nucleic Acids Research 12, p387-395). The PILEUP and BLAST algorithms can be used to calculate homology or line up sequences (typically on their default settings), for example as described in Altschul S. F. (1993) J Mol Evol 36:290-300; Altschul, S, F et al (1990) J Mol Biol 215:403-10.

Software for performing BLAST analyses is publicly available through the National Center for Biotechnology Information on the world wide web through the internet at, for example, "www.ncbi.nlm.nih.gov/". This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The homologous peptide analogues typically differ from (i) or (ii) by 1, 2, 3, 4, 5, 6, 7, 8 or more mutations (which may be substitutions, deletions or insertions). These mutations may be measured across any of the regions mentioned above in relation to calculating homology. The substitutions are preferably 'conservative'. These are defined according to the following Table. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Typically the amino acids in the analogue at the equivalent positions to amino acids in (i) or (ii) that contribute to binding the MHC molecule or are responsible for the recognition by the TCR, are the same or are conserved.

Typically the analogue peptide comprises one or more modifications, which may be natural post-translation modifications or artificial modifications. The modification may provide a chemical moiety (typically by substitution of a hydrogen, e.g. of a C-H bond), such as an amino, acetyl, hydroxy or halogen (e.g. fluorine) group or carbohydrate group. Typically the modification is present on the N or C terminus.

The analogue may comprise one or more non-natural amino acids, for example amino acids with a side chain different from natural amino acids. Generally, the non-natural amino acid will have an N terminus and/or a C terminus. The non-natural amino acid may be an L- or a D-amino acid.

The analogue typically has a shape, size, flexibility or electronic configuration that is substantially similar to (i) or (ii). It is typically a derivative of (i) or (ii). In one aspect the analogue is a fusion protein comprising the sequence of SEQ ID NO:1 or 2, or any of the other peptides mentioned herein; and non-gliadin sequence.

In one aspect the analogue is or mimics (i) or (ii) bound to a MHC class II molecule. 2, 3, 4 or more of such complexes may be associated or bound to each other, for example using a biotin/streptavidin based system, in which typically 2, 3 or 4 biotin labelled MHC molecules bind to a streptavidin moiety. This analogue typically inhibits the binding of the (i) or (ii)/MHC Class II complex to a TCR or antibody which is specific for the complex.

The analogue is typically an antibody or a fragment of an antibody, such as a Fab or (Fab)₂ fragment. The analogue may be immobilised on a solid support, particularly an analogue that mimics peptide bound to a MHC molecule.

The analogue is typically designed by computational means and then synthesised using methods known in the art. Alternatively the analogue can be selected from a library of compounds. The library may be a combinatorial library or a display library, such as a phage display library. The library of compounds may be expressed in the display library in the form of being bound to a MHC class II molecule, such as HLA-DQ2 or -DQ8. Analogues are generally selected from the library based on their ability to mimic the binding characteristics (i) or (ii). Thus they may be selected based on ability to bind a TCR or antibody which recognises (i) or (ii).

Typically analogues will be recognised by T cells to at least the same extent as any of the agents (i) or (ii), for example at least to the same extent as the equivalent epitope and preferably to the same extent as the peptide represented by SEQ ID NO:2, is recognised in any of the assays described herein, typically using T cells from coeliac disease patients. Analogues may be recognised to these extents *in vivo* and thus may be able to induce coeliac disease symptoms to at least the same extent as any of the agents mentioned herein (e.g. in a human patient or animal model).

Analogues may be identified in a method comprising determining whether a candidate substance is recognised by a T cell receptor that recognises an epitope of the disclosure, recognition of the substance indicating that the substance is an analogue. Such TCRs may be any of the TCRs mentioned herein, and may be present on T cells. Any suitable assay mentioned herein can be used to identify the analogue. In one aspect this method is carried out *in vivo.* As mentioned above preferred analogues are recognised to at least the same extent as the peptide SEQ ID NO:2, and so the method may be used to identify analogues which are recognised to this extent.

In one aspect the method comprises determining whether a candidate substance is able to inhibit the recognition of an epitope of the disclosure, inhibition of recognition indicating that the substance is an analogue.

The agent may be a product comprising at least 2, 5, 10 or 20 agents as defined by (i), (ii) or (iii). Typically the composition comprises epitopes of the disclosure (or equivalent analogues) from different gliadins, such as any of the species or variety of or types of gliadin mentioned herein. Preferred compositions comprise at least one epitope of the disclosure, or equivalent analogue, from all of the gliadins present in any of the species or variety mentioned herein, or from 2, 3, 4 or more of the species mentioned herein (such as from the panel of species consisting of wheat, rye, barley, oats and triticale). Thus, the agent may be monovalent or multivalent.

### Diagnosis

The method of diagnosis of the disclosure may be based on the detection of T cells that bind the agent or on the detection of antibodies that recognise the agent.

The T cells that recognise the agent in the method (which includes the use mentioned above) are generally T cells that have been pre-sensitised *in vivo* to gliadin. As mentioned above such antigen-experienced T cells have been found to be present in the peripheral blood.

In the method the T cells can be contacted with the agent *in vitro* or *in vivo,* and determining whether the T cells recognise the agent can be performed *in vitro* or *in vivo.* Thus the disclosure provides the agent for use in a method of diagnosis practiced on the human body. Different agents are provided for simultaneous, separate or sequential use in such a method.

The *in vitro* method is typically carried out in aqueous solution into which the agent is added. The solution will also comprise the T cells (and in certain aspects the APCs discussed below). The term 'contacting' as used herein includes adding the particular substance to the solution.

Determination of whether the T cells recognise the agent is generally accomplished by detecting a change in the state of the T cells in the presence of the agent or determining whether the T cells bind the agent. The change in state is generally caused by antigen specific functional activity of the T cell after the TCR binds the agent. The change of state may be measured inside (e.g. change in intracellular expression of proteins) or outside (e.g. detection of secreted substances) the T cells.

The change in state of the T cell may be the start of or increase in secretion of a substance from the T cell, such as a cytokine, especially IFN-γ, IL-2 or TNF-α. Determination of IFN-γ secretion is particularly preferred. The substance can typically be detected by allowing it to bind to a specific binding agent and then measuring the presence of the specific binding agent/substance complex. The specific binding agent is typically an antibody, such as polyclonal or monoclonal antibodies. Antibodies to cytokines are commercially available, or can be made using standard techniques.

Typically the specific binding agent is immobilised on a solid support. After the substance is allowed to bind the solid support can optionally be washed to remove material which is not specifically bound to the agent. The agent/substance complex may be detected by using a second binding agent that will bind the complex. Typically the second agent binds the substance at a site which is different from the site which binds the first agent. The second agent is preferably an antibody and is labelled directly or indirectly by a detectable label.

Thus the second agent may be detected by a third agent that is typically labelled directly or indirectly by a detectable label. For example the second agent may comprise a biotin moiety, allowing detection by a third agent which comprises a streptavidin moiety and typically alkaline phosphatase as a detectable label.

In one aspect the detection system which is used is the *ex-vivo* ELISPOT assay described in WO 98/23960. In that assay IFN-γ secreted from the T cell is bound by a first IFN-γ specific antibody that is immobilised on a solid support. The bound IFN-γ is then detected using a second IFN-γ specific antibody which is labelled with a detectable label. Such a labelled antibody can be obtained from MABTECH (Stockholm, Sweden). Other detectable labels which can be used are discussed below.

The change in state of the T cell that can be measured may be the increase in the uptake of substances by the T cell, such as the uptake of thymidine. The change in state may be an increase in the size of the T cells, or proliferation of the T cells, or a change in cell surface markers on the T cell.

In one aspect the change of state is detected by measuring the change in the intracellular expression of proteins, for example the increase in intracellular expression of any of the cytokines mentioned above. Such intracellular changes may be detected by contacting the inside of the T cell with a moiety that binds the expressed proteins in a specific manner and which allows sorting of the T cells by flow cytometry.

In one aspect when binding the TCR the agent is bound to an MHC class II molecule (typically HLA-DQ2 or -DQ8), which is typically present on the surface of an antigen presenting cell (APC). However as mentioned herein other agents can bind a TCR without the need to also bind an MHC molecule.

Generally the T cells which are contacted in the method are taken from the individual in a blood sample, although other types of samples which contain T cells can be used. The sample may be added directly to the assay or may be processed first. Typically the processing may comprise diluting of the sample, for example with water or buffer. Typically the sample is diluted from 1.5 to 100 fold, for example 2 to 50 or 5 to 10 fold.

The processing may comprise separation of components of the sample. Typically mononuclear cells (MCs) are separated from the samples. The MCs will comprise the T cells and APCs. Thus in the method the APCs present in the separated MCs can present the peptide to the T cells. In another aspect only T cells, such as only CD4 T cells, can be purified from the sample. PBMCs, MCs and T cells can be separated from the sample using techniques known in the art, such as those described in Lalvani et al (1997) J. Exp. Med. 186, p859-865.

In one aspect, the T cells used in the assay are in the form of unprocessed or diluted samples, or are freshly isolated T cells (such as in the form of freshly isolated MCs or PBMCs) which are used directly *ex vivo,* i.e. they are not cultured before being used in the method. Thus the T cells have not been restimulated in an antigen specific manner *in vitro.* However the T cells can be cultured before use, for example in the presence of one or more of the agents, and generally also exogenous growth promoting cytokines. During culturing the agent(s) are typically present on the surface of APCs, such as the APC used in the method. Pre-culturing of the T cells may lead to an increase in the sensitivity of the method. Thus the T cells can be converted into cell lines, such as short term cell lines (for example as described in Ota et al (1990) Nature 346, p183-187).

The APC that is typically present in the method may be from the same individual as the T cell or from a different host. The APC may be a naturally occurring APC or an artificial APC. The APC is a cell that is capable of presenting the peptide to a T cell. It is typically a B cell, dendritic cell or macrophage. It is typically separated from the same sample as the T cell and is typically co-purified with the T cell. Thus the APC may be present in MCs or PBMCs. The APC is typically a freshly isolated *ex vivo* cell or a cultured cell. It may be in the form of a cell line, such as a short term or immortalised cell line. The APC may express empty MHC class II molecules on its surface.

In the method one or more (different) agents may be used. Typically the T cells derived from the sample can be placed into an assay with all the agents which it is intended to test or the T cells can be divided and placed into separate assays each of which contain one or more of the agents.

The disclosure also provides the agents such as two or more of any of the agents mentioned herein (e.g. the combinations of agents which are present in the composition agent discussed above) for simultaneous separate or sequential use (eg. for *in vivo* use).

In one aspect agent *per se* is added directly to an assay comprising T cells and APCs. As discussed above the T cells and APCs in such an assay could be in the form of MCs. When agents that can be recognised by the T cell without the need for presentation by APCs are used then APCs are not required. Analogues which mimic the original (i) or (ii) bound to a MHC molecule are an example of such an agent.

In one aspect the agent is provided to the APC in the absence of the T cell. The APC is then provided to the T cell, typically after being allowed to present the agent on its surface. The peptide may have been taken up inside the APC and presented, or simply be taken up onto the surface without entering inside the APC.

The duration for which the agent is contacted with the T cells will vary depending on the method used for determining recognition of the peptide. Typically 10⁵ to 10⁷, preferably 5x10⁵ to 10⁶ PBMCs are added to each assay. In the case where agent is added directly to the assay its concentration is from 10⁻¹ to 10³µg/ml, preferably 0.5 to 50µg/ml or 1 to 10µg/ml.

Typically the length of time for which the T cells are incubated with the agent is from 4 to 24 hours, preferably 6 to 16 hours. When using *ex vivo* PBMCs it has been found that 0.3x10⁶ PBMCs can be incubated in 10µg/ml of peptide for 12 hours at 37°C.

The determination of the recognition of the agent by the T cells may be done by measuring the binding of the agent to the T cells (this can be carried out using any suitable binding assay format discussed herein). Typically T cells which bind the agent can be sorted based on this binding, for example using a FACS machine. The presence of T cells that recognise the agent will be deemed to occur if the frequency of cells sorted using the agent is above a "control" value. The frequency of antigen-experienced T cells is generally 1 in 10⁶ to 1 in 10³, and therefore whether or not the sorted cells are antigen-experienced T cells can be determined.

The determination of the recognition of the agent by the T cells may be measured *in vivo.* Typically the agent is administered to the host and then a response which indicates recognition of the agent may be measured. The agent is typically administered intradermally or epidermally. The agent is typically administered by contacting with the outside of the skin, and may be retained at the site with the aid of a plaster or dressing. Alternatively the agent may be administered by needle, such as by injection, but can also be administered by other methods such as ballistics (e.g. the ballistics techniques which have been used to deliver nucleic acids). EP-A-0693119 describes techniques that can typically be used to administer the agent. Typically from 0.001 to 1000 µg, for example from 0.01 to 100 µg or 0.1 to 10 µg of agent is administered.

In one aspect a product can be administered which is capable of providing the agent *in vivo.* Thus a polynucleotide capable of expressing the agent can be administered, typically in any of the ways described above for the administration of the agent. The polynucleotide typically has any of the characteristics of the polynucleotide provided by the disclosure which is discussed below. The agent is expressed from the polynucleotide *in vivo.* Typically from 0.001 to 1000 µg, for example from 0.01 to 100 µg or 0.1 to 10 µg of polynucleotide is administered.

Recognition of the agent administered to the skin is typically indicated by the occurrence of inflammation (e.g. induration, erythema or oedema) at the site of administration. This is generally measured by visual examination of the site.

The method of diagnosis based on the detection of an antibody that binds the agent is typically carried out by contacting a sample from the individual (such as any of the samples mentioned here, optionally processed in any manner mentioned herein) with the agent and determining whether an antibody in the sample binds the agent, such a binding indicating that the individual has, or is susceptible to coeliac disease. Any suitable format of binding assay may be used, such as any such format mentioned herein.

### Therapy

The identification of the immunodominant epitope and other epitopes described herein allows therapeutic products to be made which target the T cells which recognise this epitope (such T cells being ones which participate in the immune response against gliadin). These findings also allow the prevention or treatment of coeliac disease by suppressing (by tolerisation) an antibody or T cell response to the epitope(s).

Certain agents of the disclosure bind the TCR that recognises the epitope of the disclosure (as measured using any of the binding assays discussed above) and cause tolerisation of the T cell that carries the TCR. Such agents, optionally in association with a carrier, can therefore be used to prevent or treat coeliac disease.

Generally tolerisation can be caused by the same peptides which can (after being recognised by the TCR) cause antigen specific functional activity of the T cell (such as any such activity mentioned herein, e.g. secretion of cytokines). Such agents cause tolerisation when they are presented to the immune system in a 'tolerising' context.

Tolerisation leads to a decrease in the recognition of a T cell or antibody epitope by the immune system. In the case of a T cell epitope this can be caused by the deletion or anergising of T cells that recognise the epitope. Thus T cell activity (for example as measured in suitable assays mentioned herein) in response to the epitope is decreased. Tolerisation of an antibody response means that a decreased amount of specific antibody to the epitope is produced when the epitope is administered.

Methods of presenting antigens to the immune system in such a context are known and are described for example in Yoshida et al. Clin. Immunol. Immunopathol. 82, 207-215 (1997), Thurau et al. Clin. Exp. Immunol. 109, 370-6 (1997), and Weiner et al. Res. Immunol. 148, 528-33 (1997). In particular certain routes of administration can cause tolerisation, such as oral, nasal or intraperitoneal. Particular products which cause tolerisation may be administered (e.g. in a composition that also comprises the agent) to the individual. Such products include cytokines, such as cytokines that favour a Th2 response (e.g. IL-4, TGF-β or IL-10). Products or agent may be administered at a dose that causes tolerisation.

The disclosure provides a protein that comprises a sequence able to act as an antagonist of the T cell (which T cell recognises the agent). Such proteins and such antagonists can also be used to prevent or treat coeliac disease. The antagonist will cause a decrease in the T cell response. In one aspect, the antagonist binds the TCR of the T cell (generally in the form of a complex with HLA-DQ2 or -DQ8) but instead of causing normal functional activation causing an abnormal signal to be passed through the TCR intracellular signalling cascade, which causes the T cell to have decreased function activity (e.g. in response to recognition of an epitope, typically as measured by any suitable assay mentioned herein).

In one aspect the antagonist competes with epitope to bind a component of MHC processing and presentation pathway, such as an MHC molecule (typically HLA-DQ2 or -DQ8). Thus the antagonist may bind HLA-DQ2 or -DQ8 (and thus be a peptide presented by this MHC molecule), such as peptide TP (Table 10) or a homologue thereof.

Methods of causing antagonism are known in the art. In one aspect the antagonist is a homologue of the epitopes mentioned above and may have any of the sequence, binding or other properties of the agent (particularly analogues). The antagonists typically differ from any of the above epitopes (which are capable of causing a normal antigen specific function in the T cell) by 1, 2, 3, 4 or more mutations (each of which may be a substitution, insertion or deletion). Such antagonists are termed "altered peptide ligands" or "APL" in the art. The mutations are typically at the amino acid positions that contact the TCR.

The antagonist may differ from the epitope by a substitution within the sequence that is equivalent to the sequence represented by amino acids 65 to 67 of A-gliadin (such antagonists are shown in Table 9). Thus preferably the antagonist has a substitution at the equivalent of position 64, 65 or 67. Preferably the substitution is 64W, 67W, 67M or 65T.

Since the T cell immune response to the epitope of the disclosure in an individual is polyclonal, more than one antagonist may need to be administered to cause antagonism of T cells of the response which have different TCRs. Therefore the antagonists may be administered in a composition which comprises at least 2, 4, 6 or more different antagonists, which each antagonise different T cells.

The disclosure also provides a method of identifying an antagonist of a T cell (which recognises the agent), comprising contacting a candidate substance with the T cell and detecting whether the substance causes a decrease in the ability of the T cell to undergo an antigen specific response (e.g. using any suitable assay mentioned herein), the detecting of any such decrease in said ability indicating that the substance is an antagonist.

In one aspect, the antagonists (including combinations of antagonists to a particular epitope) or tolerising (T cell and antibody tolerising) agents are present in a composition comprising at least 2, 4, 6 or more antagonists or agents which antagonise or tolerise to different epitopes of the disclosure, for example to the combinations of epitopes discussed above in relation to the agents which are a product comprising more than one substance.

### Testing whether a composition is capable of causing coeliac disease

As mentioned above the disclosure provides a method of determining whether a composition is capable of causing coeliac disease comprising detecting the presence of a protein sequence which is capable of being modified by a transglutaminase to as sequence comprising the agent or epitope of the disclosure (such transglutaminase activity may be a human intestinal transglutaminase activity). Typically this is performed by using a binding assay in which a moiety which binds to the sequence in a specific manner is contacted with the composition and the formation of sequence/moiety complex is detected and used to ascertain the presence of the agent. Such a moiety may be any suitable substance (or type of substance) mentioned herein, and is typically a specific antibody. Any suitable format of binding assay can be used (such as those mentioned herein).

In one aspect, the composition is contacted with at least 2, 5, 10 or more antibodies which are specific for epitopes of the disclosure from different gliadins, for example a panel of antibodies capable of recognising the combinations of epitopes discussed above in relation to agents of the disclosure which are a product comprising more than one substance.

The composition typically comprises material from a plant that expresses a gliadin which is capable of causing coeliac disease (for example any of the gliadins or plants mentioned herein). Such material may be a plant part, such as a harvested product (e.g. seed). The material may be processed products of the plant material (e.g. any such product mentioned herein), such as a flour or food that comprises the gliadin. The processing of food material and testing in suitable binding assays is routine, for example as mentioned in Kricka LJ, J. Biolumin. Chemilumin. 13, 189-93 (1998).

### Binding assays

The determination of binding between any two substances mentioned herein may be done by measuring a characteristic of either or both substances that changes upon binding, such as a spectroscopic change.

The binding assay format may be a 'band shift' system. This involves determining whether the presence of one substance (such as a candidate substance) advances or retards the progress of the other substance during gel electrophoresis.

The format may be a competitive binding method which determines whether the one substance is able to inhibit the binding of the other substance to an agent which is known to bind the other substance, such as a specific antibody.

### Mutant gliadin proteins

The disclosure provides a gliadin protein in which an epitope sequence of the disclosure, or sequence which can be modified by a transglutaminase to provide such a sequence has been mutated so that it no longer causes, or is recognised by, a T cell response that recognises the epitope. In this context the term recognition refers to the TCR binding the epitope in such a way that normal (not antagonistic) antigen-specific functional activity of the T cell occurs.

Methods of identifying equivalent epitopes in other gliadins are discussed above. The wild type of the mutated gliadin is one which causes coeliac disease. Such a gliadin may have homology with SEQ ID NO:3, for example to the degree mentioned above (in relation to the analogue) across all of SEQ ID NO:3 or across 15, 30, 60, 100 or 200 contiguous amino acids of SEQ ID NO:3. Likewise, for other non-A-gliadins, homology will be present between the mutant and the native form of that gliadin. The sequences of other natural gliadin proteins are known in the art.

The mutated gliadin will not cause coeliac disease or will cause decreased symptoms of coeliac disease. Typically the mutation decreases the ability of the epitope to induce a T cell response. The mutated epitope may have a decreased binding to HLA-DQ2 or -DQ8, a decreased ability to be presented by an APC or a decreased ability to bind to or to be recognised (i.e. cause antigen-specific functional activity) by T cells that recognise the agent. The mutated gliadin or epitope will therefore show no or reduced recognition in any of the assays mentioned herein in relation to the diagnostic aspects of the disclosure.

The mutation may be one or more deletions, additions or substitutions of length 1 to 3, 4 to 6, 6 to 10, 11 to 15 or more in the epitope, for example across sequence SEQ ID NO:2 or across any of SEQ ID NOS: 18-22, 31-36, 39-44, and 46; or across equivalents thereof. Preferably the mutant gliadin has at least one mutation in the sequence SEQ ID NO:1. A preferred mutation is at position 65 in A-gliadin (or in an equivalent position in other gliadins). Typically the naturally occurring glutamine at this position is substituted to any of the amino acids shown in Table 3, preferably to histidine, tyrosine, tryptophan, lysine, proline, or arginine.

The disclosure thus also provides use of a mutation (such any of the mutations in any of the sequences discussed herein) in an epitope of a gliadin protein, which epitope is an epitope of the disclosure, to decrease the ability of the gliadin protein to cause coeliac disease.

In one aspect the mutated sequence is able to act as an antagonist. Thus the disclosure provides a protein that comprises a sequence which is able to bind to a T cell receptor, which T cell receptor recognises an agent of the disclosure, and which sequence is able to cause antagonism of a T cell that carries such a T cell receptor.

The disclosure also provides proteins which are fragments of the above mutant gliadin proteins, which are at least 15 amino acids long (e.g. at least 30, 60, 100, 150, 200, or 250 amino acids long) and which comprise the mutations discussed above which decrease the ability of the gliadin to be recognised. Any of the mutant proteins (including fragments) mentioned herein may also be present in the form of fusion proteins, for example with other gliadins or with non-gliadin proteins.

The equivalent wild type protein to the mutated gliadin protein is typically from a graminaceous monocotyledon, such as a plant of genus Triticum, e.g. wheat, rye, barley, oats or triticale. The protein is typically an α, αβ, β, γ or ω gliadin. The gliadin may be an A-gliadin.

### Kits

The disclosure also provides a kit for carrying out the method comprising one or more agents and optionally a means to detect the recognition of the agent by the T cell. Typically the different agents are provided for simultaneous, separate or sequential use. Typically the means to detect recognition allows or aids detection based on the techniques discussed above.

Thus the means may allow detection of a substance secreted by the T cells after recognition. The kit may thus additionally include a specific binding moiety for the substance, such as an antibody. The moiety is typically specific for IFN-γ. The moiety is typically immobilised on a solid support. This means that after binding the moiety the substance will remain in the vicinity of the T cell which secreted it. Thus "spots" of substance/moiety complex are formed on the support, each spot representing a T cell which is secreting the substance. Quantifying the spots, and typically comparing against a control, allows determination of recognition of the agent.

The kit may also comprise a means to detect the substance/moiety complex. A detectable change may occur in the moiety itself after binding the substance, such as a colour change. Alternatively a second moiety directly or indirectly labelled for detection may be allowed to bind the substance/moiety complex to allow the determination of the spots. As discussed above the second moiety may be specific for the substance, but binds a different site on the substance than the first moiety.

The immobilised support may be a plate with wells, such as a microtitre plate. Each assay can therefore be carried out in a separate well in the plate.

The kit may additionally comprise medium for the T cells, detection moieties or washing buffers to be used in the detection steps. The kit may additionally comprise reagents suitable for the separation from the sample, such as the separation of PBMCs or T cells from the sample. The kit may be designed to allow detection of the T cells directly in the sample without requiring any separation of the components of the sample.

The kit may comprise an instrument which allows administration of the agent, such as intradermal or epidermal administration. Typically such an instrument comprises plaster, dressing or one or more needles. The instrument may allow ballistic delivery of the agent. The agent in the kit may be in the form of a pharmaceutical composition.

The kit may also comprise controls, such as positive or negative controls. The positive control may allow the detection system to be tested. Thus the positive control typically mimics recognition of the agent in any of the above methods. Typically in the kits designed to determine recognition *in vitro* the positive control is a cytokine. In the kit designed to detect *in vivo* recognition of the agent the positive control may be antigen to which most individuals should response.

The kit may also comprise a means to take a sample containing T cells from the host, such as a blood sample. The kit may comprise a means to separate mononuclear cells or T cells from a sample from the host.

### Polynucleotides, cells, transgenic mammals and antibodies

The disclosure also provides a polynucleotide which is capable of expression to provide the agent or mutant gliadin proteins. Typically the polynucleotide is DNA or RNA, and is single or double stranded. The polynucleotide will preferably comprise at least 50 bases or base pairs, for example 50 to 100, 100 to 500, 500 to 1000 or 1000 to 2000 or more bases or base pairs. The polynucleotide therefore comprises a sequence which encodes the sequence of SEQ ID NO: 1 or 2 or any of the other agents mentioned herein. To the 5' and 3' of this coding sequence the polynucleotide has sequence or codons which are different from the sequence or codons 5' and 3' to these sequences in the corresponding gliadin gene.

5' and/or 3' to the sequence encoding the peptide the polynucleotide has coding or non-coding sequence. Sequence 5' and/or 3' to the coding sequence may comprise sequences which aid expression, such as transcription and/or translation, of the sequence encoding the agent. The polynucleotide may be capable of expressing the agent prokaryotic or eukaryotic cell. In one aspect the polynucleotide is capable of expressing the agent in a mammalian cell, such as a human, primate or rodent (e.g. mouse or rat) cell.

A polynucleotide of the disclosure may hybridise selectively to a polynucleotide that encodes SEQ ID NO:3 at a level significantly above background. Selective hybridisation is typically achieved using conditions of medium to high stringency (for example 0.03M sodium chloride and 0.03M sodium citrate at from about 50°C to about 60°C). However, such hybridisation may be carried out under any suitable conditions known in the art (see Sambrook et al (1989), Molecular Cloning: A Laboratory Manual). For example, if high stringency is required, suitable conditions include 0.2 x SSC at 60°C. If lower stringency is required, suitable conditions include 2 x SSC at 60°C.

Agents or proteins of the disclosure may be encoded by the polynucleotides described herein.

The polynucleotide may form or be incorporated into a replicable vector. Such a vector is able to replicate in a suitable cell. The vector may be an expression vector. In such a vector the polynucleotide is operably linked to a control sequence which is capable of providing for the expression of the polynucleotide. The vector may contain a selectable marker, such as the ampicillin resistance gene.

The polynucleotide or vector may be present in a cell. Such a cell may have been transformed by the polynucleotide or vector. The cell may express the agent. The cell will be chosen to be compatible with the said vector and may for example be a prokaryotic (bacterial), yeast, insect or mammalian cell. The polynucleotide or vector may be introduced into host cells using conventional techniques including calcium phosphate precipitation, DEAE-dextran transfection, or electroporation.

The disclosure provides processes for the production of the proteins of the disclosure by recombinant means. This may comprise (a) cultivating a transformed cell as defined above under conditions that allow the expression of the protein; and preferably (b) recovering the expressed polypeptide. Optionally, the polypeptide may be isolated and/or purified, by techniques known in the art.

The disclosure also provides TCRs which recognise (or bind) the agent, or fragments thereof which are capable of such recognition (or binding). These can be present in the any form mentioned herein (e.g. purity) discussed herein in relation to the protein of the disclosure. The disclosure also provides T cells which express such TCRs which can be present in any form (e.g. purity) discussed herein for the cells of the disclosure.

The disclosure also provides monoclonal or polyclonal antibodies which specifically recognise the agents (such as any of the epitopes of the disclosure) and which recognise the mutant gliadin proteins (and typically which do not recognise the equivalent wild-type gliadins) of the disclosure, and methods of making such antibodies. Antibodies of the disclosure bind specifically to these substances of the disclosure.

For the purposes of this disclosure, the term "antibody" includes antibody fragments such as Fv, F(ab) and F(ab)₂ fragments, as well as single-chain antibodies.

A method for producing a polyclonal antibody comprises immunising a suitable host animal, for example an experimental animal, with the immunogen and isolating immunoglobulins from the serum. The animal may therefore be inoculated with the immunogen, blood subsequently removed from the animal and the IgG fraction purified. A method for producing a monoclonal antibody comprises immortalising cells which produce the desired antibody. Hybridoma cells may be produced by fusing spleen cells from an inoculated experimental animal with tumour cells (Kohler and Milstein (1975) Nature 256, 495-497).

An immortalized cell producing the desired antibody may be selected by a conventional procedure. The hybridomas may be grown in culture or injected intraperitoneally for formation of ascites fluid or into the blood stream of an allogenic host or immunocompromised host. Human antibody may be prepared by *in vitro* immunisation of human lymphocytes, followed by transformation of the lymphocytes with Epstein-Barr virus.

For the production of both monoclonal and polyclonal antibodies, the experimental animal is suitably a goat, rabbit, rat or mouse. If desired, the immunogen may be administered as a conjugate in which the immunogen is coupled, for example via a side chain of one of the amino acid residues, to a suitable carrier. The carrier molecule is typically a physiologically acceptable carrier. The antibody obtained may be isolated and, if desired, purified.

The polynucleotide, agent, protein or antibody of the disclosure, may carry a detectable label. Detectable labels which allow detection of the secreted substance by visual inspection, optionally with the aid of an optical magnifying means, are preferred. Such a system is typically based on an enzyme label which causes colour change in a substrate, for example alkaline phosphatase causing a colour change in a substrate. Such substrates are commercially available, e.g. from BioRad. Other suitable labels include other enzymes such as peroxidase, or protein labels, such as biotin; or radioisotopes, such as ³²P or ³⁵S. The above labels may be detected using known techniques.

Polynucleotides, agents, proteins, antibodies or cells of the disclosure may be in substantially purified form. They may be in substantially isolated form, in which case they will generally comprise at least 80% e.g. at least 90, 95, 97 or 99% of the polynucleotide, peptide, antibody, cells or dry mass in the preparation. The polynucleotide, agent, protein or antibody is typically substantially free of other cellular components. The polynucleotide, agent, protein or antibody may be used in such a substantially isolated, purified or free form in the method or be present in such forms in the kit.

The disclosure also provides a transgenic non-human mammal which expresses a TCR of the disclosure. This may be any of the mammals discussed herein (e.g. in relation to the production of the antibody). Preferably the mammal has, or is susceptible, to coeliac disease. The mammal may also express HLA-DQ2 or -DQ8 or HLA-DR3-DQ2 and/or may be given a diet comprising a gliadin which cause coeliac disease (e.g. any of the gliadin proteins mentioned herein). Thus the mammal may act as an animal model for coeliac disease.

The disclosure also provides a method of identifying a product which is therapeutic for coeliac disease comprising administering a candidate substance to a mammal of the disclosure which has, or which is susceptible to, coeliac disease and determining whether substance prevents or treats coeliac disease in the mammal, the prevention or treatment of coeliac disease indicating that the substance is a therapeutic product. Such a product may be used to treat or prevent coeliac disease.

The disclosure provides therapeutic (including prophylactic) agents or diagnostic substances (the agents, proteins and polynucleotides of the disclosure). These substances are formulated for clinical administration by mixing them with a pharmaceutically acceptable carrier or diluent. For example they can be formulated for topical, parenteral, intravenous, intramuscular, subcutaneous, intraocular, intradermal, epidermal or transdermal administration. The substances may be mixed with any vehicle which is pharmaceutically acceptable and appropriate for the desired route of administration. The pharmaceutically carrier or diluent for injection may be, for example, a sterile or isotonic solution such as Water for Injection or physiological saline, or a carrier particle for ballistic delivery.

The dose of the substances may be adjusted according to various parameters, especially according to the agent used; the age, weight and condition of the patient to be treated; the mode of administration used; the severity of the condition to be treated; and the required clinical regimen. As a guide, the amount of substance administered by injection is suitably from 0.01 mg/kg to 30 mg/kg, preferably from 0.1 mg/kg to 10 mg/kg.

The routes of administration and dosages described are intended only as a guide since a skilled practitioner will be able to determine readily the optimum route of administration and dosage for any particular patient and condition.

The substances of the disclosure may thus be used to provide for a method of treatment of the human or animal body, or a diagnostic method practised on the human body. In particular they may be used to provide for a method of treating or preventing coeliac disease. The disclosure also provide the agents for use in a method of manufacture of a medicament for treating or preventing coeliac disease. Thus the disclosure provides for a method of preventing or treating coeliac disease comprising administering to a human in need thereof a substance of the disclosure (typically a non-toxic effective amount thereof).

The agent of the disclosure can be made using standard synthetic chemistry techniques, such as by use of an automated synthesizer. The agent may be made from a longer polypeptide e.g. a fusion protein, which polypeptide typically comprises the sequence of the peptide. The peptide may be derived from the polypeptide by for example hydrolysing the polypeptide, such as using a protease; or by physically breaking the polypeptide. The polynucleotide of the disclosure can be made using standard techniques, such as by using a synthesiser.

### Plant cells and plants that express mutant gliadin proteins or express proteins comprising sequences which can act as antagonists

The cell of the disclosure may be a plant cell, such as a cell of a graminaceous monocotyledonous species. The species may be one whose wild-type form expresses gliadins, such as any of the gliadin proteins mentioned herein (including gliadins with any degree of homology to SEQ ID NO:3 mentioned herein). Such a gliadin may cause coeliac disease in humans. The cell may be of wheat, maize, oats, rye, rice, barley, triticale, sorghum, or sugar cane. Typically the cell is of the Triticum genus, such as aestivum, spelta, polonicum or monococcum.

The plant cell of the disclosure is typically one which does not express a wild-type gliadin (such as any of the gliadins mentioned herein which may cause coeliac disease), or one which does not express a gliadin comprising a sequence that can be recognised by a T cell that recognises the agent. Thus if the wild-type plant cell did express such a gliadin then it may be engineered to prevent or reduce the expression of such a gliadin or to change the amino acid sequence of the gliadin so that it no longer causes coeliac disease (typically by no longer expressing the epitope).

This can be done for example by introducing mutations into 1, 2, 3 or more or all of such gliadin genes in the cell, for example into coding or non-coding (e.g. promoter regions). Such mutations can be any of the type or length of mutations discussed herein (e.g., in relation to homologous proteins). The mutations can be introduced in a directed manner (e.g., using site directed mutagenesis or homologous recombination techniques) or in a random manner (e.g. using a mutagen, and then typically selecting for mutagenised cells which no longer express the gliadin (or a gliadin sequence which causes coeliac disease)).

In the case of plants or plant cells that express a protein that comprises a sequence able to act as an antagonist such a plant or plant cell may express a wild-type gliadin protein (e.g. one which causes coeliac disease). Preferably though the presence of the antagonist sequence will cause reduced coeliac disease symptoms (such as no symptoms) in an individual who ingests a food comprising protein from the plant or plant cell.

The polynucleotide which is present in (or which was transformed into) the plant cell will generally comprise promoter capable of expressing the mutant gliadin protein the plant cell. Depending on the pattern of expression desired, the promoter may be constitutive, tissue- or stage-specific; and/or inducible. For example, strong constitutive expression in plants can be obtained with the CAMV 35S, Rubisco ssu, or histone promoters. Also, tissue-specific or stage-specific promoters may be used to target expression of protein to particular tissues in a transgenic plant or to particular stages in its development. Thus, for example seed-specific, root-specific, leaf-specific, flower-specific etc promoters may be used. Seed-specific promoters include those described by Dalta et al (Biotechnology Ann. Rev. (1997), 3, pp.269-296). Particular examples of seed-specific promoters are napin promoters (EP-A-0 255, 378), phaseolin promoters, glutenine promoters, helianthenine promoters (WO92/17580), albumin promoters (WO98/45460), oleosin promoters (WO98/45461) and ATS1 and ATS3 promoters (PCT/US98/06798).

The cell may be in any form. For example, it may be an isolated cell, e.g. a protoplast, or it may be part of a plant tissue, e.g. a callus, or a tissue excised from a plant, or it may be part of a whole plant. The cell may be of any type (e.g. of any type of plant part). For example, an undifferentiated cell, such as a callus cell; or a differentiated cell, such as a cell of a type found in embryos, pollen, roots, shoots or leaves. Plant parts include roots; shoots; leaves; and parts involved in reproduction, such as pollen, ova, stamens, anthers, petals, sepals and other flower parts.

The disclosure provides a method of obtaining a transgenic plant cell comprising transforming a plant cell with a polynucleotide or vector of the disclosure to give a transgenic plant cell. Any suitable transformation method may be used (in the case of wheat the techniques disclosed in Vasil V et al, Biotechnology 10, 667-674 (1992) may be used). Preferred transformation techniques include electroporation of plant protoplasts and particle bombardment. Transformation may thus give rise to a chimeric tissue or plant in which some cells are transgenic and some are not.

The cell of the disclosure or thus obtained cell may be regenerated into a transgenic plant by techniques known in the art. These may involve the use of plant growth substances such as auxins, giberellins and/or cytokinins to stimulate the growth and/or division of the transgenic cell. Similarly, techniques such as somatic embryogenesis and meristem culture may be used. Regeneration techniques are well known in the art and examples can be found in, e.g. US 4,459,355, US 4,536,475, US 5,464,763, US 5, 177,010, US 5, 187,073, EP 267,159, EP 604, 662, EP 672, 752, US 4,945,050, US 5,036,006, US 5,100,792, US 5,371,014, US 5,478,744, US 5,179,022, US 5,565,346, US 5,484,956, US 5,508,468, US 5,538,877, US 5,554,798, US 5,489,520, US 5,510,318, US 5,204,253, US 5,405,765, EP 442,174, EP 486,233, EP 486,234, EP 539,563, EP 674,725, WO91/02071 and WO 95/06128.

In many such techniques, one step is the formation of a callus, i.e. a plant tissue comprising expanding and/or dividing cells. Such calli are a further aspect of the disclosure as are other types of plant cell cultures and plant parts. Thus, for example, the disclosure provides transgenic plant tissues and parts, including embryos, meristems, seeds, shoots, roots, stems, leaves and flower parts. These may be chimeric in the sense that some of their cells are cells of the disclosure and some are not. Transgenic plant parts and tissues, plants and seeds of the disclosure may be of any of the plant species mentioned herein.

Regeneration procedures will typically involve the selection of transformed cells by means of marker genes.

The regeneration step gives rise to a first generation transgenic plant. The disclosure also provides methods of obtaining transgenic plants of further generations from this first generation plant. These are known as progeny transgenic plants. Progeny plants of second, third, fourth, fifth, sixth and further generations may be obtained from the first generation transgenic plant by any means known in the art.

Thus, the disclosure provides a method of obtaining a transgenic progeny plant comprising obtaining a second-generation transgenic progeny plant from a first-generation transgenic plant of the disclosure, and optionally obtaining transgenic plants of one or more further generations from the second-generation progeny plant thus obtained.

Progeny plants may be produced from their predecessors of earlier generations by any known technique. In particular, progeny plants may be produced by:
obtaining a transgenic seed from a transgenic plant of the disclosure belonging to a previous generation, then obtaining a transgenic progeny plant of the disclosure belonging to a new generation by growing up the transgenic seed; and/or
propagating clonally a transgenic plant of the disclosure belonging to a previous generation to give a transgenic progeny plant of the disclosure belonging to a new generation; and/or
crossing a first-generation transgenic plant of the disclosure belonging to a previous generation with another compatible plant to give a transgenic progeny plant of the disclosure belonging to a new generation; and optionally
obtaining transgenic progeny plants of one or more further generations from the progeny plant thus obtained.

These techniques may be used in any combination. For example, clonal propagation and sexual propagation may be used at different points in a process that gives rise to a transgenic plant suitable for cultivation. In particular, repetitive back-crossing with a plant taxon with agronomically desirable characteristics may be undertaken. Further steps of removing cells from a plant and regenerating new plants therefrom may also be carried out.

Also, further desirable characteristics may be introduced by transforming the cells, plant tissues, plants or seeds, at any suitable stage in the above process, to introduce desirable coding sequences other than the polynucleotides of the disclosure. This may be carried out by the techniques described herein for the introduction of polynucleotides of the disclosure.

For example, further transgenes may be selected from those coding for other herbicide resistance traits, e.g. tolerance to: Glyphosate (e.g. using an EPSP synthase gene (e.g. EP-A-0 293,358) or a glyphosate oxidoreductase (WO 92/000377) gene); or tolerance to fosametin; a dihalobenzonitrile; glufosinate, e.g. using a phosphinothrycin acetyl transferase (PAT) or glutamine synthase gene (cf. EP-A-0 242,236); asulam, e.g. using a dihydropteroate synthase gene (EP-A-0 369,367); or a sulphonylurea, e.g. using an ALS gene); diphenyl ethers such as acifluorfen or oxyfluorfen, e.g. using a protoporphyrogen oxidase gene); an oxadiazole such as oxadiazon; a cyclic imide such as chlorophthalim; a phenyl pyrazole such as TNP, or a phenopylate or carbamate analogue thereof.

Similarly, genes for beneficial properties other than herbicide tolerance may be introduced. For example, genes for insect resistance may be introduced, notably genes encoding *Bacillus thuringiensis (Bt)* toxins. Likewise, genes for disease resistance may be introduced, e.g. as in WO91/02701 or WO95/06128.

Typically, a protein of the disclosure is expressed in a plant of the disclosure. Depending on the promoter used, this expression may be constitutive or inducible. Similarly, it may be tissue- or stage-specific, i.e. directed towards a particular plant tissue (such as any of the tissues mentioned herein) or stage in plant development.

The disclosure also provides methods of obtaining crop products by harvesting, and optionally processing further, transgenic plants of the disclosure. By crop product is meant any useful product obtainable from a crop plant.

### Products that contain mutant gliadin proteins or proteins that comprise sequence capable of acting as an antagonist

The disclosure provides a product that comprises the mutant gliadin proteins or protein that comprises sequence capable of acting as an antagonist. This is typically derived from or comprise plant parts from plants mentioned herein which express such proteins. Such a product may be obtainable directly by harvesting or indirectly, by harvesting and further processing the plant of the disclosure. Directly obtainable products include grains. Alternatively, such a product may be obtainable indirectly, by harvesting and further processing. Examples of products obtainable by further processing are flour or distilled alcoholic beverages; food products made from directly obtained or further processed material, e.g. baked products (e.g. bread) made from flour. Typically such food products, which are ingestible and digestible (i.e. non-toxic and of nutrient value) by human individuals.

In the case of food products that comprise the protein which comprises an antagonist sequence the food product may also comprise wild-type gliadin, but preferably the antagonist is able to cause a reduction (e.g. completely) in the coeliac disease symptoms after such food is ingested.

The disclosure is illustrated by the following nonlimiting Examples. Examples relating exclusively to use of peptides not encompassed by the peptides defined in the present claims are included for reference only.

### Example 1

We carried out epitope mapping in Coeliac disease by using a set of 51 synthetic 15-mer peptides that span the complete sequence of a fully characterized a-gliadin, "A-gliadin" (see Table 1). A-Gliadin peptides were also individually treated with tTG to generate products that might mimic those produced *in vivo³.* We also sought to study Coeliac disease patients at the point of initiation of disease relapse to avoid the possibility that epitope "spreading" or "exhaustion" may have occurred, as described in experimental infectious and autoimmune diseases.

### Clinical and A-gliadin specific T cell responses with 3 and 10 day bread challenge

In a pilot study, two subjects with Coeliac disease in remission, defined by absence of serum anti-endomysial antibody (EMA), on a gluten free diet were fed four slices of standard gluten-containing white bread daily in addition to their usual gluten free diet. Subject 1 ceased bread because of abdominal pain, mouth ulcers and mild diarrhoea after three days, but Subject 2 continued for 10 days with only mild nausea at one week. The EMA became positive in Subject 2 one week after the bread challenge, indicating the bread used had caused a relapse of Coeliac disease. But in Subject 1, EMA remained negative up to two months after bread challenge. In both subjects, symptoms that appeared with bread challenge resolved within two days after returning to gluten free diet.

PBMC responses in IFNγ ELISPOT assays to A-gliadin peptides were not found before or during bread challenge. But from the day after bread withdrawal (Day 4) in Subject 1 a single pool of 5 overlapping peptides spanning A-gliadin 51-85 (Pool 3) treated with tTG showed potent IFNγ responses (see Figure 1a). In Subject 1, the PBMC IFNγ response to A-gliadin peptide remained targeted to Pool 3 alone and was maximal on Day 8. The dynamics and magnitude of the response to Pool 3 was similar to that elicited by α-chymotrypsin digested gliadin. PBMC IFNγ responses to tTG-treated Pool 3 were consistently 5 to 12-fold greater than Pool 3 not treated with tTG, and responses to α-chymotrypsin digested gliadin were 3 to 10-fold greater if treated with tTG. In Subject 2, Pool 3 treated with tTG was also the only immunogenic set of A-gliadin peptides on Day 8, but this response was weaker than Subject 1, was not seen on Day 4 and by Day 11 the response to Pool 3 had diminished and other tTG-treated pools of A-gliadin peptides elicited stronger IFNα responses (see Figure 1b).

The pilot study indicated that the initial T cell response in these Coeliac disease subjects was against a single tTG-treated A-gliadin pool of five peptides and was readily measured in peripheral blood. But if antigen exposure is continued for ten days instead of three, T cell responses to other A-gliadin peptides appear, consistent with epitope spreading.

### Coeliac disease-specific IFN-g induction by tTG-treated A-gliadin peptides

In five out of six further Coeliac disease subjects on gluten free diet (see Table 1), bread challenge for three days identified tTG-treated peptides in Pool 3, and in particular, peptides corresponding to 56-70 (12) and 60-75 (13) as the sole A-gliadin components eliciting IFNγ from PBMC (see Figure 2). IL-10 ELISPOT assays run in parallel to IFNγ ELISPOT showed no IL-10 response to tTG-treated peptides 12 or 13. In one subject, there were no IFNγ responses to any A-gliadin peptide or α-chymotrypsin digested gliadin before, during or up to four days after bread challenge. In none of these Coeliac disease subjects did EMA status change from baseline when measured for up to two months after bread challenge.

PBMC from four healthy, EMA-negative subjects with the HLA-DQ alleles α1*0501, β1*0201 (ages 28-52, 2 females) who had been challenged for three days with bread after following a gluten free diet for one month, showed no IFNγ responses above the negative control to any of the A-gliadin peptides with or without tTG treatment. Thus, induction of IFNγ in PBMC to tTG-treated Pool 3 and A-gliadin peptides 56-70 (12) and 60-75 (13) were Coeliac disease specific (7/8 vs. 0/4, p<0.01 by Chi-squared analysis).

### Fine mapping of the minimal A-gliadin T cell epitope

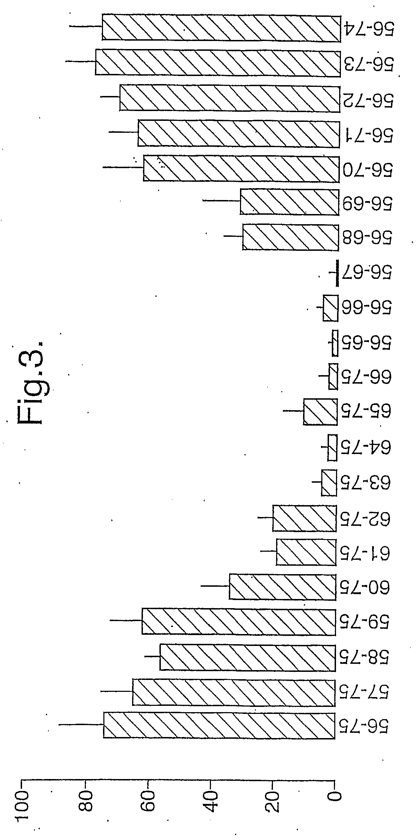

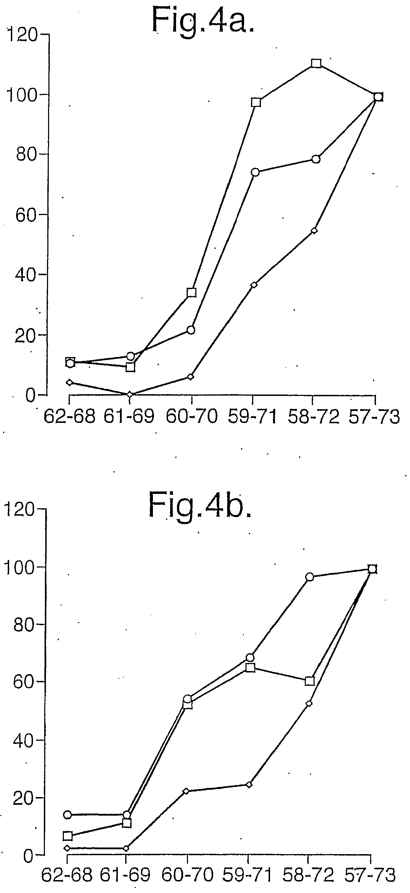

tTG-treated peptides representing truncations of A-gliadin 56-75 revealed that the same core peptide sequence QPQLP (SEQ ID NO:9) was essential for antigenicity in all of the five Coeliac disease subjects assessed (see Figure 3). PBMC IFNγ responses to tTG-treated peptides spanning this core sequence beginning with the 7-mer PQPQLPY (SEQ ID NO:4) and increasing in length, indicated that the tTG-treated 17-mer QLQPFPQPQLPYPQPQS (SEQ ID NO:10) (A-gliadin 57-73) possessed optimal activity in the IFNγ ELISPOT (see Figure 4).

### Deamidation of Q65 by tTG generates the immunodominant T cell epitope in A-gliadin

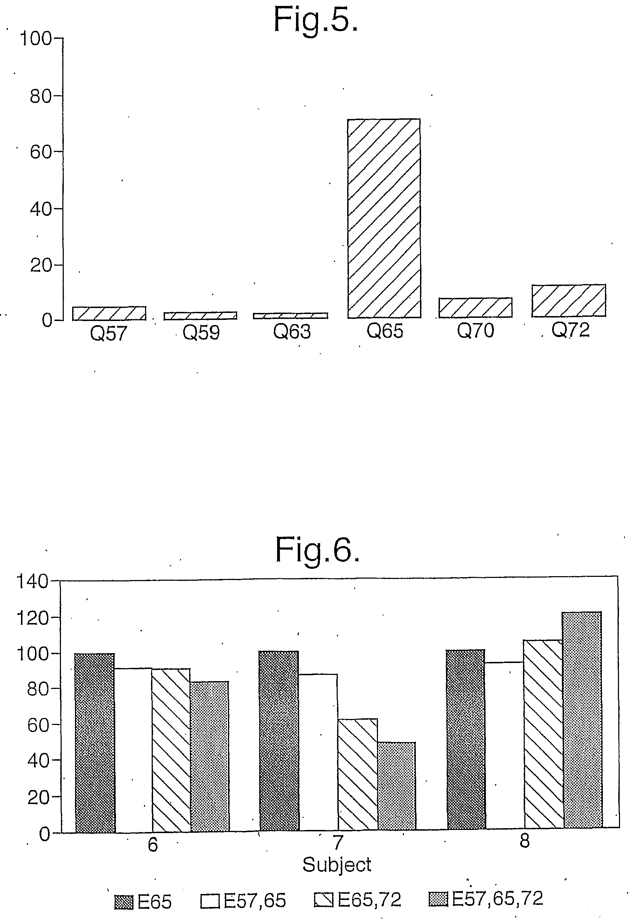

HPLC analysis demonstrated that tTG treatment of A-gliadin 56-75 generated a single product that eluted marginally later than the parent peptide. Amino acid sequencing indicated that out of the six glutamine (Q) residues contained in A-gliadin 56-75, Q65 was preferentially deamidated by tTG (see Figure 5). Bioactivity of peptides corresponding to serial expansions from the core A-gliadin 62-68 sequence in which glutamate (E) replaced Q65, was equivalent to the same peptides with Q65 after tTG-treatment (see Figure 4a). Replacement of Q57 and Q72 by E together or alone, with E65 did not enhance antigenicity of the 17-mer in the three Coeliac disease subjects studied (see Figure 6). Q57 and Q72 were investigated because glutamine residues followed by proline in gliadin peptides are not deamidated by tTG in vitro (W. Vader et al, Proceedings 8th International Symposium Coeliac Disease). Therefore, the immunodominant T cell epitope was defined as QLQPFPQPELPYPQPQS (SEQ ID NO:2).

### Immunodominant T cell epitope response is DQ2-restricted and CD4 dependent

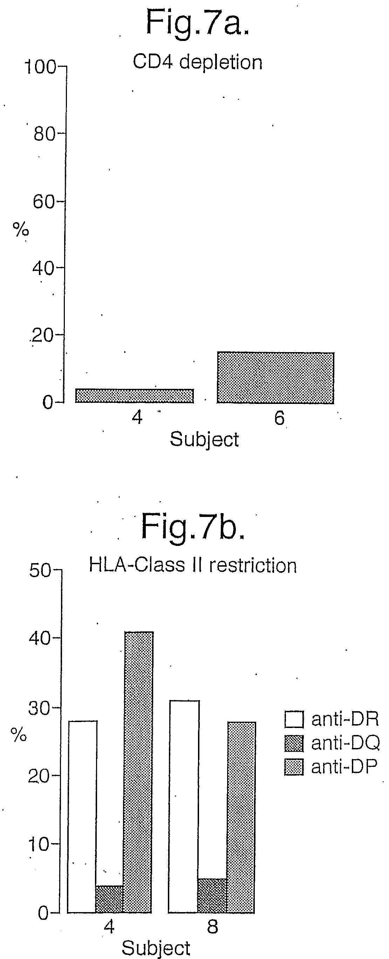

In two Coeliac disease subjects homozygous for HLA-DQ α1*0501, β1*0201, anti-DQ monoclonal antibody blocked the ELISPOT IFNγ response to tTG-treated A-gliadin 56-75, but anti-DP and -DR antibody did not (see Figure 7). Anti-CD4 and anti-CD8 magnetic bead depletion of PBMC from two Coeliac disease subjects indicated the IFNγ response to tTG-treated A-gliadin 56-75 is CD4 T cell-mediated.

### Discussion

In this study we describe a rather simple dietary antigen challenge using standard white bread to elicit a transient population of CD4 T cells in peripheral blood of Coeliac disease subjects responsive to a tTG-treated A-gliadin 17-mer with the sequence: QLQPFPQPELPYPQPQS (SEQ ID NO:2) (residues 57-73). The immune response to A-gliadin 56-75 (Q→E65) is restricted to the Coeliac disease-associated HLA allele, DQ α1*0501, β1*0201. Tissue transglutaminase action in vitro selectively deamidates Q65. Elicited peripheral blood IFNg responses to synthetic A-gliadin peptides with the substitution Q→E65 is equivalent to tTG-treated Q65 A-gliadin peptides; both stimulate up to 10-fold more T cells in the IFNg ELISPOT than unmodified Q65 A-gliadin peptides.

We have deliberately defined this Coeliac disease-specific T cell epitope using in vivo antigen challenge and short-term ex vivo immune assays to avoid the possibility of methodological artifacts that may occur with the use of T cell clones in epitope mapping. Our findings indicate that peripheral blood T cell responses to ingestion of gluten are rapid but short-lived and can be utilized for epitope mapping. In vivo antigen challenge has also shown there is a temporal hierarchy of immune responses to A-gliadin peptides; A-gliadin 57-73 modified by tTG not only elicits the strongest IFNg response in PBMC but it is also the first IFNg response to appear.

Because we have assessed only peptides spanning A-gliadin, there may be other epitopes in other gliadins of equal or greater importance in the pathogenesis of Coeliac disease. Indeed, the peptide sequence at the core of the epitope in A-gliadin that we have identified PQPQLPY (SEQ ID NO:4) is shared by several other gliadins (SwissProt and Trembl accession numbers: P02863, Q41528, Q41531, Q41533, Q9ZP09, P04722, P04724, P18573). However, A-gliadin peptides that have previously been shown to possess bioactivity in biopsy challenge and in vivo studies (for example: 31-43, 44-55, and 206-217)^{4,5} did not elicit IFNg responses in PBMC following three day bread challenge in Coeliac disease subjects. These peptides may be "secondary" T cell epitopes that arise with spreading of the immune response.

### Example 2

### The effect on T cell recognition of substitutions in the immunodominant epitope

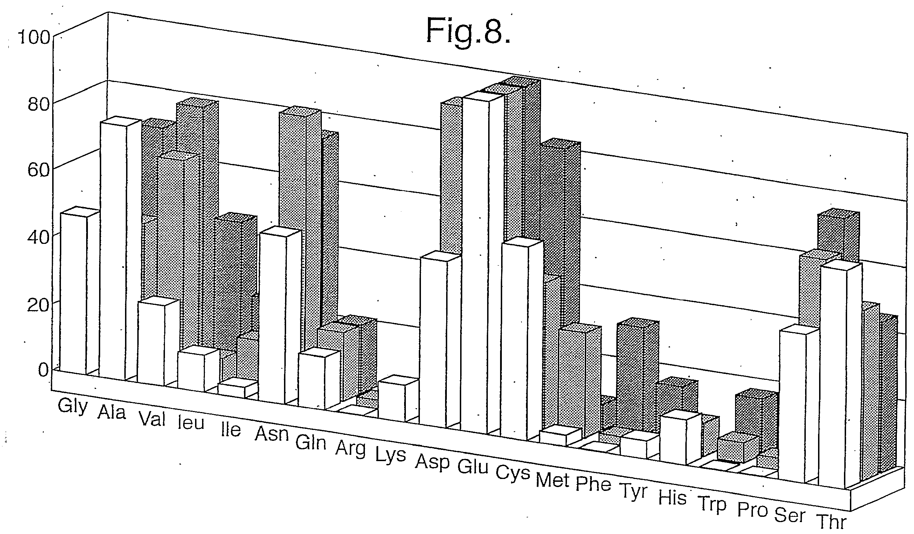

The effect of substituting the glutamate at position 65 in the 57-73 A-gliadin epitope was determined by measuring peripheral blood responses against the substituted epitopes in an IFNγ ELISPOT assay using synthetic peptides (at 50 µg/ml). The responses were measured in 3 Coeliac disease subjects 6 days after commencing gluten challenge (4 slices bread daily for 3 days). Results are shown in table 3 and Figure 8. As can be seen substitution of the glutamate to histidine, tyrosine, tryptophan, lysine, proline or arginine stimulated a response whose magnitude was less than 10% of the magnitude of the response to the immunodominant epitope. Thus mutation of A-gliadin at this position could be used to produce a mutant gliadin with reduce or absent immunoreactivity.

### Example 3

### Testing the immunoreactivity of equivalent peptides from other naturally occurring gliadins

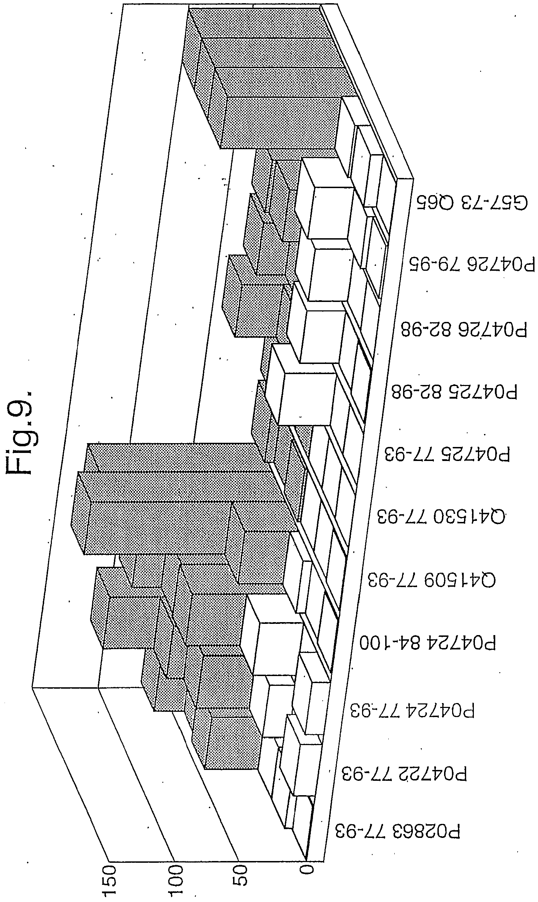

The immunoreactivity of equivalent peptides form other naturally occurring wheat gliadins was assessed using synthetic peptides corresponding to the naturally occurring sequences which were then treated with transglutaminase. These peptides were tested in an ELISPOT in the same manner and with PBMCs from the same subjects as described in Example 2. At least five of the peptides show immunoreactivity comparable to the A-gliadin 57-73 E65 peptide (after transglutaminase treatment) indicating that other gliadin proteins in wheat are also likely to induce this Coeliac disease-specific immune response (Table 4 and Figure 9).

### Methods

*Subjects:* Patients used in the study attended a Coeliac Clinic in Oxford, United Kingdom. Coeliac disease was diagnosed on the basis of typical small intestinal histology, and normalization of symptoms and small intestinal histology with gluten free diet.

*Tissue typing:* Tissue typing was performed using DNA extracted from EDTA-anticoagulated peripheral blood. HLA-DQA and DQB genotyping was performed by PCR using sequence-specific primer mixes⁶⁻⁸.

*Anti-endomysial antibody assay:* EMA were detected by indirect immunofluorescence using patient serum diluted 1:5 with monkey oesophagus, followed by FITC-conjugated goat antihuman IgA. IgA was quantitated prior to EMA, none of the subjects were IgA deficient.

*Antigen Challenge:* Coeliac disease subjects following a gluten free diet, consumed 4 slices of gluten-containing bread (50g/slice, Sainsbury's "standard white sandwich bread") daily for 3 or 10 days. EMA was assessed the week before and up to two months after commencing the bread challenge. Healthy subjects who had followed a gluten free diet for four weeks, consumed their usual diet including four slices of gluten-containing bread for three days, then returned to gluten free diet for a further six days.

IFNγ *and IL-10 ELISPOT:* PBMC were prepared from 50-100 ml of venous blood by Ficoll-Hypaque density centrifugation. After three washes, PBMC were resuspended in complete RPMI containing 10% heat inactivated human AB serum. ELISPOT assays for single cell secretion of IFNy and IL-10 were performed using commercial kits (Mabtech; Stockholm, Sweden) with 96-well plates (MAIP-S-45; Millipore, Bedford, MA) according to the manufacturers instructions (as described elsewhere⁹) with 2-5x10⁵ (IFNγ) or 0.4-1x10⁵ (IL-10) PBMC in each well. Peptides were assessed in duplicate wells, and Mycobacterium tuberculosis purified protein derivative (PPD RT49) (Serum Institute; Copenhagen, Denmark) (20 µg/ml) was included as a positive control in all assays.

*Peptides:* Synthetic peptides were purchased from Research Genetics (Huntsville, Alabama) Mass-spectroscopy and HPLC verified peptides' authenticity and >70% purity. Digestion of gliadin (Sigma; G-3375) (100 mg/ml) with α-chymotrypsin (Sigma; C-3142) 200:1 (w/w) was performed at room temperature in 0.1 M NH₄HCO₃ with 2M urea and was halted after 24 h by heating to 98°C for 10 minutes. After centrifugation (13,000g, 10 minutes), the gliadin digest supernatant was filter-sterilized (0.2 mm). Digestion of gliadin was verified by SDS-PAGE and protein concentration assessed. α-Chymotrypsin-digested gliadin (640 µg/ml) and synthetic gliadin peptides (15-mers: 160 µg/ml, other peptides: 0.1 mM) were individually treated with tTG (Sigma; T-5398) (50 µg/ml) in PBS + CaCl₂ 1 mM for 2 h at 37°C. Peptides and peptide pools were aliquotted into sterile 96-well plates and stored frozen at -20°C until use.

*Amino acid sequencing of peptides:* Reverse phase HPLC was used to purify the peptide resulting from tTG treatment of A-gliadin 56-75. A single product was identified and subjected to amino acid sequencing (automated sequencer Model 494A, Applied Biosystems, Foster City, California). The sequence of unmodified G56-75 was confirmed as: LQLQPFPQPQLPYPQPQSFP (SEQ ID NO:5), and tTG treated G56-75 was identified as: LQLQPFPQPELPYPQPQSFP (SEQ ID NO:11). Deamidation of glutamyl residues was defined as the amount (pmol) of glutamate recovered expressed as a percent of the combined amount of glutamine and glutamate recovered in cycles 2, 4, 8, 10, 15 and 17 of the amino acid sequencing. Deamidation attributable to tTG was defined as (% deamidation of glutamine in the tTG treated peptide - % deamidation in the untreated peptide) / (100 - % deamidation in the untreated peptide).

*CD4*/*CD8 and HLA Class II Restriction:* Anti-CD4 or anti-CD8 coated magnetic beads (Dynal, Oslo, Norway) were washed four times with RPMI then incubated with PBMC in complete RPMI containing 10% heat inactivated human AB serum (5x10⁶ cells/ml) for 30 minutes on ice. Beads were removed using a magnet and cells remaining counted. In vivo HLA-class II restriction of the immune response to tTG-treated A-gliadin 56-75 was established by incubating PBMC (5x10⁶ cells/ml) with anti-HLA-DR (L243), -DQ (L2), and -DP (B7.21) monoclonal antibodies (10 µg/ml) at room temperature for one hour prior to the addition of peptide.

### Example 4

### Mucosal integrin expression by gliadin -specific peripheral blood lymphocytes

Interaction between endothelial and lymphocyte adressins facilitates homing of organ-specific lymphocytes. Many adressins are known. The heterodimer α₄β₇ is specific for lamina propria gut and other mucosal lymphocytes, and α^{E}β₇ is specific and intra-epithelial lymphocytes in the gut and skin. Approximately 30% of peripheral blood CD4 T cells express α₄β₇ and are presumed to be in transit to a mucosal site, while 5% of peripheral blood T cells express α^{E}β₇. Immunomagnetic beads coated with antibody specific for α^{E} or β₇ deplete PBMC of cells expressing α^{E}β₇ or α^{E}β₇ and α₄β₇, respectively. In combination with ELISpot assay, immunomagnetic bead depletion allows determination of gliadin-specific T cell addressin expression that may identify these cells as homing to a mucosal surface. Interestingly, gluten challenge in vivo is associated with rapid influx of CD4 T cells to the small intestinal lamina propria (not intra-epithelial sites), where over 90% lymphocytes express α₄β₇.

Immunomagnetic beads were prepared and used to deplete PBMC from coeliac subjects on day 6 or 7 after commencing 3 day gluten challenge. FACS analysis demonstrated α^{E} beads depleted approximately 50% of positive CD4 T cells, while β₇ beads depleted all β₇ positive CD4 T cells. Depletion of PBMC using CD4- or βγ-beads, but not CD8- or α^{E} -beads, abolished responses in the interferon gamma ELISpot. tTG gliadin and PPD responses were abolished by CD4 depletion, but consistently affected by integrin-specific bead depletion.

Thus A-gliadin 57-73 QE65-specific T cells induced after gluten challenge in coeliac disease express the integrin, α₄β₇, present on lamina propria CD4 T cells in the small intestine.

### Example 5

### Optimal T cell Epitope Length

Previous data testing peptides from 7 to 17 amino acids in length spanning the core of the dominant T cell epitope in A-gliadin indicated that the 17mer, A-gliadin 57-73 QE65 (SEQ ID NO:2) induced maximal responses in the interferon gamma Elispot using peripheral blood mononuclear cells (PBMC) from coeliac volunteers 6 days after commencing a 3-day gluten challenge.

Peptides representing expansions form the core sequence of the dominant T cell epitope in A-gliadin were assessed in the IFN gamma ELISPOT using peripheral blood mononuclear cells (PBMC) from coeliac volunteers in 6 days after commencing a 3-day gluten challenge (n=4). Peptide 13: A-gliadin 59-71 QE65 (13mer), peptide 15: 58-72 QE65 (15mer), ..., peptide 27: 52-78 SE65 (27mer).

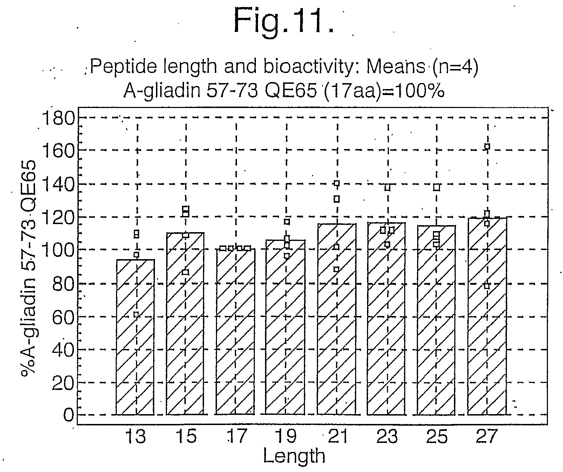

As shown in Figure 11 expansion of the A-gliadin 57-73 QE65 sequence does not substantially enhance response in the IFNgamma Elispot. Subsequent Examples characterise the agonist and antagonist activity of A-gliadin 57-73 QE65 using 17mer peptides.

### Example 6

### Comparison of A-gliadin 57-73 QE65 with other DQ2-restricted T cell epitopes in coeliac disease

Dose response studies were performed using peptides corresponding to unmodified and transglutaminase-treated peptides corresponding to T cell epitopes of gluten-specific T cell clones and lines from intestinal biopsies of coeliac subjects. Responses to peptides were expressed as percent of response to A-gliadin 57-73 QE65. All subjects were HLA-DQ2+ (none were DQ8+).

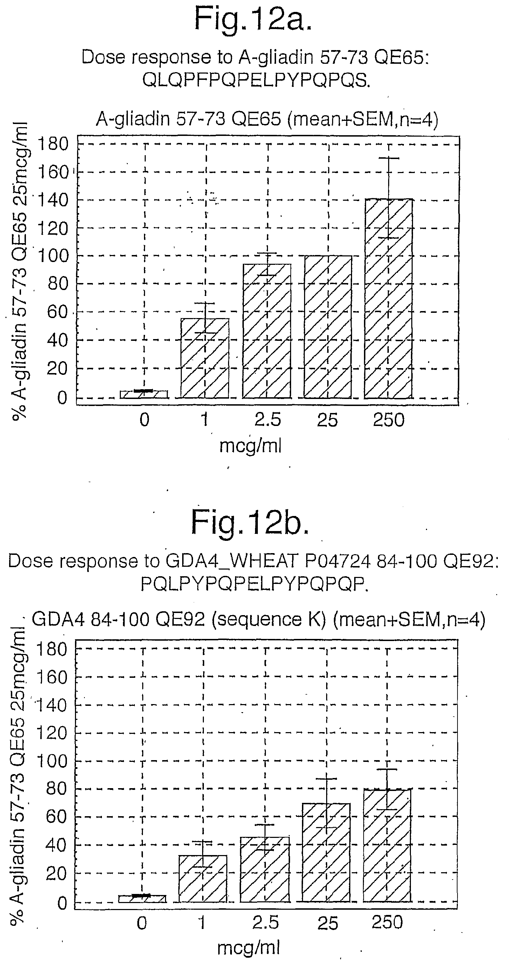

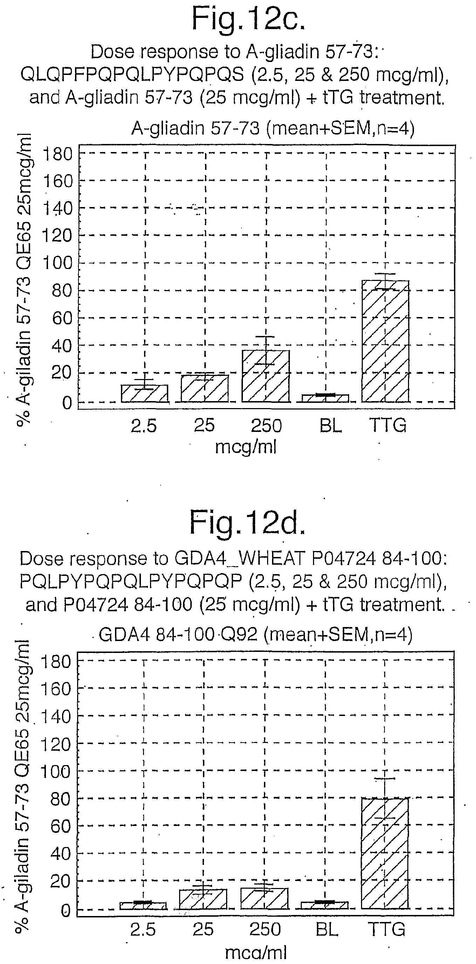

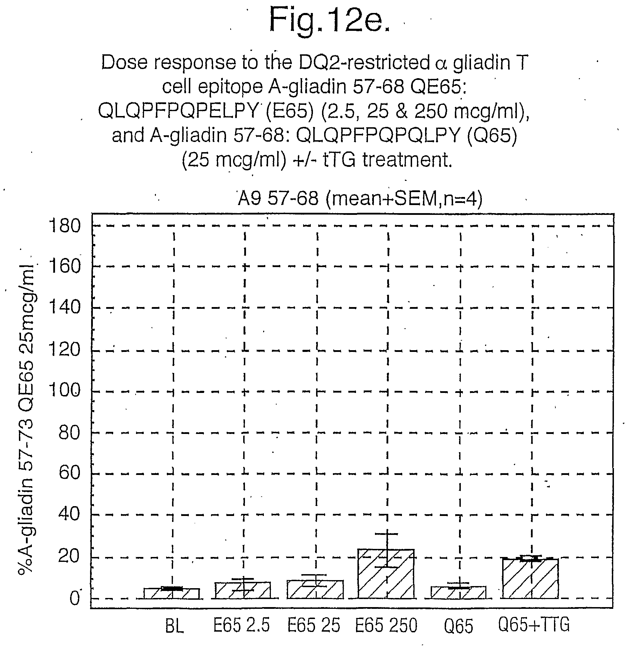

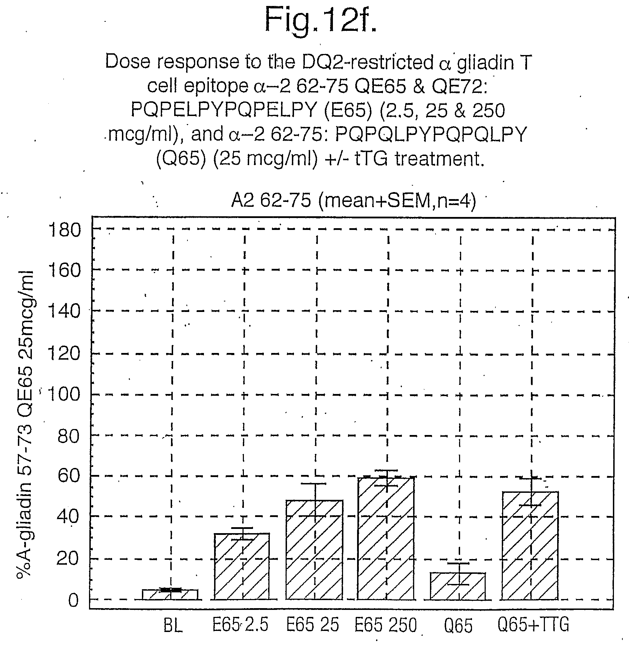

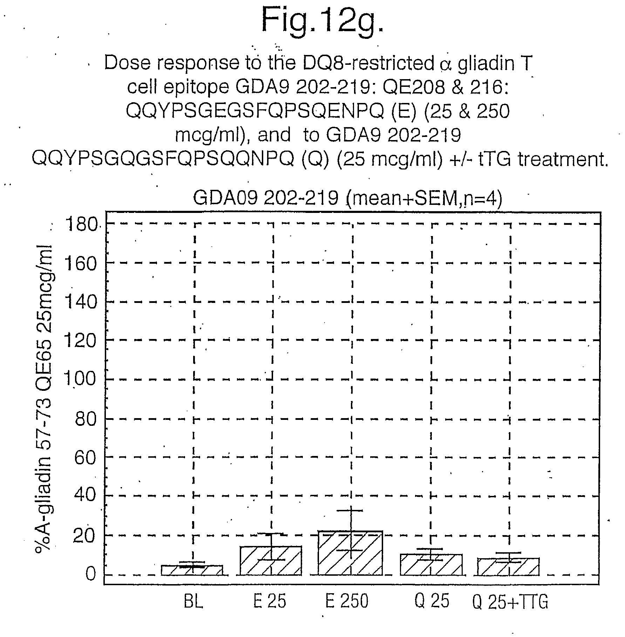

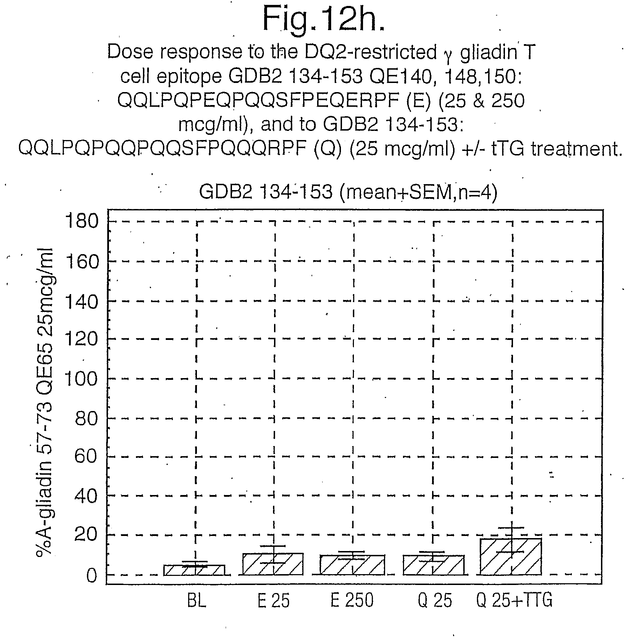

The studies indicate that A-gliadin 57-73 QE65 is the most potent gliadin peptide for induction of interferon gamma in the ELISpot assay using coeliac PBMC after gluten challenge (see Figure 12a-h, and Tables 5 and 6). The second and third epitopes are suboptimal fragments of larger peptides i.e. A-gliadin 57-73 QE65 and GDA4_WHEAT P04724-84-100 QE92. The epitope is only modestly bioactive (approximately 1/20^{th} as active as A-gliadin 57-73 QE65 after blank is subtracted).

A-gliadin 57-73 QE65 is more potent than other known T cell epitopes in coeliac disease. There are 16 polymorphisms of A-gliadin 57-73 (including the sequence PQLPY (SEQ ID NO:12)) amongst sequenced gliadin genes, their bioactivity is assessed next.

### Example 7

### Comparison of gliadin- and A-gliadin 57-73 QE65-specific responses in peripheral blood

The relative contribution of the dominant epitope, A-gliadin 57-73 QE65, to the total T cell response to gliadin in coeliac disease is a critical issue. Pepsin-trypsin and chymotrypsin-digested gliadin have been traditionally used as antigen for development of T cell lines and clones in coeliac disease. However, it is possible that these proteases may cleave through certain peptide epitopes. Indeed, chymotrypsin digestion of recombinant α9-gliadin generates the peptide QLQPFPQPELPY (SEQ ID NO:13), that is a truncation of the optimal epitope sequence QLQPFPQPELPYPQPQS (SEQ ID NO:2) (see above). Transglutaminase-treatment substantially increases the potency of chymotrypsin-digested gliadin in proliferation assays of gliadin-specific T cell clones and lines Hence, transglutaminase-treated chymotrypsin-digested gliadin (tTG gliadin) may not be an ideal antigen, but responses against this mixture may approximate the "total" number of peripheral blood lymphocyte specific for gliadin. Comparison of responses against A-gliadin 57-73 QE65 and tTG gliadin in the ELISpot assay gives an indication of the contribution of this dominant epitope to the overall immune response to gliadin in coeliac disease, and also be a measure of epitope spreading.

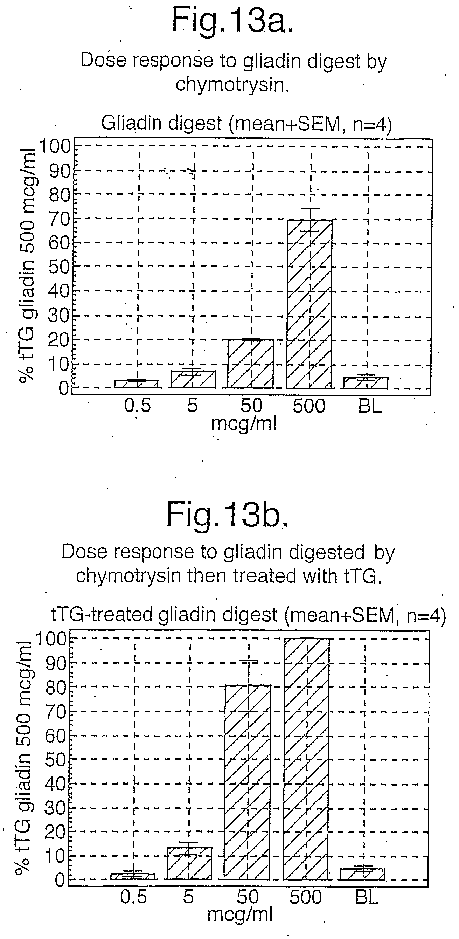

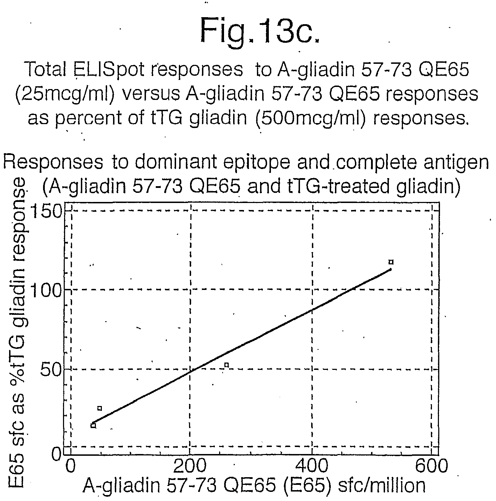

PBMC collected on day 6 or 7 after commencing gluten challenge in 4 coeliac subjects were assessed in dose response studies using chymotrypsin-digested gliadin +/- tTG treatment and compared with ELISpot responses to an optimal concentration of A-gliadin 57-73 QE65 (25mcg/ml). TTG treatment of gliadin enhanced PBMC responses in the ELISpot approximately 10-fold (tTG was comparable to blank when assessed alone) (see Figure 13a-c). In the four coeliac subjects studied, A-gliadin 57-73 QE65 (25 mcg/ml) elicited responses between 14 and 115% those of tTG gliadin (500 mcg/ml), and the greater the response to A-gliadin 57-73 QE65 the greater proportion it represented of the tTG gliadin response.

Relatively limited data suggest that A-gliadin 57-73 QE65 responses are comparable to tTG gliadin in some subjects. Epitope spreading associated with more evolved anti-gliadin T cell responses may account for the smaller contribution of A-gliadin 57-73 QE65 to "total" gliadin responses in peripheral blood in some individuals. Epitope spreading may be maintained in individuals with less strictly gluten free diets.

### Example 8

### Definition of gliadin peptides bioactive in coeliac disease: polymorphisms of A-gliadin 57-73

Overlapping 15mer peptides spanning the complete sequence of A-gliadin were assessed in order to identify the immunodominant sequence in coeliac disease. A-gliadin was the first fully sequenced alpha gliadin protein and gene, but is one of approximately 30-50 related alpha gliadin proteins in wheat. Twenty five distinct alpha-gliadin genes have been identified by searching protein data bases, Swiss-Prot and TREMBL describing a further 8 alpha-gliadins. Contained within these 25 alpha-gliadins, there are 16 distinct polymorphisms of the sequence corresponding to A-gliadin 57-73 (see Table 7).

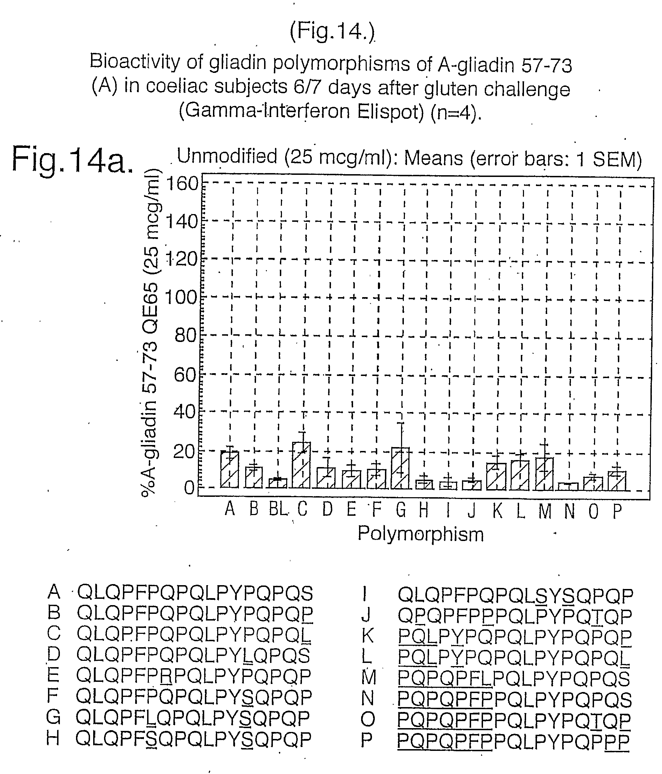

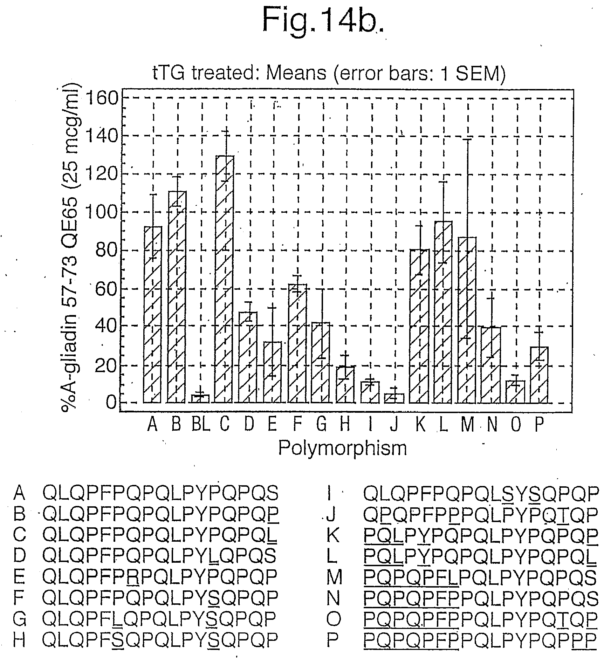

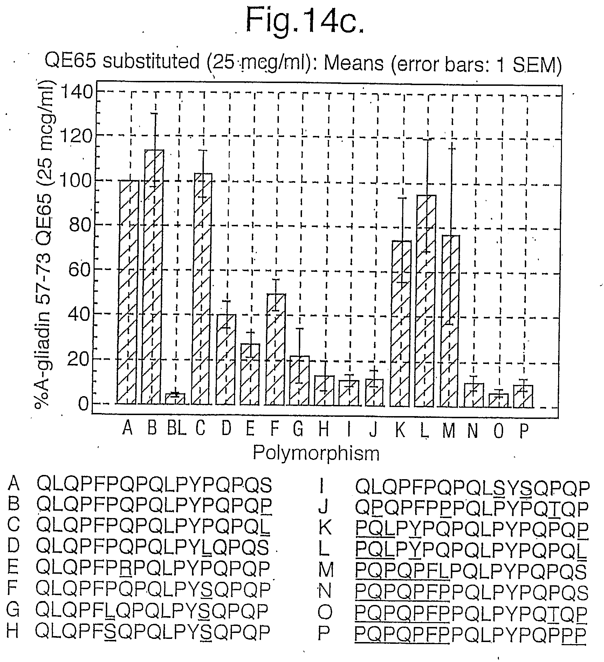

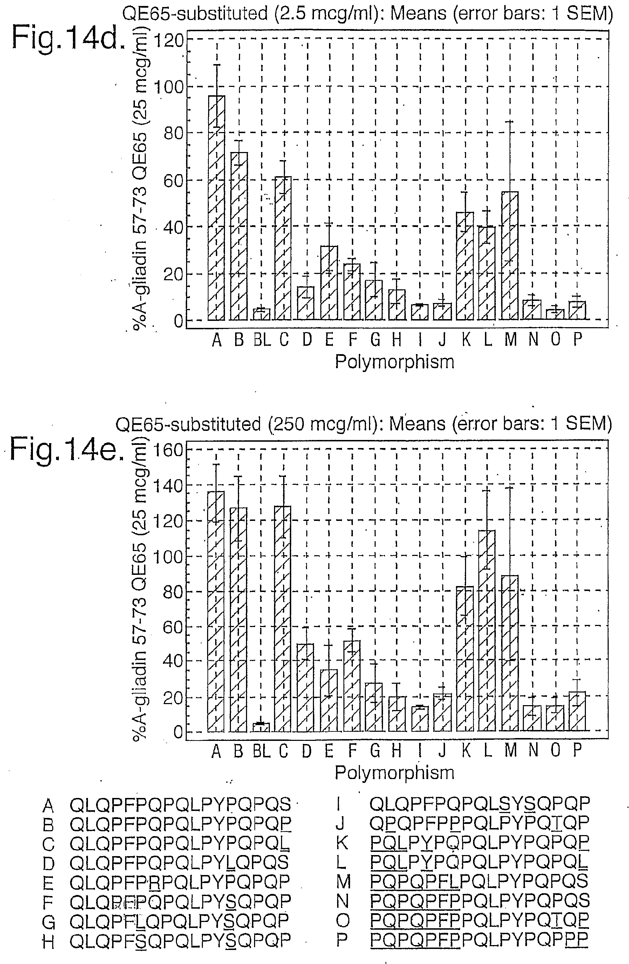

Synthetic peptides corresponding to these 16 polymorphisms, in an unmodified form, after treatment with transglutaminase in vitro, as well as with glutamate substituted at position 10 (equivalent to QE65 in A-gliadin 57-73) were assessed using PBMC from coeliac subjects, normally following a gluten free diet, day 6 or 7 after gluten challenge in interferon gamma ELISpot assays. Glutamate-substituted peptides were compared at three concentrations (2.5, 25 and 250 mcg/ml), unmodified peptide and transglutaminase-treated peptides were assessed at 25 mcg/ml only. Bioactivity was expressed as % of response associated with A-gliadin 57-73 QE65 25 mcg/ml in individual subjects (n=4). (See Fig 14).

Bioactivity of "wild-type" peptides was substantially increased (>5-fold) by treatment with transglutaminase. Transglutaminase treatment of wild-type peptides resulted in bioactivity similar to that of the same peptides substituted with glutamate at position 10. Bioactivities of five glutamate-substituted peptides (B, C, K, L, M), were >70% that of A-gliadin 57-73 QE65 (A), but none was significantly more bioactive than A-gliadin 57-73 QE65. PBMC responses to glutamate-substituted peptides at concentrations of 2.5 and 250 mcg/ml were comparable to those at 25 mcg/ml. Six glutamate-substituted gliadin peptides (H, I, J, N, O, P) were <15% as bioactive as A-gliadin 57-73 QE65. Other peptides were intermediate in bioactivity.

At least six gliadin-derived peptides are equivalent in potency to A-gliadin 57-73 QE65 after modification by transglutaminase. Relatively non-bioactive polymorphisms of A-gliadin 57-73 also exist. These data indicate that transglutaminase modification of peptides from several gliadins of *Triticum aestivum, T. uartu* and *T. spelta* may be capable of generating the immunodominant T cell epitope in coeliac disease.

Genetic modification of wheat to generate non-coeliac-toxic wheat may likely require removal or modification of multiple gliadin genes. Generation of wheat containing gliadins or other proteins or peptides incorporating sequences defining altered peptide ligand antagonists of A-gliadin 57-73 is an alternative strategy to generate genetically modified wheat that is therapeutic rather than "non-toxic" in coeliac disease.

### Example 9

### Definition of Core Epitope Sequence:

Comparison of peptides corresponding to truncations of A-gliadin 56-75 from the N- and C-terminal indicated that the core sequence of the T cell epitope is PELPY (A-gliadin 64-68). Attempts to define non-agonists and antagonists will focus on variants of A-gliadin that are substituted at residues that substantially contribute to its bioactivity.

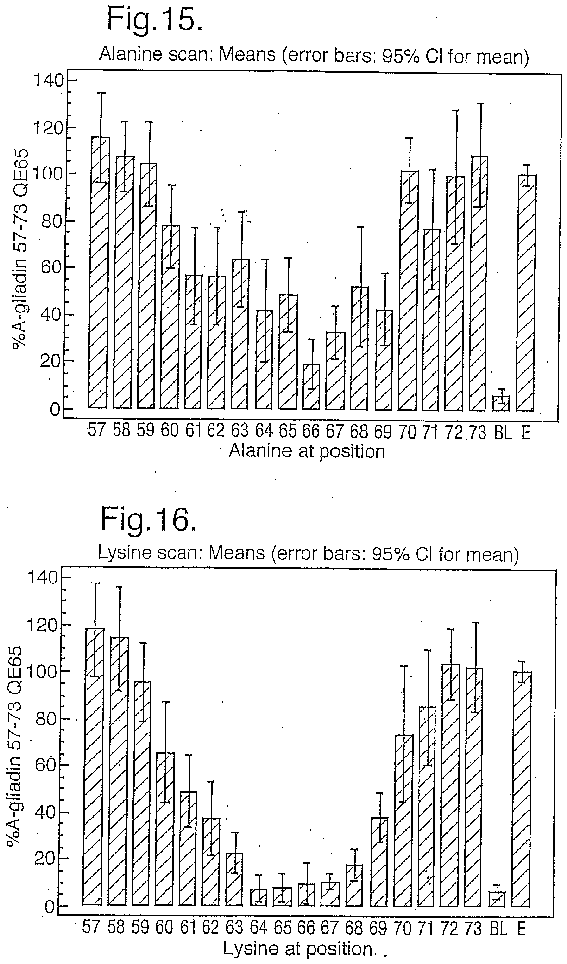

Peptides corresponding to A-gliadin 57-73 QE65 with alanine (Figure 15) or lysine (Figure 16) substituted for residues 57 to 73 were compared in the IFN gamma ELISPOT using peripheral blood mononuclear cells (PBMC) from coeliac volunteers 6 days after commencing a 3-day gluten challenge (n=8). (BL is blank, E is A-gliadin 57-73 QE65: QLQPFPQPELPYPQPQS (SEQ ID NO:2)).

It was found that residues corresponding to A-gliadin 60-70 QE65 (PFPQPELPYPQ (SEQ ID NO:14)) contribute substantially to the bioactivity in A-gliadin 57-73 QE65. Variants of A-gliadin 57-73 QE65 substituted at positions 60-70 are assessed in a 2-step procedure. Initially, A-gliadin 57-73 QE65 substituted at positions 60-70 using 10 different amino acids with contrasting properties are assessed. A second group of A-gliadin 57-73 QE65 variants (substituted with all other naturally occurring amino acids except cysteine at positions that prove are sensitive to modification) are assessed in a second round.

### Example 10

### Agonist activity of substituted variants of A-gliadin 57-73 QE65

A-gliadin 60-70 QE65 is the core sequence of the dominant T cell epitope in A-gliadin. Antagonist and non-agonist peptide variants of this epitope are most likely generated by modification of this core sequence. Initially, A-gliadin 57-73 QE65 substituted at positions 60-70 using 10 different amino acids with contrasting properties will be assessed in the IFNgamma ELISPOT using PBMC from coeliac subjects 6 days after starting 3 day gluten challenge. A second group of A-gliadin 57-73 QE65 variants (substituted with all other naturally occurring amino acids except cysteine) at positions 61-70 were also assessed. Both groups of peptides (all at 50 mcg/ml, in duplicate) were assessed using PBMC from 8 subjects and compared to the unmodified peptide (20 replicates per assay). Previous studies indicate that the optimal concentration for A-gliadin 57-73 QE65 in this assay is between 10 and 100 mcg/ml.

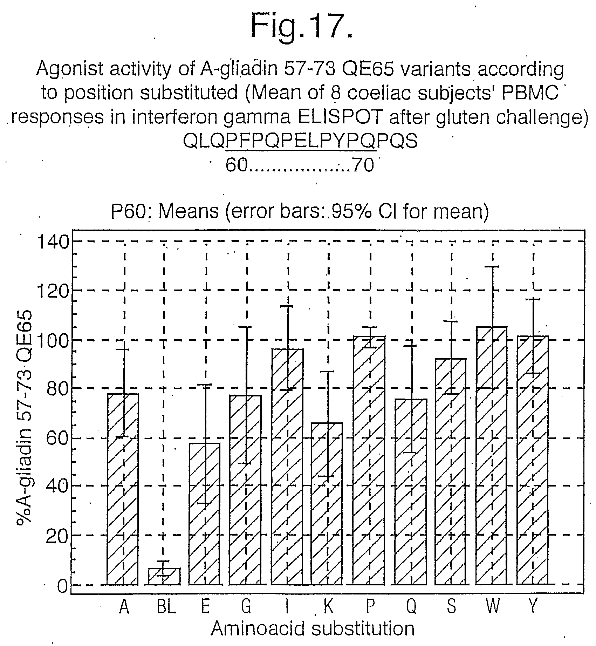

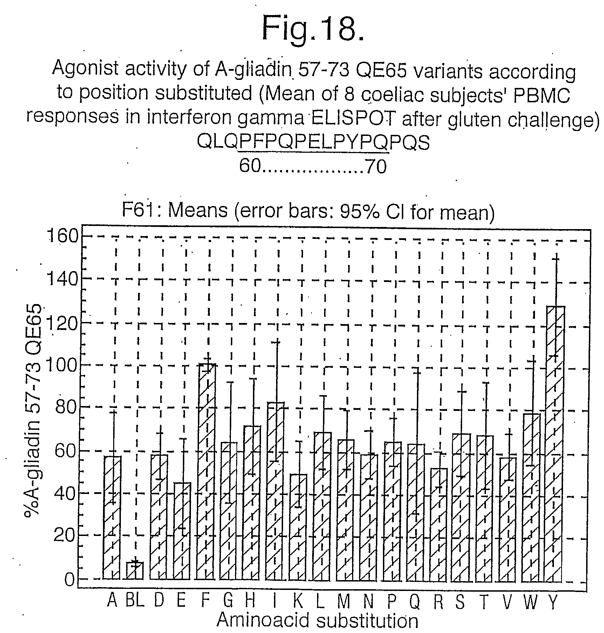

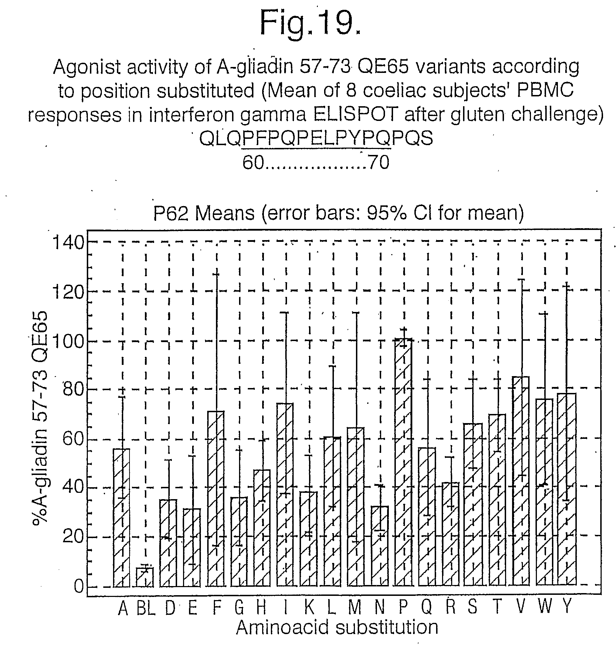

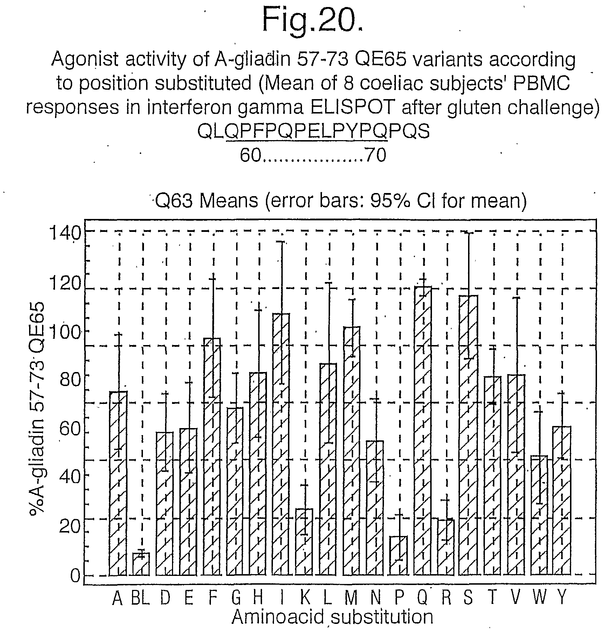

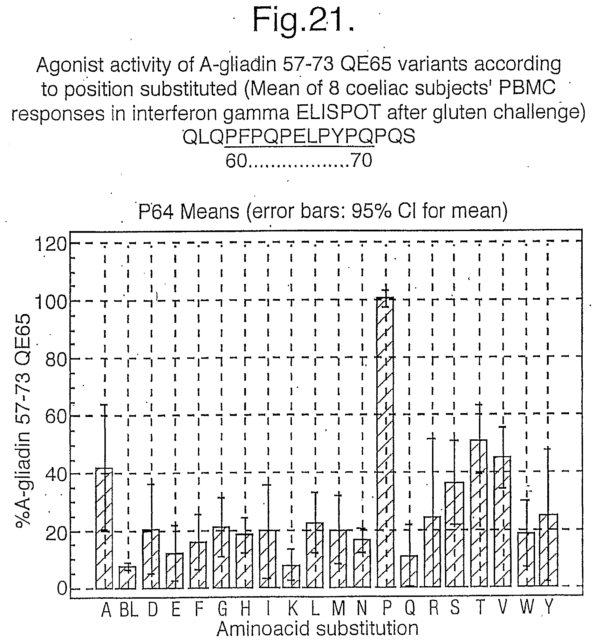

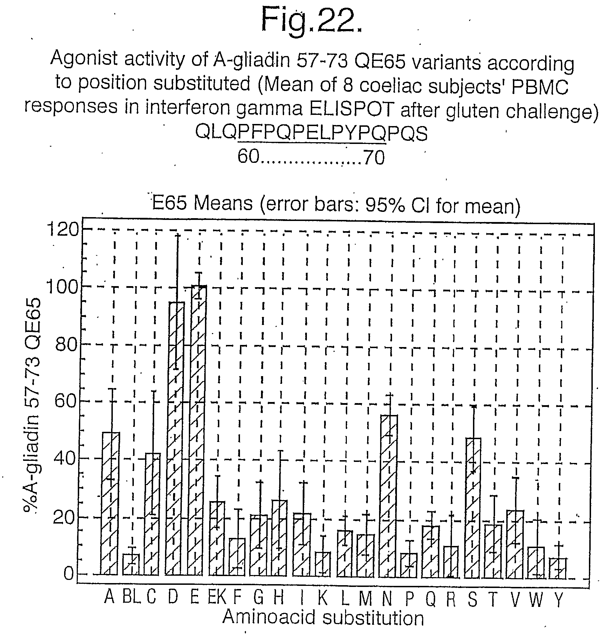

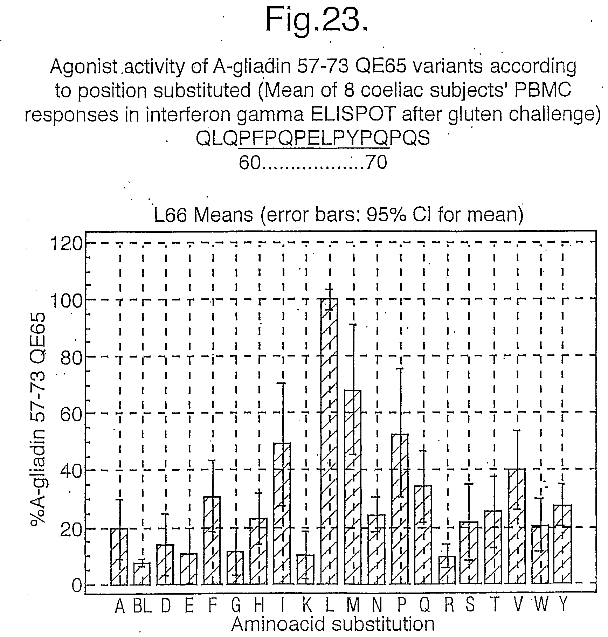

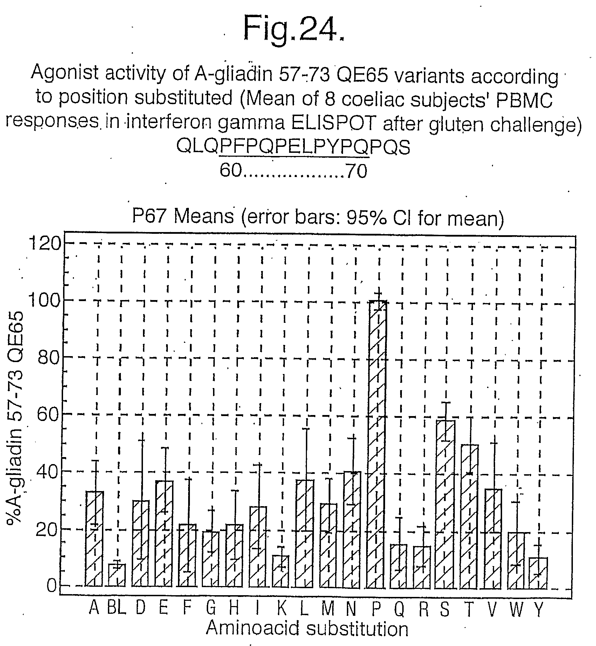

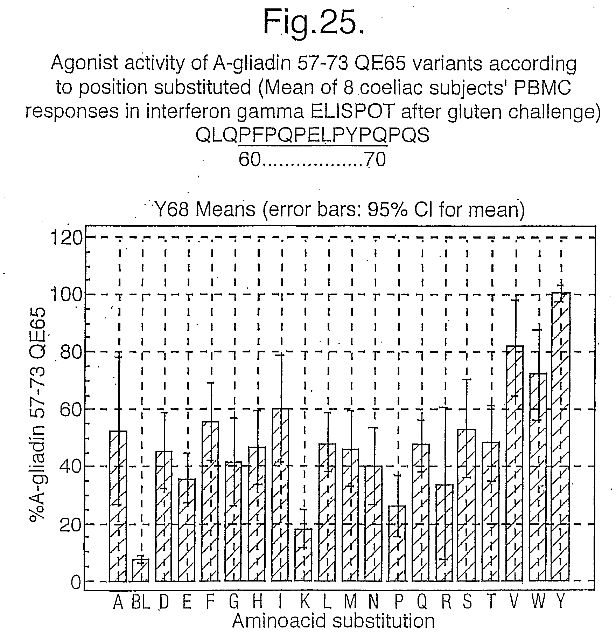

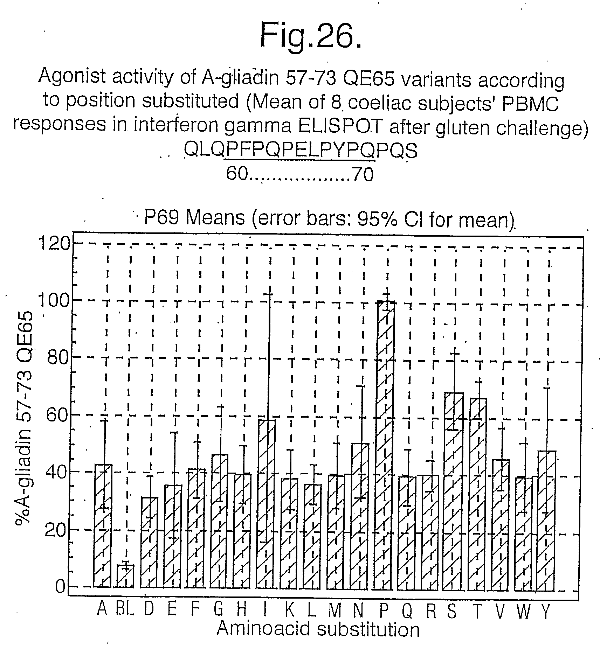

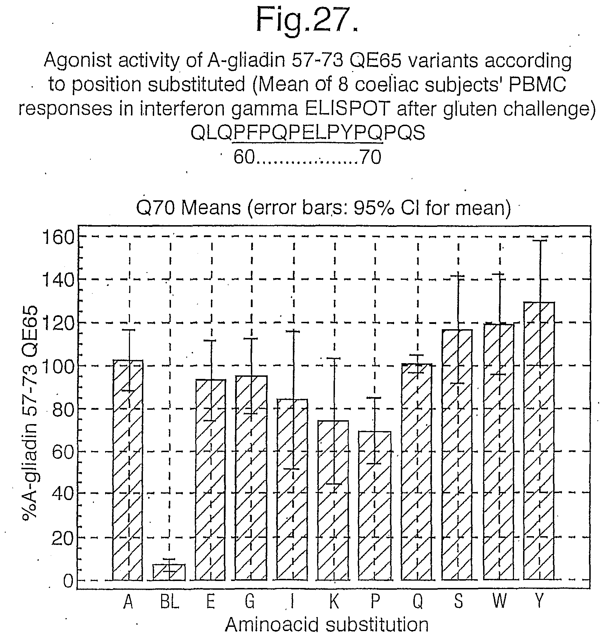

Results are expressed as mean response in spot forming cells (95% confidence interval) as % A-G 57-73 QE65 mean response in each individual. Unpaired t-tests will be used to compare ELISPOT responses of modified peptides with A-G 57-73 QE65. Super-agonists were defined as having a greater response than A-G 57-73 QE65 at a level of significance of p<0.01; partial agonists as having a response less than A-G 57-73 QE65 at a level of significance of p<0.01, and non-agonists as being not significantly different (p>0.01) from blank (buffer without peptide). Peptides with agonist activity 30% or less that of A-gliadin 57-73 QE65 were considered "suitable" partial or non-agonists to assess for antagonistic activity (see Table 8 and Figures 17-27).

The IFNgamma ELISPOT response of PBMC to A-gliadin 57-73 QE65 is highly specific at a molecular level. Proline at position 64 (P64), glutamate at 65 (E65) and leucine at position 66 (L66), and to a lesser extent Q63, P67, Y68 and P69 are particularly sensitive to modification. The substitutions Y61 and Y70 both generate super-agonists with 30% greater bioactivity than the parent peptide, probably by enhancing binding to HLA-DQ2 since the motif for this HLA molecule indicates a preference for bulky hydrophobic resides at positions 1 and 9. Eighteen non-agonist peptides were identified. Bioactivities of the variants (50 mcg/ml): P65, K64, K65 and Y65 (bioactivity 7-8%) were comparable to blank (7%). In total, 57 mutated variants of A-gliadin 57-73 QE65 were 30% or less bioactive than A-gliadin 57-73 QE65.

The molecular specificity of the peripheral blood lymphocyte (PBL) T cell response to the dominant epitope, A-gliadin 57-73 QE65, is consistently reproducible amongst HLA-DQ2+ coeliac subjects, and is highly specific to a restricted number of amino acids in the core 7 amino acids. Certain single-amino acid variants of A-gliadin 57-73 QE65 are consistently non-agonists in all HLA-DQ2+ coeliac subjects.

### Example 11

### Antagonist activity of substituted variants

The homogeneity of the PBL T cell response to A-gliadin 57-73 QE65 in HLA-DQ2+ coeliac disease suggests that altered peptide ligands (APL) capable of antagonism in PBMC ex vivo may exist, even though the PBL T cell response is likely to be poly- or oligo-clonal. APL antagonists are generally weak agonists. Fifty-seven single amino acid-substituted variants of A-gliadin 57-73 QE65 with agonist activity 30% or less have been identified and are suitable candidates as APL antagonists. In addition, certain weakly bioactive naturally occurring polymorphisms of A-gliadin 57-73 QE65 have also been identified (see below) and may be "naturally occurring" APL antagonists. It has also been suggested that competition for binding MHC may also antagonise antigen-specific T cell immune. Hence, non-gliadin peptides that do not induce IFNgamma responses in coeliac PBMC after gluten challenge but are known to bind to HLA-DQ2 may be capable of reducing T cell responses elicited by A-gliadin 57-73 QE65. Two peptides that bind avidly to HLA-DQ2 are HLA class 1 α 46-60 (HLA 1a) (PRAPWIEQEGPEYW (SEQ ID NO:15)) and thyroid peroxidase (tp) 632-645Y (IDVWLGGLLAENFLPY (SEQ ID NO:16)).

Simultaneous addition of peptide (50µg/ml) or buffer and A-gliadin 57-73 QE65 (10µg/ml) in IFNgamma ELISPOT using PBMC from coeliac volunteers 6 days after commencing 3 day gluten challenge (n=5). Results were expressed as response with peptide plus A-G 57-73 QE65 (mean of duplicates) as % response with buffer plus A-G 57-73 QE65 (mean of 20 replicates). (See Table 9).

Four single amino acid-substituted variants of A-gliadin 57-73 QE65 reduce the interferon gamma PBMC ELISPOT response to A-gliadin 57-73 QE65 (p<0.01) by between 25% and 28%, 13 other peptide variants reduce the ELISPOT response by between 18% and 24% (p<0.06). The HLA-DQ2 binder, thyroid peroxidase (tp) 632-645Y reduces PBMC interferon gamma responses to A-gliadin 57-73 QE65 by 31% (p<0.0001) but the other HLA-DQ2 binder, HLA class 1 α 46-60, does not alter responses (see Tables 9 and 10). The peptide corresponding to a transglutaminase-modified polymorphism of A-gliadin 57-73, SwissProt accession no.: P04725 82-98 QE90 (PQPQPFPPELPYPQPQS (SEQ ID NO:17)) reduces responses to A-gliadin 57-73 QE65 by 19% (p<0.009) (see Table 11).

Interferon gamma responses of PBMC to A-gliadin 57-73 QE65 in ELISPOT assays are reduced by co-administration of certain single-amino acid A-gliadin 57-73 QE65 variants, a polymorphism of A-gliadin 57-73 QE65, and an unrelated peptide known to bind HLA-DQ2 in five-fold excess. These finding suggest that altered peptide ligand antagonists of A-gliadin 57-73 QE65 exist. Not only putative APL antagonists but also certain peptides that bind HLA-DQ2 effectively reduce PBL T cell responses to A-gliadin 57-73 QE65.

These findings support two strategies to interrupt the T cell response to the dominant A-gliadin epitope in HLA-DQ2+ coeliac disease.
1. Optimisation of APL antagonists by substituting amino acids at more than one position (64-67) for use as "traditional" peptide pharmaceuticals or for specific genetic modification of gliadin genes in wheat.
2. Use of high affinity HLA-DQ2 binding peptides to competitively inhibit presentation of A-gliadin 57-73 QE65 in association with HLA-DQ2.
These two approaches may be mutually compatible. Super-agonists were generated by replacing F61 and Q70 with tyrosine residues. It is likely these super-agonists resulted from improved binding to HLA-DQ2 rather than enhanced contact with the T cell receptor. By combining these modifications with other substitutions that generate modestly effective APL antagonists might substantially enhance the inhibitory effect of substituted A-gliadin 57-73 QE65 variants.

### Example 12

### Development of interferon gamma ELISpot using PBMC and A-gliadin 57-73 QE65 and P04724 84-100 QE92 as a diagnostic for coeliac disease: Definition of immune-responsiveness in newly diagnosed coeliac disease

Induction of responsiveness to the dominant A-gliadin T cell epitope in PBMC measured in the interferon gamma ELISpot follows gluten challenge in almost all DQ2+ coeliac subjects following a long term strict gluten free diet (GFD) but not in healthy DQ2+ subjects after 4 weeks following a strict GFD. A-gliadin 57-73 QE65 responses are not measurable in PBMC of coeliac subjects before gluten challenge and pilot data have suggested these responses could not be measured in PBMC of untreated coeliacs. These data suggest that in coeliac disease immune-responsiveness' to A-gliadin 57-73 QE65 is restored following antigen exclusion (GFD). If a diagnostic test is to be developed using the ELISpot assay and PBMC, it is desirable to define the duration of GFD required before gluten challenge is capable of inducing responses to A-gliadin 57-73 QE65 and other immunoreactive gliadin peptides in blood.

Newly diagnosed DQ2+ coeliac subjects were recruited from the gastroenterology outpatient service. PBMC were prepared and tested in interferon gamma ELISpot assays before subjects commenced GFD, and at one or two weeks after commencing GFD. In addition, gluten challenge (3 days consuming 4 slices standard white bread, 200g/day) was performed at one or two weeks after starting GFD. PBMC were prepared and assayed on day six are after commencing gluten challenge. A-gliadin 57-73 QE65 (A), P04724 84-100 QE92 (B) (alone and combined) and A-gliadin 57-73 QP65 (P65) (non-bioactive variant, see above) (all 25 mcg/ml) were assessed.

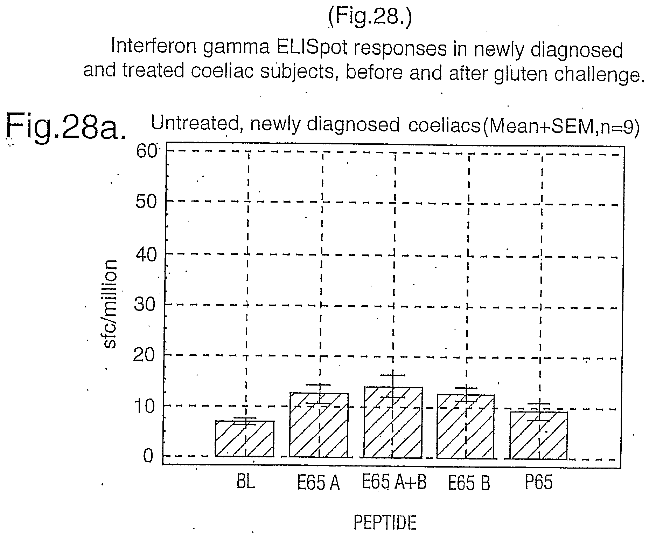

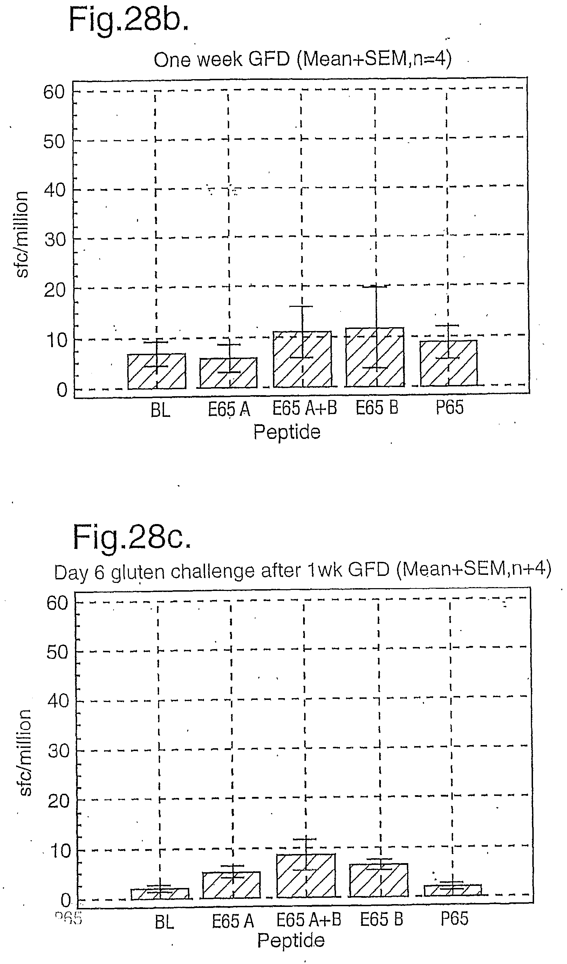

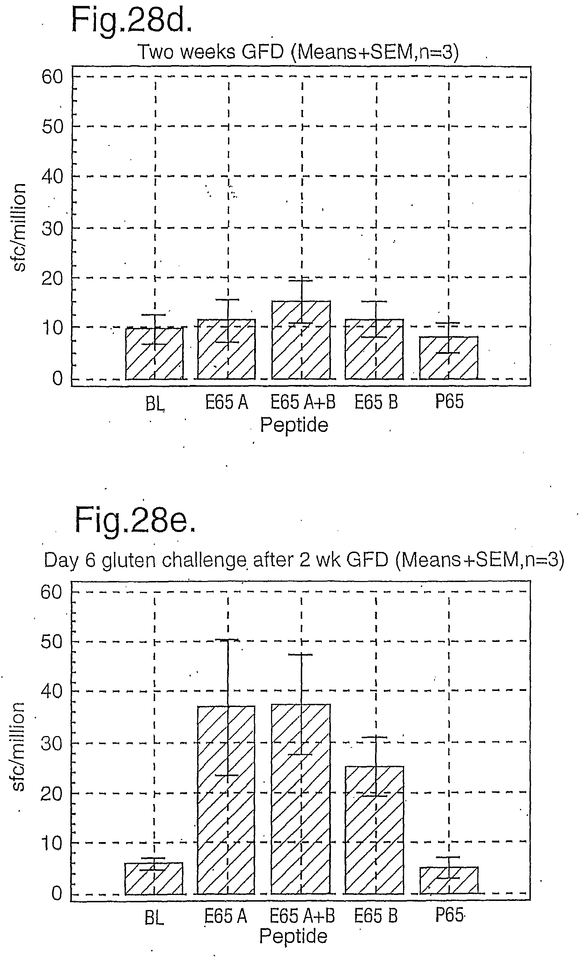

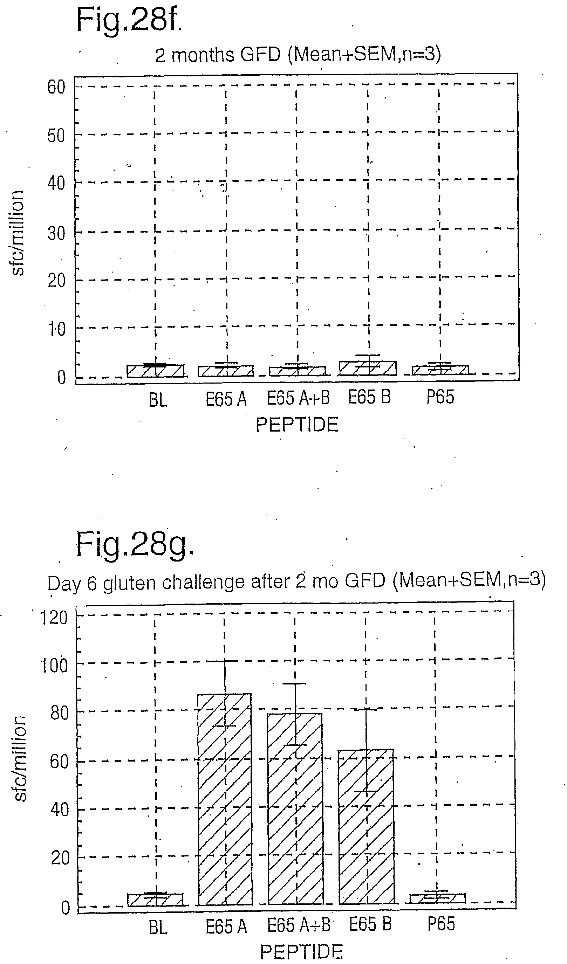

All but one newly diagnosed coeliac patient was DQ2+ (one was DQ8+) (n=11). PBMC from newly diagnosed coeliacs that were untreated, or after 1 or 2 weeks following GFD did not show responses to A-gliadin 57-73 QE65 and P04724 84-100 QE92 (alone or combined) that were not significantly different from blank or A-gliadin 57-73 QP65 (n=9) (see Figure 28). Gluten challenge in coeliacs who had followed GFD for only one week did not substantially enhance responses to A-gliadin 57-73 QE65 or P04724 84-100 QE92 (alone or combined). But gluten challenge 2 weeks after commencing GFD did induce responses to A-gliadin 57-73 QE65 and P04724 84-100 QE92 (alone or combined) that were significantly greater than the non-bioactive variant A-gliadin 57-73 QP65 and blank. Although these responses after gluten challenge at 2 weeks were substantial they appear to be less than in subjects >2 months after commencing GFD. Responses to A-gliadin 57-73 QE65 alone were equivalent or greater than responses to P04724 84-100 QE92 alone or when mixed with A-gliadin 57-73 QE65. None of the subjects experienced troubling symptoms with gluten challenge.

Immune responsiveness (as measured in PBMC after gluten challenge) to A-gliadin is partially restored 2 weeks after commencing GFD, implying that "immune unresponsiveness" to this dominant T cell epitope prevails in untreated coeliac disease and for at least one week after starting GFD. The optimal timing of a diagnostic test for coeliac disease using gluten challenge and measurement of responses to A-gliadin 57-73 QE65 in the ELISpot assay is at least 2 weeks after commencing a GFD.

Interferon gamma-secreting T cells specific to A-gliadin 57-73 QE65 cannot be measured in the peripheral blood in untreated coeliacs, and can only be induced by gluten challenge after at least 2 weeks GFD (antigen exclusion). Therefore, timing of a diagnostic test using this methodology is crucial and further studies are needed for its optimization. These finding are consistent with functional anergy of T cells specific for the dominant epitope, A-gliadin 57-73 QE65, reversed by antigen exclusion (GFD). This phenomenon has not been previously demonstrated in a human disease, and supports the possibility that T cell anergy may be inducible with peptide therapy in coeliac disease.

### Example 13

### Comprehensive Mapping of Wheat Gliadin T Cell Epitopes

Antigen challenge induces antigen-specific T cells in peripheral blood. In coeliac disease, gluten is the antigen that maintains this immune-mediated disease. Gluten challenge in coeliac disease being treated with a gluten free diet leads to the appearance of gluten-specific T cells in peripheral blood, so enabling determination of the molecular specificity of gluten T cell epitopes. As described above, we have identified a single dominant T cell epitope in a model gluten protein, A-gliadin (57-73 deamidated at Q65). In this Example, gluten challenge in coeliac patients was used to test all potential 12 amino acid sequences in every known wheat gliadin protein derived from 111 entries in Genbank. In total, 652 20mer peptides were tested in HLA-DQ2 and HLA-DQ8 associated coeliac disease. Seven of the 9 coeliac subjects with the classical HLA-DQ2 complex (HLA-DQA1*05, HLA-DQB1*02) present in over 90% of coeliacs had an inducible A-gliadin 57-73 QE65- and gliadin-specific T cell response in peripheral blood. A-gliadin 57-73 was the only significant α-gliadin T cell epitope, as well as the most potent gliadin T cell epitope, in HLA-DQ2-associated coeliac disease. In addition, there were as many as 5 families of structurally related peptides that were between 10 and 70% as potent as A-gliadin 57-73 in the interferon-γ ELISpot assay. These new T cell epitopes were derived from γ- and ω-gliadins and included common sequences that were structurally very similar, but not identical to the core sequence of A-gliadin 57-73 (core sequence: FPQPQLPYP (SEQ ID NO:18)), for example: FPQPQQPFP (SEQ ID NO:19) and PQQPQQPFP (SEQ ID NO:20). Although no homologues of A-gliadin 57-73 have been found in rye or barley, the other two cereals toxic in coeliac disease, the newly defined T cell epitopes in γ- and ω-gliadins have exact matches in rye and barley storage proteins (secalins and hordeins, respectively).

Coeliac disease not associated with HLA-DQ2 is almost always associated with HLA-DQ8. None of the seven HLA-DQ8+ coeliac subjects had inducible A-gliadin 57-73-specific T cell responses following gluten challenge, unless they also possessed the complete HLA-DQ2 complex. Two of 4 HLA-DQ8+ coeliac subjects who did not possess the complete HLA-DQ2 complex, had inducible gliadin peptide-specific T cell responses following gluten challenge. In one HLA-DQ8 subject, a novel dominant T cell epitope was identified with the core sequence LQPQNPSQQQPQ (SEQ ID NO:21). The transglutaminase-deamidated version of this peptide was more potent than the non-deamidated peptide. Previous studies suggest that the transglutaminase-deamidated peptide would have the sequence LQPENPSQEQPE (SEQ ID NO:22); but further studies are required to confirm this sequence. Amongst the healthy HLA-DQ2 (10) and HLA-DQ8 (1) subjects who followed a gluten free diet for a month, gliadin peptide-specific T cell responses were uncommon, seldom changed with gluten challenge, and were never potent T cell epitopes revealed with gluten challenge in coeliac subjects. In conclusion, there are unlikely to be more than six important T cell epitopes in HLA-DQ2-associated coeliac disease, of which A-gliadin 57-73 is the most potent. HLA-DQ2- and HLA-DQ8-associated coeliac disease do not share the same T cell specificity.

We have shown that short-term gluten challenge of individuals with coeliac disease following a gluten free diet induces gliadin-specific T cells in peripheral blood. The frequency of these T cells is maximal in peripheral blood on day 6 and then rapidly wanes over the following week. Peripheral blood gliadin-specific T cells express the integrin α4β7 that is associated with homing to the gut lamina propria. We exploited this human antigen-challenge design to map T cell epitopes relevant to coeliac disease in the archetypal gluten α-gliadin protein, A-gliadin. Using 15mer peptides overlapping by 10 amino acids with and without deamidation by transglutaminase (tTG), we demonstrated that T cells induced in peripheral blood initially target only one A-gliadin peptide, residues 57-73 in which glutamine at position 65 is deamidated. The epitope is HLA-DQ2-restricted, consistent with the intimate association of coeliac disease with HLA-DQ2.

Coeliac disease is reactivated by wheat, rye and barley exposure. The α/β-gliadin fraction of wheat gluten is consistently toxic in coeliac disease, and most studies have focused on these proteins. The gene cluster coding for α/β-gliadins is located on wheat chromosome 6C. There are no homologues of α/β-gliadins in rye or barley. However, all three of the wheat gliadin subtypes (α/β, γ, and ω) are toxic in coeliac disease. The γ- and ω-gliadin genes are located on chromosome 1A in wheat, and are homologous to the secalins and hordeins in rye and barley.

There are now genes identified for 61 α-gliadins in wheat (*Triticum aestivum*). The α-gliadin sequences are closely homologous, but the dominant epitope in A-gliadin derives from the most polymorphic region in the α-gliadin sequence. Anderson *et al* (1997) have estimated that there are a total of about 150 distinct α-gliadin genes in T. aestivum, but many are psuedogenes. Hence, it is unlikely that T-cell epitopes relevant to coeliac disease are not included within known α-gliadin sequences.

Our work has identified a group of deamidated α-gliadin peptides almost identical to A-gliadin 57-73 as potent T cell epitopes specific to coeliac disease. Over 90% of coeliac patients are HLA-DQ2+, and so far, we have only assessed HLA-DQ2+ coeliac subjects after gluten challenge. However, coeliac patients who do not express HLA-DQ2 nearly all carry HLA-DQ8. Hence, it is critical to know whether A-gliadin 57-73 and its homologues in other wheat, rye and barley gluten proteins are the only T-cell epitopes recognized by T cells induced by gluten challenge in both HLA-DQ2+ and HLA-DQ8+ coeliac disease. If this were the case, design of peptide therapeutics for coeliac disease might only require one peptide.

### Homologues of A-gliadin 57-73 as T-cell epitopes

Initial searches of SwissProt and Trembl gene databases for cereal genes coding for the core sequence of A-gliadin 57-73 (PQLPY <SEQ ID NO:12>) only revealed α/β-gliadins. However, our fine-mapping studies of the A-gliadin 57-73 QE65 epitope revealed a limited number of permissive point substitutions in the core region (PQLP) (note Q65 is actually deamidated in the epitope). Hence, we extended our search to genes in SwissProt or Trembl databases encoding for peptides with the sequence XXXXXXXPQ [ILMP] [PST] XXXXXX (SEQ ID NO:23). Homologues were identified amongst γ-gliadins, glutenins, hordeins and secalins (see Table 12). A further homologue was identified in ω-gliadin by visual search of the three ω-gliadin entries in Genbank.

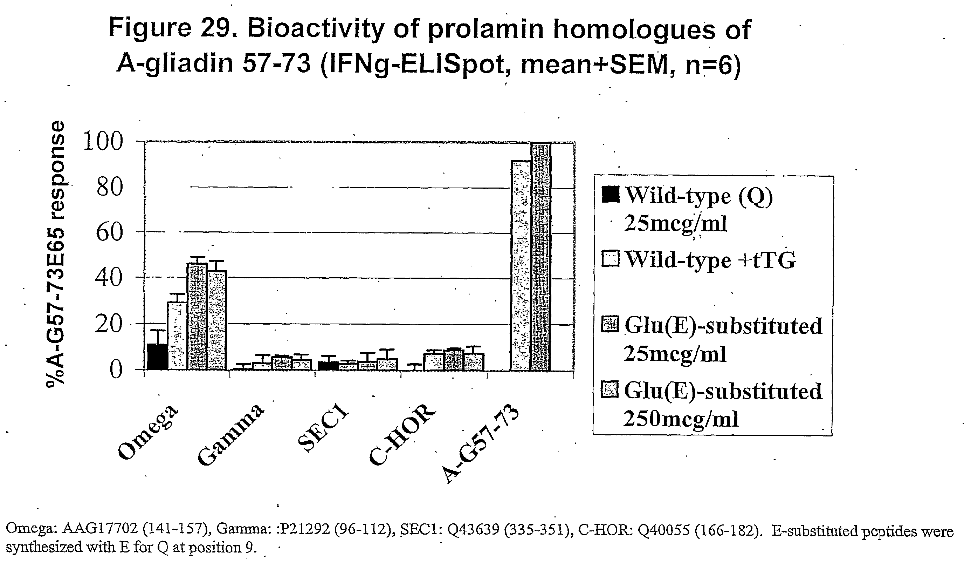

These homologues of A-gliadin 57-73 were assessed after deamidation by tTG (or synthesis of the glutamate(QE)-substituted variant in four close homologues) using the IFNγ ELISpot assay with peripheral blood mononuclear cells after gluten challenge in coeliac subjects. The ω-gliadin sequence (AAG17702 141-157) was the only bioactive peptide, approximately half as potent as A-gliadin 57-73 (see Table 12, and Figure 29). Hence, searches for homologues of the dominant A-gliadin epitope failed to account for the toxicity of γ-gliadin, secalins, and hordeins.

### Methods

### Design of a set of peptides spanning all possible wheat gliadin T-cell epitopes

In order to identify all possible T cell epitopes coded by the known wheat (Triticum aestivum) gliadin genes or gene fragments (61 α/β-, 47 γ-, and 3 ω-gliadin entries in Genbank), gene-derived protein sequences were aligned using the CustalW software (MegAlign) and arranged into phylogenetic groupings (see Table 22). Many entries represented truncations of longer sequences, and many gene segments were identical except for the length of polyglutamine repeats or rare substitutions. Hence, it was possible to rationalize all potential unique 12 amino acid sequences encoded by known wheat genes to be included in a set of 652 20mer peptides. (Signal peptide sequences were not included). Peptide sequences are listed in Table 23.

### Comprehensive epitope mapping

Healthy controls (HLA-DQ2+ n=10, and HLA-DQ8+ n=1) who had followed a gluten free diet for 4 weeks, and coeliac subjects (six HLA-DQ2, four complex heterozygotes HLA-DQ2/8, and three HLA-DQ8/X) (see Table 13) following long-term gluten free diet were studied before and on day 6 and 7 after 3-day gluten challenge (four 50g slices of standard white bread - Sainsbury's sandwich bread, each day). Peripheral blood (a total of 300ml over seven days) was collected and peripheral blood mononuclear cells (PBMC) were separated by Lymphoprep density gradient. PBMC were incubated with pools of 6 or 8 20mer peptides, or single peptides with or without deamidation by tTG in overnight interferon gamma (IFNγ) ELISpot assays.

Peptides were synthesized in batches of 96 as Pepsets (Mimotopes Inc., Melbourne Australia). Approximately 0.6 micromole of each of 652 20mers was provided. Two marker 20mer peptides were included in each set of 96 (VLQQHNIAHGSSQVLQESTY - peptide 161 (SEQ ID NO:24), and IKDFHVYFRESRDALWKGPG (SEQ ID NO:25)) and were characterized by reverse phase-HPLC and amino acid sequence analysis. Average purities of these marker peptides were 50% and 19%, respectively. Peptides were initially dissolved in acetonitrile (10%) and Hepes 100mM to 10mg/ml.

The final concentration of individual peptides in pools (or alone) incubated with PBMC for the IFNγ ELISpot assays was 20 µg/ml. Five-times concentrated solutions of peptides and pools in PBS with calcium chloride 1mM were aliquotted and stored in 96-well plates according to the template later used in ELISpot assays. Deamidated peptides and pools of peptides were prepared by incubation with guinea pig tissue tTG (Sigma T5398) in the ratio 100:32 µg/ml for two hours at 37°C. Peptides solutions were stored at -20°C and freshly thawed prior to use.

Gliadin (Sigma G3375) (100 mg/ml) in endotoxin-free water and 2M urea was boiled for 10 minutes, cooled to room temperature and incubated with filter (0.2 µm)-sterilised pepsin (Sigma P6887) (2 mg/ml) in HCl 0.02M or chymotrypsin (C3142) (4mg/ml) in ammonium bicarbonate (0.2M). After incubation for 4 hours, pepsin-digested gliadin was neutralized with sodium hydroxide, and then both pepsin- and chymotrypsin-digested gliadin were boiled for 15 minutes. Identical incubations with protease in which gliadin was omitted were also performed. Samples were centrifuged at 15 000g, then protein concentrations were estimated in supernatants by the BCA method (Pierce, USA). Before final use in IFNγ ELISpot assays, aliquots of gliadin-protease were incubated with tTG in the ratio 2500:64 µg/ml.

IFNγ ELISpot assays (Mabtech, Sweden) were performed in 96-well plates (MAIP S-45, Millipore) in which each well contained 25µl of peptide solution and 100µl of PBMC (2-8x10⁵/well) in RPMI containing 10% heat inactivated human AB serum. Deamidated peptide pools were assessed in one 96-well ELISpot plate, and peptides pools without deamidation in a second plate (with an identical layout) on both day 0 and day 6. All wells in the plate containing deamidated peptides included tTG (64 µg/ml). In each ELISpot plate there were 83 wells with peptide pools (one unique pool in each well), and a series of wells for "control" peptides (peptides all >90% purity, characterized by MS and HPLC, Research Genetics): P04722 77-93 (QLQPFPQPQLPYPQPQP (SEQ ID NO:26)), P04722 77-93 QE85 (in duplicate) (QLQPFPQPELPYPQPQP (SEQ ID NO:27)), P02863 77-93 (QLQPFPQPQLPYSQPQP (SEQ ID NO:28)), P02863 77-93 QE85 (QLQPFPQPELPYSQPQP (SEQ ID NO:29)), and chymotrypsin-digested gliadin (500 µg/ml), pepsin-digested gliadin (500 µg/ml), chymotrypsin (20 µg/ml) alone, pepsin (10 µg/ml) alone, and blank (PBS+/-tTG) (in triplicate).

After development and drying, IFNγ ELISpot plates were assessed using the MATP automated ELISpot plate counter. In HLA-DQ2 healthy and coeliac subjects, induction of spot forming cells (sfc) by peptide pools in the IFNγ ELISpot assay was tested using a one-tailed Wilcoxon Matched-Pairs Signed-Ranks test (using SPSS software) applied to spot forming cells (sfc) per million PBMC minus blank on day 6 versus day 0 ("net response"). Significant induction of an IFNγ response to peptide pools in PBMC by *in vivo* gluten challenge was defined as a median "net response" of at least 10 sfc/million PBMC and p<0.05 level of significance. Significant response to a particular pool of peptides on day 6 was followed by assessment of individual peptides within each pool using PBMC drawn the same day or on day 7.

For IFNγ ELISpot assays of individual peptides, bioactivity was expressed as a percent of response to P04722 77-93 QE85 assessed in the same ELISpot plate. Median response to blank (PBS alone) was 0.2 (range 0-5) sfc per well, and the positive control (P04722 77-93 QE85) 76.5 (range: 25-282) sfc per well using a median of 0.36 million (range: 0.3-0.72) PBMC. Hence, median response to blank expressed as a percentage of P04722 77-93 QE65 was 0.2% (range: 0-6.7). Individual peptides with mean bioactivity greater than10% that of P04722 QE85 were analyzed for common structural motifs.

### Results

### Healthy HLA-DQ2 subjects

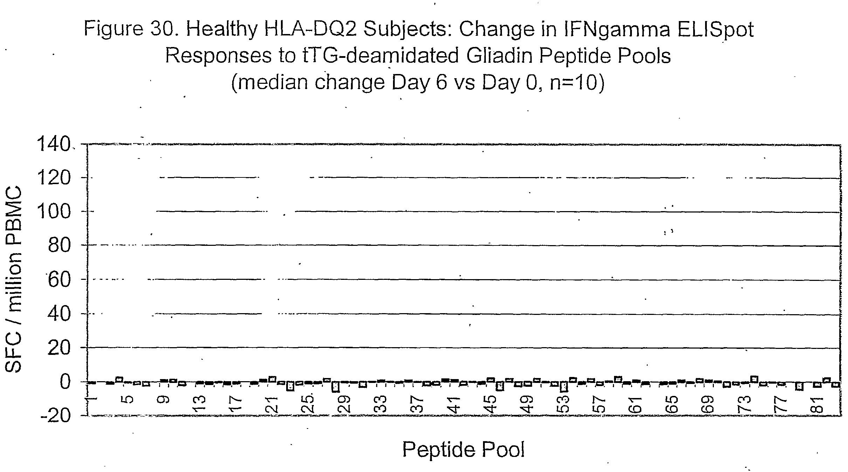

None of the healthy HLA-DQ2+ subjects following a gluten free diet for a month had IFNγ ELISpot responses to homologues of A-gliadin 57-73 before or after gluten challenge. However, in 9/10 healthy subjects, gluten challenge was associated with a significant increase in IFNγ responses to both peptic- and chymotryptic-digests of gliadin, from a median of 0-4 sfc/million on day 0 to a median of 16-29 sfc/million (see Table 14). Gliadin responses in healthy subjects were unaffected by deamidation (see Table 15). Amongst healthy subjects, there was no consistent induction of IFNγ responses to specific gliadin peptide pools with gluten challenge (see Figure 30, and Table 16). IFNγ ELISpot responses were occasionally found, but these were weak, and not altered by deamidation. Many of the strongest responses to pools were also present on day 0 (see Table 17, subjects H2, H8 and H9). Four healthy subjects did show definite responses to pool 50,and the two with strongest responses on day 6 also had responses on day 0. In both subjects, the post-challenge responses to pool 50 responses were due to peptide 390 (QQTYPQRPQQPFPQTQQPQQ (SEQ ID NO:30)).

### HLA-DQ2 coeliac subjects

Following gluten challenge in HLA-DQ2+ coeliac subjects, median IFNγ ELISpot responses to P04722 77-93 E85 rose from a median of 0 to 133 sfc/million (see Table 4). One of the six coeliac subjects (C06) did not respond to P04722 77-93 QE85 (2 sfc/million) and had only weak responses to gliadin peptide pools (maximum: Pool 50+tTG 27 sfc/million). Consistent with earlier work, bioactivity of wild-type P04722 increased 6.5 times with deamidation by tTG (see Table 15). Interferon-gamma responses to gliadin-digests were present at baseline, but were substantially increased by gluten challenge from a median of 20 up to 92 sfc/million for chymotryptic-gliadin, and from 44 up to 176 sfc/million for peptide-gliadin. Deamidation of gliadin increased bioactivity by a median of 3.2 times for chymotryptic-gliadin and 1.9 times for peptic-gliadin (see Table 15). (Note that the acidity required for digestion by pepsin is likely to result in partial deamidation of gliadin.)

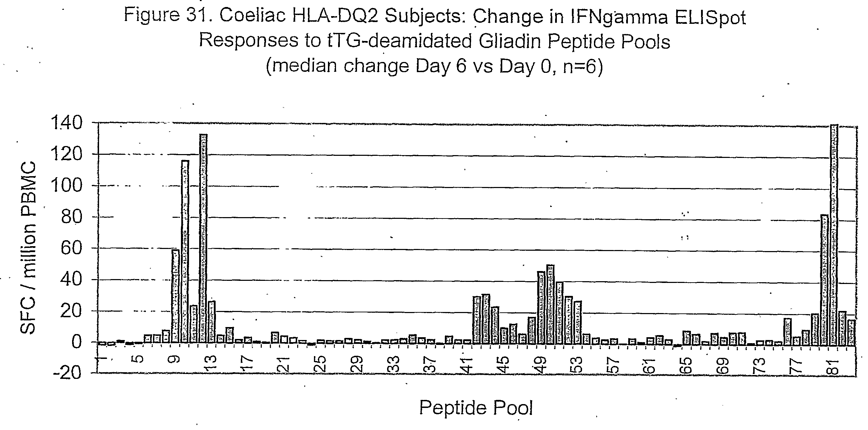

In contrast to healthy subjects, gluten challenge induced IFNγ ELISpot responses to 22 of the 83 tTG-treated pools including peptides from α-, γ- and ω-gliadins (see Figure 31, and Table 17). Bioactivity of pools was highly consistent between subjects (see Table 18). IFNγ ELISpot responses elicited by peptide pools were almost always increased by deamidation (see Table 17). But enhancement of bioactivity of pools by deamidation was not as marked as for P04722 77-73 Q85, even for pools including homologues of A-gliadin 57-73. This suggests that Pepset peptides were partially deamidated during synthesis or in preparation, for example the Pepset peptides are delivered as salts of trifluoracetic acid (TFA) after lyophilisation from a TFA solution.

One hundred and seventy individual tTG-deamidated peptides from 21 of the most bioactive pools were separately assessed. Seventy-two deamidated peptides were greater than 10% as bioactive as P04722 77-93 QE85 at an equivalent concentration (20 µg/ml) (see Table 19). The five most potent peptides (85-94% bioactivity of P04722 QE85) were previously identified α-gliadin homologues A-gliadin 57-73. Fifty of the bioactive peptides were not homologues of A-gliadin 57-73, but could be divided into six families of structurally related sequences (see Table 20). The most bioactive sequence of each of the peptide families were: PQQPQQPQQPFPQPQQPFPW (SEQ ID NO:31) (peptide 626, median 72% bioactivity of P04722 QE85), QQPQQPFPQPQQPQLPFPQQ (SEQ ID NO:32) (343, 34%), QAFPQPQQTFPHQPQQQFPQ (SEQ ID NO:33) (355, 27%), TQQPQQPFPQQPQQPFPQTQ (SEQ ID NO:34) (396, 23%), PIQPQQPFPQQPQQPQQPFP (SEQ ID NO:35) (625, 22%), PQQSFSYQQQPFPQQPYPQQ (SEQ ID NO:36) (618, 18%) (core sequences are underlined). All of these sequences include glutamine residues predicted to be susceptible to deamidation by transglutaminase (e.g. QXP, QXPF (SEQ ID NO:37), QXX [FY] (SEQ ID NO:38)) (see Vader et al 2002). Some bioactive peptides contain two core sequences from different families.

Consistent with the possibility that different T-cell populations respond to peptides with distinct core sequences, bioactivity of peptides from different families appear to be additive. For example, median bioactivity of tTG-treated Pool 81 was 141% of P04722 QE85, while bioactivity of individual peptides was in rank order: Peptide 631 (homologue of A-gliadin 57-73) 61%, 636 (homologue of 626) 51%, and 635 19%, 629 16%, and 634 13% (all homologues of 396).

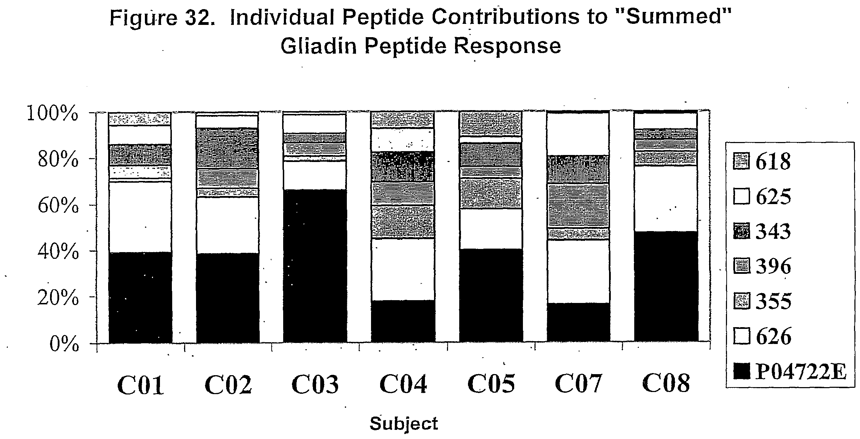

Although likely to be an oversimplification, the contribution of each "peptide family" to the summed IFNγ ELISpot response to gliadin peptides was compared in the HLA-DQ2+ coeliac subjects (see Figure 32). Accordingly, the contribution of P04722 77-73 E85 to the summed response to gliadin peptides is between 1/5 and 2/3.

Using the peptide homology search programme, WWW PepPepSearch, which can be accessed through the world wide web of the internet at, for example, "cbrg.inf.ethz.ch/subsection3_1_5.html.", and by direct comparison with Genbank sequences for rye secalins, exact matches were found for the core sequences QQPFPQPQQPFP (SEQ ID NO:39) in barley hordeins (HOR8) and rye secalins (A23277, CAA26449, AAG35598), QQPFPQQPQQPFP (SEQ ID NO:40) in barley hordeins (HOG1 and HOR8), and for PIQPQQPFPQQP (SEQ ID NO:41) also in barley hordeins (HOR8).

### HLA-DQ8-associated coeliac disease

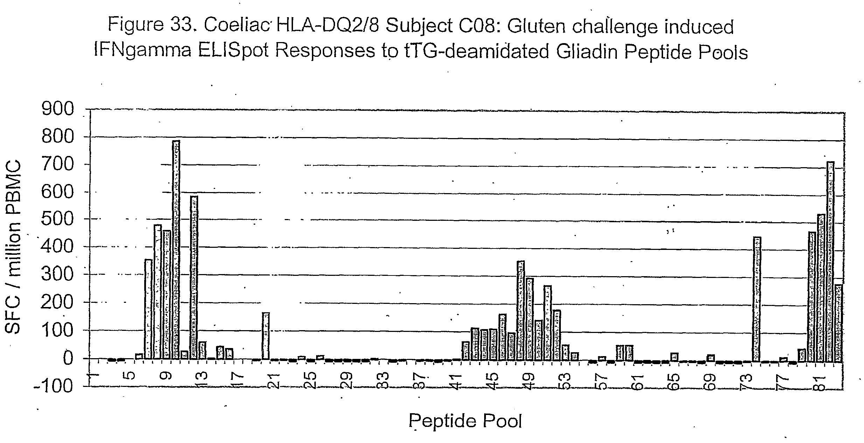

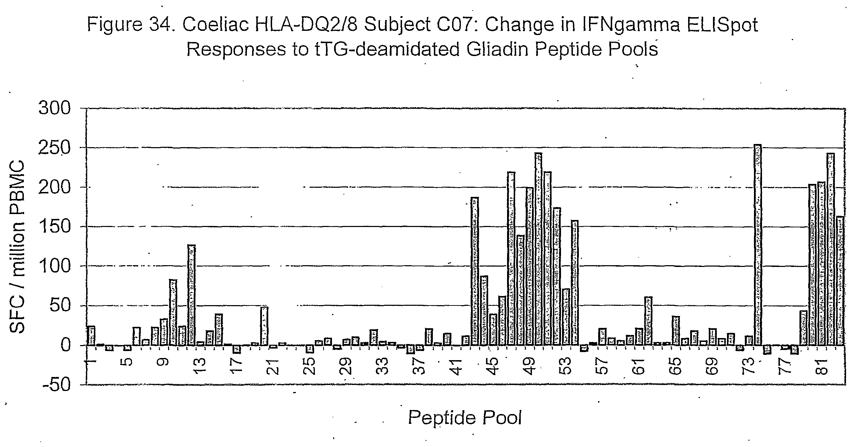

Seven HLA-DQ8+ coeliac subjects were studied before and after gluten challenge. Five of these HLA-DQ8+ (HLA-DQA0*0301-3, HLA-DQB0*0302) subjects also carried one or both of the coeliac disease-associated HLA-DQ2 complex (DQA0*05, DQB0*02). Two of the three subjects with both coeliac-associated HLA-DQ complexes had potent responses to gliadin peptide pools (and individual peptides including P04722 77-93 E85) that were qualitatively and quantitatively identical to HLA-DQ2 coeliac subjects (see Figures 33 and 34, and Table 18). Deamidated peptide pool 74 was bioactive in both HLA-DQ2/8 subjects, but only in one of the 6 HLA-DQ2/X subjects. Pretreatment of pool 74 with tTG enhances bioactivity between 3.8 and 22-times, and bioactivity of tTG-treated pool 74 in the three responders is equivalent to between 78% and 350% the bioactivity of P04722 77-93 E85. Currently, it is not known which peptides are bioactive in Pool 74 in subject C02, C07, and C08.

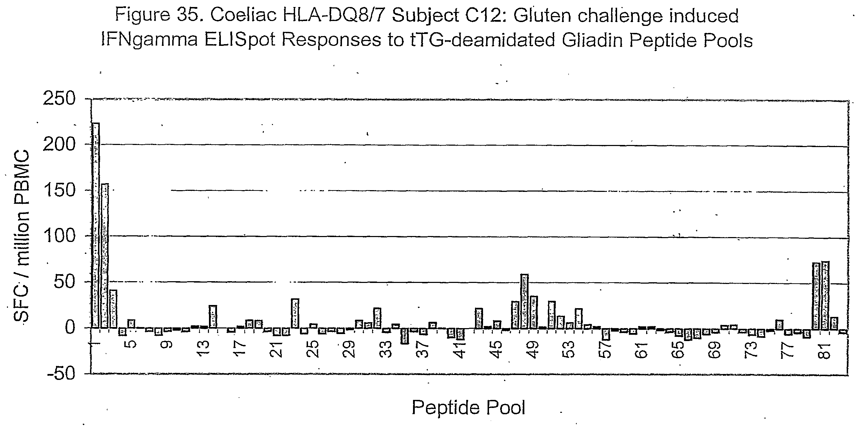

Two of the four HLA-DQ8 coeliac subjects that lacked both or one of the HLA-DQ2 alleles associated with coeliac disease showed very weak IFNγ ELISpot responses to gliadin peptide pools, but the other two did respond to both protease-digested gliadin and specific peptide pools. Subject C12 (HLA-DQ7/8) responded vigorously to deamidated Pools 1-3 (see Figure 35). Assessment of individual peptides in these pools identified a series of closely related bioactive peptides including the core sequence LQPQNPSQQQPQ (SEQ ID NO:42) (see Table 20). Previous work (by us) has demonstrated that three glutamine residues in this sequence are susceptible to tTG-mediated deamidation (underlined). Homology searches using WWW PepPepSearch have identified close matches to LQPQNPSQQQPQ (SEQ ID NO:43) only in wheat α-gliadins.

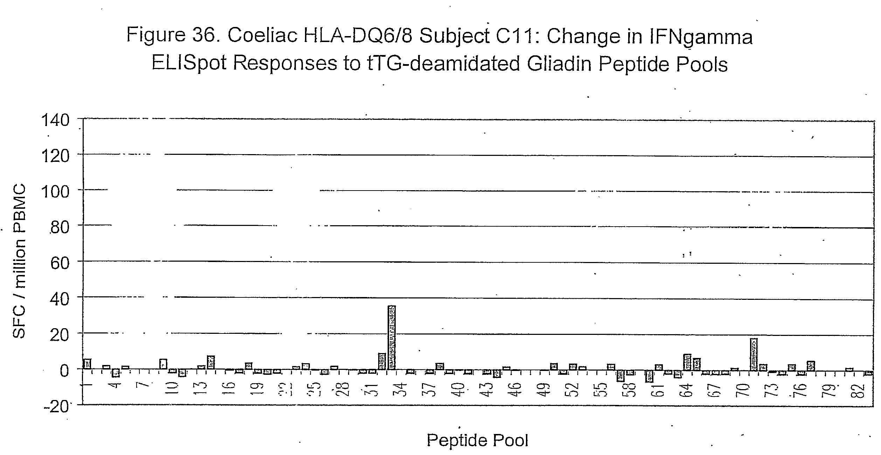

The fourth HLA-DQ8 subject (C11) had inducible IFNγ ELISpot responses to tTG-treated Pool 33 (see Figure 36). Pools 32 and 33 include polymorphisms of a previously defined HLA-DQ8 restricted gliadin epitope (QQYPSGQGSFQPSQQNPQ (SEQ ID NO:44)) active after deamidation by tTG (underlined Gln are deamidated and convey bioactivity) (van der Wal et al 1998). Currently, it is not known which peptides are bioactive in Pool 33 in subject C11.

Comprehensive T cell epitope mapping in HLA-DQ2-associated coeliac disease using in vivo gluten challenge and a set of 652 peptides spanning all known 12 amino acid sequences in wheat gliadin has thus identified at least 72 peptides at 10% as bioactive as the known α-gliadin epitope, A-gliadin 57-73 E65. However, these bioactive peptides can be reduced to a set of perhaps as few as 5 distinct but closely related families of peptides. Almost all these peptides are rich in proline, glutamine, phenylalanine, and/or tyrosine and include the sequence PQ (QL) P (FY) P (SEQ ID NO:45). This sequence facilitates deamidation of Q in position 2 by tTG. By analogy with deamidation of A-gliadin 57-68 (Arentz-Hansen 2000), the enhanced bioactivity of these peptides generally found with deamidation by tTG may be due to increased affinity of binding for HLA-DQ2.

Cross-reactivity amongst T cells in vivo recognizing more than one of these bioactive gliadin peptides is possible. However, if each set of related peptides does activate a distinct T cell population in vivo, the epitope corresponding to A-gliadin 57-73 E65 is the most potent and is generally recognized by at least 40% of the peripheral blood T cells that secrete IFNγ in response to gliadin after gluten challenge.

No gliadin-peptide specific responses were found in HLA-DQ2/8 coeliac disease that differed qualitatively from those in HLA-DQ2/X-associated coeliac disease. However, peripheral blood T cells in HLA-DQ8+ coeliac subjects without both HLA-DQ2 alleles did not recognize A-gliadin 57-73 E65 homologues. Two different epitopes were dominant in two HLA-DQ8+ coeliacs. The dominant epitope in one of these HLA-DQ8+ individuals has not been identified previously (LQPQNPSQQQPQ (SEQ ID NO:46)).

Given the teaching herein, design of an immunotherapy for coeliac disease utilizing all the commonly recognised T cell epitopes is practical and may include fewer than six distinct peptides. Epitopes in wheat γ- and ω-gliadins are also present in barley hordeins and rye secalins.

### Example 14

Several ELISpot assays were performed as previously described and yielded the following results and/or conclusions:

### Examination of multiple α-gliadin polymorphisms with PQLPY

Potent agonists of A-gliadin 57-73QE (G01) include QLQPFPQPELPYPQPQS (G01), PQL-Y---------------------P (G10), and PQPQPFL-(G12). Less potent include ----------------------L------P (G04),R------------------P (G05), and ----------------------S------P (G06). Less potent yet include --------L------------S------P (G07), --------S----- -------S------P (G08), -------------------S--S------P (G09), and PQPQPFP------------------ (G13). Dashes indicate identity with the G01 sequence in the particular position.

### Gluten challenge induces A-gliadin 57-73 QE65 T cells only after two weeks of gluten-free diet in newly diagnosed coeliac disease

Additional analyses indicated that tTG-deamidated gliadin responses change after two weeks of gluten-free diet in newly diagnosed coeliac disease. Other analyses indicated that deamidated gliadin-specific T cells are CD4⁺α₄β₇⁺ HLA-DQ2 restricted.

### Optimal epitope (clones versus gluten challenge)

A "dominant" epitope is defined by γIFN ELISpot after gluten challenge. QLQPFPQPELPYPQPQS (100% ELISpot response). Epitopes defined by intestinal T cell clones: QLQPFPQPELPY (27%), PQPELPYPQPELPY (52%), and QQLPQPEQPQQSFPEQERPF (9%).

### Dominance among individual peptide responses

Dominance depends on wheat or rye. For wheat, dominant peptides include peptide numbers 89, 90 and 91 (referring to sequence numbers in Table 23). For rye, dominant peptides include peptide numbers 368, 369, 370, 371, and 372 (referring to sequence numbers in Table 23). Some peptides, including 635 and 636 (referring to sequence numbers in Table 23) showed activity in both rye and wheat.

### In vivo gluten challenge allows T cell epitope hierarchy to be defined for coeliac disease

The epitope hierarchy is consistent among HLA-DQ2⁺ coeliacs but different for HLA-DQ8⁺ coeliacs. The hierarchy depends on what cereal is consumed. Deamidation generates almost all gliadin epitopes. HLA-DQ2, DQ8, and DR4 present deamidated peptides. HLA-DQ2/8-associated coeliac disease preferentially present DQ2-associated gliadin epitopes. Gliadin epitopes are sufficiently restricted to justify development of epitope-based therapeutics.

Other analyses indicated the following: HLA-DR3-DQ2 (85-95%) and HLA-DR4-DQ8 (5-15%).

Other analyses indicated the following:

| | HLA-DQ | HLA-DQA1 allele | HLA-DQB1 allele | Duodenal histology | Gluten free | EMA on gluten (on GFD) |
|---|---|---|---|---|---|---|
| C01 | 2,6 | 102/6, 501 | 201, 602 | SVA | 1 yr | +(-) |
| C02 | 2,2 | 501 | 201 | SVA | 1 yr | +(-) |
| C03 | 2,5 | 101/4/5, 501 | 201, 501 | PVA | 1 yr | +(-) |
| C04 | 2,5 | 101/4/5, 501 | 201, 501 | SVA | 7 yr | +(-) |
| C05 | 2,2 | 201, 501 | 201, 202 | SVA | 4 mo | +(ND) |
| C06 | 2,2 | 201, 501 | 201, 202 | SVA | 2 yr | +(-) |
| C07 | 2,8 | 301-3, 501 | 201, 302 | SVA | 1 yr | +(-) |
| C08 | 2,8 | 301-3, 501 | 201, 302/8 | SVA | 11 yr | ND (-) |
| C09 | 2,8 | 301-3, 501 | 201, 302 | SVA | 29 yr | +(-) |
| C10 | 2,8 | 201, 301-3 | 202, 302 | IEL | 1 yr | +(-) |
| C11 | 6,8 | 102/6, 301-3 | 602/15, 302/8 | IEL | 9 mo | - (ND) |
| C12 | 8,7 | 301-3, 505 | 302, 301/9-10 | SVA | 2 yr | - (-) |
| C13 | 8,8 | 301 | 302 | SVA | 1 yr | + (+) |

Another analysis was carried out to determine the bioactivity of individual tTG-deamidated peptides in pools 1-3 in subject C12. The results are as follows (sequence numbers refer to the peptides listed in Table 23): Sequence 8 (100%), Sequence 5 (85%), Sequence 6 (82%), Sequence 3 (77%), Sequence 1 (67%), Sequence 2 (59%), Sequence 9 (49%), Sequence 7 (49%), Sequence 10 (33%), Sequence 4 (15%), Sequence 12 (8%), Sequence 11 (0%), Sequence 23 (26%), Sequence 14 (18%), Sequence 15 (18%), Sequence 17 (18%), Sequence 16 (13%), Sequence 14 (8%), Sequence 22 (5%), Sequence 18 (3%), Sequence 19 (3%), Sequence 20 (0%), Sequence 21 (0%). The predicted deamidated sequence is LQPENPSQEQPE.

### Individual ELISpot responses by PBMC (Spot forming cells determined by ELISpot Reader)

| Peptide (see Table 23) | C01 | C02 | C03 | C04 | C05 |
|---|---|---|---|---|---|
| 65 | 16 | 2 | 1 | 2 | 3 |
| 66 | 32 | 6 | 13 | 0 | 6 |
| 67 | 16 | 3 | 4 | 0 | 4 |
| 68 | 25 | 8 | 4 | 2 | 2 |
| 69 | 4 | 0 | 0 | 0 | 0 |
| 70 | 2 | 1 | 0 | 0 | 0 |
| 71 | 1 | 1 | 0 | 0 | 1 |
| 72 | 0 | 0 | 0 | 0 | 0 |
| 73 | 95 | 21 | 42 | 31 | 31 |
| 74 | 122 | 15 | 29 | 21 | 28 |
| 75 | 5 | 1 | 2 | 2 | 5 |
| 76 | 108 | 13 | 28 | 16 | 22 |
| 77 | 3 | 0 | 1 | 0 | 1 |
| 78 | 21 | 2 | 3 | 5 | 3 |
| 79 | 20 | 0 | 2 | 0 | 2 |
| 80 | 5 | 2 | 0 | 0 | 3 |
| 81 | 4 | 1 | 2 | 3 | 1 |
| 82 | 3 | 3 | 5 | 2 | 2 |
| 83 | 14 | 2 | 0 | 0 | 1 |
| 84 | 3 | 0 | 0 | 0 | 0 |
| 85 | 14 | 1 | 2 | 1 | 2 |
| 86 | 11 | 0 | 2 | 0 | 2 |

### Cross-reactivity

To deal with data from 652 peptides in 29 subjects, or to determine when a particular response is a true positive peptide-specific T-cell response, or to determine when a response to a peptide is due to cross-reactivity with another structurally related peptide, expression of a particular peptide response can be as a percentage of a "dominant" peptide response. Alternately, the expression can be a "relatedness" as correlation coefficients between peptide responses, or via bioinformatics.

### Additional epitopes

A representative result is as follows:
Combination of peptides with P04722E (all 20mcg/ml) (n=4)

| | Alone | P04722E+ |
|---|---|---|
| Pep 626 | 60 | 135 |
| P04722E | 100 | 110 |
| HLAa | 0 | 85 |

(expressed as percent P04722E)
626+tT: PQQPQQPQQPFPQPQQPFPW
P04724E: QLQPFPQPELPYPQPQL

TTG-deamidation of peptide 626 (n=12)
No tTG = 100%
TTG = 170%

### Substitution at particular positions

Substitution of Peptide 626 PQQP [Q1] QP [Q2] QPFPQP [Q3] QPFPV (n=12)

| | Glu | Arg |
|---|---|---|
| Q1 | 95 | 90 |
| Q2 | 145 | 80 |
| Q3 | 155 | 10 |

(expressed as percent wild-type peptide)

Bioactivity of tTG-treated 15mers spanning Peptide 626/627 (PQQPQQPQQPFPQPQQPFPWQP) (n=8)

| | |
|---|---|
| P1-15 | 5 |
| P2-16 | 4 |
| P3-17 | 3 |
| P4-18 | 38 |
| P5-19 | 65 |
| P6-20 | 95 |
| P7-21 | 65 |
| P8-22 | 90 |

(expressed as percent of maximal 15mer response)

### Multiple epitopes:

P04724E: QLQPFPQPQLPYPQPQL
626+tTG: PQQPQQPQQPFPQPQQPFPW
Minimal epitope: QPQQPFPQPQQPFPW

Immunomagnetic depletion of PBMC by beads coated with anti-CD4 and by anti-integrin β₇ depleted IFNγ ELISpot responses, while immunomagnetic depletion of PBMC by beads coated with anti-CD8 or anti-alphaE integrin. Thus, the PBMC secreting IFNγ are CD4+ and α₄β₇+, associated with homing to the lamina propria in the gut.

Blocked by anti-DQ antibody but not by anti-DR antibody in heterozygotes and homozygotes for HLA-DQ2. This may imply multiple epitopes within one sequence.

### T cell epitopes in coeliac disease

Other investigators have characterized certain intestinal T cell clone epitopes. See, e.g., Vader et al., Gastroenterology 2002, 122:1729-37; Arentz-Hansen et al., Gastroenterology 2002, 123:803-809. These are examples of epitopes whose relevance is at best unclear because of the in vitro techniques used to clone T cells.

### Intestinal versus peripheral blood clones

Intestinal: 1) intestinal biopsies, 2) T cell clones raised against peptic-tryptic digest of gluten, 3) all HLA-DQ2 restricted, 4) clones respond to gliadin deamidated by transglutaminase.

Peripheral blood: 1) T cell clones raised against gluten are HLA-DR, DQ and DP restricted. Result: Intestinal T cell clones can be exclusively used to map coeliac disease associated epitopes
GDA_9Wheat 307 aa Definition Alpha/Beta-Gliadin MM1 Precursor (Prolamin)
Accession P18573 -- Genbank (which is incorporated herein by reference in its entirety)

### Intestinal T cell clone epitopes

A definition of intestinal T cell clone epitopes can be found in, for example, Arentz-Hansen et al., J Exp Med. 2000, 191:603-12. Also disclosed therein are gliadin epitopes for intestinal T cell clones. Deamidated QLQPFPQPQLPY is an epitope, with a deamidated sequence of QLQPFPQPELPY. There is an HLA-DQ2 restriction. A homology search shows other bioactive rAlpha-gliadins include PQPQLPY singly or duplicated. A majority of T cell clones respond to either/or DQ2-αI: QLQPFPQPELPY DQ2-αII: PQPELPYPQPELPY

### Dominant gliadin T cell epitopes

All deamidated by transglutaminase.
Peripheral blood day 6 after gluten challenge: A-gliadin 57-73: QLQPFPQPELPYPQPQS
Intestinal T cell clones: DQ2-αI: QLQPFPQPELPY DQ2-αII: PQPELPYPQPELPY

### Intestinal T-cell Clone Epitope Mapping

| | | |
|---|---|---|
| α-Gliadins | A1 | PFPQPQLPY |
| | A2 | PQPQLPYPQ |
| | A3 | PYPQPQLPY |
| | Glia-20 | PQQPYPQPQPQ |
| Γ-Gliadins | G1 | PQQSFPQQQ |
| | G2 | IIPQQPAQ |
| | G3 | FPQQPQQPYPQQP |
| | G4 | FSQPQQQFPQPQ |
| | G5 | LQPQQPFPQQPQQPYPQQPQ |
| | Glu-21 | QSEQSQQPFPQQF |
| | Glu-5 | Q (IL) PQQPQQF |
| Glutenin | Glt-156 | PFSQQQQSPF |
| | Glt-17 | PFSQQQQQ |

### Gluten exposure and induction of IFNγ-secreting A-Gliadin 57-73QE65-specific T cells in peripheral blood

Untreated coeliac disease, followed by gluten free diet for 1, 2, or 8 weeks, followed by gluten exposure (3 days bread 200g/day), followed by gluten free diet
Result 1: Duration of gluten free diet and IFNγ ELISpot responses on day 0 and day 6 of gluten challenge: A-gliadin 57-73 QE65 (results expressed as IFNγ specific spots/million PPBMC)
Day 0: none (5), 1 week (1), 2 weeks (2), 8 weeks (1)
Day 6: none (0), 1 week (4), 2 weeks (28), 8 weeks (48)
Result 2: Duration of gluten free diet and IFNγ ELISpot responses on day 0 and day 6 of gluten challenge: tTG-gliadin (results expressed as IFNγ specific spots/million PPBMC)
Day 0: none (45), 1 week (62), 2 weeks (5), 8 weeks (5)
Day 6: none (0), 1 week (67), 2 weeks (40), 8 weeks (60)
Result 3: Duration of gluten free diet and IFNγ ELISpot responses on day 0 and day 6 of gluten challenge: A-gliadin 57-73 P65 (results expressed as IFNγ specific spots/million PPBMC)
Day 0: none (1), 1 week (2), 2 weeks (1), 8 weeks (1)
Day 6: none (0), 1 week (0), 2 weeks (0), 8 weeks (0)
Result 4: Duration of gluten free diet and IFNγ ELISpot responses on day 0 and day 6 of gluten challenge: PPD (results expressed as IFNγ specific spots/million PPBMC)
Day 0: none (90), 1 week (88), 2 weeks (210), 8 weeks (150)
Day 6: none (0), 1 week (100), 2 weeks (210), 8 weeks (100)
Result 5: Duration of gluten free diet and IFNγ ELISpot responses on day 0 and day 6 of gluten challenge: tTG (results expressed as IFNγ specific spots/million PPBMC)
Day 0: none (5), 1 week (4), 2 weeks (3), 8 weeks (2)
Day 6: none (0), 1 week (4), 2 weeks (1), 8 weeks (2)

### Gluten challenge in HLA-DQ2 coeliac disease on long term gluten

Characterization of anti-gliadin T cell response was carried out in peripheral blood on day 6-8 after 3-day gluten challenge.
Result 1: PBMC Day 6 Long-term gluten free diet (preincubation with anti-HLA-DR and -DQ antibody) (expressed as % inhibition)
DR-: tTG-gliadin 100 mcg/ml (105), A-gliadin 57-73 QE65 50 mcg/ml (90), PPD 5 mcg/ml (30)
DQ-: tTG-gliadin 100 mcg/ml (5), A-gliadin 57-73 QE65 50 mcg/ml (22), PPD 5 mcg/ml (78).
Result 2: PBMC Day 6 Long-term gluten free diet (expressed as % CD8-depleted PBMC response)
B7 depletion: tTG-gliadin n=6 (7), A-gliadin 57-73 n=9 (6), PPD n=8 (62)
AE depletion: tTG-gliadin n=6 (120), A-gliadin 57-73 n=9 (80), PPD n=8 (110).
CD4 depletion: tTG-gliadin n=6 (10), A-gliadin 57-73 n=9 (9), PPD n=8 (10).

### Therapeutic peptides include, but are not limited to

QLQPFPQPQLPYPQPQS (AG01)
QLQPFPQPQLPYPQPQP (AG02)
QLQPFPQPQLPYPQPQL (AG03)
QLQPFPQPQLPYLQPQP (AG04)
QLQPFPRPQLPYPQPQP (AG05)
QLQPFPQPQLPYSQPQP (AG06)
QLQPFLQPQLPYSQPQP (AG07)
QLQPFSQPQLPYSQPQP (AG08)
QLQPFPQPQLSYSQPQP (AG09)
PQLPYPQPQLPYPQPQP (AG10)
PQLPYPQPQLPYPQPQL (AG11)
PQPQPFLPQLPYPQPQS (AG12)
PQPQPFPPQLPYPQPQS (AG13)
PQPQPFPPQLPYPQYQP (AG14)
PQPQPFPPQLPYPQPPP (AG015)

Briefly after oral antigen challenge, specificities of peripheral blood T cells reflect those of intestinal T cell clones. In peripheral blood, epitopes of intestinal T cell clones are sub-optimal compared to A-gliadin 57-73 QE65, which is an optimal α-gliadin epitope.

### Example 15

ELISpot assays were also carried out for mapping purposes as follows.

### Fine-mapping the dominant DQ-8 associated epitope

| Sequence / sfc | tTG-treated sequence / sfc |
|---|---|
| VPQLQPQNPSQQQPQEQV / 76 | RWPVPQLQPQNPSQQ / 60 |
| | WPVPQLQPQNPSQQQ / 90 |
| VPQLQPENPSQQQPQEQV /3 | PVPQLQPQNPSQQQP / 130 |
| VPQLQPRNPSQQQPQEQV / 76 | VPQLQPQNPSQQQPQ / 140 |
| | PQLQPQNPSQQQPQE / 59 |
| VPQLQPQNPSQEQPQEQV / 100 | QLQPQNPSQQQPQEQ / 95 |
| VPQLQPQNPSQRQPQEQV / 1 | LQPQNPSQQQPQEQV / 30 |
| | QPQNPSQQQPQEQVP / 4 |
| VPQLQPQNPSQQQPEEQV / 71 | |
| VPQLQPQNPSQQQPREQV / 27 | DQ8 Gliadin Epitope |
| | GDA09 202Q / 6 |
| VPQLQPQNPSQEQPEEQV / 81 | GDA09 202E / 83 |
| VPQLQPENPSQQQPEEQV / 2 | GDA09 202Q+tTG / 17 |
| VPQLQPENPSQEQPQEQV / 6 | BI + tTG / 0 |
| VPQLQPENPSQEQPEEQV / 5 | BI /0 |

### Fine-mapping dominant epitope (2)

**Pool 33 (deamidated) / sfc**

| | |
|---|---|
| A2b3 301 | qqyp sgqg ffqp sqqn pqaq / 2 |
| A2b5 301 | qqyp sgqg ffqp fqqn pqaq / 1 |
| A3a1 301 | qqyp sgqg ffqp sqqn pqaq / 0 |
| A3b1 301 | qqyp ssqv sfqp sqln pqaq / 0 |
| A3b2 301 | qqyp ssqg sfqp sqqn pqaq / 2 |
| A4a 301 | eqyp sgqv sfqs sqqn pqaq /28 |
| A1b1 309 | sfrp sqqn plaq gsvq pqql / 2 |
| A1a1 309 | sfrp sqqn pqaq gsvq pqql / 2 |

### Example 16

### Bioactivity of gliadin epitopes in IFNγ-ELISpot (25 mcg/ml, n=6) (expressed as % A-gliadin 57-73 QE65 response)

DQ2-AII: wild type (WT) (4), WT + tTG (52), Glu-substituted (52)
DQ2-AI: wild type (WT) (2), WT + tTG (22), Glu-substituted (28)
GDA09: wild type (WT) (1), WT + tTG (7), Glu-substituted (8)
A-G31-49: wild type (WT) (2), WT + tTG (3), Glu-substituted (0)

### Dose response of A-Gliadin 57-73 QE65 (G01E) (n=8) (expressed as %G01E maximal response)

0.025 mcg/ml (1), 0.05 mcg/ml (8), 0.1 mcg/ml (10), 0.25 mcg/ml (22), 0.5 mcg/ml (38), 1 mcg/ml (43), 2.5 mcg/ml (52), 5 mcg/ml (70), 10 mcg/ml (81), 25 mcg/ml (95), 50 mcg/ml (90), 100 mcg/ml (85).
IFNγ ELISpot response to gliadin epitopes alone or mixed with A-gliadin 57-75 (G01E) (all 50 mcg/ml, tTG-gliadin 100 mcg/ml, PPD 5 mcg/ml; n=9) (expressed as % G01E response)
Alone: DQ2-A1 (20), DQ2-A2 (55), Omega G1 (50), tTG Gliadin (80), PPD (220), DQ2 binder (0)
G01E+: DQ2-A1 (90), DQ2-A2 (95), Omega G1 (100), tTG Gliadin (120), PPD (280), DQ2 binder (80)

### Effect of alanine and lysine substitution of A-gliadin 57-73 QE65 on IFNγ ELISpot responses in individual coeliac subjects (n=8)

### Epitope sequence: QLQPFPQPELPYPQPQS

Alanine substitution at positions 57-59 and 72-73 showed little to no decrease in % A-gliadin 57-73 QE65 response. Alanine substitution at positions 60-62 and 68-71 showed moderate decrease in % A-gliadin 57-73 QE65 response. Alanine substitution at positions 63-67 showed most decrease in % A-gliadin 57-73 QE65 response.

Effect of lysine substitution of A-gliadin 57-73 QE65 on IFNγ ELISpot responses in individual coeliac subjects (n=8);
Epitope sequence: QLQPFPQPELPYPQPQS

Lysine substitution at positions 57-59 and 71-73 showed little to no decrease in % A-gliadin 57-73 QE65 response. Lysine substitution at positions 60-61 and 69-70 showed moderate decrease in % A-gliadin 57-73 QE65 response. Lysine substitution at positions 62-68 showed most decrease in % A-gliadin 57-73 QE65 response.

### Example 17

Table 24 shows the results of analyses examining the 652 peptides with several patients challenged with wheat or rye.

### References

1. Molberg O, et al. Nature Med. 4, 713-717 (1998).
2. Quarsten H, et al. Eur. J. Immunol. 29, 2506-2514 (1999).
3. Greenberg CS et al. FASEB 5, 3071-3077 (1991).
4. Mantzaris G, Jewell D. Scand. J. Gastroenterol. 26, 392-398 (1991).
5. Mauri L, et al. Scand. J. Gastroenterol. 31, 247-253 (1996).
6. Bunce M, et al. Tissue Antigens 46, 355-367 (1995).
7. Olerup O, et al. Tissue antigens 41, 119-134 (1993).
8. Mullighan CG, et al. Tissue-Antigens. 50, 688-92 (1997).
9. Plebanski M et al. Eur. J. Immunol. 28, 4345-4355 (1998).
10. Anderson DO, Greene FC. The alpha-gliadin gene family. II. DNA and protein sequence variation, subfamily structure, and origins of pseudogenes. Theor Appl Genet (1997) 95:59-65.
11. Arentz-Hansen H, Korner R, Molberg O, Quarsten H, Van der Wal Y, Kooy YMC, Lundin KEA, Koning F, Roepstorff P, Sollid LM, McAdam SN. The intestinal T cell response to alpha-gliadin in adult celiac disease is focused on a single deamidated glutamine targeted by tissue transglutaminase. J Exp Med. 2000; 191:603-12.
12. Vader LW, de Ru A, van der Wal, Kooy YMC, Benckhuijsen W, Mearin ML, Drijfhout JW, van Veelen P, Koning F. Specificity of tissue transglutaminase explains cereal toxicity in celiac disease. J Exp Med 2002; 195:643-649.
13. van der Wal Y, Kooy Y, van Veelan P, Pena S, Mearin L, Papadopoulos G, Koning F. Selective deamidation by tissue transglutaminase strongly enhances gliadin-specific T cell reactivity. J Immunol. 1998; 161:1585-8.
14. van der Wal Y, Kooy Y, van Veelan P, Pena S, Mearin L, Molberg O, Lundin KEA, Sollid L, Mutis T, Benckhuijsen WE, Drijfhout JW, Koning F. Proc Natl Acad Sci USA 1998; 95:10050-10054.
15. Vader W, Kooy Y, Van Veelen P et al. The gluten response in children with celiac disease is directed toward multiple gliadin and glutenin peptides. Gastroenterology 2002, 122:1729-37
16. Arentz-Hansen H, McAdam SN, Molberg O, et al. Celiac lesion T cells recognize epitopes that cluster in regions of gliadin rich in proline residues. Gastroenterology 2002, 123:803-809.

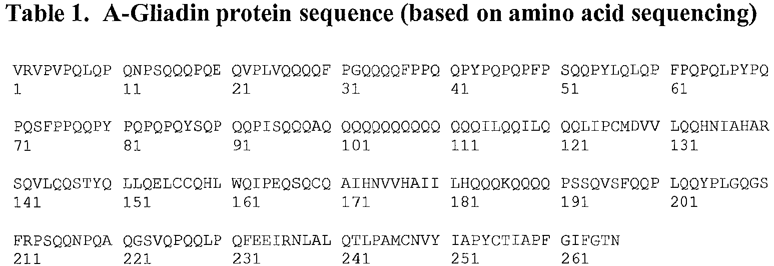

**Table 2. Coeliac disease subjects studied**

| | **Age Sex** | **Gluten free diet** | **HLA-DQ2** | **Bread challenge** | **Symptoms with bread** |
|---|---|---|---|---|---|
| 1 | 64 f | 14 yr | Homozygote | 3 days | Abdominal pain, lethargy, mouth ulcers, diarrhoea |
| 2 | 57 m | 1 yr | Heterozygote | 10 days | Lethargy, nausea |
| 3 | 35 f | 7 yr | Heterozygote | 3 days | Nausea |
| 4 | 36 m | 6 wk | Homozygote | 3 days | Abdominal pain, mouth ulcers, diarrhoea |
| 5 | 26 m | 19 yr | Heterozygote | 3 days | None |
| 6 | 58 m | 35 yr | Heterozygote | 3 days | None |
| 7 | 55 m | 1 yr | Heterozygote | 3 days | Diarrhoea |
| 8 | 48 f | 15 yr | Homozygote | 3 days | Abdominal pain, diarrhoea |

**Table 3**

| Aminoacid at position 65 | Range | Mean |
|---|---|---|
| Glutamate | (100) | 100% |
| Asparagine | (50-84) | 70% |
| Aspartate | (50-94) | 65% |
| Alanine | (44-76) | 64% |
| Cysteine | (45-83) | 62% |
| Serine | (45-75) | 62% |
| Valine | (24-79) | 56% |
| Threozine | (46-66) | 55% |
| Glycine | (34-47) | 40% |
| Leucine | (8-46) | 33% |
| Glutamine | (16-21) | 19% |
| Isoleucine | (3-25) | 14% |
| Methionine | (3-32) | 14% |
| Phenylalanine | (0-33) | 12% |
| Histidine | (0-13) | 8% |
| Tyrosine | (0-17) | 8% |
| Tryptophan | (0-17) | 8% |
| Lysine | (0-11) | 4% |
| Proline | (0-4) | 2% |
| Arginine | (0-2) | 1% |

**Table 4**

| lisopt response | | Peptide sequence | Corresponding residues in gliadin protein sequences (Accession no.) | |
|---|---|---|---|---|
| to TG (1-13) | TG | QLQPFPQPQLPYPQPQS | 57-73 | α-Gliadin (T. aestivum) Q41545 |
| | 100 (100) | QLQPFPQPELPYPQPQS | 57-73 | α-Gliadin (T. aestivum) Q41545 |
| (1-7) | 53 (44-67) | QLQPPPQPQLPYSQPQP | 77-93 | α/β-Gliadin precursor (Tricetum. aestivum) P02863 |
| | | | 76-92 | α-Gliadin (T. aestivum) Q41528 |
| | | | 77-93 | α-Gliadin storage protein (T. aestivum Q41531 |
| | | | 57-73 | α-Gliadin mature peptide (T. aestivum) Q41533 |
| | | | 77-93 | α-Gliadin precursor (T. spelta) Q9ZP09 |
| 2 (0-20) | 83 (61-113) | QLQPFPQPQLPYPQPQP | 77-93 | α/β-Gliadin A-II precursor (T. aestivum) P0472 |
| 9 (0-33) | 33 (74-97) | QLQPFPQPQLPYPQPQL | 77-93 | α/β-Gliadin A-IV precursor (T. aestivum) P04724 |
| | | | 77-93 | α/β-Gliadin MMI precursor (T. aestivum) P18573 |
| (0-7) | 109 (41-152) | PQLPYPQPQLPYPQPQP | 84-100 | α/β-Gliadin A-IV procursor (T. aestivum) P04724 |
| ID | | PQLPYPQPQLPYPQPQL | 84-100 | α/β-Gliadin MMI precursor (T. aestivum) P18573 |
| (0-1) | 3 (0-7) | QLQPFLQPQLPYSQPQP | 77-93 | α/β-Gliadin A-I precursor (T. aestivum) P04721 |
| | | | 71-93 | α-Gliadin (T. aestivum) Q41509 |
| (0-0) | 2 (0-7) | QLQPFSQPQLPYSQPQP | 77-93 | α-Gliadin storage proteic (T. aestivum) Q41330 |
| ID | | PQPQPFPPQLPYPQTQP | 77-93 | α/β-Gliadin A-III procursor (T. aestivum) P04725 |
| 7 (0-40) | 24 (11-43) | PQPQPFPPQLPYPQPQS | 62-98 | α/β-Gliadin A-V precursor (T. aestivum) P04725 |
| 0 (0-30) | 19 (11-33) | PQPQPFPPQLPYPQPPP | 62-95 | α/β-Gliadin clone PW1215 precursor (T. aestivum) P04726 |
| | | | 82-98 | α/β-Gliadin (T. uractu) Q41632 |
| 0 (0-30) | 21 (11-33) | PQPQPFLPQLPYPQPQS | 79-95 | α/β-Gliadin clone PW3142 precursor (T. aestivum) P04726 |
| | | | 79-95 | α-Gliadin (T. aestivum) Q41529 |
| | | | 79-95 | α/β-Gliadin precursor (T. aestivum) Q41546 |

**Table 5. T cell epitopes described in coeliac disease**

| Source | Restriction | Frequency | Sequence* |
|---|---|---|---|
| Gamma -gliadin | DQ2 | 3/NS (iTCC) | QQLPQPEQPQQSFPEQERPF |
| Alpha-gliadin | DQ2 | 12/17 (iTCL) | QLQPFPQPELPY |
| Alpha-gliadin | DQ2 | 11/17 (iTCL) | PQPELPYPQPELPY |
| Alpha-gliadin | DQ2 | 1/23 (bTCC) | LGQQQPFPPQQPYPQPQPF |
| Alpha-gliadin | DQ8 | 3/NS (iTCC) | QQYPSGEGSFQPSQENPQ |
| Glutenin | DQ8 | 1/1 (iTCC) | GQQGYYPTSPQQSGQ |
| Alpha-gliadin | DQ2 | 11/12 in vivo | QLQPFPQPELPYPQPQS |

| | | | |
|---|---|---|---|
| NS not stated in original publication, iTCC intestinal T cell clone, iTCL intestinal polyclonal T cell line, bTCC peripheral bl T cell clone *All peptides are the products of transglutaminase modifying wild type gluten peptides except the fourth and sixth peptides | | | |

**Table 6. Relative bioactivity of gliadin T cell epitopes in coeliac PBMC after gluten challenge**

| Sequence* | ELISpot response as % A-gliadin 57-73 QE65 (all 25mcg/ml) | | | | | |
|---|---|---|---|---|---|---|
| | Wild type | | Wildtype+tTG | | E-substituted | |
| QQLPQPEQPQQSFPEQERPF | 9 | (3) | 18 | (7) | 10 | (5) |
| QLQPFPQPELPY | 6 | (2) | 19 | (1) | 8 | (3) |
| PQPELPYPQPELPY | 13 | (6) | 53 | (8) | 48 | (9) |
| QQYPSGEGSFQPSQENPQ | 10 | (3) | 9 | (3) | 14 | (8) |
| QLQPFPQPELPYPQPQS | 18 | (7) | 87 | (7) | 100 | |
| PQLPYPQPELPYPQPQP | 14 | (4) | 80 | (17) | 69 | (20) |

| | | | | | | |
|---|---|---|---|---|---|---|
| * sequence refers that of transglutaminase (tTG) modified peptide and the T cell epitope. Wild type is the unmodified gliadi peptide. Data from 4 subjects. Blank was 5 (1) %. | | | | | | |

**Table 7. Polymorphisms of A-gliadin 57-73**

| A. Sequences derived from Nordic autumn wheat strain Mjoelner | |
|---|---|
| Alpha-gliadin protein (single letter code refers to Fig. 14 peptides) | Polymorphism |
| Q41545 A-gliadin (from sequenced protein) 57-73 (A) | QLQPFPQPQLPYPQPQS |
| Gli alpha 1,6: (EMBL: AJ133605 & AJ133602 58-74) (J) | QPQPFPPPQLPYPQTQP |
| Gli alpha 3,4,5: (EMBL: AJ133606, AJ133607, AJ133608 57-73) (I) | QLQPFPQPQLSYSQPQP |
| Gli alpha 7: (EMBL: AJ133604 57-73) (E) | QLQPFPRPQLPYPQPQP |
| Gli alpha 8, 9, 11: (EMBL: ) (F) | QLQPFPQPQLPYSQPQP |
| Gli alpha 10: (EMBL: AJ133610 57-73) (D) | QLQPFPQPQLPYLQPQS |

| **B. SWISSPROT and TREMBL scan (10.12.99) for gliadins XXXXXXXPQLPYXXXXX containing the sequence:** | |
|---|---|
| Wheat (Triticum aestivum unless stated) gliadin accession number | Polymorphism |
| Q41545 A-gliadin (from sequenced protein) 57-73 (A) | QLQPFPQPQLPYPQPQS |
| SWISSPROT: | |
| GDA0_WHEAT P02863 77-93 (F) | QLQPFPQPQLPYSQPQP |
| GDA1_WHEAT P04721 77-93 (G) | QLQPFLQPQLPYSQPQP |
| GDA2_WHEAT P04722 77-93 (B) | QLQPFPQPQLPYPQPQP |
| GDA3_WHEAT P04723 77-93 (O) | PQPQPFPPQLPYPQTQP |
| GDA4_WHEAT P04724 77-93 (C) | QLQPFPQPQLPYPQPQL |
| GDA4_WHEAT P04724 84-100 (K) | PQLPYPQPQLPYPQPQP |
| GDA5_WHEAT P04725 82-98 (N) | PQPQPFPPQLPYPQPQS |
| GDA6_WHEAT P04726 82-98 (P) | PQPQPFPPQLPYPQPPP |
| GDA7_WHEAT P04727 79-95 (M) | PQPQPFLPQLPYPQPQS |
| GDA9_WHEAT P18573 77-93 (C) | QLQPFPQPQLPYPQPQL |
| GDA9_WHEAT P18573 84-100 (L) | PQLPYPQPQLPYPQPQL |
| GDA9_WHEAT P18573 91-107 (K) | PQLPYPQPQLPYPQPQP |
| TREMBL | QLQPFLQPQLPYSQPQP |
| Q41509 ALPHA-GLIADIN 77-93 (G) | QLQPFPQPQLPYSQPQP |
| Q41528 ALPHA-GLIADIN 76-92 (F) | PQPQPFLPQLPYPQPQS |
| Q41529 ALPHA-GLIADIN 79-95 (M) | QLQPFSQPQLPYSQPQP |
| Q41530 ALPHA-GLIADIN 77-93 (H) | QLQPFPQPQLPYSQPQP |
| Q41531 ALPHA-GLIADIN 77-93 (F) | QLQPFPQPQLPYSQPQP |
| Q41533 ALPHA-GLIADIN 57-73 (F) | PQPQPFLPQLPYPQPQS |
| Q41546 ALPHA/BETA-GLIADIN 79-95 (M) | PQPQPFPPQLPYPQPPP |
| Q41632 ALPHA/BETA-TYPE GLIADIN. Triticum urartu 82-98 (P) | QLQPFPQPQLPYSQPQP |
| Q9ZP09 ALPHA-GLIADIN Triticum spelta 77-93 (F) | |

**Table 11. Antagonism of A-gliadin 57-73 QE65 interferon gamma ELISpot response by naturally occurring polymorphisms of A-gliadin 57-73 QE65 (P is significance level in unpaired t-test).**

| **A-gliadin 57-73 QE65 polymorphism** | | **% Inhibit.** | **P** |
|---|---|---|---|
| **P04725 82-98 QE90** | **PQPQPFPPELPYPQPQS** | **19** | **0.009** |
| **Q41509 77-93 QE85** | **QLQPFLQPELPYSQPQP** | **11** | **0.15** |
| **Gli ∀ 1,6 58-74 QE66** | **QPQPFPPPELPYPQTQP** | **11** | **0.11** |
| **P04723 77-93 QE85** | **PQPQPFPPELPYPQTQP** | **10** | **0.14** |
| **Gli V 3-5 57-73 QE65** | **QLQPFPQPELSYSQPQP** | **7** | **0.34** |
| **P02863 77-93 QE85** | **QLQPFPQPELPYSQPQP** | **6** | **0.35** |
| **Q41509 77-93 QE85** | **QLQPFLQPELPYSQPQP** | **6** | **0.41** |
| **P04727 79-95 QE65** | **PQPQPFLPELPYPQPQS** | **6** | **0.39** |
| **P04726 82-98 QE90** | **PQPQPFPPELPYPQPPP** | **5** | **0.43** |

**Table 8. Bioactivity of substituted variants of A-gliadin 57-73 QE65 (Subst) compared to unmodified A-gliadin 57-73 QE65 (G) (mean 100%, 95% CI 97-104) and blank (no peptide, bl) (mean 7.1%, 95% CI: 5.7-8.5)**

| **Subst** | **%** | **P vs G** | **Subst** | **%** | **P vs G** | **Subst** | **%** | **P vs G** | **Subst** | **%** | **P vs G** | **P vs bl** |
|---|---|---|---|---|---|---|---|---|---|---|---|---|
| **Super-agonists** | | | **F62** | **71** | **0.001** | **H62** | **47** | **<0.0001** | **N66** | **24** | **<0.0001** | |
| **Y61** | **129** | **<0.0001** | **V63** | **70** | **<0.0001** | **G69** | **47** | **<0.0001** | **R64** | **24** | **<0.0001** | |
| **Y70** | **129** | **0.0006** | **S69** | **70** | **<0.0001** | **N63** | **47** | **<0.0001** | **K63** | **23** | **<0.0001** | |
| **Agonists** | | | **H63** | **70** | **<0.0001** | **H68** | **47** | **<0.0001** | **V65** | **23** | **<0.0001** | |
| **W70** | **119** | **0.017** | **F63** | **70** | **0.008** | **M68** | **46** | **<0.0001** | **H66** | **23** | **<0.0001** | |
| **K57** | **118** | **0.02** | **P70** | **69** | **<0.0001** | **D68** | **46** | **<0.0001** | **H67** | **22** | **<0.0001** | |
| **Y59** | **117** | **0.04** | **T62** | **69** | **<0.0001** | **V69** | **46** | **<0.0001** | **L64** | **22** | **<0.0001** | |
| **A57** | **116** | **0.046** | **L61** | **69** | **<0.0001** | **G63** | **45** | **<0.0001** | **S66** | **22** | **<0.0001** | |
| **S70** | **116** | **0.045** | **S61** | **69** | **<0.0001** | **V64** | **45** | **<0.0001** | **F67** | **21** | **<0.0001** | |
| **K58** | **114** | **0.08** | **T61** | **69** | **<0.0001** | **E61** | **45** | **<0.0001** | **W66** | **21** | **<0.0001** | |
| **W59** | **110** | **0.21** | **T63** | **69** | **<0.0001** | **A69** | **43** | **<0.0001** | **G64** | **21** | **<0.0001** | |
| **A73** | **109** | **0.24** | **M66** | **68** | **<0.0001** | **R62** | **42** | **<0.0001** | **G65** | **21** | **<0.0001** | |
| **159** | **108** | **0.37** | **T69** | **67** | **<0.0001** | **G68** | **42** | **<0.0001** | **D64** | **21** | **<0.0001** | |
| **G59** | **108** | **0.34** | **K60** | **66** | **<0.0001** | **A64** | **42** | **<0.0001** | **165** | **21** | **<0.0001** | |
| **A58** | **108** | **0.35** | **S62** | **66** | **<0.0001** | **C65** | **42** | **<0.0001** | **M64** | **20** | **<0.0001** | **<0.0001** |
| **W60** | **105** | **0.62** | **M61** | **66** | **<0.0001** | **N67** | **41** | **<0.0001** | **G67** | **19** | **<0.0001** | **<0.0001** |
| **A59** | **104** | **0.61** | **P61** | **65** | **<0.0001** | **W63** | **41** | **<0.0001** | **T65** | **19** | **<0.0001** | **0.003** |
| **K72** | **104** | **0.65** | **M62** | **64** | **<0.0001** | **F69** | **41** | **<0.0001** | **A66** | **19** | **<0.0001** | **<0.0001** |
| **S59** | **103** | **0.76** | **Q61** | **64** | **<0.0001** | **N68** | **40** | **<0.0001** | **164** | **19** | **<0.0001** | **0.0003** |
| **K73** | **102** | **0.8** | **G61** | **64** | **<0.0001** | **V66** | **40** | **<0.0001** | **R63** | **19** | **<0.0001** | **<0.0001** |
| **A70** | **102** | **0.81** | **A63** | **64** | **<0.0001** | **H69** | **40** | **<0.0001** | **W67** | **19** | **<0.0001** | **<0.0001** |
| **Y60** | **101** | **0.96** | **L62** | **60** | **<0.0001** | **M69** | **40** | **<0.0001** | **K68** | **18** | **<0.0001** | **<0.0001** |
| **A72** | **100** | **0.94** | **168** | **60** | **<0.0001** | **R69** | **40** | **<0.0001** | **H64** | **18** | **<0.0001** | **<0.0001** |
| **S63** | **98** | **0.67** | **S67** | **59** | **<0.0001** | **W69** | **40** | **<0.0001** | **W64** | **18** | **<0.0001** | **0.0001** |
| **K59** | **96** | **0.46** | **N61** | **59** | **<0.0001** | **Q69** | **39** | **<0.0001** | **Q65** | **18** | **<0.0001** | **0.0002** |
| **160** | **96** | **0.5** | **169** | **59** | **<0.0001** | **L67** | **38** | **<0.0001** | **F64** | **16** | **<0.0001** | **0.0008** |
| **G70** | **95** | **0.41** | **V61** | **58** | **<0.0001** | **K69** | **38** | **<0.0001** | **L65** | **16** | **<0.0001** | **0.0022** |
| **D65** | **95** | **0.44** | **D61** | **58** | **<0.0001** | **K62** | **38** | **<0.0001** | **N64** | **16** | **<0.0001** | **<0.0001** |
| **E70** | **93** | **0.27** | **E60** | **57** | **<0.0001** | **E67** | **37** | **<0.0001** | **F65** | **16** | **<0.0001** | **0.12** |
| **163** | **92** | **0.19** | **A61** | **57** | **<0.0001** | **L69** | **37** | **<0.0001** | **Q67** | **15** | **<0.0001** | **0.0012** |
| **s60** | **92** | **0.23** | **Q62** | **56** | **<0.0001** | **S64** | **36** | **<0.0001** | **M65** | **14** | **<0.0001** | **0.015** |
| **P59** | **88** | **0.08** | **F68** | **56** | **<0.0001** | **G62** | **36** | **<0.0001** | **D66** | **14** | **<0.0001** | **0.013** |
| **M63** | **87** | **0.03** | **N65** | **56** | **<0.0001** | **E69** | **36** | **<0.0001** | **R67** | **14** | **<0.0001** | **0.002** |
| **K71** | **85** | **0.047** | **A62** | **56** | **<0.0001** | **E68** | **36** | **<0.0001** | | **Non-agonists** | | |
| **V62** | **84** | **0.04** | **A68** | **53** | **<0.0001** | **V67** | **35** | **<0.0001** | **P63** | **13** | **<0.0001** | **0.012** |
| **170** | **84** | **0.04** | **P66** | **53** | **<0.0001** | **D62** | **35** | **<0.0001** | **E64** | **12** | **<0.0001** | **0.053** |
| **161** | **83** | **0.01** | **R61** | **53** | **<0.0001** | **R68** | **34** | **<0.0001** | **W65** | **11** | **<0.0001** | **0.24** |
| **V68** | **82** | **0.0045** | **S68** | **53** | **<0.0001** | **Q66** | **34** | **<0.0001** | **Q64** | **11** | **<0.0001** | **0.15** |
| **E59** | **81** | **0.01** | **Y63** | **52** | **<0.0001** | **A67** | **33** | **<0.0001** | **G66** | **11** | **<0.0001** | **0.07** |
| **Partial agonists** | | | **N69** | **51** | **<0.0001** | **N62** | **32** | **<0.0001** | **R65** | **11** | **<0.0001** | **0.26** |
| **W61** | **79** | **0.002** | **E63** | **51** | **<0.0001** | **F66** | **31** | **<0.0001** | **Y67** | **10** | **<0.0001** | **0.13** |
| **A60** | **78** | **0.002** | **T64** | **51** | **<0.0001** | **E62** | **31** | **<0.0001** | **E66** | **10** | **<0.0001** | **0.17** |
| **Y62** | **78** | **0.006** | **T67** | **51** | **<0.0001** | **D69** | **31** | **<0.0001** | **K66** | **10** | **<0.0001** | **0.21** |
| **G60** | **77** | **0.003** | **Y69** | **50** | **<0.0001** | **D67** | **30** | **<0.0001** | **R66** | **10** | **<0.0001** | **0.23** |
| **A71** | **77** | **0.003** | **D63** | **50** | **<0.0001** | **M67** | **29** | **<0.0001** | **K67** | **10** | **<0.0001** | **0.11** |
| **W62** | **76** | **0.0009** | **A65** | **49** | **<0.0001** | **Y66** | **28** | **<0.0001** | **P65** | **8** | **<0.0001** | **0.57** |
| **Q60** | **76** | **0.001** | **K61** | **49** | **<0.0001** | **167** | **28** | **<0.0001** | **K64** | **8** | **<0.0001** | **0.82** |
| **L63** | **74** | **0.0002** | **166** | **49** | **<0.0001** | **H65** | **26** | **<0.0001** | **K65** | **8** | **<0.0001** | **0.63** |
| **162** | **74** | **0.0005** | **T68** | **48** | **<0.0001** | **P68** | **26** | **<0.0001** | **Y65** | **7** | **<0.0001** | **0.9** |
| **K70** | **74** | **0.001** | **S65** | **48** | **<0.0001** | **Y64** | **25** | **<0.0001** | | | | |
| **H61** | **72** | **<0.0001** | **L68** | **48** | **<0.0001** | **EK65** | **25** | **<0.0001** | | | | |
| **W68** | **72** | **<0.0001** | **Q68** | **48** | **<0.0001** | **T66** | **25** | **<0.0001** | | | | |

**Table 9. Antagonism of A-gliadin 57-73 QE65 interferon gamma ELISPOT response by substituted variants of A-gliadin 57-73 QE65 (Subst) (P is significance level in unpaired t-test). Agonist activity (% agonist) of peptides compared to A-gliadin 57-73 QE65 is also shown.**

| **Subst** | **% Inhibit.** | **P** | **% agonist.** | **Subst** | **% Inhibit.** | **P** | **% agonist.** |
|---|---|---|---|---|---|---|---|
| **Antagonists** | | | | **65R** | **13** | **0.18** | **11** |
| **65T** | **28** | **0.004** | **19** | **65M** | **13** | **0.16** | **14** |
| **67M** | **27** | **0.0052** | **29** | **68P** | **13** | **0.16** | **26** |
| **64W** | **26** | **0.007** | **18** | **63R** | **13** | **0.19** | **19** |
| **67W** | **25** | **0.0088** | **19** | **66G** | **12** | **0.19** | **11** |
| **Potential antagonists** | | | | **65Q** | **12** | **0.2** | **18** |
| | | | | | | | |
| **671** | **24** | **0.013** | **10** | **65Y** | **12** | **0.22** | **7** |
| **67Y** | **24** | **0.013** | **21** | **66S** | **12** | **0.22** | **22** |
| **64G** | **21** | **0.03** | **21** | **67F** | **11** | **0.25** | **21** |
| **64D** | **21** | **0.029** | **16** | **66R** | **10** | **0.29** | **10** |
| **65L** | **20** | **0.046** | **26** | **67K** | **10** | **0.29** | **10** |
| **66N** | **20** | **0.037** | **24** | **64F** | **10** | **0.29** | **16** |
| **65H** | **20** | **0.038** | **16** | **65F** | **9** | **0.41** | **16** |
| **64N** | **19** | **0.05** | **16** | **63P** | **8** | **0.42** | **13** |
| **64Y** | **19** | **0.06** | **25** | **65EK** | **8** | **0.39** | **25** |
| **66Y** | **19** | **0.048** | **28** | **64Q** | **7** | **0.49** | **11** |
| **64E** | **19** | **0.049** | **12** | **641** | **5** | **0.6** | **21** |
| **67A** | **18** | **0.058** | **30** | **68K** | **5** | **0.56** | **19** |
| **67H** | **18** | **0.052** | **22** | **67Q** | **5** | **0.61** | **18** |
| **Non-antagonists** | | | | **65G** | **5** | **0.62** | **15** |
| **65V** | **17** | **0.07** | **23** | **64M** | **4** | **0.7** | **20** |
| **651** | **17** | **0.086** | **21** | **66H** | **4** | **0.66** | **23** |
| **66T** | **17** | **0.069** | **25** | **66 E** | **3** | **0.76** | **10** |
| **65W** | **15** | **0.11** | **11** | **66D** | **1** | **0.9** | **14** |
| **67R** | **15** | **0.13** | **14** | **63K** | **1** | **0.88** | **23** |
| **65P** | **15** | **0.13** | **8** | **64H** | **1** | **0.93** | **18** |
| **65K** | **15** | **0.11** | **8** | **66K** | **0** | **0.98** | **10** |
| **66W** | **15** | **0.12** | **21** | **64K** | **-2** | **0.88** | **8** |
| **67G** | **14** | **0.14** | **19** | **64L** | **-11** | **0.26** | **22** |
| **66A** | **14** | **0.14** | **19** | | | | |

**Table 10. Inhibition of A-gliadin 57-73 QE65 interferon gamma ELISPOT response by peptides known to bind HLA-DQ2 (P is significance level in unpaired t-test).**

| **Peptide** | **% Inhibit.** | **P** |
|---|---|---|
| **TP** | **31** | **<0.0001** |
| **HLA1a** | **0** | **0.95** |

**Table 12. Prolamin homologues of A-gliadin 57-73 (excluding alpha/beta-gliadins)**

| **Prolamin** | **Accession number** | **Sequence** | **% Bioactivity*** |
|---|---|---|---|
| Wheat: α-gliadin | A-gliadin (57-73) | **QLQPFPQPQLPYPQPQS** | 100 (0) |
| Wheat: ω-gliadin | AAG17702 (141-157) | PQ...........................F......QSE | 32 (6.4) |
| Barley: C-hordein | Q40055 (166-182) | ...QPFPL...............F............Q | 2.3 (2.0) |
| Wheat γ-gliadin | P21292 (96-112) | ...QTFPQ...............F......QPQ | 2.1 (4.2) |
| Rye: secalin | Q43639 (335-351) | ...QPSPQ...............F............Q | 1.6 (1.4) |
| Barley: γ-hordein | P80198 (52-68) | ...QPFPQ...............HQHQFP | -1.0 (1.8) |
| Wheat: LMW glutenin | P16315 (67-83) | LQ...QPIL............FS...Q...Q | -0.9 (1.0) |
| Wheat: HMW glutenin | P08489 (718-734) | HGYYPTS.........SGQGQRP | 6.4 (4.0) |
| Wheat γ-gliadin | P04730 (120-136) | ...QCCQQL......I...QQSRYQ | 0.7 (0.9) |
| Wheat: LMW glutenin | P10386 (183-199) | ...QCCQQL......I...QQSRYE | -0.7 (0.5) |
| Wheat: LMW glutenin | 049958 (214-230) | ...QCCRQL......I...EQSRYD | -1.1 (0.3) |
| Barley: B1-hordein | P06470 (176-192) | ...QCCQQL......I...EQFRHE | 1.8 (1.4) |
| Barley: B-hordein | 040026 (176-192) | ...QCCQQL......ISEQFRHE | 0.5 (0.9) |

| | | | |
|---|---|---|---|
| *Bioactivity is expressed as 100x(spot forming cells with peptide 25mcg/ml plus tTG 8mcg/ml minus blank)/(spot forming cells with A-gliadin 57-73 25mcg/ml plus tTG 8mcg/ml minus blank) (mean (SEM), n=5). Peptides were preincubated with tTG for 2h 37°C. Note, Q is deamidated in A-gliadin 57-73 by tTG. | | | |

**Table 13. Clinical details of coeliac subjects.**

| | **HLA-DQ** | **HLA-DQA1 alleles** | **HLA-DQB1 alleles** | **Duodenal histology** | **Gluten free** | **EMA on gluten (on GFD)** |
|---|---|---|---|---|---|---|
| **C01** | 2,6 | 102/6, 501 | 201,602 | SVA | 1 yr | + (-) |
| **C02** | 2,2 | 501 | 201 | SVA | 1 yr | + (-) |
| **C03** | 2,5 | 101/4/5, 501 | 201,501 | PVA | 1 yr | + (-) |
| **C04** | 2,5 | 101/4-5, 501 | 201,501 | SVA | 7 yr | + (-) |
| **C05** | 2,2 | 201,501 | 201, 202 | SVA | 4 mo | + (ND) |
| **C06** | 2,2 | 201,501 | 201,202 | SVA | 2 yr | + (-) |
| **C07** | 2,8 | 301-3,501 | 201, 302 | SVA | 1 yr | + (-) |
| **C08** | 2,8 | 301-3,501 | 201, 302/8 | SVA | 11 yr | ND (-) |
| **C09** | 2,8 | 301-3,501 | 201, 302 | SVA | 29 yr | + (-) |
| **C10** | 2,8 | 201,301-3 | 202, 302 | IEL | 1 yr | + (-) |
| **C11** | 6,8 | 102/6, 301-3 | 602/15, 302/8 | IEL | 9 mo | - (ND) |
| **C12** | 8,7 | 301-3, 505 | 302, 301/9-10 | SVA | 2 yr | - (-) |
| **C13** | 8,8 | 301 | 302 | SVA | 1yr | + (+) |

| | | | | | | |
|---|---|---|---|---|---|---|
| SVA subtotal villous atrophy, PVA partial villous atrophy, IEL increased intra-epithelial atrophy, GFD gluten-free diet, ND not done. | | | | | | |

**Table 14. HLA-DQ2+ Coeliac (C01-6) and healthy control (H01-10) IFNγ ELISpot responses to control peptides (20 µg/ml) and gliadin (500 µg/ml) before and after gluten challenge (sfc/million PBMC minus response to PBS alone)**

| **Peptide** | **Healthy Day 0** | **Healthy Day 6** | **Coeliac Day 0** | **Coeliac Day 6** |
|---|---|---|---|---|
| P04722 77-93 | 0 (-4 to 17) | 0 (-5 to 9) | -2 (-3 to 0) | **27 (0-100)*** |
| P04722 77-93 + tTG | 0 (-5 to 4) | 0 (-9 to 3) | 0 (-4 to 11) | **141 (8 to 290)**** |
| P04722 77-93 QE85 | 0 (-5 to 5) | 0 (-3 to 4) | 0 (-6 to 14) | **133 (10 to 297)*** |
| P02863 77-93 | 0 (-4 to 13) | 2 (-3 to 5) | -2 (-3 to 2) | **8 (-2 to 42)**** |
| P02863 77-93 +tTG | -1 (-5 to 4) | -1 (-4 to 11) | 1 (-4 to 6) | **65 (8-164)**** |
| P02863 77-93 QE85 | 0 (-4 to 13) | 0 (-4 to 14) | -1 (-4 to 6) | **42 (-2 to 176)*** |
| Gliadin chymotrypsin | 2 (-5 to 20) | **18 (0 to 185)*** | 20(11 to 145) | 92 (50 to 154) |
| Gliadin chymotrypsin + tTG | 0 (-1 to 28) | **16 (-9 to 171)*** | 55 (29 to 248) | **269 (206 to 384)**** |
| Chymotrypsin | 0 (-4 to 5) | 1 (-4 to 11) | -2 (-5 to 5) | 1 (-4 to 8) |
| Chymotrypsin + tTG | 0 (-5 to 8) | 6 (0 to 29) | -2(-3 to 11) | **2 (-3 to 18)*** |
| Gliadin pepsin | 4 (-4 to 28) | **29 (0 to 189)***** | 44 (10 to 221) | **176 (54 to 265)**** |
| Gliadin pepsin + tTG | 2 (-3 to 80) | **27 (-4 to 241)***** | 61 (8 to 172) | **280 (207 to 406)**** |
| Pepsin | 0 (-4 to 10) | 0 (-3 to 12) | 0 (-2 to 3) | 2 (-2 to 8) |
| Pepsin + tTG | 0 (-3 to 8) | 0 (-5 to 9) | 1 (-6 to 3) | 0 (-3 to 14) |
| PBS alone | 4 (0 to 6) | 2 (0 to 6) | 4 (1 to 12) | 4 (0 to 4) |
| PBS + tTG | 3 (0 to 8) | 3 (0 to 11) | 4 (2 to 10) | 4 (2 to 11) |

| | | | | |
|---|---|---|---|---|
| Day 6 vs. Day 0: *P<0.05 **P,0.02, ***P<0.01 by one-tailed Wilcoxon Matched-Pairs Signed-Ranks test | | | | |

**Table 15. Effect of deamidation by tTG to gliadin (0.5 mg/ml) and A-gliadin 57-73 homologues on IFNγ ELISpot responses in HLA-DQ2+ coeliac (C01-6) and healthy control subjects (H01-10) (median ratio tTG:no tTG pretreatment, range)**

| **Peptide** | **Healthy Day 6** | **Coeliac Day 0** | **Coeliac Day 6** |
|---|---|---|---|
| Gliadin chymotrypsin | 0.94 (0.4-9.0) | 2.1 (0.8-6.8)* | 3.2 (1.8 -4.2)** |
| Gliadin pepsin | 1.4 (0.5-1.4) | 1.4 (0.8-4.0)* | 1.9 (1.1-4.4)** |
| P04722 77-93 Q85 | | | 6.5 (2.3-12)** |
| P04722 77-93 E85 | | | 0.7 (0.6-1.1) |
| P02863 77-93 Q85 | | | 7.5 (3.9-19.9)** |
| P02863 77-93 E85 | | | 1.0 (0.8-1.2) |

| | | | |
|---|---|---|---|
| TTG>no tTG: *P<0.05 **P,0.02, ***P<0.01 by one-tailed Wilcoxon Matched-Pairs Signed-Ranks test | | | |

**Table 16. Healthy subjects: IFNγ ELISpot Responses (>10 sfc/million PBMC and >4 x buffer only) to tTG-treated gliadin peptide Pools on Day 6 of gluten challenge (sfc/million PBMC) (italic: response also present on Day 0):**

| | | | | | | | | | | | | |
|---|---|---|---|---|---|---|---|---|---|---|---|---|
| **Group 1 - HLA-DQ2 (DQA1*0501-5, DQB1*0201)** | | | | | | | | | | | | |
| **Group 2 - HLA-DQ8 (DQA1*0301, DQB1*0302) and absent or "incomplete" DQ2 (only** | | | | | | | | | | | | |
| **DQA1*0501-5 or DQB1*0201)** | | | | | | | | | | | | |

| | **Group 1** | | | | | | | | | | | **Group 2** |
|---|---|---|---|---|---|---|---|---|---|---|---|---|
| **Subject** | H01 | H02 | H03 | H04 | H05 | H06 | H07 | H08 | H09 | H10 | | H11 |
| **HLA-DQ** | 2,6 | 2,7 | 2,8 | 2,5 | 2,6 | 2,6 | 2,6 | 2,7 | 2,5 | 2,5 | | 8,8 |
| **Pool 1** | . | . | . | . | . | . | . | . | . | . | | . |
| **2** | . | . | . | . | . | . | . | . | . | . | | . |
| **3** | . | . | . | . | . | . | . | . | . | . | | . |
| **4** | . | . | . | . | . | . | . | . | 13 | . | | . |
| **5** | . | 17 | . | . | . | . | . | . | ***24*** | . | | . |
| **6** | . | . | . | . | . | . | . | . | ***31*** | . | | . |
| **7** | . | . | . | . | . | . | . | . | . | . | | . |
| **8** | . | . | . | . | . | . | . | . | . | . | | . |
| **9** | . | . | . | . | . | . | . | . | . | . | | . |
| **10** | . | . | . | . | . | . | . | . | . | . | | . |
| **11** | . | . | . | . | . | . | . | . | . | . | | . |
| **12** | . | . | . | . | . | . | . | . | . | . | | . |
| **13** | . | . | . | . | . | . | . | . | . | . | | . |
| **14** | . | . | . | . | . | . | . | . | . | . | | . |
| **15** | . | . | . | . | . | . | . | . | . | . | | . |
| **16** | . | . | . | . | . | . | . | . | . | . | | . |
| **17** | . | . | . | . | . | . | . | . | . | . | | . |
| **18** | . | . | . | . | . | . | 20 | . | . | . | | . |
| **19** | . | . | . | . | . | . | . | . | . | . | | . |
| **20** | . | 11 | . | . | . | . | . | . | . | . | | . |
| **21** | . | 11 | . | . | . | . | . | . | 27 | . | | . |
| **22** | . | . | . | . | . | . | . | . | . | . | | . |
| **23** | . | ***43*** | . | . | . | . | . | . | . | . | | . |
| **24** | . | . | . | . | . | . | . | . | . | . | | . |
| **25** | . | 11 | . | . | . | . | . | . | . | . | | . |
| **26** | . | . | . | . | . | . | . | . | . | . | | . |
| **27** | . | . | . | . | . | . | . | . | . | . | | . |
| **28** | . | . | . | . | . | . | . | . | . | . | | . |
| **29** | . | . | . | . | . | . | . | . | . | . | | . |
| **30** | . | . | . | . | . | . | . | 23 | . | . | | . |
| **31** | . | . | . | . | . | . | . | . | . | . | | . |
| **32** | . | . | . | . | . | . | . | . | . | . | | . |
| **33** | . | 20 | . | . | . | . | . | . | . | . | | . |
| **34** | . | . | . | . | . | . | . | . | . | . | | . |
| **35** | . | 11 | . | . | . | . | . | . | . | . | | . |
| **36** | . | . | . | . | . | . | . | . | . | . | | . |
| **37** | . | . | . | . | . | . | . | ***18*** | . | . | | . |
| **38** | 14 | . | . | . | . | . | . | 12 | . | . | | . |
| **39** | . | . | . | . | . | . | . | 11 | . | . | | . |
| **40** | . | 14 | . | . | . | . | . | 17 | . | . | | . |
| **41** | . | . | . | . | . | . | . | . | . | . | | . |
| **42** | . | . | . | . | . | . | . | . | . | . | | . |
| **43** | . | . | . | . | . | . | . | . | 11 | . | | . |
| **44** | . | 14 | . | . | . | . | . | . | . | . | | . |
| **45** | . | 11 | . | . | . | . | . | . | . | . | | . |
| **46** | . | . | . | . | . | . | . | . | . | . | | . |
| **47** | . | . | . | . | . | . | . | . | . | . | | . |
| **48** | . | . | . | . | . | . | . | . | . | . | | . |
| **49** | . | . | . | . | . | . | . | . | . | . | | . |
| **50** | . | 14 | . | . | 12 | . | . | ***22*** | . | ***14*** | | . |
| **51** | . | . | . | . | . | . | . | . | . | . | | . |
| **52** | . | 14 | . | . | . | . | . | . | . | . | | . |
| **53** | . | 26 | . | . | . | . | . | . | . | . | | . |
| **54** | . | . | . | . | . | . | . | 12 | . | . | | . |
| **55** | . | . | . | . | . | . | . | . | . | . | | . |
| **56** | . | . | . | . | . | . | . | . | . | . | | . |
| **57** | . | 23 | . | . | . | . | 12 | . | . | . | | . |
| **58** | . | 14 | . | . | . | . | . | . | . | . | | . |
| **59** | . | . | . | . | . | . | . | . | . | . | | . |
| **60** | . | . | . | . | . | . | . | . | . | . | | . |
| **61** | . | 23 | . | . | . | . | . | ***11*** | ***11*** | . | | . |
| **62** | . | . | . | . | . | . | . | . | . | . | | . |
| **63** | . | . | . | . | . | . | . | . | . | . | | . |
| **64** | . | 20 | . | . | . | . | . | . | . | . | | . |
| **65** | . | . | . | . | . | . | . | . | . | . | | . |
| **66** | . | 14 | . | . | . | . | . | . | . | . | | . |
| **67** | . | 11 | . | . | . | . | . | . | . | . | | . |
| **68** | . | 20 | . | . | . | . | . | ***20*** | . | . | | . |
| **69** | . | 20 | . | . | . | . | . | . | . | . | | . |
| **70** | . | . | . | . | . | . | . | . | . | . | | . |
| **71** | . | . | . | . | . | . | . | . | . | . | | 16 |
| **72** | . | 11 | . | . | . | . | . | . | . | . | | . |
| **73** | . | 14 | . | . | . | . | . | . | . | . | | . |
| **74** | . | . | . | . | . | . | . | . | . | . | | . |
| **75** | . | . | . | . | . | . | . | . | . | . | | . |
| **76** | . | 14 | . | . | . | . | . | . | . | . | | . |
| **77** | . | .. | . | . | . | . | . | . | . | . | | . |
| **78** | . | 11 | . | . | . | . | . | . | . | . | | . |
| **79** | . | 11 | . | . | 19 | . | . | . | . | . | | . |
| **80** | . | . | . | . | . | . | . | . | . | . | | . |
| **81** | . | . | . | . | . | . | . | . | . | . | | . |
| **82** | . | . | . | . | . | . | . | . | . | . | | . |
| **83** | . | . | . | . | . | . | . | . | . | . | | . |
| **P04722 77-93** | . | . | . | . | . | . | . | . | . | . | | . |
| **P04722 77-93** E | . | . | . | . | . | . | . | . | . | . | | . |
| **P04722 77-93 E** | . | . | . | . | . | . | . | . | . | . | | . |
| **P02863 77-93** | . | . | . | . | . | . | . | 11 | . | . | | . |
| **P02863 77-93 E** | . | . | . | . | . | . | . | . | . | . | | . |
| **Giladin+C** | ***171*** | 40 | 25 | 16 | 10 | . | 18 | 14 | . | 17 | | ***90*** |
| **Chymotrypsin** | 29 | 26 | 18 | . | . | . | . | . | 22 | . | | . |
| **Gliadin+Pepsin** | ***241*** | 151 | 29 | 24 | 48 | . | 16 | 45 | . | 19 | | ***35*** |
| **Pepsin** | . | . | . | . | . | . | . | . | . | . | | . |

**Table 17: tTG-deamidated gliadin peptide pools showing significant increase in IFN gamma responses between Day 0 and Day 6 of gluten challenge in HLA-DQ2 coeliac subjects C01-6 (Day 6 -Day 0 response, and ratio of responses to tTG-deamidated pool and same pool without tTG treatment)**

| **Pool** | **IFNg ELISpot (Median sfc/million)** | **tTG: no tTG (Median)** | **Pool** | **IFNg ELISpot (Median sfc/million)** | **tTG: no tTG (Median)** |
|---|---|---|---|---|---|
| **9** | 59*** | 1.0 | **49** | 46*** | 1.4 |
| **10** | 116** | 1.7 | **50** | 50*** | 4.6 |
| **11** | 24*** | 2.5 | **51** | 40*** | 1.7 |
| **12** | 133*** | 1.1 | **52** | 30*** | 3.1 |
| **13** | 26** | 2.1 | **53** | 27** | 1.4 |
| **42** | 30** | 1.2 | **76** | 17*** | 1.1 |
| **43** | 32*** | 1.3 | **79** | 20*** | 0.9 |
| **44** | 24*** | 1.5 | **80** | 83*** | 1 |
| **45** | 10*** | 1.1 | **81** | 141*** | 1.1 |
| **46** | 12*** | 2.1 | **82** | 22*** | 1.5 |
| **48** | 17*** | 1.4 | **83** | 16** | 1.8 |

| | | | | | |
|---|---|---|---|---|---|
| Day 6 vs. Day 0 **P<0.02, ***P<0.01 by one-tailed Wilcoxon Matched-Pairs Signed-Ranks test | | | | | |

**Table 18. Coeliac subjects: IFNγ ELISpot Responses >10 sfc/million PBMC and >4 x buffer only to tTG-treated Pepset Pools on Day 6 of gluten challenge (sfc/million PBMC) (italic: response also present on Day 0):**

| | | | | | | | | | | | | | | | |
|---|---|---|---|---|---|---|---|---|---|---|---|---|---|---|---|
| **Group 1 - HLA-DQ2 (DQA1*0501-5, DQB1*0201/2),** | | | | | | | | | | | | | | | |
| **Group 2 - HLA-DQ2/8 (DQA1*0501-5, *0301, and DQB1*0201/2, *0302), and** | | | | | | | | | | | | | | | |
| **Group 3 - HLA-DQ8 (DQA1*0301, DQB1*0302) and absent or "incomplete" DQ2 (only DQA1*0501-5 or DQB1*0201/2)** | | | | | | | | | | | | | | | |

| | **Group 1:** | | | | | | | **Group 2:** | | | | **Group 3** | | | |
|---|---|---|---|---|---|---|---|---|---|---|---|---|---|---|---|
| **Subject** | C01 | C02 | C03 | C04 | C05 | C06 | | C07 | C08 | C09 | | C10 | C11 | C12 | C13 |
| **HLA-DQ** | 2,6 | 2,2 | 2,5 | 2,5 | 2,2 | 2,2 | | 2,8 | 2,8 | 2,8 | | 2,8 | 6,8 | 7,8 | 8,8 |
| **Pool 1** | . | | | | | | | 23 | | | | | . | 223 | |
| **2** | . | | | | | | | | | | | | . | 155 | |
| **3** | . | | | | | | | | | | | | . | 41 | |
| **4** | 11 | | | | | | | | | | | 22 | . | | |
| **5** | . | | | | | | | | | | | . | . | | |
| **6** | 18 | | | 21 | | | | 20 | 17 | | | . | . | | |
| **7** | . | | | | | | | | 353 | | | . | . | | |
| **8** | 11 | 64 | | | | 14 | | 20 | 480 | | | . | . | | 13 |
| **9** | 93 | 127 | | 92 | 25 | | | 32 | 460 | | | . | . | | 18 |
| **10** | 175 | 491 | 58 | 200 | 48 | | | 84 | ***787*** | | | . | . | | |
| **11** | 32 | 118 | | 33 | 14 | | | 26 | 27 | | | 12 | . | | |
| **12** | 204 | 379 | 54 | 225 | 61 | | | 129 | 587 | | | 12 | . | | |
| **13** | 93 | 142 | | 29 | 18 | | | | ***60*** | | | . | . | | 11 |
| **14** | . | 45 | | 21 | | | | 17 | | | | . | . | | |
| **15** | 18 | 30 | | | | | | 38 | 43 | | | . | . | | |
| **16** | . | | | | | | | | 37 | | | . | . | | |
| **17** | . | | | | | | | | | | | . | . | | |
| **18** | . | | | | | | | | | | | . | . | | |
| **19** | 11 | | | | | | | | | | | . | . | | |
| **20** | 11 | 215 | | | | | | 51 | 167 | | | . | . | | |
| **21** | . | | | | | | | | | 11 | | . | . | | |
| **22** | . | 21 | | | | | | | | | | . | . | | |
| **23** | . | 18 | | 21 | | | | | | | | 12 | . | | |
| **24** | . | 15 | | | | | | | 10 | | | . | . | | |
| **25** | . | ***15*** | | | | | | | | | | 12 | . | | |
| **26** | . | ***18*** | | | | | | | 13 | | | 12 | . | | |
| **27** | . | ***15*** | | | | | | | | | | . | . | | |
| **28** | . | | | | | | | | | | | . | . | | 11 |
| **29** | . | | | | | | | 11 | | | | . | . | | |
| **30** | 11 | | | | | | | 11 | | | | . | . | | |
| **31** | . | 70 | | | | | | | | | | . | . | | |
| **32** | . | 18 | | | | | | 20 | | | | . | . | | |
| **33** | 11 | | | 10 | | | | ***14*** | | ***11*** | | . | 40 | | ***11*** |
| **34** | . | | | | | | | | | 11 | | . | . | | |
| **35** | . | | | | | | | | | | | . | . | | |
| **36** | . | | | | | | | | | | | . | . | | |
| **37** | . | | | 23 | | 14 | | | | | | . | . | | |
| **38** | . | 24 | | 19 | | | | 20 | | | | . | . | | |
| **39** | . | 49 | | 15 | | | | | | ***11*** | | . | . | | |
| **40** | . | | | | | | | 14 | | | | . | . | | |
| **41** | | 21 | | | | | | | | | | . | . | | |
| **42** | 39 | 42 | | 44 | 21 | | | 11 | 63 | | | 12 | . | | |
| **43** | 50 | 91 | 13 | 75 | 14 | | | 190 | 113 | | | . | . | 21 | |
| **44** | 32 | 97 | 17 | 96 | 13 | | | 87 | 107 | | | . | . | | |
| **45** | | 21 | 10 | 100 | 11 | | | 38 | 110 | | | . | . | | |
| **46** | 14 | 55 | | 102 | 18 | | | 63 | ***163*** | | | . | . | | |
| **47** | 14 | 58 | | 38 | | | | 223 | 97 | | | . | . | 31 | |
| **48** | 21 | 106 | | 60 | 14 | | | 144 | 353 | | | . | . | 57 | |
| **49** | 75 | 170 | 17 | 142 | 30 | | | 202 | 293 | | | . | . | 39 | |
| **50** | 57 | ***245*** | 23 | 140 | 61 | ***27*** | | 248 | ***143*** | | | . | . | | 11 |
| **51** | 68 | 106 | 10 | 127 | | | | 220 | ***267*** | | | . | . | 29 | |
| **52** | 43 | 121 | | 79 | 13 | 16 | | 175 | 180 | | | . | . | | |
| **53** | 36 | 94 | | 92 | 29 | | | 69 | 53 | | | . | . | | |
| **54** | 36 | | | 35 | 11 | | | 166 | 27 | | | . | . | 19 | 13 |
| **55** | . | | | | | | | | | | | . | . | | |
| **56** | 29 | | | | | | | | | ***11*** | | . | . | | |
| **57** | . | 36 | | | | | | 20 | 13 | | | . | . | | |
| **58** | . | | | | | | | | | | | . | . | | |
| **59** | . | | 10 | | | | | | 53 | | | . | . | | |
| **60** | . | 18 | | 15 | | | | 11 | 53 | | | . | . | | |
| **61** | . | | | | | | | 20 | | | | . | . | | |
| **62** | 14 | 18 | | 13 | | | | 60 | | | | . | . | | |
| **63** | . | | 10 | | | 14 | | | | | | 28 | . | | |
| **64** | . | 15 | | | | | | | | | | 18 | . | | |
| **65** | . | 36 | | 25 | ***23*** | | | 35 | 27 | | | . | ***11*** | | |
| **66** | . | | | 31 | 11 | 10 | | 17 | | | | . | . | | |
| **67** | . | | | 17 | | | | 17 | | | | . | . | | |
| **68** | . | | 19 | ***127*** | | 14 | | | | | | . | . | | |
| **69** | . | 15 | | 10 | | | | ***20*** | 20 | | | . | . | | |
| **70** | . | 12 | 31 | | 13 | 10 | | | | | | . | . | | |
| **71** | 11 | 21 | 13 | | | | | 14 | | | | . | 18 | | |
| **72** | . | | | | | 16 | | | | | | . | . | | |
| **73** | . | | | 13 | | ***14*** | | 11 | | | | . | . | | |
| **74** | . | 239 | | | | | | 254 | 447 | | | . | . | | |
| **75** | . | | | | | | | | | | | . | . | | |
| **76** | 18 | 21 | 19 | 15 | | | | | | | | . | . | 12 | |
| **77** | . | 88 | | | | 10 | | | 13 | | | . | . | | |
| **78** | . | 18 | 17 | 69 | | | | | | | | . | . | | |
| **79** | 11 | 85 | | 44 | 29 | 12 | | 44 | 43 | | | . | . | | |
| **80** | 132 | 133 | 33 | 240 | 39 | 12 | | 208 | 467 | | | 12 | . | 70 | |
| **81** | 171 | 318 | 113 | 367 | 104 | 12 | | 211 | 530 | | | . | . | 74 | |
| **82** | 18 | 300 | 17 | 125 | 32 | ***16*** | | 241 | 723 | | | . | . | | |
| **83** | 14 | 164 | | 31 | 21 | | | 163 | 277 | | | 15 | . | | |
| **P04722 77-93** | 211 | 291 | 75 | 281 | 66 | | | 78 | ***740*** | | | . | . | | |
| **P04722 77-93** E | 164 | 297 | 108 | 221 | 64 | 10 | | 84 | 653 | | | . | . | | |
| **P04722 77-93** E | 161 | 182 | 98 | 256 | 73 | ***16*** | | 63 | 500 | | | . | . | | |
| **P02863 77-93** | 139 | 164 | 35 | 94 | 36 | | | 29 | 603 | | | . | . | | |
| **P02863 77-93** E | 46 | 176 | 19 | 88 | 41 | | | 23 | 520 | | | . | . | | |
| **Gliadin+C** | ***214*** | ***273*** | ***265*** | ***360*** | ***384*** | ***206*** | | ***278*** | ***543*** | 17 | | | ***25*** | ***527*** | 71 |
| **Chymotrypsin** | . | | | | | ***18*** | | | | | | . | . | | |
| **Gliadin+Pepsin** | ***239*** | ***315*** | 269 | ***406*** | ***207*** | 292 | | ***357*** | ***557*** | | | ***42*** | ***89*** | ***335*** | 87 |
| **Pepsin** | . | | | | | 14 | | | | | | | | | |

**Table 19. Deamidated peptides with mean bioactivity > 10% of P04722 E85 (20 µg/ml) in HLA-DQ2 coeliac subjects C01-5**

| **Rank** | **No.** | **Sequence** | **Mean (SEM)** | **Rank** | **No.** | **Sequence** | **Mean (SEM)** |
|---|---|---|---|---|---|---|---|
| | 89 | PQLPYPQPQLPYPQPQLPYP | 94(18) | **37** | 413 | SKQPQQPFPQPQQPQQSFPQ | 18(4) |
| ***2** | 91 | PQPFPPQLPYPQPQLPYPQP | 89(12) | **38** | 380 | QPQQPQQPFPQPQQPQLPFP | 18(6) |
| ***3** | 74 | MQLQPFPQPQLPYPQPQLPY | 88(14) | **39** | 618 | PQQSFSYQQQPFPQQPYPQQ | 18(7) |
| ***4** | 90 | PQLPYPQPQLPYPQPQPFRP | 87(16) | ***40** | 78 | LQLQPFPRPQLPYPQPQPFR | 17(8) |
| ***5** | 76 | LQLQPFPQPQLPYPQPQPFR | 85(15) | **41** | 390 | QQTYPQRPQQPFPQTQQPQQ | 17(9) |
| **6** | 626 | PQQPQQPQQPFPQPQQPFPW | 72(23) | **42** | 348 | QQTFPQPQQTFPHQPQQQFP | 16(10) |
| **7** | 627 | QPFPQPQQPFPWQPQQPFPQ | 66(30) | **43** | 409 | QPQQPFPQLQQPQQPLPQPQ | 16(2) |
| ***8** | 631 | FPQQPQQPFPQPQLPFPQQS | 61(12) | **44** | 382 | QQPFPQQPQQPFPQTQQPQQ | 16(6) |
| **9** | 636 | PQQPQQPFPQPQQPIPVQPQ | 51(10) | **45** | 629 | PFPQTQQSFPLQPQQPFPQQ | 16(5) |
| ***10** | 73 | LQLQPFPQPQLPYPQPQLPY | 49(11) | **46** | 643 | PLQPQQPFPQQPQQPFPQQP | 16(6) |
| **11** | 412 | SQQPQQPFPQPQQQFPQPQQ | 34(19) | **47** | 389 | QQPFPQTQQPQQPFPQQPQQ | 16(6) |
| **12** | 343 | QQPQQPFPQPQQPQLPFPQQ | 34(11) | **48** | 350 | QQIFPQPQQTFPHQPQQAFP | 15(8) |
| ***13** | 68 | LQLQPFPQPQLPYLQPQPFR | 33(10) | **49** | 65 | PFPSQQPYPQPQPFPQPQPF | 15(5) |
| ***14** | 66 | LQLQPFPQPQLPYSQPQPFR | 32(7) | **50** | 349 | QQIFPQPQQTFPHQPQQQFP | 15(9) |
| ***15** | 96 | PQPFPPQLPYPQPQSFPPQQ | 28(6) | **51** | 610 | PWQQQPLPPQQSFSQQPPFS | 15(11) |
| **16** | 393 | QLPFPQQPQQPFPQPQQPQQ | 27(8) | ***52** | 81 | PQPQPFPPQLPYPQTQPFPP | 15(5) |
| **17** | 355 | QAFPQPQQTFPHQPQQQFPQ | 27(15) | ***53** | 75 | MQLQPFPQPQPFPPQLPYPQ | 14(5) |
| ***18** | 67 | LQLQPFPQPQLPYSQPQQFR | 26(6) | **54** | 368 | QQFPQPQQPQQPFPQQPQQQ | 14(7) |
| **19** | 335 | QQQQPFPQPQQPQQPFPQPQ | 25(11) | ***55** | 82 | PQPQPFPQPQPFPPQLPYPQ | 14(3) |
| ***20** | 95 | PQPFLPQLPYPQPQSFPPQQ | 24(6) | ***56** | 80 | LQLQPFPQPQPFPPQLPYPQ | 14(4) |
| **21** | 396 | TQQPQQPFPQQPQQPFPQTQ | 23(9) | **57** | 624 | FTQPQQPTPIQPQQPFPQQP | 14(6) |
| **22** | 609 | SCISGLERPWQQQPLPPQQS | 23(18) | **58** | 407 | QPQQPFPQSQQPQQPFPQPQ | 14(5) |
| **23** | 385 | QQPFPQPQQPQLPFPQQPQQ | 23(7) | **59** | 337 | QQQPFPQPQQPFCQQPQRTI | 13(4) |
| **24** | 375 | PQQPFPQPQQPQQPFPQPQQ | 23(10) | **60** | 634 | PQQLQQPFPLQPQQPFPQQP | 13(3) |
| **25** | 406 | QPQQPFPQLQQPQQPFPQPQ | 22(8) | **61** | 388 | QQPYPQQPQQPFPQTQQPQQ | 13(3) |
| **26** | 625 | PIQPQQPFPQQPQQPQQPFP | 22(9) | **62** | 641 | FPELQQPIPQQPQQPFPLQP | 13(7) |
| **27** | 378 | QQPQQPFPQQPQQQFPQPQQ | 22(10) | **63** | 399 | QQPFPQTQQPQQPFPQLQQP | 13(5) |
| **28** | 371 | PQQQFIQPQQPFPQQPQQTY | 22(10) | **64** | 387 | QQTFPQQPQLPFPQQPQQPF | 13(4) |
| **29** | 642 | PQQPQQPFPLQPQQPFPQQP | 20(8) | **65** | 628 | PFPWQPQQPFPQTQQSFPLQ | 12(4) |
| **30** | 635 | PLQPQQPFPQQPQQPFPQPQ | 19(5) | ***66** | 88 | PQPFPPQLPYSQPQPFRPQQ | 12(3) |
| ***31** | 93 | PQPFPPQLPYPQPQPFRPQQ | 19(5) | **67** | 408 | QPQQPFPQSKQPQQPFPQPQ | 12(5) |
| **32** | 377 | PQQQFPQPQQPQQPFPQQPQ | 19(9) | ***68** | 77 | LQLQPFPQPQPFPPQLPYPQ | 11(4) |
| **33** | 411 | LQQPQQPFPQPQQQLPQPQQ | 19(4) | **69** | 370 | PQQQFLQPQQPFPQQPQQPY | 11(5) |
| **34** | 415 | SQQPQQPFPQPQQPQQSFPQ | 18(5) | ***70** | 79 | LQLQPFPQPQPFLPQLPYPQ | 11(5) |
| ***35** | 94 | PQPFPPQLPYPQPPPFSPQQ | 18(3) | **71** | 379 | QQPQQQFPQPQQPQQPFPQP | 11(5) |
| **36** | 329 | PSGQVQWPQQQPFPQPQQPF | 18(4) | **72** | 397 | PQQPQQPFPQTQQPQQPFPQ | 11(3) |
| * Indicates homologue of A-gliadin 57-73 with the core sequence PQLP(Y/F) | | | | | | | |

**Table 20. Peptides >10% as bioactive as P04722 QE65 grouped by structure.**

| Rank | Peptide no. (Pool) Gliadin-ubtype | Sequence | IFNg ELISpot response compared to P04722 77-93 QE85: mean (SEM) |
|---|---|---|---|
| **Group 1: Homologues of A-gliadin 57-73** | | | |
| | P04722 77-93 | **QLQPFPQPQLPYPQPQP** | |
| 1 | 89 (12) α | PQL...Y.................................LPYP | 94 (18) |
| 2 | 91 (12) α | PQPFPPQL...Y........................... | 89 (12) |
| 3 | 74 (10) α | M................................................LPY | 88 (14) |
| 4 | 90 (12) α | PQL...Y.................................PFRP | 87 (16) |
| 5 | 76 (10) α | L................................................PFR | 85 (15) |
| 8 | 631 (81) ω | FPQQPQ...........................F......QS | 61 (12) |
| 10 | 73 (10) α | L................................................LPY | 49 (11) |
| 13 | 68 (9) α | L....................................L.........PFR | 33 (10) |
| 14 | 66 (9) α | L....................................S.........PFR | 32 (7) |
| 18 | 67 (9) α | L...................................S.........QFR | 26 (6) |
| 20 | 95 (13) α | PQPFL..............................FPPQQ | 24 (6) |
| 31 | 93 (12) α | PQPFP ........................... PFRPQQ | 19 (5) |
| 35 | 94 (12) α | PQPFP........................PPFSPQQ | 18 (3) |
| 40 | 78 (10) α | L..................R...........................PFR | 17 (8) |
| 52 | 81 (11) α | PQPQPFP.....................T...PFPP | 15 (5) |
| 53 | 75 (10) α | MQLQPFPQPQPF........................ | 14 (5) |
| 55 | 82(11) α | PQPQPFPQPQPF........................ | 14 (3) |
| 56 | 80 (10) α | LQLQPFPQPQPF........................ | 14 (4) |
| 66 | 88 (11) α | PQPFP..................S.........PFRPQQ | 12 (3) |
| 68 | 77 (10) α | LQLQPFPQPQPFP..................... | 11 (4) |
| 70 | 79 (10) α | LQLQPFPQPQPFL..................... | 11 (5) |

| **Group 2: Homologues of peptide 626** | | | |
|---|---|---|---|
| | | **QQPFPQPQQPFP** | |
| 6 | 626(80) ω | PQQPQQP....................................W | 72 (23) |
| 7 | 627(80) ω | .................................WQPQQPFPQ | 66 (30) |
| 9 | 636(81) ω | PQQP ..............................I...VQPQ | 51 (10) |
| 11 | 412(53) γ | SQQP...........................Q.........PQQ | 34 (19) |
| 33 | 411(53) γ | LQQP...........................Q.........PQQ | 19 (4) |
| 36 | 329(42) γ | PSGQVQWPQ................................. | 18 (4) |
| 41 | 390(50) γ | QQTYPQRP..................T.........QQ | 17 (9) |
| 59 | 337(43) γ | Q.................................CQQPQRTI | 13 (4) |
| 61 | 388(50) γ | QQPYPQQP..................T.........QQ | 13 (3) |

| **Group 3: Homologues of peptide 355** | | | |
|---|---|---|---|
| | | **FPQPQQTFPHQPQQQFP** | |
| 17 | 355(46) γ | QA...................................................Q | 27 (15) |
| 42 | 348(45) γ | QQT....._{.......}....................................... | 16 (10) |
| 48 | 350(45) γ | QQL.........................................A...... | 15 (8) |
| 50 | 349(45) γ | QQI................................................... | 15 (9) |

| **Group 4: Homologues of Peptide 396** | | | |
|---|---|---|---|
| | | **QQPFPQQPQQPFP** | |
| 21 | 396(51) γ | TQQP.......................................QTQ | 23 (9) |
| 27 | 378(49) γ | QQP.......................................QPQQ | 22 (10) |
| 28 | 371(48) γ | PQQQFIQP.................................TY | 22 (10) |
| 29 | 642(82) ω | PQQP...............L.....................QQP | 20 (8) |
| 30 | 635(81) ω | PLQP.......................................QPQ | 19 (5) |
| 44 | 382(49) γ | .......................................QTQQPQQ | 16 (6) |
| 45 | 629(81) ω | PFPQT......S......L.....................QQ | 16 (5) |
| 46 | 643(82) ω | PLQP.......................................QQP | 16 (6) |
| 60 | 634(81) ω | PQQL...............L.....................QQP | 13 (3) |
| 64 | 387(50) γ | ......T..................L.........QQPQQPF | 13 (4) |
| 62 | 641(82) ω | FPEL.........I...........................LQP | 13 (7) |
| | | | |

| **Group 5: Homologues of Peptide 343 (overlap Groups 2 and 4)** | | | |
|---|---|---|---|
| | | **QQPFPQPQQPQLPFPQ** | |
| 12 | 343(44) γ | QQP................................................Q | 34 (11) |
| 16 | 393(51) γ | QLPFPQQP.................................. | 27 (8) |
| 19 | 335(43) γ | QQ.................................Q............PQ | 25 (11) |
| 23 | 385(50) γ | ...............................................QPQQ | 23 (7) |
| 24 | 375(48) γ | P................................Q............PQQ | 23 (10) |
| 25 | 406(52) γ | QP..................L............Q............PQ | 22 (8) |
| 32 | 377(49) γ | P......Q........................Q............QPQ | 19 (9) |
| 34 | 415(53) γ | SQQP.................................QS......... | 18 (5) |
| 37 | 413(53) γ | SKQP.................................QS......... | 18 (4) |
| 38 | 380(49) γ | QPQQP............................................. | 18 (6) |
| 43 | 409(53) γ | QP..................L............Q...L......PQ | 16 (2) |
| 47 | 389(50) γ | ..................T............Q............QPQQ | 16 (6) |
| 58 | 407(52) γ | QP..................S............Q............PQ | 14 (5) |
| 63 | 399(51) γ | ..................T............Q............LQQP | 13 (5) |
| 67 | 408(52) γ | QP..................SK.........Q............PQ | 12 (5) |
| 71 | 379(49) γ | QQP......Q........................Q............P | 11 (5) |
| 72 | 397(51) γ | PQQP..................T............Q............ | 11 (3) |

| **Group 6: Peptide 625** | | | |
|---|---|---|---|
| | | **PIQPQQPFPQQP** | |
| 26 | 625(80) ω | **....................................QQPQQPFP** | 22 (9) |
| 57 | 624(80) ω | FTQPQQPT.................................... | 14 (6) |
| 65 | 628(80) ω | PF...W........................TQQSFPLQ | 12(4) |
| **Group 7: Peptide 618** | | | |
| 39 | 618(79) ω | **PQQSFSYQQQPFPQQPYPQQ** | 18 (7) |

| **Table 21. Bioactivity of individual tTG-deamidated Pools 1-3 peptides in Subject C12:** | | | | | |
|---|---|---|---|---|---|
| No. | Sequence | % | No. | Sequence | % |
| 8 | AVRWPVPQLQPQNPSQQQPQ | 100 | 23 | LQPQNPSQQQPQEQVPLMQQ | 26 |
| 5 | **M**VRV**T**VPQ.................................... | 85 | 14 | ....................................EQVPLVQQ | 18 |
| 6 | AVRV**S**VPQ.................................... | 82 | 15 | ...........................H......EQVPLVQQ | 18 |
| 3 | **M**VRVPVPQ...........................H...... | 77 | 17 | ....................................**K**QVPLVQQ | 18 |
| 1 | AVR**F**PVPQ...........................L...... | 67 | 16 | ............D.....................EQVPLVQQ | 13 |
| 2 | **M**VRVPVPQ.................................... | 59 | 13 | ....................................EQVPLVQQ | 8 |
| 9 | AVRVPVPQ......L........................... | 49 | 22 | .........K........................EQVPLVQQ | 5 |
| 7 | AVRVPVPQ.................................... | 49 | 18 | ......L...........................EQVPLVQE | 3 |
| 10 | **M**VRVPVPQ......L........................... | 33 | 19 | ......L...........................EQVPLVQE | 3 |
| 4 | **M**VRVP**M**PQ............D..................... | 15 | 20 | P.....................P.........**G**QVPLVQQ | 0 |
| 12 | AVRVPVPQ.........K........................ | 8 | 21 | P.....................P.........**R**QVPLVQQ | 0 |
| 11 | AVRVPVPQP.....................P......... | 0 | | | |
| Core sequence of epitope is underlined. Predicted deamidated sequence is: LQP**E**NPSQ**E**QP**E** | | | | | |

**Table 22: Phylogenetic groupings of wheat (Triticum aestivum) gliadins**

| | **Alpha/beta-gliadins (n=61)** | | |
|---|---|---|---|
| A1a1 | AAA96525, EEWTA, P02863 | A1b13 | B22364, P04271 |
| A1a2 | CAB76963 | A2a1 | AAB23109, CAA35238, P18573, S10015 |
| A1a3 | AAA96276 | A2a2 | CAB76964 |
| A1a4 | CAA26384, S07923 | A2b1 | P04724, T06500, AAA348282 |
| Ala5 | AAA34280 | A2b2 | D22364 |
| Ala6 | P04728 | A2b3 | P04722, T06498, AAA34276 |
| A1b1 | CAB76962 | A2b4 | C22364 |
| A1b2 | CAB76961 | A2b5 | CAB76956 |
| Alb3 | BAA12318 | A3a1 | AAA34277, CAA26383, P04726, S07361 |
| A1b4 | CAB76960 | A3a2 | 1307187B,A27319, |
| Alb5 | CAB76958 | A3b1 | AAA96522 |
| Alb6 | CAB76959 | A3b2i | AAA34279, P04727, |
| Alb7 | CAB76955 | A3b2ii | CAA26385, S07924 |
| A1b8 | AAA96524 | A3b3 | A22364, AAA34278, AAB23108, C61218, P04725 |
| A1b9 | CAA10257 | A4a | P04723, AAA34283, T06504 |
| A1b10 | AAA96523, T06282 | A4b | E22364 |
| A1b11 | AAA17741, S52124 | A4c | CAB76957 |
| A1b12 | AAA34281 | A4d | CAB76954 |

| | **Gamma-gliadins (n=47)** | | Gamma-gliadins |
|---|---|---|---|
| GI1a | P08079, AAA34288, PS0094, CAC11079, AAD30556, CAC11057, CAC11065, CAC11056 | GI5a | AAK84774, AAK84772 |
| GI1b | CAC11089, CAC11064, CAC11080, CAC11078, AAD30440 | GI5b | AAK84773 |
| GIlc | CAC11087 | GI5c | AAK84776 |
| GI1d | CAC11088 | GI6a | JA0153, P21292, AAA34272, 1507333A |
| GI1e | CAC11055 | GI6b | AAK84777 |
| GI2a | JS0402, P08453, AAA34289 | GI6c | 1802407A, AAK84775, AAK84780 |
| GI2b | AAF42989, AAK84779, AAK84779 | GI7 | AAB31090 |
| GI3a | AAK84778 | GIIa | AAA34287, P04730, S07398 |
| GI3b | CAB75404 | GIIb | 1209306A |
| GI3c | BAA11251 | GIII1a | P04729 |
| GI4 | EEWTG, P06659, AAA34274 | GIII1b | AAA34286 |

| | **Omega-gliadins (n=3)** | | |
|---|---|---|---|
| Ola | AAG17702 | | |
| Olb | P02865 | | |
| O1c | A59156 | | |

**Table 23. Synthetic peptides spanning all known wheat gliadin 12mers**

| Protein Position* Sequence | No. | Protein Position* Sequence | No. |
|---|---|---|---|
| **POOL 1** | | **POOL 43** | . |
| A1A1 20 AVRF PVPQ LQPQ NPSQ QLPQ | 1 | GI2A 33 QQQ**L** VPQ**L** QQP**L** SQQP QQTF | 331 |
| A1A2 20 **M**VRV PVPQ LQPQ NPSQ Q**Q**PQ | 2 | GI3A 33 QQQ**P F**PQ**P H**QP**F** SQQP QQTF | 332 |
| A1B1 20 MVRV PVPQ LQPQ NPSQ Q**H**PQ | 3 | GI4 33 QQQ**P** F**L**Q**P H**QP**F** SQQP QQIF | 333 |
| A1B2 20 MVRV P**M**PQ LQPQ **D**PSQ Q**Q**PQ | 4 | GI5A 33 QQQQ PFPQ PQQP FSQQ PQQI | 334 |
| A1B7 20 MVRV **T**VPQ LQPQ NPSQ QQPQ | 5 | GI5B 33 QQQQ PFPQ PQQP QQPF PQPQ | 335 |
| A1B8 20 AVRV **S**VPQ LQPQ NPSQ QQPQ | 6 | GI5C 33 QQQP FRQP QQPF YQQP QHTF | 336 |
| A1B8 20 AVRV PVPQ LQPQ NPSQ QQPQ | 7 | GI6A 33 QQQP FPQP QQPF CQQP QRTI | 337 |
| A1B10 20 AVR**W** PVPQ LQPQ NPSQ QQPQ | 8 | GI6C 42 QQQP FPQP QQPF CEQP QRTI | 338 |
| **POOL 2** | . | **POOL 44** | . |
| A2B3 20 AVRV PVPQ LQ**L**Q NPSQ QQPQ | 9 | GI1A 42 **H**QPF SQQP QQTF PQPQ QTFP | 339 |
| A2B5 20 **M**VRV PVPQ LQ**L**Q NPSQ QQPQ | 10 | GI2A 42 **Q**QPL SQQP QQTF PQPQ QTFP | 340 |
| A3A1 20 AVRV PVPQ **P**QPQ NPSQ **P**QPQ | 11 | GI4 42 **H**QP**F** SQQP QQIF PQPQ QTFP | 341 |
| A3B1 20 AVRV PVPQ LQ**PK** NPSQ QQPQ | 12 | GI5A 42 QQPF SQQP QQIF PQPQ QTFP | 342 |
| A1A1 28 LQPQ NPSQ Q**L**PQ EQVP LVQQ | 13 | GI5B 42 QQPQ QPFP QPQQ PQLP FPQQ | 343 |
| A1A2 28 LQPQ NPSQ Q**Q**PQ EQVP LVQQ | 14 | GI5C 42 QQPF YQQP QHTF PQPQ QTCP | 344 |
| A1B1 28 LQPQ NPSQ Q**H**PQ EQVP LVQQ | 15 | GI6A 42 QQPF CQQP QRTI PQPH QTFH | 345 |
| A1B2 28 LQPQ **D**PSQ Q**Q**PQ EQVP LVQQ | 16 | GI6B 42 QQPF CQQP QQTI PQPH QTFH | 346 |
| **POOL 3** | . | **POOL 45** | . |
| A2B1 28 LQPQ NPSQ QQPQ **K**QVP LVQQ | 17 | GI6C 42 QQPF CEQP QRTI PQPH QTFH | 347 |
| A2B3 28 LQ**L**Q NPSQ QQPQ EQVP LVQ**E** | 18 | GI1A 50 QQTF PQPQ QTFP HQPQ QQFP | 348 |
| A2B5 28 LQ**L**Q NPSQ QQPQ EQVP LVQE | 19 | GI4 50 QQ**I**F PQPQ QTFP HQPQ QQFP | 349 |
| A3A1 28 **P**Q**P**Q NPSQ **P**QPQ **G**QVP LVQQ | 20 | GI5A 50 QQIF PQPQ QTFP HQPQ QAFP | 350 |
| A3A2 28 PQPQ NPSQ PQPQ **R**QVP LVQQ | 21 | GI6A 50 QRTI PQPH QTFH HQPQ QTFP | 351 |
| A3B1 28 LQP**K** NPSQ QQPQ EQVP LVQQ | 22 | GI5A 58 QTFP HQPQ QAFP QPQQ TFPH | 352 |
| A4A 28 LQPQ NPSQ QQPQ EQVP L**M**QQ | 23 | GI6A 58 QTFH HQPQ QTFP QPQQ TYPH | 353 |
| A1A1 36 QLPQ EQVP LVQQ QQFL GQQQ | 24 | GI6C 58 QTFH HQPQ QTFP QPEQ TYPH | 354 |
| **POOL 4** | . | **POOL 46** | . |
| A1B1 36 Q**H**PQ EQVP LVQQ QQFL GQQQ | 25 | GI5A 66 QAFP QPQQ TFPH QPQQ QFPQ | 355 |
| A1B2 36 Q**Q**PQ EQVP LVQQ QQFL GQQQ | 26 | GI5C 66 QHTF PQPQ QTCP HQPQ QQFP | 356 |
| A1B12 36 QQPQ EQVP LVQQ QQF**L** GQQQ | 27 | GI6A 66 QTFP QPQQ TYPH QPQQ QFPQ | 357 |
| A2A1 36 QQPQ EQVP LVQQ QQF**P** GQQQ | 28 | GI6C 66 QTFP QP**E**Q TYPH QPQQ QFPQ | 358 |
| A2B1 36 QQPQ **K**QVP LVQQ QQFP GQQQ | 29 | GI1A 73 QTFP HQPQ QQ**F**P QPQQ PQQQ | 359 |
| A2B3 36 QQPQ EQVP LVQ**E** QQF**Q** GQQQ | 30 | GI2A 73 QTFP HQPQ QQ**V**P QPQQ PQQP | 360 |
| A3A1 36 **P**QPQ **G**QVP LVQQ QQFP GQQQ | 31 | GI3A 73 QTFP HQPQ QQ**FS** QPQQ PQQQ | 361 |
| A3A2 36 PQPQ **R**QVP LVQQ QQFP GQQQ | 32 | GI5C 73 QTCP HQPQ QQFP QPQQ PQQP | 362 |
| **POOL 5** | . | **POOL 47** | . |
| A4A 36 QQPQ EQVP L**M**QQ QQ**Q**F PGQQ | 33 | GI6A 73 QTYP HQPQ QQFP QTQQ PQQP | 363 |
| A1A1 44 LVQQ QQFL GQQQ PFPP QQPY | 34 | GI1A 81 QQ**FP** QPQQ PQQQ F**L**QP QQPF | 364 |
| A1B1 44 LVQQ QQFL GQQQ **S**FPP QQPY | 35 | GI2A 81 QQ**VP** QPQQ PQQP F**L**QP QQPF | 365 |
| A1B12 44 LVQQ QQF**L** GQQQ PFPP QQPY | 36 | GI3A 81 QQ**FS** QPQQ PQQQ F**I**QP QQPF | 366 |
| A2A1 44 LVQQ QQF**P** GQQQ PFPP QQPY | 37 | GI4 81 QQ**FP** QPQQ PQQQ F**L**QP **R**QPF | 367 |
| A2B3 44 LVQE QQF**Q** GQQQ PFPP QQPY | 38 | GI5A 81 QQFP QPQQ PQQP FPQQ PQQQ | 368 |
| A3A1 44 LVQQ QQFP GQQQ **Q**FPP QQPY | 39 | GI6A 81 QQFP QTQQ PQQP FPQP QQTF | 369 |
| A4A 44 L**M**QQ QQ**Q**F PGQQ **EQ**FP PQQP | 40 | GI1A 89 PQQQ F**L**QP QQPF PQQP QQP**Y** | 370 |
| **POOL 6** | . | **POOL 48** | . |
| A4D 44 LMQQ QQQF PGQQ E**R**FP PQQP | 41 | GI3A 89 PQQQ F**I**QP QQPF PQQP QQ**TY** | 371 |
| A1A1 53 GQQQ PFPP QQPY PQPQ PFPS | 42 | GI3B 89 PQQQ F**I**QP QQP**Q** QTYP Q**RPQ** | 372 |
| A1A3 53 GQQQ PFPP QQPY PQPQ FPSQ | 43 | GI4 89 PQQQ F**L**QP **R**QPF **PQ**QP QQPY | 373 |
| A1B1 53 GQQQ **S**FPP QQPY PQPQ PFPS | 44 | GI5A 89 PQQP FPQQ PQQQ FPQP QQPQ | 374 |
| A2B1 53 GQQQ PFPP QQPY PQ**Q**Q PFPS | 45 | GI5C 89 PQQP FPQP QQPQ QPFP QPQQ | 375 |
| A3A1 53 GQQQ **Q**FPP QQPY PQPQ PFPS | 46 | GI6A 89 PQQP FPQP QQTF PQQP QLPF | 376 |
| A4A 53 GQQ**E Q**FPP QQPY P**HQ**Q PFPS | 47 | **POOL 49** | . |
| A4D 53 GQQE **R**FPP QQPY PHQQ PFPS | 48 | GI5A 97 PQQQ FPQP QQPQ QPFP Q**QP**Q | 377 |
| **POOL 7** | . | GI5A 105 QQPQ QPFP QQPQ QQFP QPQQ | 378 |
| A1A1 61 QQPY PQPQ PFPS Q**L**PY LQLQ | 49 | GI5A 113 QQPQ QQFP QPQQ PQQP FPQP | 379 |
| A1A3 61 QQPY PQPQ FPSQ LPYL QLQP | 50 | GI5A 121 QPQQ PQQP FPQP QQPQ LPFP | 380 |
| A1B1 61 QQPY PQPQ PFPS Q**Q**PY LQLQ | 51 | GI1A 126 QQPF PQQP QQP**Y** PQ**Q**P Q**QPF** | 381 |
| A2B1 61 QQPY PQ**Q**Q PFPS QQPY **M**QLQ | 52 | GI2A 126 QQPF PQQP QQP**F** PQ**T**Q Q**PQQ** | 382 |
| A4A 61 QQPY P**HQ**Q PFPS QQ**PY PQP**Q | 53 | GI3A 126 QQPF PQQP QQ**TY** PQ**R**P QQPF | 383 |
| A1A1 69 PFPS Q**L**PY LQLQ PFPQ PQLP | 54 | GI4 126 **R**QPF **PQ**QP QQPY PQQP QQPF | 384 |
| A1B1 69 PFPS Q**Q**PY LQLQ PFPQ PQLP | 55 | **POOL 50** | . |
| A1B10 69 PFPS QQPY LQLQ PF**S**Q PQLP | 56 | GI5A 126 QQPF PQPQ QPQL PFPQ QPQQ | 385 |
| **POOL 8** | . | GI5C 126 QQPF PQPQ QAQL PFPQ QPQQ | 386 |
| A1B11 69 PFPS QQPY LQLQ PF**L**Q PQLP | 57 | QQTF PQQP QQPF GI6A 126 QLPF | |
| A1B12 69 PFPS QQPY LQLQ PFLQ PQ**PF** | 58 | GI1A 134 QQP**Y** PQQP QQPF P**QT**Q QPQQ | 388 |
| A2A1 69 PFPS QQPY LQLQ PFPQ PQLP | 59 | GI2A 134 QQP**F** PQ**TQ QPQQ** P**FP**Q QPQQ | 389 |
| A2B1 69 PFPS QQPY **M**QLQ PFPQ PQ**LP** | 60 | GI3A 134 QQ**TY** PQ**R**P QQPF P**Q**TQ QPQQ | 390 |
| A2B2 69 PFPS QQPY **M**QLQ PFPQ PQ**PF** | 61 | G15A 134 QPQL PFPQ QPQQ QPQQ PFPQ | 391 |
| A2B4 69 PFPS QQPY LQLQ PFPQ PQPF | 62 | GI5C 134 QAQL PFPQ QPQQ PLPQ PQQP | 392 |
| A2B5 69 PFPS QQPY LQLQ PFP**R** PQLP | 63 | **POOL 51** | . |
| A4A 69 PFPS QQ**PY PQP**Q PFPP QLPY | 64 | GI6A 134 QLPF PQQP QQPF PQPQ QPQQ | 393 |
| **POOL 9** | . | GI2A 142 QPQQ PFPQ QPQQ PFPQ TQQP | 394 |
| A4B 69 PFPS QQPY PQPQ PFPQ PQPF | 65 | GI2A 150 QPQQ PFPQ TQQP QQPF PQQP | 395 |
| A1A1 77 LQLQ PFPQ PQLP YSQP QPFR | 66 | GI2A 158 TQQP QQPF PQQP QQPF PQTQ | 396 |
| A1A4 77 LQLQ PFPQ PQLP YSQP Q**Q**FR | 67 | GI2A 166 PQQP QQPF PQTQ QPQQ PFPQ | 397 |
| A1B1 77 LQLQ PFPQ PQLP Y**L**QP QPFR | 68 | GI1A 170 QQPF PQTQ QPQQ **L**FPQ **S**QQP | 398 |
| A1B4 77 LQLQ PFPQ PQL**S** YSQP QPFR | 69 | GI2A 170 QQPF PQTQ QPQQ **P**FPQ **L**QQP | 399 |
| A1B10 77 LQLQ PF**S**Q PQLP YSQP QPFR | 70 | GI3A 170 QQPF PQTQ QPQQ **P**FPQ **S**QQP | 400 |
| A1B11 77 LQLQ PF**L**Q PQLP YSQP QPFR | 71 | **POOL 52** | . |
| A1B12 77 LQLQ PFLQ PQ**PF PPQL** PYSQ | 72 | GI4 170 QQPF PQTQ QPQQ PFPQ **SK**QP | 401 |
| **POOL 10** | . | GI5A 170 QQPF PQPQ QPQQ PFPQ LQQP | 402 |
| A2A1 77 LQLQ PFPQ PQLP YPQP QLPY | 73 | GI5C 170 QQPL PQPQ QPQQ PFPQ SQQP | 403 |
| A2B1 77 **M**QLQ PFPQ PQ**LP YPQ**P QLPY | 74 | GI6A 170 QQPF PQPQ QPQQ PFPQ SQQP | 404 |
| A2B2 77 **M**QLQ PFPQ PQ**PF PP**QL PYPQ | 75 | GI1A 178 QPQQ **L**FPQ **S**QQP QQ**Q**F **S**QPQ | 405 |
| A2B3 77 LQLQ PFPQ PQLP Y**P**QP QPFR | 76 | GI2A 178 QPQQ **P**FPQ **L**QQP QQPF **P**QPQ | 406 |
| A2B4 77 LQLQ PFPQ PQPF PPQL PYPQ | 77 | GI3A 178 QPQQ **P**FPQ **S**QQP QQPF PQPQ | 407 |
| A2B5 77 LQLQ PFP**R** PQLP YPQP QPFR | 78 | GI4 178 QPQQ PFPQ **SK**QP QQPF PQPQ | 408 |
| A3B1 77 LQLQ PFPQ PQPF **L**PQL PYPQ | 79 | **POOL 53** | . |
| A3B3 77 LQLQ PFPQ PQPF **P**PQL PYPQ | 80 | GI5A 178 QPQQ PFPQ LQQP QQPL PQPQ | 409 |
| **POOL 11** | . | GI1A 186 **S**QQP QQQF **S**QPQ QQ**FP** QPQQ | 410 |
| A4A 77 **PQP**Q PFPP QLPY PQ**T**Q PF**P**P | 81 | GI2A 186 **L**QQP QQPF **P**QPQ QQ**L**P QPQQ | 411 |
| A4B 77 PQPQ PFPQ PQPF PPQL PYPQ | 82 | GI3A 186 **S**QQP QQPF **P**QPQ QQ**F**P QPQQ | 412 |
| A1A1 85 PQLP YSQP QPFR PQQP YPQP | 83 | GI4 186 **SK**QP QQPF PQPQ QP**Q**Q **SFP**Q | 413 |
| A1A6 85 PQLP YSQP Q**Q**FR PQQP YPQP | 84 | GI5A 186 LQQP QQP**L** PQPQ QPQQ PFPQ | 414 |
| A1B1 85 PQLP Y**L**QP QPFR PQQP YPQP | 85 | GI5C 186 SQQP QQPF PQPQ QPQQ SFPQ | 415 |
| A1B4 85 PQL**S** YSQP QPFR PQQP YPQP | 86 | GI1A 194 **S**QPQ QQ**F**P QPQQ PQQS FPQQ | 416 |
| A1B6 85 PQLS YSQP QPFR PQQ**L** YPQP | 87 | **POOL 54** | . |
| A1B12 85 PQ**PF PP**QL **P**YSQ PQPF RPQQ | 88 | GI2A 194 **P**QPQ QQ**L**P QPQQ PQQS FPQQ | 417 |
| **POOL 12** | . | GI3A 194 **P**QPQ QQFP QPQQ PQQS FPQQ | 418 |
| A2A1 85 PQLP YPQP QLPY PQPQ LPYP | 89 | GI4 194 PQPQ QP**Q**Q **SFP**Q QQPS LIQQ | 419 |
| A2B1 85 PQ**LP YPQ**P QLPY PQPQ PFRP | 90 | GI5A 194 PQPQ QPQQ PFPQ QQQP LIQP | 420 |
| A2B2 85 PQ**PF PP**QL PYPQ PQLP YPQP | 91 | GI5C 194 PQPQ QPQQ SFPQ QQQP LIQP | 421 |
| A2B3 85 PQLP Y**P**QP QPFR PQQP YPQP | 92 | GI1A 202 QPQQ PQQS FPQQ Q**P**PF IQ**P**S | 422 |
| A2B4 85 PQPF PPQL PYPQ PQPF RPQQ | 93 | GI2A 202 QPQQ PQQS FPQQ Q**R**PF IQ**P**S | 423 |
| A3A1 85 PQPF PPQL PYPQ P**P**PF **S**PQQ | 94 | GI3A 202 QPQQ PQQS FPQQ QP**SL** IQ**Q**S | 424 |
| **POOL 13** | . | **POOL 55** | . |
| A3B1 85 PQPF **L**PQL PYPQ PQ**S**F **P**PQQ | 95 | GI1A 210 FPQQ Q**P**P**F** IQPS LQQQ **V**NPC | 425 |
| A3B3 85 PQPF **P**PQL PYPQ PQ**S**F **P**PQQ | 96 | GI2A 210 FPQQ Q**R**P**F** IQPS LQQQ **L**NPC | 426 |
| A4A 85 QLPY PQ**T**Q PF**P**P QQPY PQPQ | 97 | GI3A 210 FPQQ Q**PSL** IQ**Q**S LQQQ **L**NPC | 427 |
| A4B 85 PQPF PPQL PYPQ TQPF PPQQ | 98 | GI5A 210 FPQQ QQPL IQPY LQQQ MNPC | 428 |
| A2A1 106 LPYP QPQP FRPQ QPYP Q**S**QP | 99 | GI6A 210 FPQQ QQPA IQSF LQQQ MNPC | 429 |
| A2B1 106 LPYP QPQP FRPQ Q**S**YP QPQP | 100 | GI1A 218 IQ**P**S LQQQ **V**NPC KNFL LQQ**C** | 430 |
| A3A1 106 LPYP QP**P**P F**S**PQ QPYP QPQP | 101 | GI2A 218 IQ**P**S LQQQ **L**NPC KNIL LQQ**S** | 431 |
| A3B1 106 **L**PQL PYPQ PQ**S**F **P**PQQ PYPQ | 102 | GI3A 218 IQ**Q**S LQQQ **L**NPC KNFL LQQ**C** | 432 |
| **POOL 14** | . | **POOL 56** | . |
| A4A 106 PPQL PYPQ **T**QPF **P**PQQ PYPQ | 103 | GI5A 218 IQPY LQQQ MNPC KNYL LQQC | 433 |
| A1A1 112 QPFR PQQP YPQP QPQY SQPQ | 104 | GI6A 218 IQSF LQQQ MNPC KNFL LQQC | 434 |
| A1B6 112 QPFR PQQ**L** YPQP QPQY SQPQ | 105 | GI1A 226 **V**NPC KNFL LQQ**C** KP**V**S LVSS | 435 |
| A2A1 112 QPFR PQQP YPQ**S** QPQY SQPQ | 106 | GI2A 226 **L**NPC KNIL LQQ**S** KP**A**S LVSS | 436 |
| A2B1 112 QPFR PQQ**S** YPQP QPQY SQPQ | 107 | GI3A 226 **L**NPC KNFL LQQ**C** KPVS LVSS | 437 |
| A3A1 112 **P**PF**S** PQQP YPQP QPQY **P**QPQ | 108 | GI5A 226 MNPC KNYL LQQC NPVS LVSS | 438 |
| A3B1 112 Q**S**F**P** PQQP YPQ**Q R**P**K**Y **L**QPQ | 109 | GI6A 226 MNPC KNFL LQQC NHVS LVSS | 439 |
| A3B2 112 Q**S**F**P** PQQP YPQ**Q R**P**M**Y **L**QPQ | 110 | GI1A 234 LQQ**C** KP**V**S LVSS LWS**M** IWPQ | 440 |
| **POOL 15** | . | **POOL 57** | . |
| A3B3 112 Q**S**F**P** PQQ**P** YPQ**Q** QP**Q**Y LQPQ | 111 | GI2A 234 LQQ**S** KP**A**S LVSS LWS**I** IWPQ | 441 |
| A4A 112 QPF**P** PQQP YPQP QPQY **P**QPQ | 112 | GI3A 234 LQQ**C** KP**V**S LVSS LWS**M** ILP**R** | 442 |
| A1A1 120 YPQP QPQY SQPQ QPIS QQQQ | 113 | GI5A 234 LQQC NPVS LVSS LVSM ILPR | 443 |
| A1B3 120 YPQP QPQY SQPQ **E**PIS QQQQ | 114 | GI6A 234 LQQC NHVS LVSS LVSI ILPR | 444 |
| A2A1 120 YPQ**S** QPQY SQPQ QPIS QQQQ | 115 | GI1A 242 LVSS LWS**M** I**W**P**Q** SDCQ VMRQ | 445 |
| A3A1 120 YPQP QPQY **P**QPQ QPIS QQQ**A** | 116 | GI2A 242 LVSS L**W**SI I**W**P**Q** SDCQ VMRQ | 446 |
| A3B1 120 YPQ**Q R**P**K**Y **L**QPQ QPIS QQQ**A** | 117 | GI3A 242 LVSS LWS**M** I**L**P**R** SDCQ VMRQ | 447 |
| A3B2 120 YPQ**Q R**PMY **L**QPQ QPIS QQQ**A** | 118 | GI4 242 LVSS LWS**I** I**L**PP SDCQ VMRQ | 448 |
| **POOL 16** | . | **POOL 58** | . |
| A3B3 120 YPQ**Q** QP**Q**Y LQPQ QPIS QQQA | 119 | GI5A242 LVSS LVSM ILPR SDCK VMRQ | 449 |
| A1A1 128 SQPQ QPIS QQQQ QQQQ QQQQ | 120 | GI5C 242 LVSS LVSM ILPR SDCQ VMQQ | 450 |
| A1B3 128 SQPQ **E**PIS QQQQ QQQQ QQQ**I** | 121 | GI6A 242 LVSS LVSI ILPR SDCQ VMQQ | 451 |
| A3A1 128 **P**QPQ QPIS QQQ**A** QQQQ QQQQ | 122 | GI1A 250 I**W**P**Q** SDCQ VMRQ QCCQ QLAQ | 452 |
| A1A1 138 QQQQ QQQQ QQQQ Q**Q**QQ ILQQ | 123 | GI3A 250 I**L**P**R** SDCQ VMRQ QCCQ QLAQ | 453 |
| A1A6 138 QQQQ QQQQ QQQQ Q**E**QQ ILQQ | 124 | GI4 250 I**L**P**P** SDCQ VMRQ QCCQ QLAQ | 454 |
| A1B11 138 QQQQ QQQQ QQQQ QQQQ IIQQ | 125 | GI5A 250 ILPR SDCK VMRQ QCCQ QLAR | 455 |
| A2A1 138 QQQQ QQQQ QQ**K**Q QQQQ QQQI | 126 | GI5C 250 ILPR SDCQ VMQQ QCCQ QLAQ | 456 |
| **POOL 17** | . | **POOL 59** | . |
| A4B 139 AQQQ QQQQ QQQQ QQQQ TLQQ | 127 | GI1A 258 VMRQ QCCQ QLAQ IPQQ LQCA | 457 |
| A1A1 146 QQQQ Q**Q**QQ ILQQ ILQQ QLIP | 128 | GI5A 258 VMRQ QCCQ QLAR IPQQ LQCA | 458 |
| A1A6 146 QQQQ Q**E**QQ ILQQ ILQQ QLIP | 129 | GI5C 258 VMQQ QCCQ QLAQ IPRQ LQCA | 459 |
| A1B6 146 QQQQ Q**E**QQ ILQQ **M**LQQ QLIP | 130 | GI6A 258 VMQQ QCCQ QLAQ IPQQ LQCA | 460 |
| A1B10 146 QQQQ Q**E**QQ ILQQ ILQQ QL**T**P | 131 | GI1A 266 QLAQ IPQQ LQCA AIH**T II**HS | 461 |
| A1B11 146 QQQQ QQQQ I**I**QQ ILQQ QLIP | 132 | GI1B 266 QLAQ IPQQ LQCA AIH**T VI**HS | 462 |
| A2A1 146 QQ**K**Q QQQQ QQQI LQQI LQQQ | 133 | GI2A 266 QLAQ IPQQ LQCA AIH**S VV**HS | 463 |
| A3A2 146 QQQQ QQQQ IL**P**Q ILQQ QLIP | 134 | GI3A 266 QLAQ IPQQ LQCA AIH**S IV**HS | 464 |
| **POOL 18** | . | **POOL 60** | . |
| A4A 146 QQQQ QQQQ **T**LQQ ILQQ QLIP | 135 | GI5A 266 QLAR IPQQ LQCA AIHG IVHS | 465 |
| A1A1 163 ILQQ ILQQ QLIP CMDV VLQQ | 136 | GI5C 266 QLAQ IPRQ LQCA AIHS VVHS | 466 |
| A1B6 163 ILQQ **M**LQQ QLIP CMDV VLQQ | 137 | GI6A 266 QLAQ IPQQ LQCA AIHS VAHS | 467 |
| A1B10 163 ILQQ ILQQ QL**T**P CMDV VLQQ | 138 | GI1A 274 LQCA AIH**T II**HS IIMQ Q**E**QQ | 468 |
| A2B1 163 ILQQ ILQQ QLIP C**R**DV VLQQ | 139 | GI1B 274 LQCA AIH**T VI**HS IIMQ Q**E**QQ | 469 |
| A3A2 163 IL**P**Q ILQQ QLIP CRDV VLQQ | 140 | GI2A 274 LQCA AIH**S VV**HS IIMQ Q**Q**QQ | 470 |
| A4A 163 **T**LQQ ILQQ QLIP CRDV VLQQ | 141 | **POOL 61** | . |
| A1A1 171 QLIP CMDV VLQQ HN**I**A HGRS | 142 | GI3A 274 LQCA AIH**S IV**HS IIMQ Q**E**QQ | 471 |
| **POOL 19** | . | GI4 274 LQCA AIH**S VV**HS IIMQ Q**E**QQ | 472 |
| A1A3 171 QLIP CMDV VLQQ HN**K**A HGRS | 143 | GI5A 274 LQCA AIHG IVHS IIMQ QEQQ | 473 |
| A1B2 171 QLIP CMDV VLQQ HN**L**A HGRS | 144 | GI6A 274 LQCA AIHS VAHS IIMQ QEQQ | 474 |
| A1B7 171 QLIP CMDV VLQQ HNI**V** HGRS | 145 | GI1A 282 **II**HS IIMQ Q**E**QQ **E**QQQ GMHI | 475 |
| A1B10 171 QL**T**P CMDV VLQQ HNIA **R**GRS | 146 | GI1B 282 **VI**HS IIMQ QEQQ QGMH ILLP | 476 |
| A1B11 1 171 QLIP CMDV VLQQ HNI**V** HG**K**S | 147 | GI2A 282 **VV**HS IIMQ Q**Q**QQ **Q**QQQ QGID | 477 |
| A2A1 171 QLIP CRDV VLQQ HSIA YGSS | 148 | GI3A 282 **IV**HS IIMQ Q**E**QQ **E**Q**R**Q GVQI | 478 |
| A2B1 171 QLIP CRDV VLQQ H**S**IA HGSS | 149 | **POOL 62** | . |
| A2B3 171 QLIP CRDV VLQQ H**N**IA HG**S**S | 150 | GI4 282 **VV**HS IIMQ QEQQ EQ**L**Q GVQI | 479 |
| **POOL 20** | . | GI5A 282 IVHS IIMQ QEQQ QQQQ QQQQ | 480 |
| A3A1 171 QLIP CRDV VLQQ HNIA H**AR**S | 151 | GI5C 282 VVHS IVMQ QEQQ QGIQ ILRP | 481 |
| A3B1 171 QLIP CRDV VLQQ HNIA H**A**SS | 152 | GI6A 282 VAHS IIMQ QEQQ QGVP ILRP | 482 |
| A1A1 179 VLQQ HN**I**A HGRS QVLQ QSTY | 153 | GI1A 290 Q**E**QQ **E**QQQ G**MHI** LLPL YQQQ | 483 |
| A1A3 179 VLQQ HN**K**A HGRS QVLQ QSTY | 154 | GI2A 290 Q**Q**QQ QQQQ Q**GID** IFLP LSQH | 484 |
| A1B2 179 VLQQ HN**L**A HGRS QVLQ QSTY | 155 | GI2B 290 QQQQ QQQQ QG**M**H IFLP LSQ**Q** | 485 |
| A1B7 179 VLQQ HNI**V** HGRS QVLQ QSTY | 156 | GI3A 290 QEQQ EQ**R**Q GVQI L**V**PL **S**QQQ | 486 |
| A1B10 179 VLQQ HNIA **R**GRS QVLQ QSTY | 157 | **POOL 63** | . |
| A1B11 179 VLQQ HNI**V** HG**K**S QVLQ QSTY | 158 | GI4 290 Q**E**QQ EQLQ GVQI LVPL SQQQ | 487 |
| **POOL 21** | . | GI5A 290 QEQQ QQQQ QQQQ QQQG IQIM | 488 |
| A2A1 179 VLQQ H**S**IA YG**S**S QVLQ QSTY | 159 | GI5C 290 QEQQ QGIQ ILRP LFQL VQGQ | 489 |
| A2B1 179 VLQQ H**S**IA HGSS QVLQ QSTY | 160 | GI6A 290 QEQQ QGVP ILRP LFQL AQGL | 490 |
| A2B3 179 VLQQ H**N**IA HG**S**S QVLQ **E**STY | 161 | GI5A 298 QQQQ QQQG IQIM RPLF QLVQ | 491 |
| A3A1 179 VLQQ HNIA H**AR**S QVLQ QSTY | 162 | GI1A 305 G**MH**I **L**LPL **Y**Q**QQ** QVGQ G**T**LV | 492 |
| A3B1 179 VLQQ HNIA H**A**SS QVLQ QSTY | 163 | GI2A 305 G**ID**I **F**LPL **S**Q**HE** QVGQ G**S**LV | 493 |
| A4A 179 VLQQ HNIA HASS QVLQ QS**S**Y | 164 | GI2B 305 G**MH**I FLPL SQQQ QVGQ G**S**LV | 494 |
| A1A1 187 HGRS QVLQ QSTY QLL**Q** ELCC | 165 | **POOL 64** | . |
| A1A3 187 HGRS QVLQ QSTY QLL**R** ELCC | 166 | GI3A 305 G**VQ**I **LV**PL **S**QQQ QVGQ GTLV | 495 |
| **POOL 22** | . | GI4 305 GVQI LVPL SQQQ QVGQ G**I**LV | 496 |
| A1B8 187 HGRS QVLQ QSTY QLL**R E**LCC | 167 | GI5A 305 GIQI MRPL FQLV QGQG IIQP | 497 |
| A1B11 187 HG**K**S QVLQ QSTY QLLQ ELCC | 168 | GI5C 305 GIQI LRPL FQLV QGQG IIQP | 498 |
| A2A1 187 YGSS QVLQ QSTY QL**V**Q **Q**LCC | 169 | GI6A 305 GVPI LRPL FQLA QGLG IIQP | 499 |
| A2B1 187 HGSS QVLQ QSTY QL**V**Q Q**F**CC | 170 | GI1A 313 **Y**Q**QQ** QVGQ G**T**LV QGQG IIQP | 500 |
| A2B3 187 HG**S**S QVLQ ESTY QL**V**Q **Q**LCC | 171 | GI2A 313 **S**Q**HE** QVGQ G**S**LV QGQG IIQP | 501 |
| A3A1 187 H**AR**S QVLQ QSTY Q**P**LQ QLCC | 172 | GI2B 313 **S**Q**QQ** QVGQ G**S**LV QGQG IIQP | 502 |
| A3B1 187 H**A**SS QVLQ QSTY QLLQ QLCC | 173 | **POOL 65** | . |
| A4A 187 H**A**SS QVLQ QS**S**Y Q**Q**LQ QLCC | 174 | GI3A 313 SQQQ QVGQ G**T**LV QGQG IIQP | 503 |
| **POOL 23** | . | GI4 313 SQQQ QVGQ G**I**LV QGQG IIQP | 504 |
| A1A1 195 QSTY QLL**Q** ELCC QHLW QIPE | 175 | GI1A 321 G**T**LV QGQG IIQP QQPA QLEA | 505 |
| A1A3 195 QSTY QLL**R** ELCC QHLW QIPE | 176 | GI2A 321 G**S**LV QGQG IIQP QQPA QLEA | 506 |
| A1B8 195 QSTY QLL**R E**LCC QHLW QIPE | 177 | GI5A 321 FQLV QGQG IIQP QQPA QLEV | 507 |
| A2A1 195 QSTY QL**V**Q **Q**LCC QQLW QIPE | 178 | GI6A 321 FQLA QGLG IIQP QQPA QLEG | 508 |
| A2B1 195 QSTY QL**V**Q Q**F**CC QQLW QIPE | 179 | GI1A 329 IIQP QQPA QLE**A** IRS**L** VLQT | 509 |
| A3A1 195 QSTY Q**P**LQ QLCC QQLW QIPE | 180 | GI3A 329 IIQP QQPA QLE**V** IRS**L** VLQT | 510 |
| A3B1 195 QSTY QLLQ QLCC QQL**L** QIPE | 181 | **POOL 66** | . |
| A4A 195 QS**S**Y Q**Q**LQ QLCC QQL**F** QIPE | 182 | GI3C 329 IIQP QQPA QLE**V** IRS**S** VLQT | 511 |
| **POOL 24** | . | GI5C 329 IIQP QQPA QYEV IRSL VLRT | 512 |
| A1A1 203 ELCC QHLW QIPE QSQC QAIH | 183 | GI6A 329 IIQP QQPA QLEG IRSL VLKT | 513 |
| A1B6 203 ELCC QHLW QI**L**E QSQC QAIH | 184 | GI1A 337 QLE**A** IRSL VLQT LP**T**M CNVY | 514 |
| A1B10 203 ELCC QHLW QIPE **KL**QC QAIH | 185 | GI2A 337 QLE**A** IRSL VLQT LP**S**M CNVY | 515 |
| A2A1 203 **Q**LCC Q**Q**LW QIPE QS**R**C QAIH | 186 | GI3A 337 QLEV IRSL VLQT LA**T**M CNVY | 516 |
| A2B1 203 Q**F**CC QQLW QIPE QSRC QAIH | 187 | GI3C 337 QLEV IRS**S** VLQT LATM CNVY | 517 |
| A3B1 203 QLCC QQL**L** QIPE QSRC QAIH | 188 | GI5A 337 QLEV IRSL VLGT LPTM CNVF | 518 |
| **POOL 25** | . | **POOL 67** | . |
| A3B3 203 GLCC QQLL QIPE QS**Q**C QAIH | 189 | GI5C 337 QYEV IRSL VLRT LPNM CNVY | 519 |
| A4A 203 Q**L**CC QQL**F** QIPE QSRC QAIH | 190 | GI6A 337 QLEG IRSL VLKT LPTM CNVY | 520 |
| A1A1 211 QIPE QSQC QAIH NWH AIIL | 191 | GI1A 345 VLQT LP**T**M CNVY VPPE CS**II** | 521 |
| A1B3 211 QIPE QSQC QAI**Q** NWH AIIL | 192 | GI2A 345 VLQT LP**S**M CNVY VPPE CS**IM** | 522 |
| A1B6 211 QI**L**E QSQC QAIH NVVH AIIL | 193 | GI3A 345 VLQT LA**T**M CNVY VPP**Y** CS**TI** | 523 |
| A1B9 211 QIPE QSQC QAIH **K**WH AIIL | 194 | GI5A 345 VLGT LPTM CNVF VPPE CSTT | 524 |
| A1B10 QIPE **KL**QC QAIH NWH AIIL | 195 | GI5C 345 VLRT LPNM CNVY VRPD CSTI | 525 |
| A2A1 211 QIPE QS**R**C QAIH NVVH AIIL | 196 | GI6A 345 VLKT LPTM CNVY VPPD CSTI | 526 |
| **POOL 26** | . | **POOL 68** | . |
| A3B3 211 QIPE QS**Q**C QAIH NV**A**H AII**M** | 197 | GI1A 353 CNVY VPP**E** CS**II K**APF **SSV**V | 527 |
| A4A 211 QIPE QSRC QAIH NVVH AIIL | 198 | GI2A 353 CNVY VPP**E** CS**IM R**APF **ASI**V | 528 |
| A1A1 219 QAIH NVVH AIIL HQQQ KQQQ | 199 | GI3A 353 CNVY VPP**Y** CS**TI R**APF **A**SIV | 529 |
| A1A6 219 QAIH NWH AIIL HQQQ **Q**KQQ | 200 | GI5A 353 CNVF VPPE CSTT KAPF ASIV | 530 |
| A1B3 219 QAI**Q** NVVH AIIL HQQQ KQQQ | 201 | GI5C 353 CNVY VRPD CSTI NAPF ASIV | 531 |
| 219 QAIH **K**VVH AIIL HQQQ KQQQ | 202 | GI6A 353 CNVY VPPD CSTI NVPY ANID | 532 |
| A1B13 219 QAIH NVVH AIIL HQQQ **QQQQ** | 203 | GI1A 361 CS**II K**APF **S**S**V**V AGIG GQ | 533 |
| A2B3 219 QAIH NVVH AIIL HQQ**H HHH**Q | 204 | GI2A 361 CS**IM R**APF **A**SIV AGIG GQ | 534 |
| **POOL 27** | . | **POOL 69** | . |
| A3A1 219 QAIH NVVH AIIL HQQQ **R**QQQ | 205 | GI3A 361 CS**TI R**APF **ASI**V AGIG GQYR | 535 |
| A3B1 219 QAIH NVVH AII**M** HQQ**E** QQQQ | 206 | GI4 361 CSTI RAPF ASIV A**S**IG GQ | 536 |
| A3B3 219 QAIH NV**A**H AII**M** HQQQ QQQQ | 207 | GI5A 361 CSTT KAPF ASIV ADIG GQ | 537 |
| A4A 219 QAIH NVVH AIIL H**HH**Q QQQQ | 208 | GI5C 361 CSTINAPF ASIV AGIS GQ | 538 |
| A1A1 227 AIIL HQQQ KQQQ QPSS QVSF | 209 | GI6A 361 CSTI NVPY ANID AGIG GQ | 539 |
| A1A6 227 AIIL HQQQ **Q**KQQ QQPS SQFS | 210 | GII 1 PQQP FPLQ PQQS FLWQ SQQP | 540 |
| A1B2 227 AIIL HQQQ KQQQ Q**L**SS QVSF | 211 | GII 9 PQQS FLWQ SQQP FLQQ PQQP | 541 |
| A1B10 227 AIIL HQQQ KQQQ PSSQ VSFQ | 212 | GII 17 SQQP FLQQ PQQP SPQP QQVV | 542 |
| **POOL 28** | . | **POOL 70** | . |
| A1B13 227 AIIL HQQQ **QQQQ EQKQ QLQQ** | 213 | GII 25 PQQP SPQP QQVV QIIS PATP | 543 |
| A2A1 227 AIIL HQQQ QQQQ QQQQ QPLS | 214 | GII 33 QQVV QIIS PATP TTIP SAGK | 544 |
| A2B3 227 AIIL HQQ**H HHH**Q QQQQ QQQQ | 215 | GII 41 PATP TTIP SAGK PTSA PFPQ | 545 |
| A2B4 227 AIIL HQQH HHHQ EQKQ QLQQ | 216 | GII 49 SAGK PTSA PFPQ QQQQ HQQL | 546 |
| A3A1 227 AIIL HQQQ **R**QQQ PSSQ VS**L**Q | 217 | GII 57 PFPQ QQQQ HQQL AQQQ IPVV | 547 |
| A3B1 227 AII**M** HQQ**E** QQQQ **LQQQ QQQQ** | 218 | GII 65 HQQL AQQQ IPVV QPSI LQQL | 548 |
| A3B3 227 AII**M** HQQQ QQQQ EQKQ QLQQ | 219 | GII 73 IPW QPSI LQQL NPCK VFLQ | 549 |
| A4A 227 AIIL H**HH**Q QQQQ QPSS QVS**Y** | 220 | GII 81 LQQL NPCK VFLQ QQCS PVAM | 550 |
| **POOL 29** | . | **POOL 71** | . |
| A1A1 235 KQQQ QPSS QVSF QQPL QQYP | 221 | GII 89 VFLQ QQCS PVAM PQRL ARSQ | 551 |
| A1A6 235 KQQ**Q** QPSS Q**F**SF QQPL QQYP | 222 | GII 97 PVAM PQRL ARSQ MLQQ SSCH | 552 |
| A1B2 235 KQQQ Q**L**SS QVSF QQP**Q** QQYP | 223 | GII 105 ARSQ MLQQ SSCH VMQQ QCCQ | 553 |
| A1B10 235 KQQQ PSSQ VSFQ QP**Q**Q QYPL | 224 | GII 113 SSCH VMQQ QCCQ QLPQ IPQQ | 554 |
| A1B13 235 **QQQQ EQKQ QLQQ QQQQ QQQL** | 225 | GII 121 QCCQ QLPQ IPQQ SRYQ AIRA | 555 |
| A2B4 235 HHHQ EQKQ QLQQ QQQQ QQQL | 226 | GII 127B PQIP QQSR YEAI RAII YSII | 556 |
| A3A1 235 **R**QQQ PSSQ VS**L**Q QPQQ QYPS | 227 | GII 129 IPQQ SRYQ AIRA IIYS IILQ | 557 |
| A3B1 235 QQQQ **LQQQ QQQQ LQQ**Q QQQQ | 228 | GII 137 AIRA IIYS IILQ EQQQ VQGS | 558 |
| **POOL 30** | . | **POOL 72** | . |
| A4A 235 QQQQ QPSS QVSY QQPQ **E**QYP | 229 | GII 145 IILQ EQQQ VQGS IQSQ QQQP | 559 |
| A1B13 243 **QLQQ QQQQ QQQL QQQQ Q**KQQ | 230 | GII 153 VQGS IQSQ QQQP QQLG QCVS | 560 |
| A1B13 251 **QQQL QQQQ Q**KQQ QQPS SQVS | 231 | GII 161 QQQP QQLG QCVS QPQQ QSQQ | 561 |
| A2A1 260 QQQQ QQQQ QPLS QVSF QQPQ | 232 | GII 169 QCVS QPQQ QSQQ QLGQ QPQQ | 562 |
| A2B1 260 QQQQ QQQQ QPLS QVCF QQSQ | 233 | GII 177 QSQQ QLGQ QPQQ QQLA QGTF | ₅63 |
| A2B3 260 **HHH**Q QQQQ QQQQ QPLS QVSF | 234 | GII 185 QPQQ QQLA QGTF LQPH QIAQ | 564 |
| A3B1 260 QQQQ QQQQ QPSS QVSF QQPQ | 235 | **POOL 73** | . |
| A2A1 289 QP**L**S QVSF QQPQ QQYP SGQG | 236 | GII 193 QGTF LQPH QIAQ LEVM TSIA | 565 |
| **POOL 31** | . | GII 201 QIAQ LEVM TSIA LRIL PTMC | 566 |
| A2B1 289 QP**L**S QV**C**F QQ**S**Q QQYP SGQG | 237 | GII 209 TSIA LRIL PTMC SVNV PLYR | 567 |
| A3B1 289 QPSS QVSF QQPQ QQYP S**S**Q**V** | 238 | GII 217 PTMC SVNV PLYR TTTS VPFG | 568 |
| A1A1 293 QVSF QQPL QQYP LGQG SFRP | 239 | GII 225 PLYR TTTS VPFG VGTG VGAY | 569 |
| A1A6 293 Q**F**SF QQPL QQYP LGQG SFRP | 240 | GIII 1A 1 TITR TFPIPTIS SNNN HHFR | 570 |
| A1B2 293 QVSF QQP**Q** QQYP LGQG SFRP | 241 | GIII 1A 9 PTIS SNNN HHFR SNSN HHFH | 571 |
| A2A1 293 QVSF QQP**Q** QQYP **S**GQG SF**Q**P | 242 | GIII 1A 17 HHFR SNSN HHFH SNNN QFYR | 572 |
| A2B1 293 QV**C**F QQ**S**Q QQYP SGQG SFQP | 243 | **POOL 74** | . |
| A2B3 293 QVSF QQPQ QQYP **S**GQG **F**F**Q**P | 244 | GIII 1A 25 HHFH SNNN QFYRNNNS PGHN | 573 |
| **POOL 32** | . | GIII 1A 33 QFYR NNNS PGHN NPLN NNNS | 574 |
| A2B5 293 QVSF QQPQ QQYP **S**GQG **F**FQP | 245 | GIII 1A 41 PGHN NPLN NNNS PNNN SPSN | 575 |
| A3A1 293 QVS**L** QQPQ QQYP SGQG **F**FQP | 246 | GIII 1A 49 NNNS PNNN SPSN HHNN SPNN | 576 |
| A3B1 293 QVSF QQPQ QQYP S**S**QV SFQP | 247 | GIII 1A 57 SPSN HHNN SPNN NFQY HTHP | 577 |
| A3B2 293 QVSF QQPQ QQYP S**S**QG SFQP | 248 | GIII 1A 65 SPNN NFQY HTHP SNHK NLPH | 578 |
| A4A 293 QVS**Y** QQPQ EQYP SGQ**V** SFQ**S** | 249 | GIII 1A 73 HTHP SNHK NLPH TNNI QQQQ | 579 |
| A1A1 301 QQYP LGQG SFRP SQQN PQAQ | 250 | GIII 1A 81 NLPH TNNI QQQQ PPFS QQQQ | 580 |
| A1B2 301 QQYP LGQG SFRP SQQN **S**QAQ | 251 | **POOL 75** | . |
| A2A1 301 QQYP **S**GQG SF**Q**P SQQN PQAQ | 252 | GIII 1A 89 QQQQ PPFS QQQQ PPFS QQQQ | 581 |
| **POOL 33** | . | GIII 1A 97 QQQQ PPFS QQQQ PVLP QQSP | 582 |
| A2B3 301 QQYP **S**GQG FF**Q**P SQQN PQAQ | 253 | GIII 1A 105 QQQQ PVLP QQSP FSQQ QQLV | 583 |
| A2B5 301 QQYP SGQG **F**FQP **F**QQN PQAQ | 254 | GIII 1A 113 QQSP FSQQ QQLV LPPQ QQQQ | 584 |
| A3A1 301 QQYP SGQG **F**FQP SQQN PQAQ | 255 | GIII 1A 121 QQLV LPPQ QQQQ QLVQ QQIP | 585 |
| A3B1 301 QQYP S**S**Q**V** SFQP SQLN PQAQ | 256 | GIII 1A 129 QQQQ QLVQ QQIP IVQP SVLQ | 586 |
| A3B2 301 QQYP S**S**QG SFQP SQQN PQAQ | 257 | GIII 1A 137 QQIP IVQP SVLQ QLNP CKVF | 587 |
| A4A 301 **E**QYP SGQ**V** SFQ**S** SQQN PQAQ | 258 | GIII 1A 145 SVLQ QLNP CKVF LQQQ CSPV | 588 |
| A1B1 309 SFRP SQQN P**L**AQ GSVQ PQQL | 259 | **POOL 76** | . |
| A1A1 309 SFRP SQQN PQAQ GSVQ PQQL | 260 | GIII 1A 153 CKVF LQQQ CSPV AMPQ RLAR | 589 |
| **POOL 34** | . | GIII 1A 161 CSPV AMPQ RLAR SQMW QQSS | 590 |
| A1A3 309 SFRP SQQN PQ**T**Q GSVQ PQQL | 261 | GIII 1A 169 RLAR SQMW QQSS CHVM QQQC | 591 |
| A1B2 309 SFRP SQQN **S**QAQ GSVQ PQQL | 262 | GIII 1A 177 QQSS CHVM QQQC CQQL QQIP | 592 |
| A1B3 309 SFRP SQQN PQ**D**Q GSVQ PQQL | 263 | GIII 1A 185 QQQC CQQL QQIP EQSR YEAI | 593 |
| A1B4 309 SFRP SQQN P**R**AQ GSVQ PQQL | 264 | GIII 1A 193 QQIP EQSR YEAI RAII YSII | 594 |
| A2A1 309 SF**Q**P SQQN PQAQ GSVQ PQQL | 265 | GIII 1A 201 YEAI RAII YSII LQEQ QQGF | 595 |
| A2B3 309 **F**F**Q**P SQQN PQAQ GSFQ PQQL | 266 | GIII 1A 209 YSII LQEQ QQGF VQPQ QQQP | 596 |
| A2B5 309 **F**FQP **F**QQN PQAQ GSFQ PQQL | 267 | **POOL 77** | |
| A3A1 309 **F**FQP SQQN PQAQ GSVQ PQQL | 268 | GIII 1A 217 QQGF VQPQ QQQP QQSG QGVS | 597 |
| **Pool 35** | . | GIII 1A 225 QQQP QQSG QGVS QSQQ QSQQ | 598 |
| A3B1 309 SFQP SQ**L**N PQAQ GSVQ PQQL | 269 | GIII 1A 233 QGVS QSQQ QSQQ QLGQ CSFQ | 599 |
| A3B1 309 SFQP SQ**L**N PQAQ GSVQ PQQL | 270 | GIII 1A 241 QSQQ QLGQ CSFQ QPQQ QLGQ | 600 |
| A3B2 309 SFQP SQQN PQAQ GSVQ PQQL | 271 | GIII 1A 249 CSFQ QPQQ QLGQ QPQQ QQQQ | 601 |
| A4A 309 SFQ**S** SQQN PQAQ GSVQ PQQL | 272 | GIII 1A 257 QLGQ QPQQ QQQQ QVLQ GTFL | 602 |
| A1A1 317 PQAQ GSVQ PQQMPQFE EIRN | 273 | GIII 1A 263 QQQQ QVLQ GTFL QPHQ IAHL | 603 |
| A1A3 317 PQ**T**Q GSVQ PQQLPQFE EIRN | 274 | GIII 1A 271 GTFL QPHQ IAHL EAVT SIAL | 604 |
| A1A6 317 PQAQ GSVQ PQQL PQFE IRNL | 275 | **POOL 78** | . |
| A1B1 317 P**L**AQ GSVQ PQQL PQFE EIRN | 276 | GIII 1A 279 IAHL EAVT SIAL RTLP TMCS | 605 |
| **POOL 36** | . | GIII 1A 287 SIAL RTLP TMCS VNVP LYSA | 606 |
| A1B3 317 PQ**D**Q GSVQ PQQL PQFE EIRN | 277 | GIII 1A 295 TMCS VNVP LYSA TTSV PFGV | 607 |
| A1B4 317 P**R**AQ GSVQ PQQL PQFE EIRN | 278 | GIII 1A 303 LYSA TTSV PFGV GTGV GAY | 608 |
| A2B3 317 PQAQ GS**F**Q PQQL PQFE EIRN | 279 | GIII 1B 26 SCIS GLER PWQQ QPLP PQQS | 609 |
| A2B5 317 PQAQ GS**F**Q PQQL PQFE AIRN | 280 | GIII 1B 34 PWQQ QPLP PQQS FSQQ PPFS | 610 |
| A3B1 317 PQAQ GSVQ PQQL PQF**A** EIRN | 281 | GIII 1B 42 PQQS FSQQ PPFS QQQQ QPLP | 611 |
| A4A 317 PQAQ GSVQ PQQL PQF**Q** EIRN | 282 | GIII 1B 50 PPFS QQQQ QPLP QQPS FSQQ | 612 |
| **Pool 37** | . | **Pool 79** | . |
| A1A1 325 PQQL PQFE EIRN LALQ TLPA | 283 | GIII 1B 58 QPLP QQPS FSQQ QPPF SQQQ | 613 |
| A1A6 325 PQQL PQFE IRNL ALQT LPAM | 284 | GII 1B 66 FSQQ QPPF SQQQ PILS QQPP | 614 |
| A1B12 325 PQQL PQFE EIRN LA**RK** | 285 | GIII 1B 74 SQQQ PILS QQPP FSQQ QQPV | 615 |
| A2A1 325 PQQL PQFE EIRN LALE TLPA | 286 | O 1A 17 ATAA RELN PSNK ELQS PQQS | 616 |
| A2B5 325 PQQL PQFE **A**IRN LALQ TLPA | 287 | O 1A 25 PSNK ELQS PQQS FSYQ QQPF | 617 |
| A3B1 325 PQQL PQF**A** EIRN LALQ TLPA | 288 | O 1A 33 PQQS FSYQ QQPF PQQP YPQQ | 618 |
| A4A 325 PQQL PQF**Q** EIRN LALQ TLPA | 289 | O 1A 41 QQPF PQQP YPQQ PYPS QQPY | 619 |
| A1A1 333 EIRN LALQ TLP**A** MCNV YIPP | 290 | O 1A 49 YPQQ PYPS QQPY PSQQ PFPT | 620 |
| **POOL 38** | . | **POOL 80** | . |
| A1A3 333 EIRN LALQ TLP**S** MCNV YIPP | 291 | O 1A 57 QQPY PSQQ PFPT PQQQ FPEQ | 621 |
| A2A1 333 EIRN LALE TLPA MCNV YIPP | 292 | O 1A 65 PFPT PQQQ FPEQ SQQP FTQP | 622 |
| A3A1 333 EIRN LALQ TLP**R** MCNV YIPP | 293 | O 1A 73 FPEQ SQQP FTQP QQPT PIQP | 623 |
| A1A1 341 TLPA MCNV YIPP YCTI APFG | 294 | O 1A 81 FTQP QQPT PIQP QQPF PQQP | 624 |
| A1A3 341 TLP**S** MCNV YIPP YCTI APFG | 295 | O 1A 89 PIQP QQPF PQQP QQPQ QPFP | 625 |
| A1B1 341 TLPA MCNV YIPP YCTI **V**PFG | 296 | O 1A 97 PQQP QQPQ QPFP QPQQ PFPW | 626 |
| A1B4 341 TLPA MCNV YIPP YC**AM** APFG | 297 | O 1A 105 QPFP QPQQ PFPW QPQQ PFPQ | 627 |
| A1B9 341 TLPA MCNV YIPP YCTI TPFG | 298 | O 1A 113 PFPW QPQQ PFPQ TQQS FPLQ | 628 |
| **Pool 39** | . | **POOL 81** | . |
| A2A1 341 TLPA MCNV YIPP YCTI AP**V**G | 299 | O 1A 121 PFPQ TQQS FPLQ PQQP FPQQ | 629 |
| A2B2 341 TLPA MCNV YIPP YC**ST** TIAP | 300 | O 1A 129 FPLQ PQQP FPQQ PQQP FPQP | 630 |
| A3A1 341 TLP**R** MCNV YIPP YC**ST** TIAP | 301 | O 1A 137 FPQQ PQQP FPQP QLPF PQQS | 631 |
| A3A2 341 TLPR MCNV YIPP YCST T**T**AP | 302 | O 1A 145 FPQP QLPF PQQS EQII PQQL | 632 |
| A3B1 341 TLPA MCNV YIPP **H**C**ST** TIAP | 303 | O 1A 153 PQQS EQII PQQL QQPF PLQP | 633 |
| A1A1 349 YIPP YCTI APFG IFGT NYR | 304 | O 1A 161 PQQL QQPF PLQP QQPF PQQP | 634 |
| A1B1 349 YIPP YCTI **V**PFG IFGT NYR | 305 | O 1A 169 PLQP QQPF PQQP QQPF PQPQ | 635 |
| A1B4 349 YIPP YC**AM** APFG IFGT NYR | 306 | O 1A 177 PQQP QQPF PQPQ QPIP VQPQ | 636 |
| **Pool 40** | . | **Pool 82** | . |
| A1B5 349 YIPP YCT**M** APFG IFGT NYR | 307 | O 1A 185 PQPQ QPIP VQPQ QSFP QQSQ | 637 |
| A1B9 349 YIPP YCTI **T**PFG IFGT N | 308 | O 1A 193 VQPQ QSFP QQSQ QSQQ PFAQ | 638 |
| A2A1 349 YIPP YCTI AP**V**G IFGT NYR | 309 | O 1A 201 QQSQ QSQQ PFAQ PQQL FPEL | 639 |
| A2B2 349 YIPP YC**ST** TIAP VGIF GTN | 310 | O 1A 209 PFAQ PQQL FPEL QQPI PQQP | 640 |
| A3A2 349 YIPP YCST TTAP FGIF GTN | 311 | O 1A 217 FPEL QQPI PQQP QQPF PLQP | 641 |
| A3B1 349 YIPP **H**C**ST** TIAP FGIF GTN | 312 | O 1A 225 PQQP QQPF PLQP QQPF PQQP | 642 |
| A3B3 349 YIPP HCST TIAP FGI**S** GTN | 313 | O 1A 233 PLQP QQPF PQQP QQPF PQQP | 643 |
| A4D 350 IPPY CSTT IAPF GIFG TNYR | 314 | O 1A 241 PQQP QQPF PQQP QQSF PQQP | 644 |
| **Pool 41** | | **POOL 83** | . |
| GI1A 17 GTAN **M**QVD PS**S**Q VQW**P** QQQ**P** | 315 | O 1A 249 PQQP QQSF PQQP QQPY PQQQ | 645 |
| GI2A 17 GTAN **I**QVD PS**G**Q VQW**L** QQQL | 316 | O 1A 257 PQQP QQPY PQQQ PYGS SLTS | 646 |
| GI3A 17 **A**TAN **M**QVD PS**G**Q VPW**P** QQQ**P** | 317 | O 1A 265 PQQQ PYGS SLTS IGGQ | 647 |
| GI3B 19 **M**N IQVD PSGQ VPWP QQQP FP | 318 | O 1B 1 ARQL NPSD QELQ SPQQ LYPQ | 648 |
| GI4 17 ATAN MQ**A**D PS**G**Q VQW**P** QQQ**P** | 319 | O 1B 9 QELQ SPQQ LYPQ QPYP QQPY | 649 |
| GI5A 17 **T**TAN IQVD PSGQ VQWP QQQQ | 320 | O 1C 1 SRLL SPRG KELH TPQE QFPQ | 650 |
| GI5C 17 ATAN MQVD PSGQ VQWP QQQP | 321 | O 1C 9 KELH TPQE QFPQ QQQF PQPQ | 651 |
| GI7 20 QIVF PSGQ VQWP QQQQ PFP | 322 | 1C 17 QFPQ QQQF PQPQ QFPQ | 652 |
| **Pool 42** | | | |
| GI1A 25 PS**S**Q VQW**P** QQQ**P** VPQ**P H**QP**F** | 323 | | |
| GI2A 25 PS**G**Q VQW**L** QQQ**L** VPQ**L Q**QP**L** | 324 | | |
| GI3A 25 PS**G**Q VPW**P** QQQ**P F**PQ**P H**QP**F** | 325 | | |
| GI4 25 PS**G**Q VQW**P** QQQ**P FL**Q**P H**QP**F** | 326 | | |
| GI5A 25 PSGQ VQWP QQQQ PFPQ PQQP | 327 | | |
| GI5C 25 PSGQ VQWP QQQP FRQP QQPF | 328 | | |
| GI6A 25 PSGQ VQWP QQQP FPQP QQPF | 329 | | |
| GI1A 33 QQQ**P** VPQ**P H**QPF SQQP QQTF | 330 | | |

| | | | |
|---|---|---|---|
| *Position of N-terminal residue in α-, γ1-, γ2-, γ3-, or ω consensus sequence | | | |

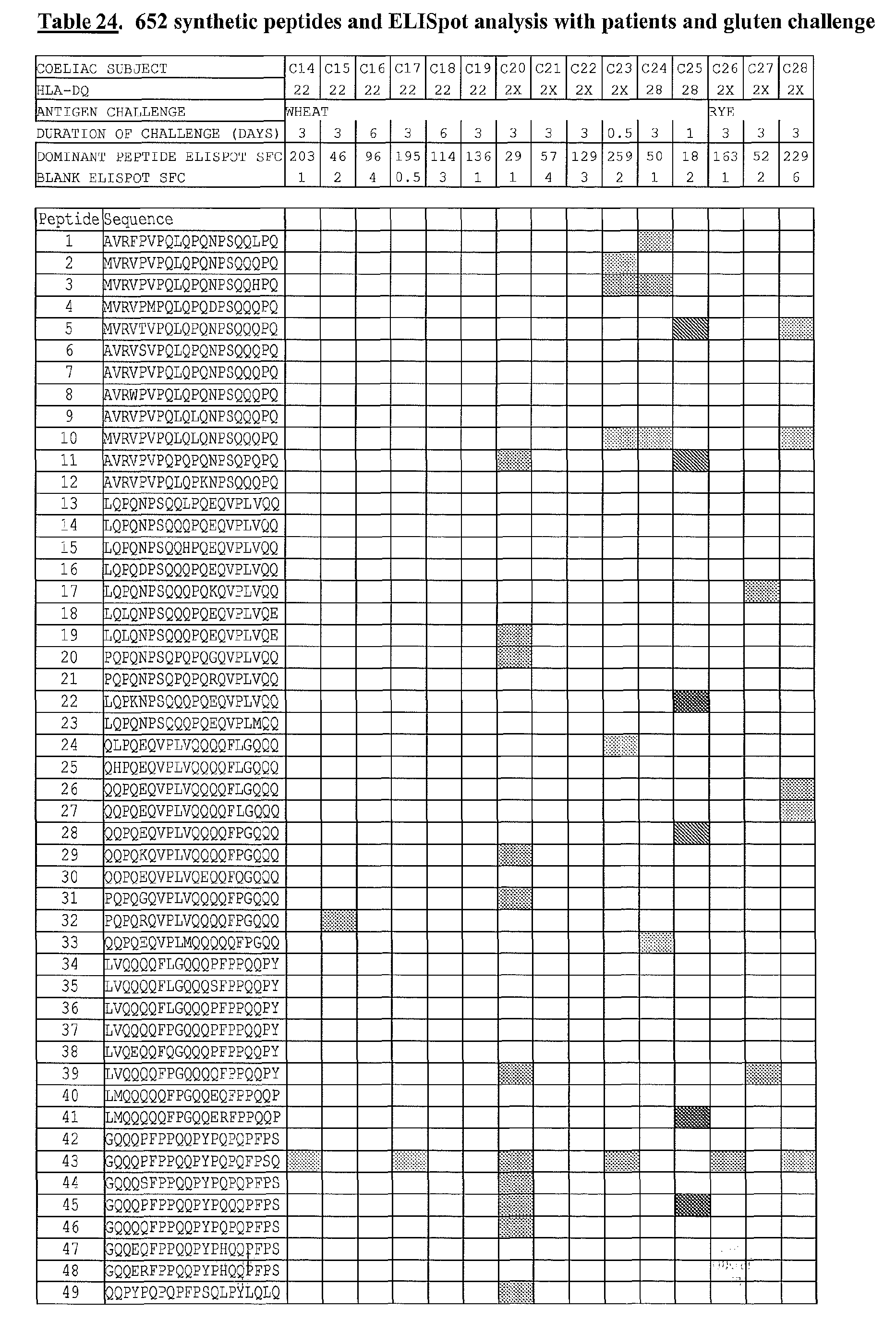

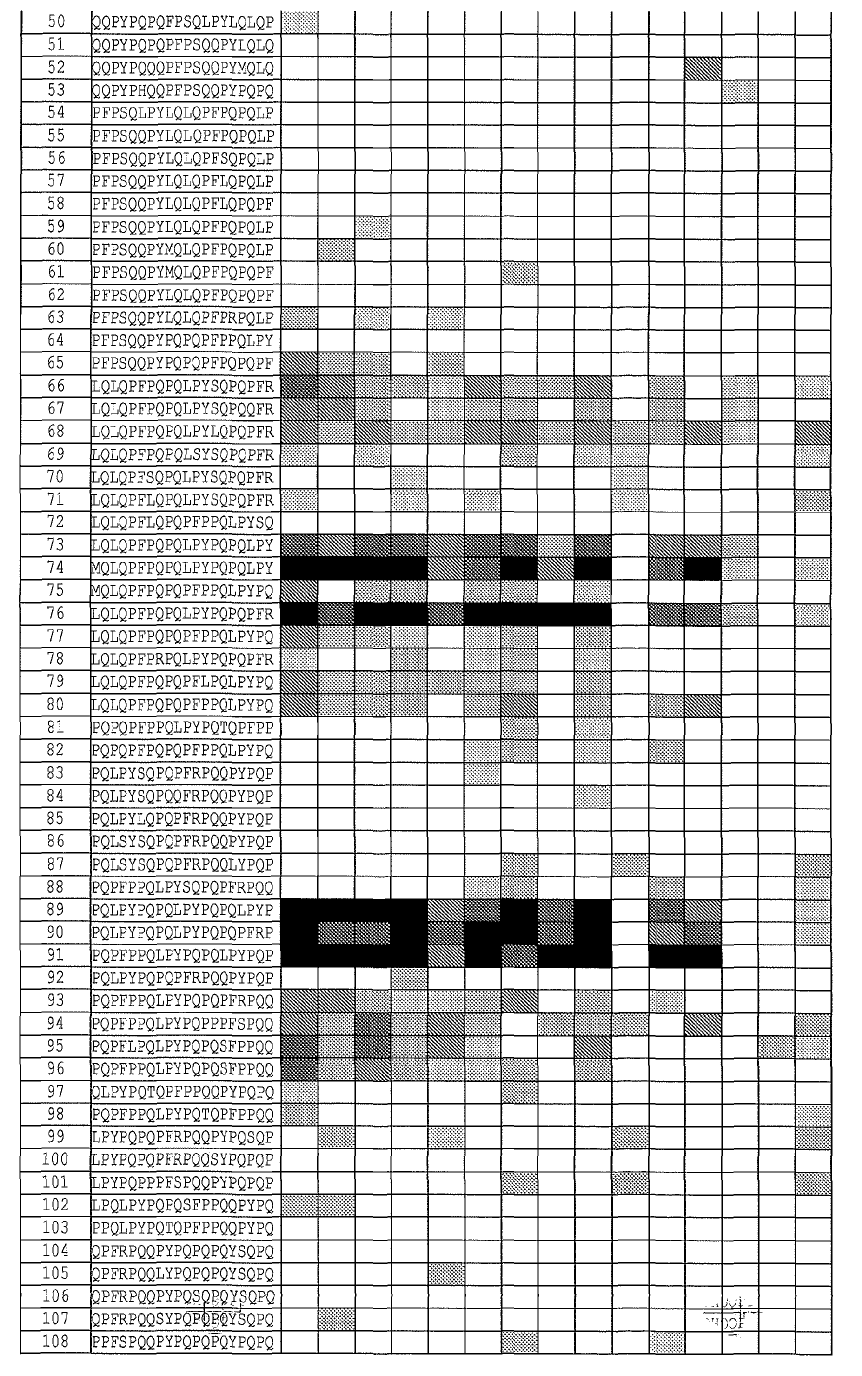

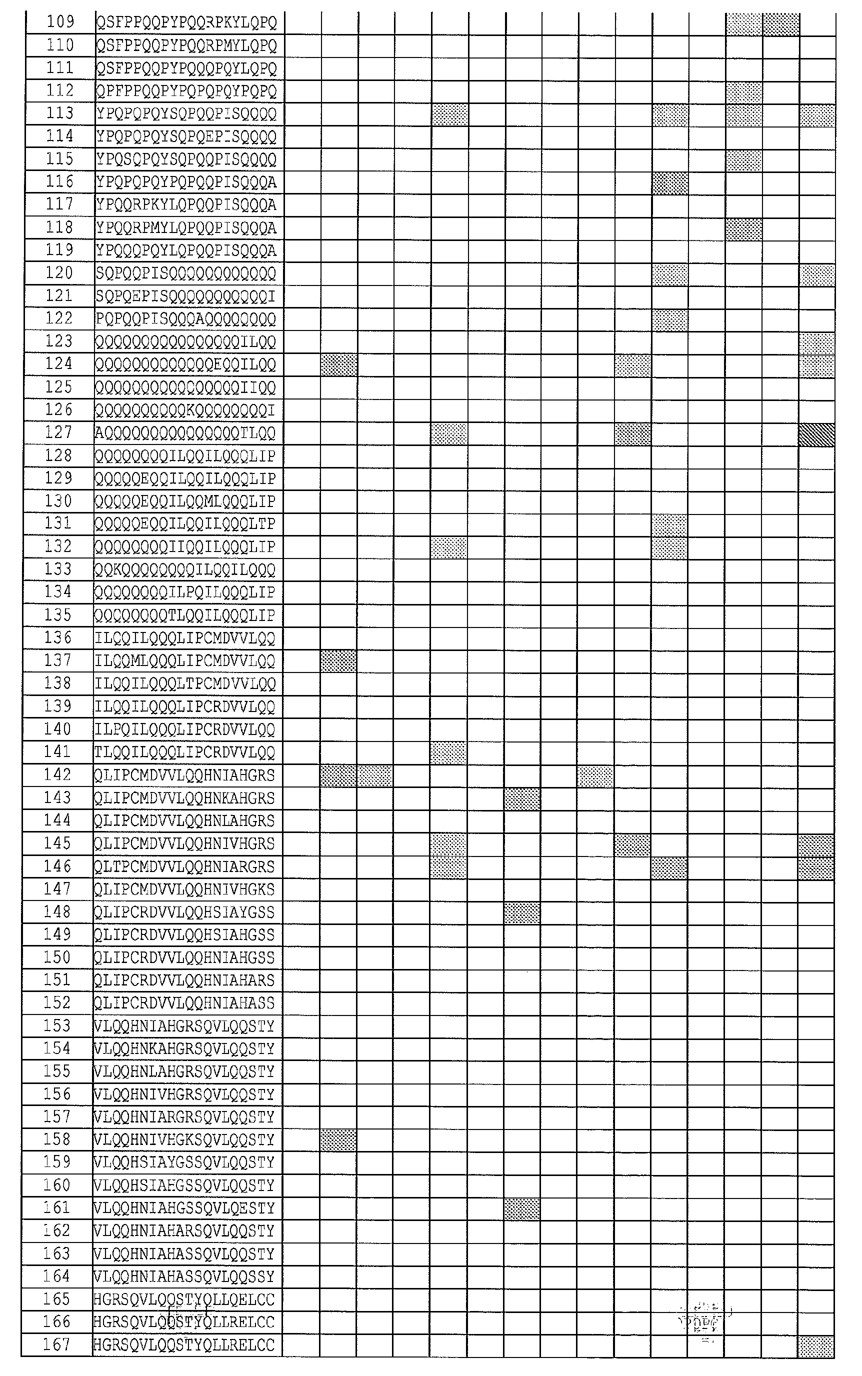

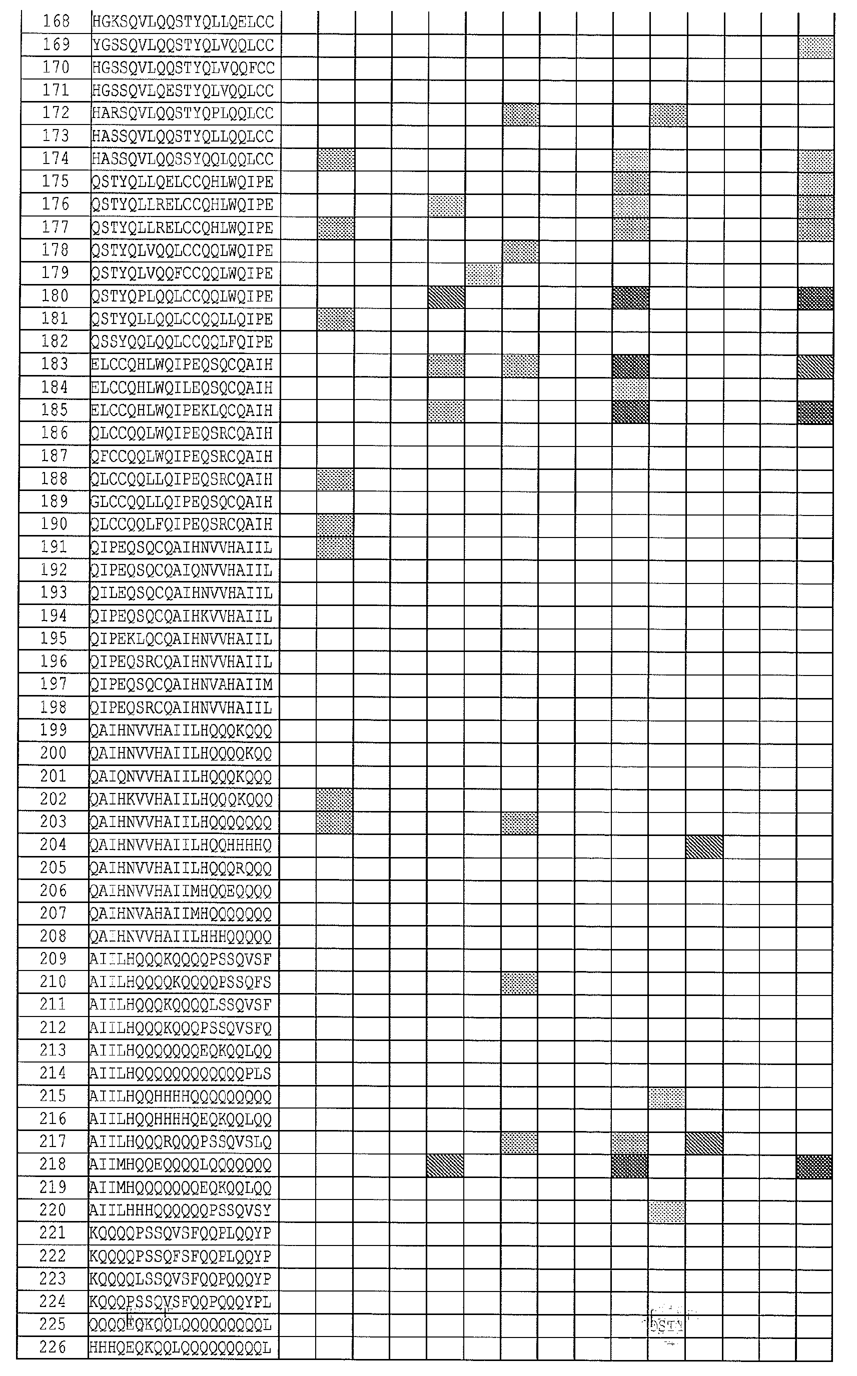

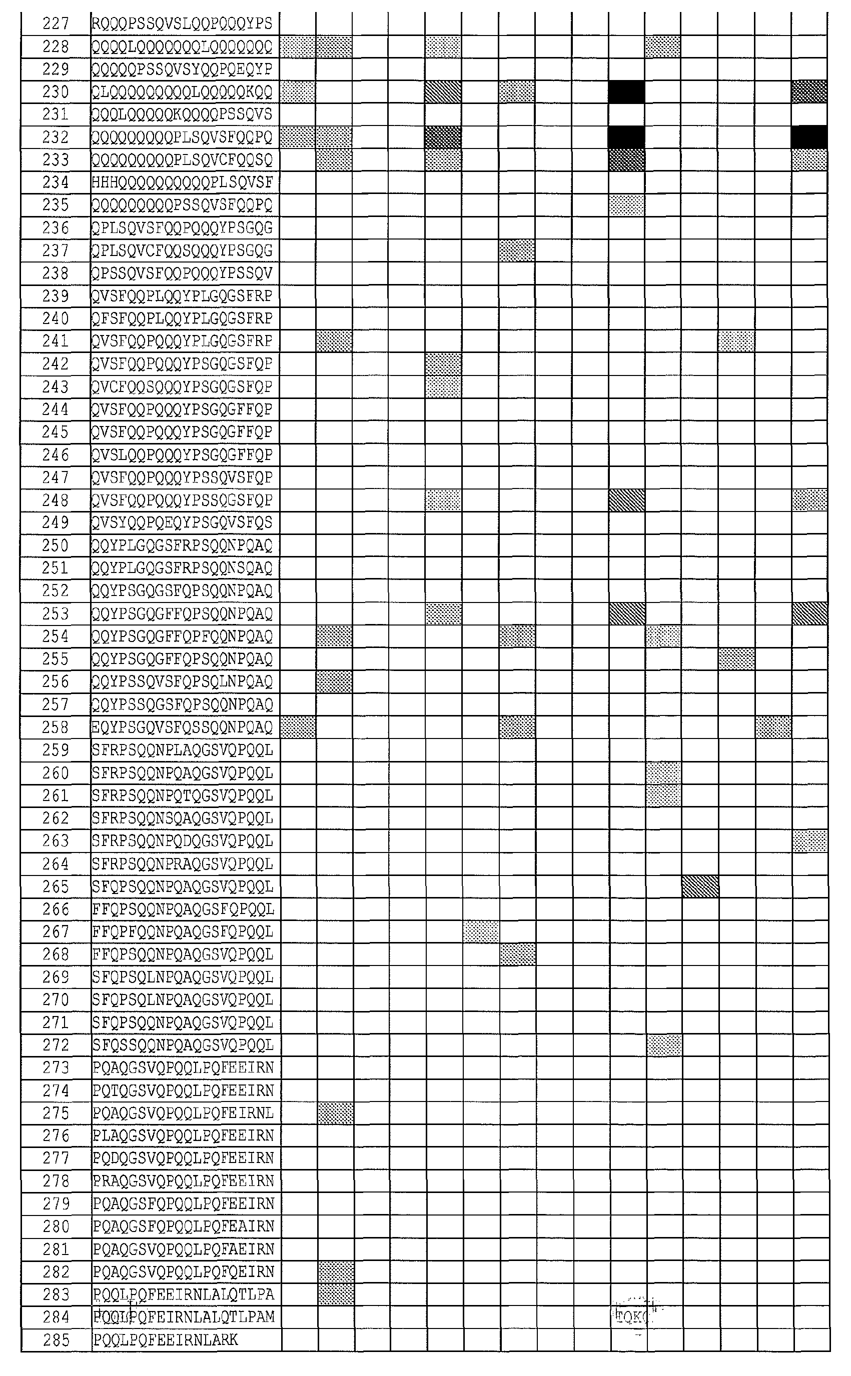

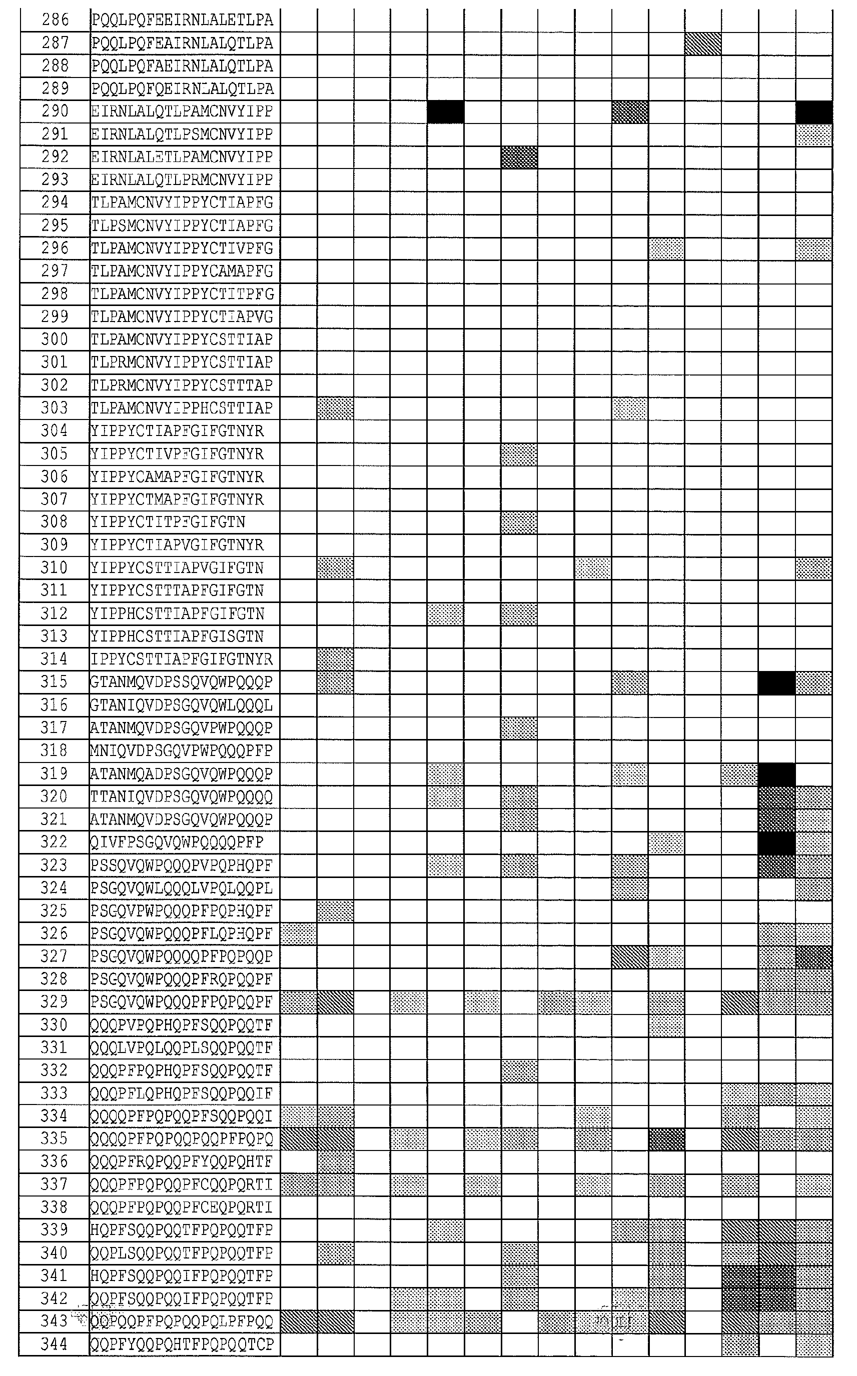

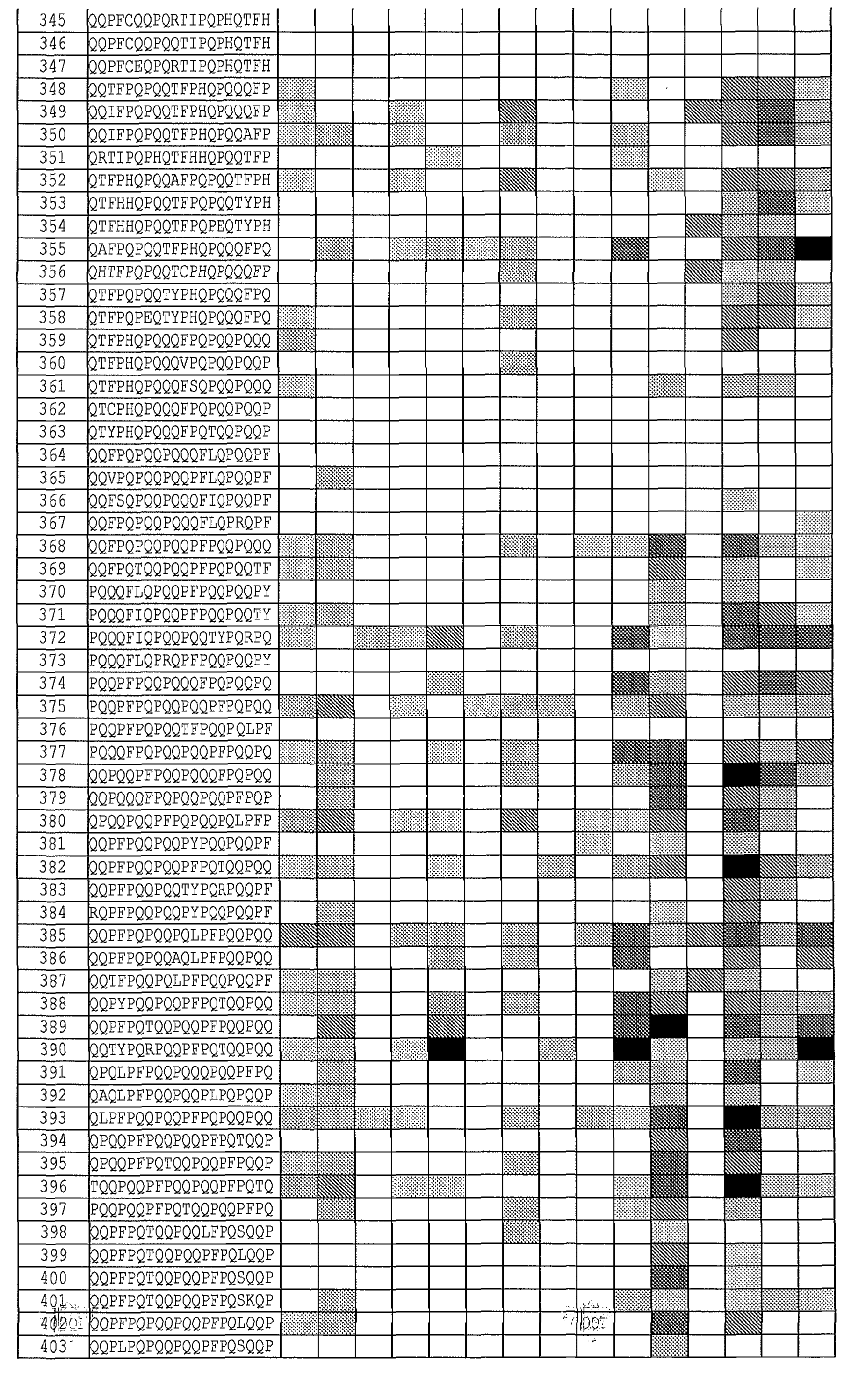

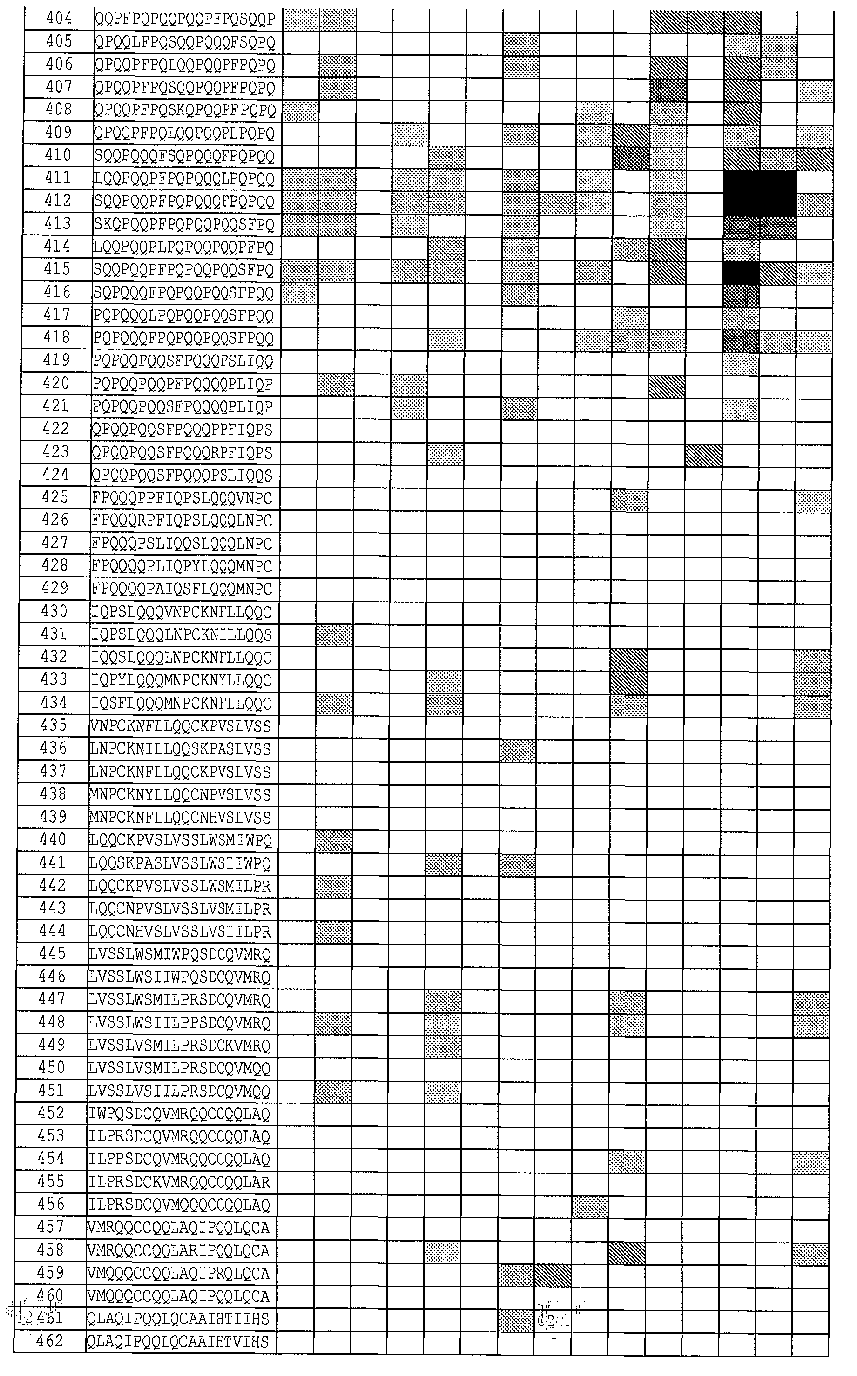

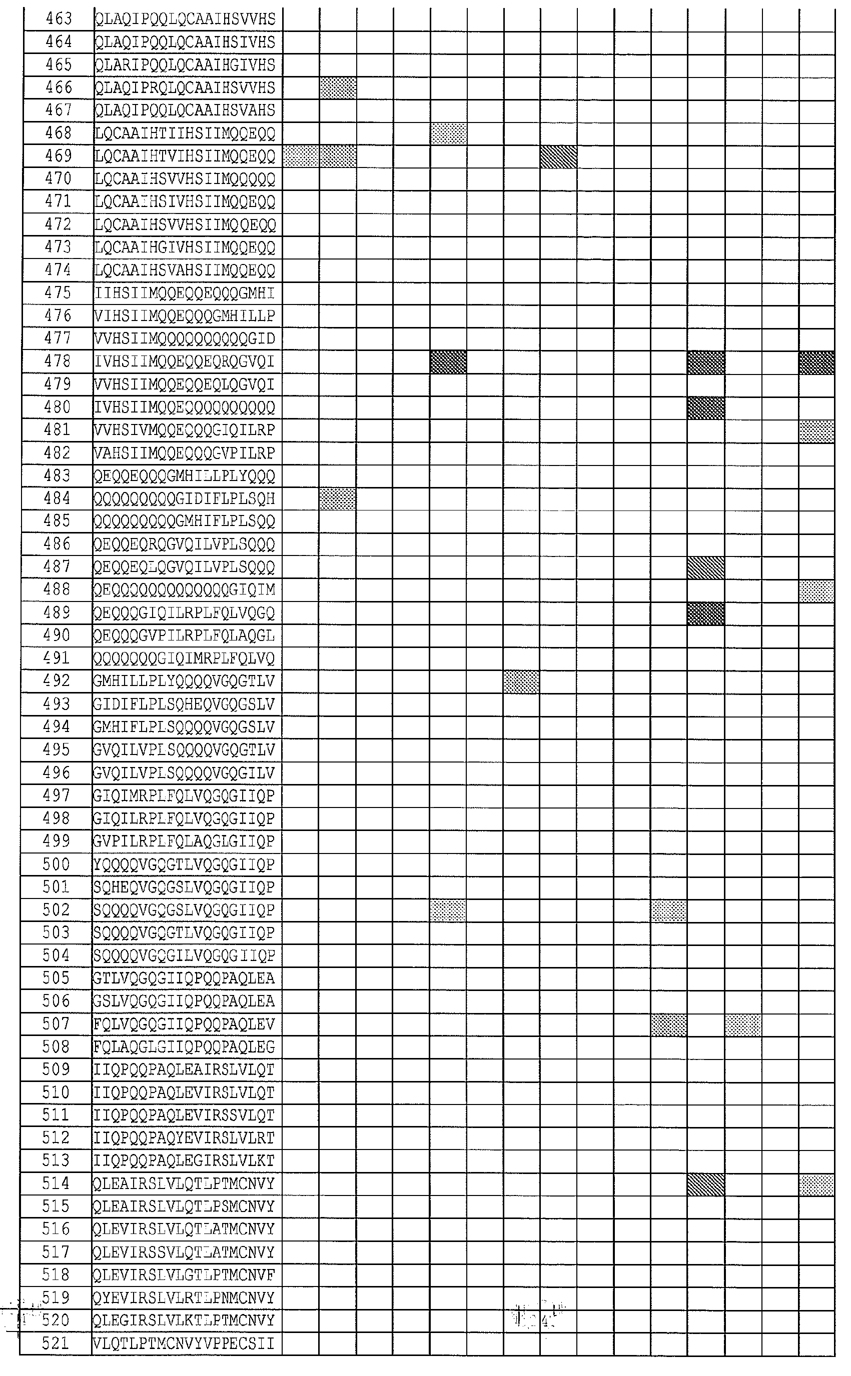

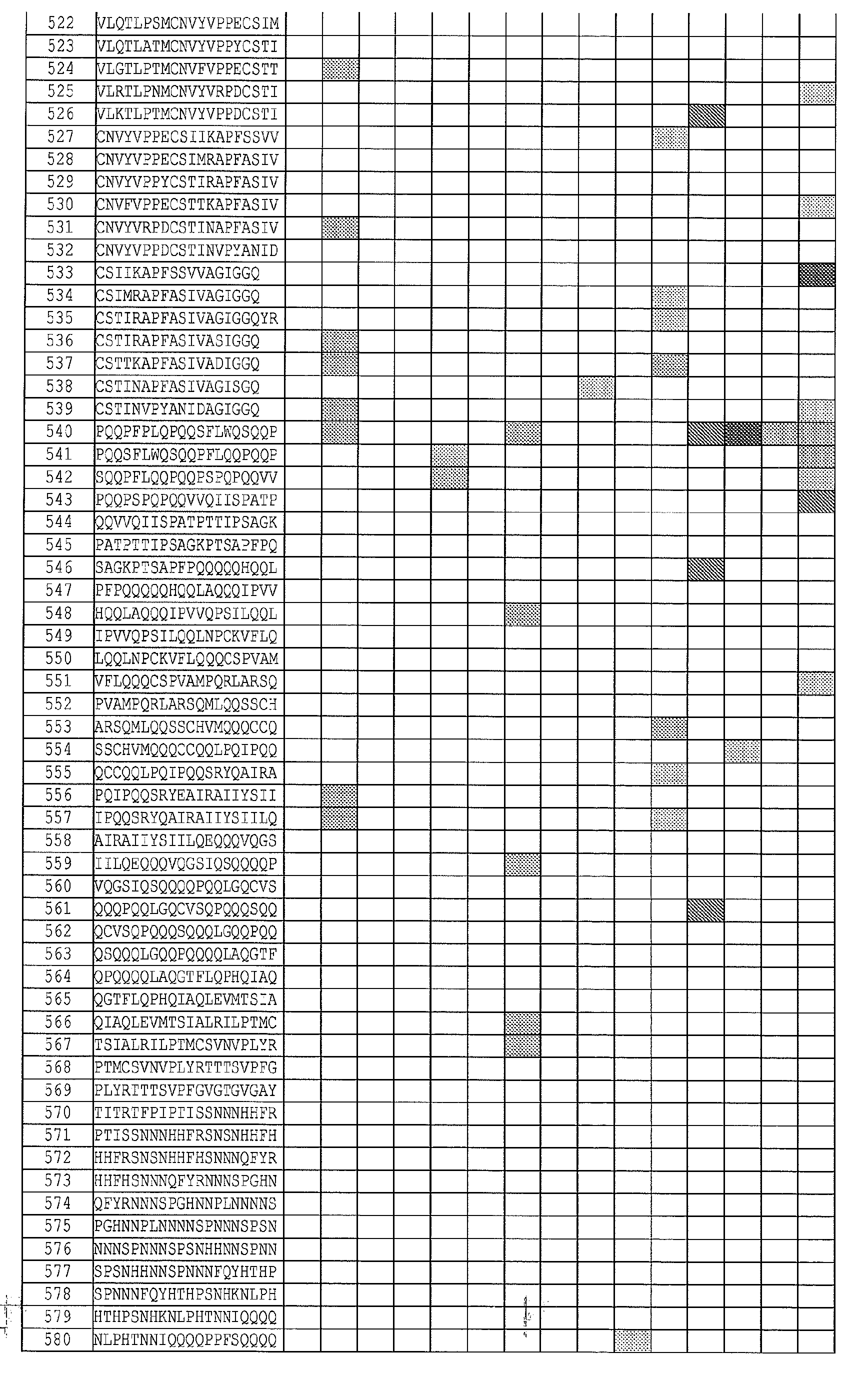

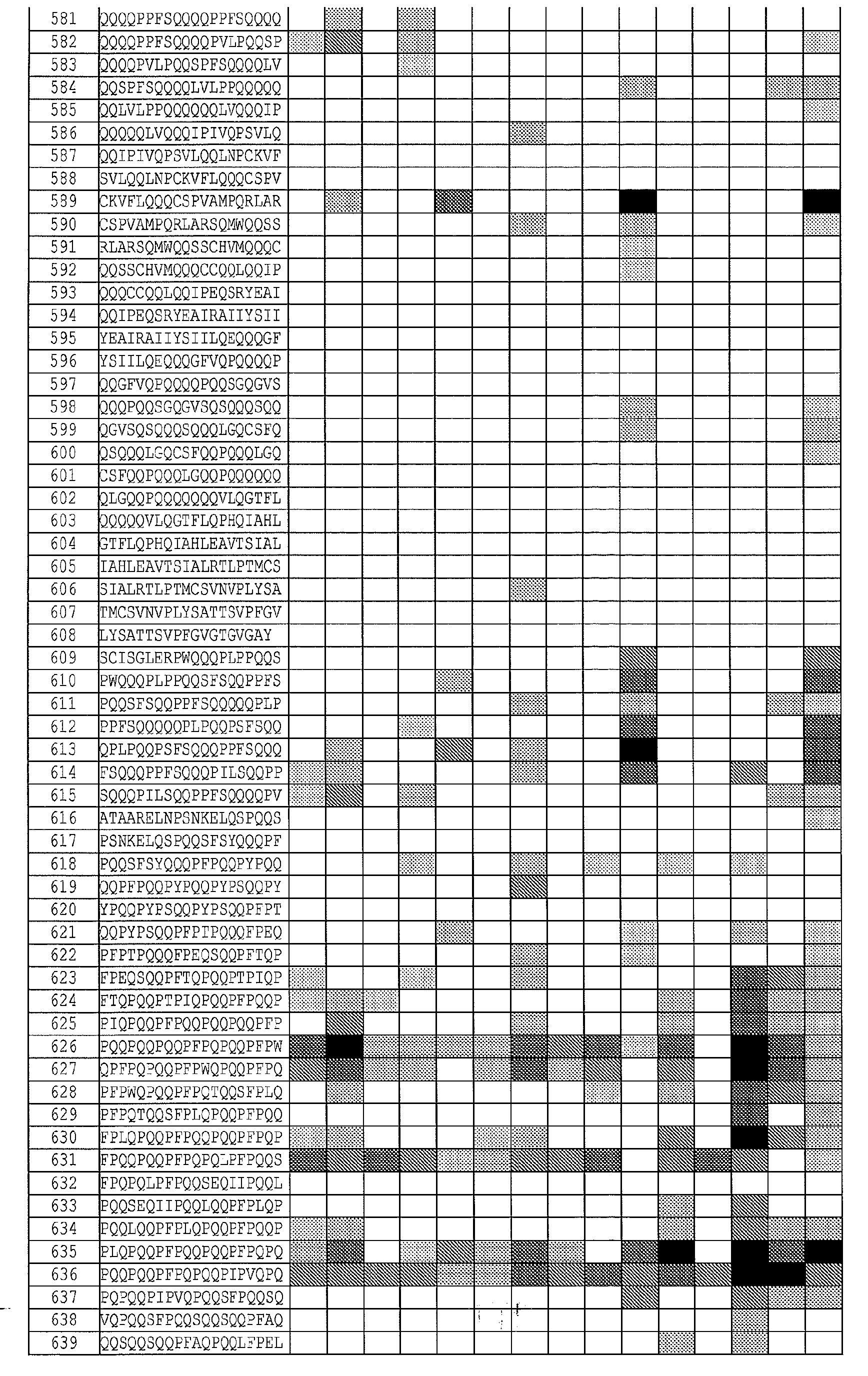

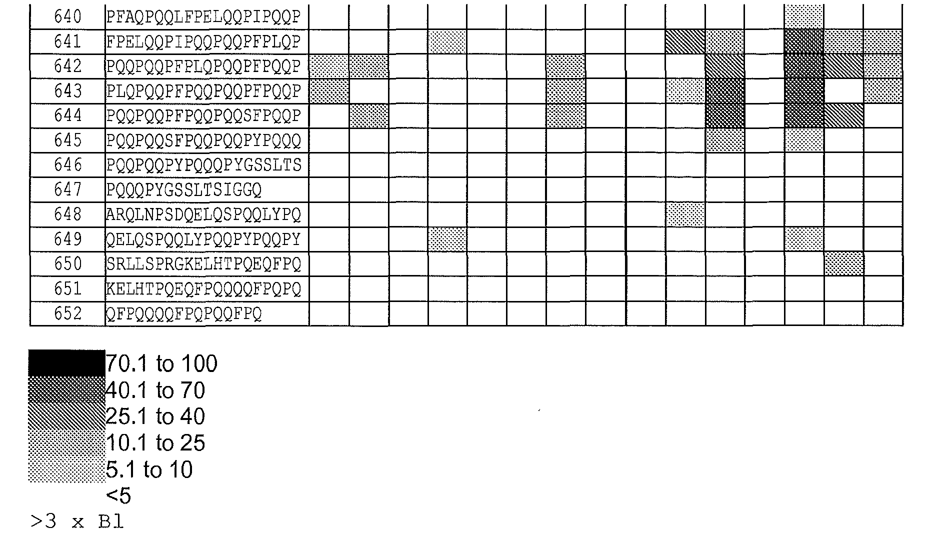

## Claims

1. A peptide for use in a method of therapy, the peptide comprising at least one T-Cell epitope, wherein the peptide is not more than 50 amino acids in length and comprises a sequence obtainable by transglutaminase deamidating the sequence PQQPQQPFP; provided that the peptide:
(i) is not an epitope that is selected from the group consisting of QQPYPEQPEQPFPQ, QQPFPEQPEQPFPQ, QLPFPEQPEQPFPQ and PFPQPEQPEQPFPQ; and
(ii) is not a peptide obtained by tissue transglutaminase treatment of the peptide PQQPFPQPQQPQQPFPQSQQ.

2. A peptide for use according to claim 1, which comprises the sequence PEQPEQPFP.

3. A peptide for use in a method of therapy, the peptide comprising at least one T-Cell epitope, wherein the peptide is not more than 50 amino acids in length and comprises a sequence obtainable by transglutaminase deamidating a sequence selected from the group consisting of PQQPQQPQQPFPQPQQPFPW, QQPFPQQPQQPFP, PQQPQQPFPQPQQPIPVQPQ, TQQPQQPFPQQPQQPFPQTQ, PIQPQQPFPQQPQQPQQPFP, PQQPQQPQQPFPQPQQPFPWQP, QQQQPFPQPQQPQQPFPQPQ, QQFPQPQQPQQPFPQQPQQQ, PQQPFPQPQQPQQPFPQPQQ, PQQQFPQPQQPQQPFPQQPQ, QQPQQQFPQPQQPQQPFPQP, QPQQPQQPFPQPQQPQLPFP, QQPFPQQPQQPFPQTQQPQQ, QQPYPQQPQQPFPQTQQPQQ, QLPFPQQPQQPFPQPQQPQQ, QPQQPFPQQPQQPFPQTQQP, TQQPQQPFPQQPQQPFPQTQ, PQQPQQPFPQTQQPQQPFPQ, QQPFPQPQQPQQPFPQLQQP, QQPLPQPQQPQQPFPQSQQP, QQPFPQPQQPQQPFPQSQQP, LQQPQQPLPQPQQPQQPFPQ, PQPQQPQQPFPQQQQPLIQP, FPLQPQQPFPQQPQQPFPQP, FPQQPQQPFPQPQLPFPQQS, PLQPQQPFPQQPQQPFPQPQ, FPELQQPIPQQPQQPFPLQP, PQQPQQPFPLQPQQPFPQQP, PLQPQQPFPQQPQQPFPQQP and PQQPQQPFPQQPQQSFPQQP.

4. A peptide for use according to claim 3, which comprises a sequence selected from the group consisting of PEQPEQPEQPFPQPEQPFPW, EQPFPEQPEQPFP, PEQPEQPFPQPEQPIPVQPQ, TEQPEQPFPEQPEQPFPQTQ, PIQPEQPFPEQPEQPEQPFP, PEQPEQPEQPFPQPEQPFPWQP, QQEQPFPQPEQPEQPFPQPQ, QEFPQPEQPEQPFPEQPQQQ, PEQPFPQPEQPEQPFPQPQQ, PEQEFPQPEQPEQPFPEQPQ, EQPEQEFPQPEQPEQPFPQP, QPEQPEQPFPQPEEPELPFP, EQPFPEQPEQPFPQTEQPQQ, EQPYPEQPEQPFPQTEQPQQ, ELPFPEQPEQPFPQPEQPQQ, QPEQPFPEQPEQPFPQTEQP, TEQPEQPFPEQPEQPFPQTQ, PEQPEQPFPQTEQPEQPFPQ, EQPFPQPEQPEQPFPQLEQP, EQPLPQPEQPEQPFPQSEQP, EQPFPQPEQPEQPFPQSEQP, LEQPEQPLPQPEQPEQPFPQ, PQPEQPEQPFPQQEEPLIQP, FPLQPEQPFPEQPEQPFPQP, FPEQPEQPFPEPELPFPQQS, PLQPEQPFPEQPEQPFPQPQ, FPELEQPIPEQPEQPFPLQP, PEQPEQPFPLQPEQPFPEQP, PLQPEQPFPEQPEQPFPEQP and PEQPEQPFPEQPEQSFPEQP.

5. A peptide for use according to any one of claims 1 to 4, which comprises a wheat epitope, a barley epitope, and a rye epitope.

6. The peptide as defined in any one of claims 1 to 5, for use in treating or preventing coeliac disease;
provided that the peptide:
(i) is not an epitope that is selected from the group consisting of QQPYPEQPEQPFPQ, QQPFPEQPEQPFPQ, QLPFPEQPEQPFPQ and PFPQPEQPEQPFPQ; and
(ii) is not a peptide obtained by tissue transglutaminase treatment of the peptide PQQPFPQPQQPQQPFPQSQQ.

7. A pharmaceutical composition comprising: (a) a peptide as defined in any one of claims 1-5, (b) a pharmaceutically acceptable carrier or diluent, and, optionally, (c) a peptide comprising at least one epitope comprising a sequence selected from PQPELPY (SEQ ID NO:1) and QLQPFPQPELPYPQPQS (SEQ ID NO:2);
provided that the peptide in (a):
(i) is not an epitope that is selected from the group consisting of QQPYPEQPEQPFPQ, QQPFPEQPEQPFPQ, QLPFPEQPEQPFPQ and PFPQPEQPEQPFPQ; and
(ii) is not a peptide obtained by tissue transglutaminase treatment of the peptide PQQPFPQPQQPQQPFPQSQQ.

8. An isolated peptide comprising at least one T-Cell epitope, wherein the peptide is not more than 50 amino acids in length and comprises a sequence obtainable by transglutaminase deamidating a sequence selected from the group consisting of PQQPQQPQQPFPQPQQPFPW, PQQPQQPFPQPQQPIPVQPQ, TQQPQQPFPQQPQQPFPQTQ, PIQPQQPFPQQPQQPQQPFP, PQQPQQPQQPFPQPQQPFPWQP, QQQQPFPQPQQPQQPFPQPQ, QQFPQPQQPQQPFPQQPQQQ, PQQPFPQPQQPQQPFPQPQQ, PQQQFPQPQQPQQPFPQQPQ, QQPQQQFPQPQQPQQPFPQP, QPQQPQQPFPQPQQPQLPFP, QQPFPQQPQQPFPQTQQPQQ, QQPYPQQPQQPFPQTQQPQQ, QLPFPQQPQQPFPQPQQPQQ, QPQQPFPQQPQQPFPQTQQP, TQQPQQPFPQQPQQPFPQTQ, PQQPQQPFPQTQQPQQPFPQ, QQPFPQPQQPQQPFPQLQQP, QQPLPQPQQPQQPFPQSQQP, QQPFPQPQQPQQPFPQSQQP, LQQPQQPLPQPQQPQQPFPQ, PQPQQPQQPFPQQQQPLIQP, FPLQPQQPFPQQPQQPFPQP, FPQQPQQPFPQPQLPFPQQS, PLQPQQPFPQQPQQPFPQPQ, FPELQQPIPQQPQQPFPLQP, PQQPQQPFPLQPQQPFPQQP, PLQPQQPFPQQPQQPFPQQP and PQQPQQPFPQQPQQSFPQQP.

9. A peptide according to claim 8, which comprises a sequence selected from the group consisting of PEQPEQPEQPFPQPEQPFPW, PEQPEQPFPQPEQPIPVQPQ, TEQPEQPFPEQPEQPFPQTQ, PIQPEQPFPEQPEQPEQPFP, PEQPEQPEQPFPQPEQPFPWQP, QQEQPFPQPEQPEQPFPQPQ, QEFPQPEQPEQPFPEQPQQQ, PEQPFPQPEQPEQPFPQPQQ, PEQEFPQPEQPEQPFPEQPQ, EQPEQEFPQPEQPEQPFPQP, QPEQPEQPFPQPEEPELPFP, EQPFPEQPEQPFPQTEQPQQ, EQPYPEQPEQPFPQTEQPQQ, ELPFPEQPEQPFPQPEQPQQ, QPEQPFPEQPEQPFPQTEQP, TEQPEQPFPEQPEQPFPQTQ, PEQPEQPFPQTEQPEQPFPQ, EQPFPQPEQPEQPFPQLEQP, EQPLPQPEQPEQPFPQSEQP, EQPFPQPEQPEQPFPQSEQP, LEQPEQPLPQPEQPEQPFPQ, PQPEQPEQPFPQQEEPLIQP, FPLQPEQPFPEQPEQPFPQP, FPEQPEQPFPEPELPFPQQS, PLQPEQPFPEQPEQPFPQPQ, FPELEQPIPEQPEQPFPLQP, PEQPEQPFPLQPEQPFPEQP, PLQPEQPFPEQPEQPFPEQP and PEQPEQPFPEQPEQSFPEQP.

10. A peptide according to claim 8 or 9, which comprises a wheat epitope, a barley epitope, and a rye epitope.

11. A composition comprising a first HLA-DQ2-restricted agent and a second HLA-DQ8-restricted agent, wherein the composition comprises a peptide as defined in any one of claims 8 to 10.

12. A composition comprising a peptide comprising a wheat epitope, a peptide comprising a barley epitope and/or a peptide comprising a rye epitope, wherein the composition comprises a peptide as defined in any one of claims 8 to 10.

13. A composition comprising: (a) a peptide as defined in any one of claims 8 to 10; and (b) a peptide comprising at least one epitope comprising a sequence selected from PQPELPY (SEQ ID NO:1) and QLQPFPQPELPYPQPQS (SEQ ID NO:2).

14. A method of diagnosing coeliac disease, or susceptibility to coeliac disease, in an individual comprising:
contacting a sample from the host with at least one peptide as defined in any one of claims 8 to 10; and
determining *in vitro* whether T cells in the sample recognise the peptide;
recognition by the T cells indicating that the individual has, or is susceptible to, coeliac disease.

15. A kit for carrying out a method according to claim 14, comprising a peptide as defined in any one of claims 8 to 10 and a means to detect the recognition of the peptide by a T cell, wherein optionally the means to detect recognition comprises an antibody to IFN-γ.

## Patentansprüche

1. Peptid für die Verwendung in einem Therapieverfahren, wobei das Peptid wenigstens ein T-Zellenepitop umfasst, wobei das Peptid nicht mehr als 50 Aminosäuren lang ist und eine Sequenz umfasst, die dadurch erhalten werden kann, dass Transglutaminase die Sequenz PQQPQQPFP deamidiert;
unter der Voraussetzung, dass das Peptid:
(i) kein Epitop ist, das aus der Gruppe ausgewählt ist, bestehend aus QQPYPEQPEQPFPQ, QQPFPEQPEQPFPQ, QLPFPEQPEQPFPQ und PFPQPEQPEQPFPQ; und
(ii) kein Peptid ist, das durch eine Gewebetransglutaminasebehandlung des Peptids PQQPFPQPQQPQQPFPQSQQ erhalten wird.

2. Peptid für die Verwendung nach Anspruch 1, das die Sequenz PEQPEQPFP umfasst.

3. Peptid für die Verwendung in einem Therapieverfahren, wobei das Peptid wenigstens ein T-Zellenepitop umfasst, wobei das Peptid nicht mehr als 50 Aminosäuren lang ist und eine Sequenz umfasst, die dadurch erhalten werden kann, dass Transglutaminase eine Sequenz deamidiert, ausgewählt aus der Gruppe bestehend aus PQQPQQPQQPFPQPQQPFPW, QQPFPQQPQQPFP, PQQPQQPFPQPQQPIPVQPQ, TQQPQQPFPQQPQQPFPQTQ, PIQPQQPFPQQPQQPQQPFP, PQQPQQPQQPFPQPQQPFPWQP, QQQQPFPQPQQPQQPFPQPQ, QQFPQPQQPQQPFPQQPQQQ, PQQPFPQPQQPQQPFPQPQQ, PQQQFPQPQQPQQPFPQQPQ, QQPQQQFPQPQQPQQPFPQP, QPQQPQQPFPQPQQPQLPFP, QQPFPQQPQQPFPQTQQPQQ, QQPYPQQPQQPFPQTQQPQQ, QLPFPQQPQQPFPQPQQPQQ, QPQQPFPQQPQQPFPQTQQP, TQQPQQPFPQQPQQPFPQTQ, PQQPQQPFPQTQQPQQPFPQ, QQPFPQPQQPQQPFPQLQQP, QQPLPQPQQPQQPFPQSQQP, QQPFPQPQQPQQPFPQSQQP, LQQPQQPLPQPQQPQQPFPQ, PQPQQPQQPFPQQQQPLIQP, FPLQPQQPFPQQPQQPFPQP, FPQQPQQPFPQPQLPFPQQS, PLQPQQPFPQQPQQPFPQPQ, FPELQQPIPQQPQQPFPLQP, PQQPQQPFPLQPQQPFPQQP, PLQPQQPFPQQPQQPFPQQP und PQQPQQPFPQQPQQSFPQQP.

4. Peptid für die Verwendung nach Anspruch 3, das eine Sequenz umfasst, ausgewählt aus der Gruppe bestehend aus PEQPEQPEQPFPQPEQPFPW, EQPFPEQPEQPFP, PEQPEQPFPQPEQPIPVQPQ, TEQPEQPFPEQPEQPFPQTQ, PIQPEQPFPEQPEQPEQPFP, PEQPEQPEQPFPQPEQPFPWQP, QQEQPFPQPEQPEQPFPQPQ, QEFPQPEQPEQPFPEQPQQQ, PEQPFPQPEQPEQPFPQPQQ, PEQEFPQPEQPEQPFPEQPQ, EQPEQEFPQPEQPEQPFPQP, QPEQPEQPFPQPEEPELPFP, EQPFPEQPEQPFPQTEQPQQ, EQPYPEQPEQPFPQTEQPQQ, ELPFPEQPEQPFPQPEQPQQ, QPEQPFPEQPEQPFPQTEQP, TEQPEQPFPEQPEQPFPQTQ,PEQPEQPFPQTEQPEQPFPQ, EQPFPQPEQPEQPFPQLEQP, EQPLPQPEQPEQPFPQSEQP, EQPFPQPEQPEQPFPQSEQP, LEQPEQPLPQPEQPEQPFPQ, PQPEQPEQPFPQQEEPLIQP, FPLQPEQPFPEQPEQPFPQP, FPEQPEQPFPEPELPFPQQS, PLQPEQPFPEQPEQPFPQPQ, FPELEQPIPEQPEQPFPLQP, PEQPEQPFPLQPEQPFPEQP, PLQPEQPFPEQPEQPFPEQP und PEQPEQPFPEQPEQSFPEQP.

5. Peptid für die Verwendung nach einem der Ansprüche 1 bis 4, das ein Weizenepitop, ein Gerstenepitop und ein Roggenepitop umfasst.

6. Peptid nach einem der Ansprüche 1 bis 5 für die Verwendung beim Behandeln oder Verhindern der Zöliakie;
vorausgesetzt, dass das Peptid:
(i) kein Epitop ist, das aus der Gruppe ausgewählt ist, bestehend aus QQPYPEQPEQPFPQ, QQPFPEQPEQPFPQ, QLPFPEQPEQPFPQ und PFPQPEQPEQPFPQ; und
(ii) kein Peptid ist, das durch eine Gewebetransglutaminasebehandlung des Peptids PQQPFPQPQQPQQPFPQSQQ erhalten wird.

7. Pharmazeutische Zusammensetzung, umfassend: (a) ein Peptid nach einem der Ansprüche 1-5, (b) einen pharmazeutisch unbedenklichen Träger oder Verdünner und optional (c) ein Peptid, das wenigstens ein Epitop umfasst, das eine Sequenz umfasst, ausgewählt aus PQPELPY (SEQ ID NO: 1) und QLQPFPQPELPYPQPQS (SEQ ID NO: 2) umfasst; vorausgesetzt, dass das Peptid in (a):
(i) kein Epitop ist, das aus der Gruppe ausgewählt ist, bestehend aus QQPYPEQPEQPFPQ, QQPFPEQPEQPFPQ, QLPFPEQPEQPFPQ und PFPQPEQPEQPFPQ; und
(ii) kein Peptid ist, das durch eine Gewebetransglutaminasebehandlung des Peptids PQQPFPQPQQPQQPFPQSQQ erhalten wird.

8. Isoliertes Peptid, umfassend wenigstens ein T-Zellenepitop, wobei das Peptid nicht mehr als 50 Aminosäuren lang ist und eine Sequenz umfasst, die dadurch erhalten werden kann, dass Transglutaminase eine Sequenz deamidiert, ausgewählt aus der Gruppe bestehend aus PQQPQQPQQPFPQPQQPFPW, PQQPQQPFPQPQQPIPVQPQ, TQQPQQPFPQQPQQPFPQTQ, PIQPQQPFPQQPQQPQQPFP, PQQPQQPQQPFPQPQQPFPWQP, QQQQPFPQPQQPQQPFPQPQ, QQFPQPQQPQQPFPQQPQQQ, PQQPFPQPQQPQQPFPQPQQ, PQQQFPQPQQPQQPFPQQPQ, QQPQQQFPQPQQPQQPFPQP, QPQQPQQPFPQPQQPQLPFP, QQPFPQQPQQPFPQTQQPQQ, QQPYPQQPQQPFPQTQQPQQ, QLPFPQQPQQPFPQPQQPQQ, QPQQPFPQQPQQPFPQTQQP, TQQPQQPFPQQPQQPFPQTQ, PQQPQQPFPQTQQPQQPFPQ, QQPFPQPQQPQQPFPQLQQP, QQPLPQPQQPQQPFPQSQQP, QQPFPQPQQPQQPFPQSQQP, LQQPQQPLPQPQQPQQPFPQ, PQPQQPQQPFPQQQQPLIQP, FPLQPQQPFPQQPQQPFPQP, FPQQPQQPFPQPQLPFPQQS, PLQPQQPFPQQPQQPFPQPQ, FPELQQPIPQQPQQPFPLQP, PQQPQQPFPLQPQQPFPQQP, PLQPQQPFPQQPQQPFPQQP und PQQPQQPFPQQPQQSFPQQP.

9. Peptid nach Anspruch 8, das eine Sequenz umfasst, ausgewählt aus der Gruppe bestehend aus PEQPEQPEQPFPQPEQPFPW, PEQPEQPFPQPEQPIPVQPQ, TEQPEQPFPEQPEQPFPQTQ, PIQPEQPFPEQPEQPEQPFP, PEQPEQPEQPFPQPEQPFPWQP, QQEQPFPQPEQPEQPFPQPQ, QEFPQPEQPEQPFPEQPQQQ, PEQPFPQPEQPEQPFPQPQQ, PEQEFPQPEQPEQPFPEQPQ, EQPEQEFPQPEQPEQPFPQP, QPEQPEQPFPQPEEPELPFP, EQPFPEQPEQPFPQTEQPQQ, EQPYPEQPEQPFPQTEQPQQ, ELPFPEQPEQPFPQPEQPQQ, QPEQPFPEQPEQPFPQTEQP,TEQPEQPFPEQPEQPFPQTQ, PEQPEQPFPQTEQPEQPFPQ, EQPFPQPEQPEQPFPQLEQP, EQPLPQPEQPEQPFPQSEQP, EQPFPQPEQPEQPFPQSEQP, LEQPEQPLPQPEQPEQPFPQ, PQPEQPEQPFPQQEEPLIQP, FPLQPEQPFPEQPEQPFPQP, FPEQPEQPFPEPELPFPQQS, PLQPEQPFPEQPEQPFPQPQ, FPELEQPIPEQPEQPFPLQP, PEQPEQPFPLQPEQPFPEQP, PLQPEQPFPEQPEQPFPEQP und PEQPEQPFPEQPEQSFPEQP.

10. Peptid nach Anspruch 8 oder 9, das ein Weizenepitop, ein Gerstenepitop und ein Roggenepitop umfasst.

11. Zusammensetzung, umfassend einen ersten HLA-DQ2-einschränkenden Wirkstoff und einen zweiten HLA-DQ8-einschränkenden Wirkstoff, wobei die Zusammensetzung ein Peptid nach einem der Ansprüche 8 bis 10 umfasst.

12. Zusammensetzung, umfassend ein Peptid, das ein Weizenepitop umfasst, ein Peptid, das ein Gerstenepitop umfasst, und/oder Peptid, das ein Roggenepitop umfasst, wobei die Zusammensetzung ein Peptid nach einem der Ansprüche 8 bis 10 umfasst.

13. Zusammensetzung, umfassend: (a) ein Peptid nach einem der Ansprüche 8 bis 10; und (b) ein Peptid, das wenigstens ein Epitop umfasst, das eine Sequenz umfasst, ausgewählt aus PQPELPY (SEQ ID NO: 1) und QLQPFPQPELPYPQPQS (SEQ ID NO: 2).

14. Verfahren zum Diagnostizieren der Zöliakie oder der Anfälligkeit für die Zöliakie in einem Individuum, umfassend:
Inberührungbringen einer Probe von dem Wirt mit wenigstens einem Peptid nach einem der Ansprüche 8 bis 10; und
Bestimmen *in vitro,* ob die T-Zellen in der Probe das Peptid erkennen; wobei eine Erkennung durch die T-Zellen anzeigt, dass das Individuum Zöliakie aufweist oder für diese anfällig ist.

15. Kit zum Ausführen eines Verfahrens nach Anspruch 14, umfassend ein Peptid nach einem der Ansprüche 8 bis 10 und ein Mittel zum Nachweisen der Erkennung des Peptids durch eine T-Zelle, wobei optional das Mittel zum Nachweisen der Erkennung einen Antikörper IFN-γ umfasst.

## Revendications

1. Peptide destiné à une utilisation dans une méthode de thérapie, le peptide comprenant au moins un épitope de cellule T, le peptide n'étant de pas plus de 50 acides aminés en longueur et comprenant une séquence pouvant être obtenue par désamidation par transglutaminase de la séquence PQQPQQPFP ; à condition que le peptide :
(i) ne soit pas un épitope qui est choisi dans le groupe constitué par QQPYPEQPEQPFPQ, QQPFPEQPEQPFPQ, QLPFPEQPEQPFPQ et PFPQPEQPEQPFPQ et
(ii) ne soit pas un peptide obtenu par traitement à la transglutaminase de tissu du peptide PQQPFPQPQQPQQPFPQSQQ.

2. Peptide destiné à une utilisation selon la revendication 1, qui comprend la séquence PEQPEQPFP.

3. Peptide destiné à une utilisation dans une méthode de thérapie, le peptide comprenant au moins un épitope de cellule T, le peptide n'étant pas de plus de 50 acides aminés en longueur et comprenant une séquence pouvant être obtenue par désamidation par transglutaminase d'une séquence choisie dans le groupe constitué par PQQPQQPQQPFPQPQQPFPW, QQPFPQQPQQPFP, PQQPQQPFPQPQQPIPVQPQ, TQQPQQPFPQQPQQPFPQTQ, PIQPQQPFPQQPQQPQQPFP, PQQPQQPQQPFPQPQQPFPWQP, QQQQPFPQPQQPQQPFPQPQ, QQFPQPQQPQQPFPQQPQQQ, PQQPFPQPQQPQQPFPQPQQ, PQQQFPQPQQPQQPFPQQPQ, QQPQQQFPQPQQPQQPFPQP, QPQQPQQPFPQPQQPQLPFP, QQPFPQQPQQPFPQTQQPQQ, QQPYPQQPQQPFPQTQQPQQ, QLPFPQQPQQPFPQPQQPQQ, QPQQPFPQQPQQPFPQTQQP, TQQPQQPFPQQPQQPFPQTQ, PQQPQQPFPQTQQPQQPFPQ, QQPFPQPQQPQQPFPQLQQP, QQPLPQPQQPQQPFPQSQQP, QQPFPQPQQPQQPFPQSQQP, LQQPQQPLPQPQQPQQPFPQ, PQPQQPQQPFPQQQQPLIQP, FPLQPQQPFPQQPQQPFPQP, FPQQPQQPFPQPQLPFPQQS, PLQPQQPFPQQPQQPFPQPQ, FPELQQPIPQQPQQPFPLQP, PQQPQQPFPLQPQQPFPQQP, PLQPQQPFPQQPQQPFPQQF et PQQPQQPFPQQPQQSFPQQP.

4. Peptide destiné à une utilisation selon la revendication 3, qui comprend une séquence choisie dans le groupe constitué par PEQPEQPEQPFPQPEQPFPW, EQPFPEQPEQPFP, PEQPEQPFPQPEQPIPVQPQ, TEQPEQPFPEQPEQPFPQTQ, PIQPEQPFPEQPEQPEQPFP, PEQPEQPEQPFPQPEQPFPWQP, QQEQPFPQPEQPEQPFPQPQ, QEFPQPEQPEQPFPEQPQQQ, PEQPFPQPEQPEQPFPQPQQ,PEQEFPQPEQPEQPFPEQPQ, EQPEQEFPQPEQPEQPFPQP, QPEQPEQPFPQPEEPELPFP, EQPFPEQPEQPFPQTEQPQQ, EQPYPEQPEQPFPQTEQPQQ, ELPFPEQPEQPFPQPEQPQQ, QPEQPFPEQPEQPFPQTEQP, TEQPEQPFPEQPEQPFPQTQ, PEQPEQPFPQTEQPEQPFPQ, EQPFPQPEQPEQPFPQLEQP, EQPLPQPEQPEQPFPQSEQP, EQPFPQPEQPEQPFPQSEQP, LEQPEQPLPQPEQPEQPFPQ, PQPEQPEQPFPQQEEPLIQP, FPLQPEQPFPEQPEQPFPQP, FPEQPEQPFPEPELPFPQQS, PLQPEQPFPEQPEQPFPQPQ, FPELEQPIPEQPEQPFPLQP, PEQPEQPFPLQPEQPFPEQP, PLQPEQPFPEQPEQPFPEQP et PEQPEQPFPEQPEQSFPEQP.

5. Peptide destiné à une utilisation selon l'une quelconque des revendications 1 à 4, qui comprend un épitope de blé, un épitope d'orge et un épitope de seigle.

6. Peptide tel que défini selon l'une quelconque des revendications 1 à 5, destiné à une utilisation dans le traitement ou la prévention de la maladie cœliaque ; à condition que le peptide :
(i) ne soit pas un épitope qui est choisi dans le groupe constitué par QQPYPEQPEQPFPQ, QQPFPEQPEQPFPQ, QLPFPEQPEQPFPQ et PFPQPEQPEQPFPQ ; et
(ii) ne soit pas un peptide obtenu par traitement à la transglutaminase de tissu du peptide PQQPFPQPQQPQQPFPQSQQ.

7. Composition pharmaceutique comprenant : (a) un peptide tel que défini dans l'une quelconque des revendications 1 à 5, (b) un support ou diluent pharmaceutiquement acceptable, et, éventuellement, (c) un peptide comprenant au moins un épitope comprenant une séquence choisie parmi PQPELPY (SEQ ID n° : 1) et QLQPFPQPELPYPQPQS (SEQ ID n° : 2) ; à condition que le peptide dans (a) :
(i) ne soit pas un épitope qui est choisi dans le groupe constitué par QQPYPEQPEQPFPQ, QQPFPEQPEQPFPQ, QLPFPEQPEQPFPQ et PFPQPEQPEQPFPQ ; et
(ii) ne soit pas un peptide obtenu par traitement par traitement de la transglutaminase de tissu du peptide PQQPFPQPQQPQQPFPQSQQ.

8. Peptide isolé comprenant au moins un épitope de cellule T, le peptide n'étant pas de plus de 50 acides aminés en longueur et comprenant une séquence pouvant être obtenue par désamidation par transglutaminase d'une séquence choisie dans le groupe constitué par PQQPQQPQQPFPQPQQPFPW, PQQPQQPFPQPQQPIPVQPQ, TQQPQQPFPQQPQQPFPQTQ, PIQPQQPFPQQPQQPQQPFP, PQQPQQPQQPFPQPQQPFPWQP, QQQQPFPQPQQPQQPFPQPQ, QQFPQPQQPQQPFPQQPQQQ, PQQPFPQPQQPQQPFPQPQQ, PQQQFPQPQQPQQPFPQQPQ, QQPQQQFPQPQQPQQPFPQP, QPQQPQQPFPQPQQPQLPFP, QQPFPQQPQQPFPQTQQPQQ, QQPYPQQPQQPFPQTQQPQQ, QLPFPQQPQQPFPQPQQPQQ, QPQQPFPQQPQQPFPQTQQP, TQQPQQPFPQQPQQPFPQTQ, PQQPQQPFPQTQQPQQPFPQ, QQPFPQPQQPQQPFPQLQQP, QQPLPQPQQPQQPFPQSQQP, QQPFPQPQQPQQPFPQSQQP, LQQPQQPLPQPQQPQQPFPQ, PQPQQPQQPFPQQQQPLIQP, FPLQPQQPFPQQPQQPFPQP, FPQQPQQPFPQPQLPFPQQS, PLQPQQPFPQQPQQPFPQPQ, FPELQQPIPQQPQQPFPLQP, PQQPQQPFPLQPQQPFPQQP, PLQPQQPFPQQPQQPFPQQP et PQQPQQPFPQQPQQSFPQQP.

9. Peptide selon la revendication 8, qui comprend une séquence choisie dans le groupe constitué par PEQPEQPEQPFPQPEQPFPW, PEQPEQPFPQPEQPIPVQPQ, TEQPEQPFPEQPEQPFPQTQ, PIQPEQPFPEQPEQPEQPFP, PEQPEQPEQPFPQPEQPFPWQP, QQEQPFPQPEQPEQPFPQPQ, QEFPQPEQPEQPFPEQPQQQ, PEQPFPQPEQPEQPFPQPQQ,
PEQEFPQPEQPEQPFPEQPQ, EQPEQEFPQPEQPEQPFPQP, QPEQPEQPFPQPEEPELPFP, EQPFPEQPEQPFPQTEQPQQ, EQPYPEQPEQPFPQTEQPQQ, ELPFPEQPEQPFPQPEQPQQ, QPEQPFPEQPEQPFPQTEQP, TEQPEQPFPEQPEQPFPQTQ, PEQPEQPFPQTEQPEQPFPQ, EQPFPQPEQPEQPFPQLEQP, EQPLPQPEQPEQPFPQSEQP, EQPFPQPEQPEQPFPQSEQP, LEQPEQPLPQPEQPEQPFPQ, PQPEQPEQPFPQQEEPLIQP, FPLQPEQPFPEQPEQPFPQP, FPEQPEQPFPEPELPFPQQS, PLQPEQPFPEQPEQPFPQPQ, FPELEQPIPEQPEQPFPLQP, PEQPEQPFPLQPEQPFPEQP, PLQPEQPFPEQPEQPFPEQP et PEQPEQPFPEQPEQSFPEQP.

10. Peptide selon la revendication 8 ou 9, qui comprend un épitope de blé, un épitope d'orge et un épitope de seigle.

11. Composition comprenant un premier agent restrient à HLA-DQ2 et un deuxième agent restrient à HLA-DQ8, la composition comprenant un peptide tel que défini dans l'une quelconque des revendications 8 à 10.

12. Composition comprenant un peptide comprenant un épitope de blé, un peptide comprenant un épitope d'orge et/ou un peptide comprenant un épitope de seigle, la composition comprenant un peptide tel que défini dans l'une quelconque des revendications 8 à 10.

13. Composition comprenant : (a) un peptide tel que défini dans l'une quelconque des revendications 8 à 10 ; et (b) un peptide comprenant au moins un épitope comprenant une séquence choisie parmi PQPELPY (SEQ ID n° : 1) et QLQPFPQPELPYPQPQS (SEQ ID n° : 2).

14. Méthode de diagnostic de la maladie cœliaque ou vulnérabilité à la maladie cœliaque, chez un individu comprenant :
la mise en contact d'un échantillon provenant de l'hôte avec au moins un peptide tel que défini dans l'une quelconque des revendications 8 à 10 ; et
la détermination *in vitro* de savoir si les cellules T dans l'échantillon reconnaissent le peptide ;
la reconnaissance par les cellules T indiquant que l'individu présente, ou est susceptible de présenter, la maladie cœliaque.

15. Trousse destinée à effectuer un procédé selon la revendication 14, comprenant un peptide tel que défini dans l'une quelconque des revendications 8 à 10 et un moyen de détecter la reconnaissance du peptide par une cellule T, le moyen de détecter une reconnaissance comprenant éventuellement un anticorps à IFN-γ.
